# EUROPEAN PATENT APPLICATION

(11) **EP 4 843 982 A1**
(43) Date of publication of application: **25.11.2026**
(21) Application number: 24918376.5
(22) Date of filing: 31.12.2024
(51) Int. Cl.: C07D 401/14, A61K 51/08, A61K 47/54, A61P 35/00, A61P 9/10, A61P 25/00, A61P 29/00

(54) **FAP INHIBITOR**

(30) Priority: 18.01.2024 CN 202410075967
(71) Applicant: Grand Sirtex (Chengdu) Medical Technology Co., Ltd., Chengdu, Sichuan 611138 (CN); Serilink Biotechnology Co., Ltd., Sha Tin, N.T. Hong Kong (HK)
(72) Inventor: SHI, Yaxin, Chengdu, Sichuan 611138 (CN); TANG, Weikun, Chengdu, Sichuan 611138 (CN); LI, Xuechen, Hong Kong Science Park, Sha Tin, Hong Kong (CN); LIANG, Shuheng, Chengdu, Sichuan 611138 (CN); CHOW, Hoi Yee, Hong Kong Science Park, Sha Tin, Hong Kong (CN); HUANG, Wentao, Chengdu, Sichuan 611138 (CN); TAM, Kam Pui, Hong Kong Science Park, Sha Tin, Hong Kong (CN); WU, Xiaoyan, Chengdu, Sichuan 611138 (CN); HE, Yun, Chengdu, Sichuan 611138 (CN); QIAO, Nina, Chengdu, Sichuan 611138 (CN); ZHANG, Yiran, Chengdu, Sichuan 611138 (CN); YANG, Ju, Chengdu, Sichuan 611138 (CN)
(74) Representative: Cabinet Becker et Associés
(86) International application number: PCT/CN2024/144376
(87) International publication number: WO 2025/152768

(57) **Abstract**

Provided in the present invention is an FAP inhibitor compound represented by formula (A). Compared with the prior art, the inhibitor compound can improve the uptake and retention time in a target organ so that the therapeutic or diagnostic effect is improved, the uptake in non-target organs is reduced, and the safety is improved.

## Description

This application claims the priority to Chinese patent application No. 202410075967.X entitled "FAP Inhibitor," filed by the applicant with the China National Intellectual Property Administration on January 18, 2024, the entire contents of which are incorporated herein by reference.

### Technical Field

The present invention belongs to the field of pharmaceutical technology, specifically ralates to an FAP inhibitor.

### Background Art

Fibroblast activation protein (FAP), also known as proline endopeptidase FAP or seprase, is a type II transmembrane serine protease. In normal adults, FAP is highly expressed on the surface of activated fibroblasts in the stroma of more than 90% of epithelioid malignant tumors, including breast cancer, colorectal cancer, skin cancer, prostate cancer, and pancreatic cancer, but is almost not expressed in other tissues, or benign or precancerous epithelial lesions. Furthermore, studies have found that FAP is also significantly expressed in diseases associated with inflammation and cellular fibrosis, including wound healing, rheumatoid arthritis, osteoarthritis, liver cirrhosis, pulmonary fibrosis, and ventricular remodeling following myocardial infarction.

Studies have shown that inhibiting FAP can affect the motility of CAFs and tumor cells, inhibit the growth of CAFs, and effectively slow down tumor progression. Therefore, FAP has become a target of great interest. With the continuous optimization of small molecule inhibitors of FAP in recent years, radionuclide-labeled fibroblast activation protein inhibitors (FAPIs) have gradually become a new strategy in tumor targeted diagnosis and therapy. However, there is still room for improvement in the therapeutic and diagnostic effects of existing FAP-targeted nuclide therapies.

### Summary

The existing FAP-targeted nuclide conjugates have the problems of rapid clearance from tumor tissues and short tumor retention time, which hinder their therapeutic application. The inventors of the present application, while attempting to introduce a covalent warhead into a nuclide-labeled FAP-targeted compound, unexpectedly found that:
1). A hydrolyzed covalent warhead, such as sulfonic acid group as the hydrolysis product of a fluorosulfonyl group, and vicinal diol and enol groups as the hydration and rearrangement results of an epoxy moiety, can endow the compound with excellent properties and effects, such as enhancing tumor-specific uptake and functioning of prolonging tumor retention time;
2). The attachment position and/or linker length of the covalent warhead have a significant impact on its performance, such as enhanced tumor-specific uptake and prolonged tumor retention time.

Specific aspects of the present invention include:
The present invention first provides a compound represented by Formula (A), or a stereoisomer, tautomer, isotopically labeled form, pharmaceutically acceptable salt, or prodrug thereof, 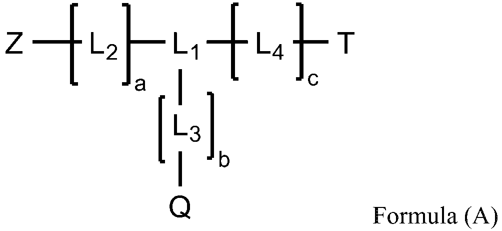
wherein Z is a payload, T is a targeting moiety,
Q is selected from the structure represented by any one of the following Formulas (Q-1) to (Q-21): 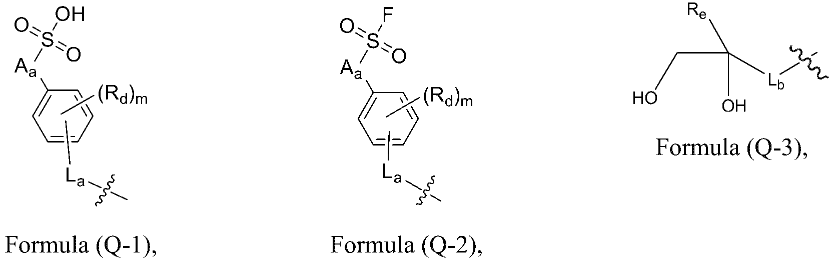 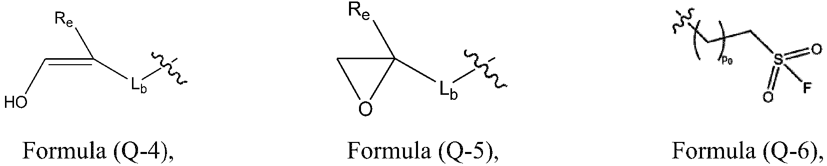 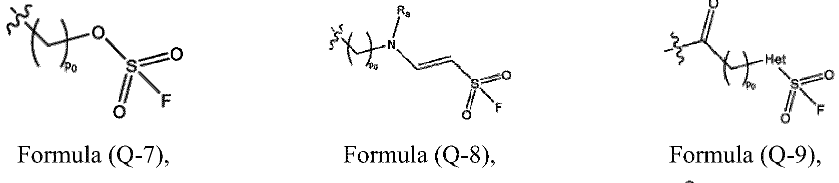 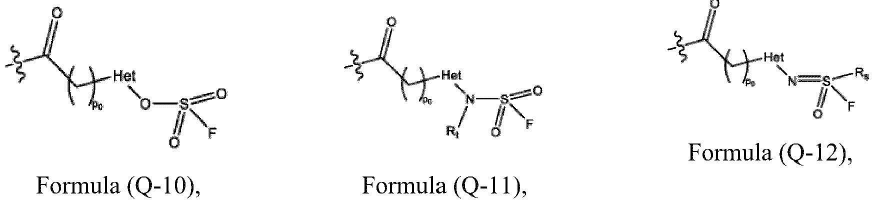 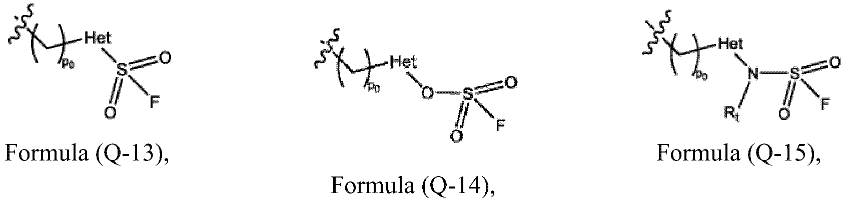 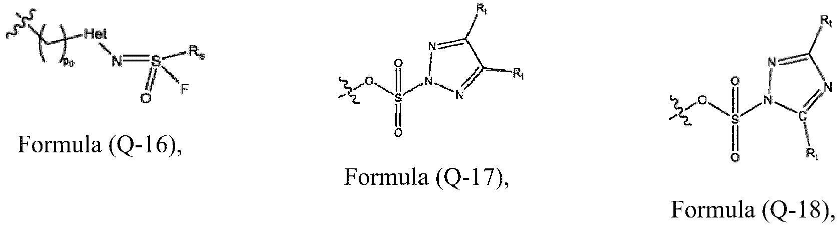 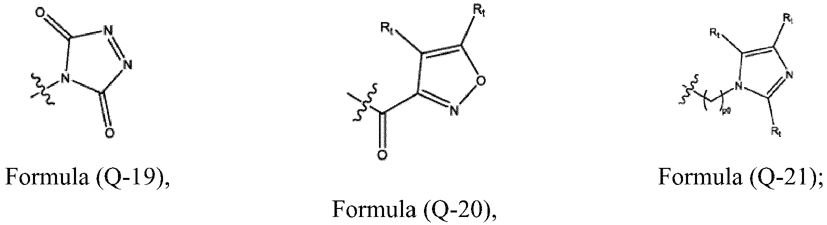
L₁, L₂, L₃, L₄, Aₐ, Lₐ, and L_{b} are the same or different, and are each independently selected from a bond or a linking group,
a, b, c, m, and p₀ are the same or different, and are each independently selected from an integer of 0 to 6 (e.g., 0, 1, 2, 3, 4, 5, 6),
R_{d} and Rₑ are the same or different, and are each independently selected from hydrogen, halogen, OH, cyano, nitro, amino, C₁₋₂₀ alkyl, C₁₋₂₀ alkoxy, and halogenated C₁₋₂₀ alkyl;
Het is an optionally substituted saturated heterocyclylene or heteroarylene;
Rₛ is selected from hydrogen, optionally substituted alkyl, optionally substituted cycloalkyl, optionally substituted heterocycloalkyl, optionally substituted aryl, and optionally substituted heteroaryl;
each Rₜ is independently selected from hydrogen, halogen, nitro, cyano, optionally substituted mercapto, optionally substituted amino, optionally substituted selenyl, optionally substituted C₁₋₂₀ alkyl, optionally substituted C₃₋₂₀ cycloalkyl, optionally substituted heterocycloalkyl, optionally substituted aryl, and optionally substituted heteroaryl;
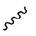 denotes the attachment site of the group.

In some embodiments, Q is selected from the structures represented by Formulas (Q-1-1), (Q-2-1), (Q-3), (Q-4), or (Q-5) below: 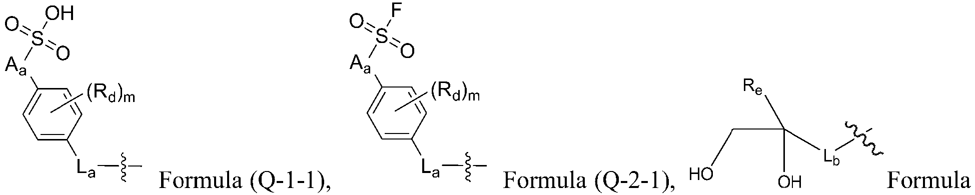 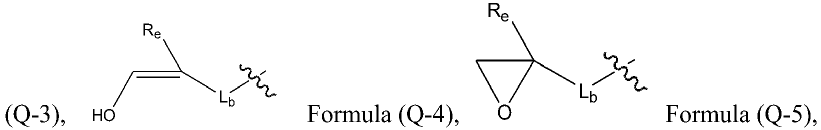
L₁, L₂, L₃, L₄, Aₐ, Lₐ, and L_{b} are the same or different, and are each independently selected from a bond or a linking group,
a, b, c, and m are the same or different, and are each independently selected from an integer of 0 to 6 (e.g., 0, 1, 2, 3, 4, 5, 6),
R_{d} and Rₑ are the same or different, and are each independently selected from hydrogen, halogen, OH, cyano, nitro, amino, C₁₋₂₀ alkyl, C₁₋₂₀ alkoxy, and halogenated C₁₋₂₀ alkyl;
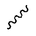 denotes the attachment site of the group.

A second aspect of the present invention also provides the use of a compound represented by Formula (A-0) in the preparation of the compound represented by Formula (A) wherein Q is Formula (Q-1), Formula (Q-3), or Formula (Q-4), 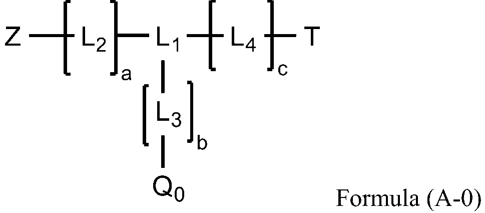 wherein Q₀ is selected from the structure represented by Formula (Q-2) or (Q-5) below: 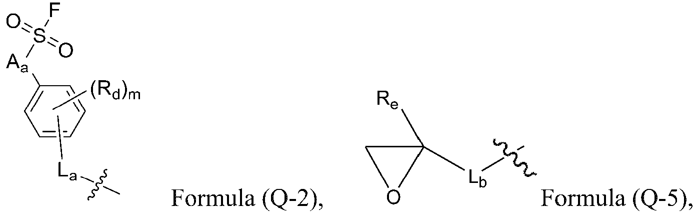 in Formula (A-0), Z, T, L₁, L₂, L₃, L₄, L_{b}, Aₐ, Lₐ, a, b, c, m, R_{d}, and Rₑ are as defined in Formula (A) above.

A third aspect of the present invention provides a compound represented by Formula (B) below: 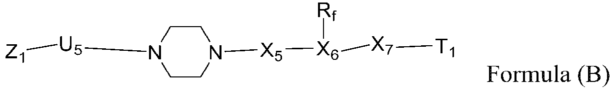
wherein Z₁ is a payload, T₁ is a targeting moiety,
U₅, X₅, X₆, and X₇ are the same or different, and are each independent selected from a linking group;
R_{f} is selected from the structure represented by Formula (Q-2-2) or Formula (Q-5-1) below: 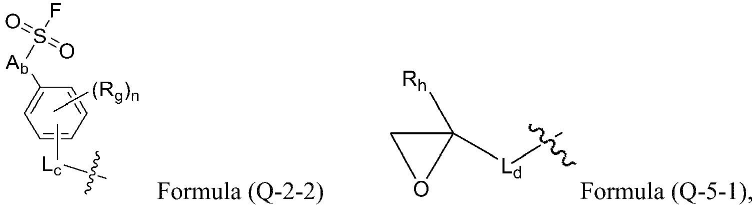
L_{c}, L_{d}, and A_{b} are the same or different, and are each independently selected from a linking group,
n is selected from an integer of 0 to 6 (e.g., 0, 1, 2, 3, 4, 5, 6),
R_{g} and Rₕ are the same or different, and are each independently selected from hydrogen, halogen, -OH, cyano, nitro, amino, C₁₋₂₀ alkyl, C₁₋₂₀ alkoxy, and halogenated C₁₋₂₀ alkyl; 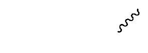 denotes the attachment site of the group.

In some embodiments, R_{f} is selected from the structure represented by Formula (Q-2-3) below:

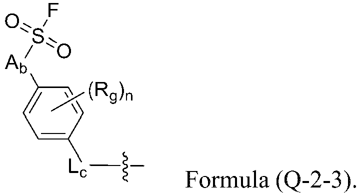

A fourth aspect of the present invention further provides a compound represented by Formula (D): 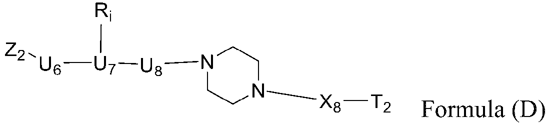
wherein Z₂ is a payload, T₂ is a targeting moiety,
U₆, U₇, U₈, and X₈ are the same or different, and are each independent selected from a linking group;
Rᵢ is selected from the structure represented by Formula (Q-2-4) or Formula (Q-5-2) below: 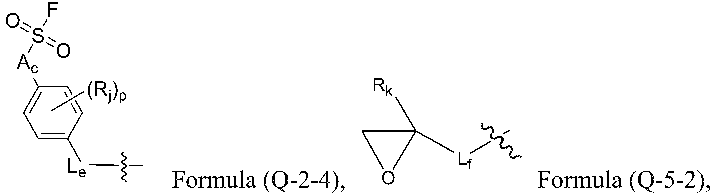
Lₑ, A_{c}, and L_{f} are the same or different, and are each independently selected from a linking group,
p is selected from an integer of 0 to 6 (e.g., 0, 1, 2, 3, 4, 5, 6),
Rⱼ and Rₖ are selected from hydrogen, halogen, OH, cyano, nitro, amino, C₁₋₂₀ alkyl, C₁₋₂₀ alkoxy, and halogenated C₁₋₂₀ alkyl;
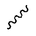 denotes the attachment site of the group.

In some embodiments, Rᵢ is selected from the structure represented by Formula (Q-2-5) below:

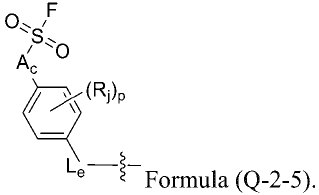

The present invention further provides a compound represented by Formula (A-4), or a stereoisomer, tautomer, isotopically labeled form, pharmaceutically acceptable salt, or prodrug thereof, 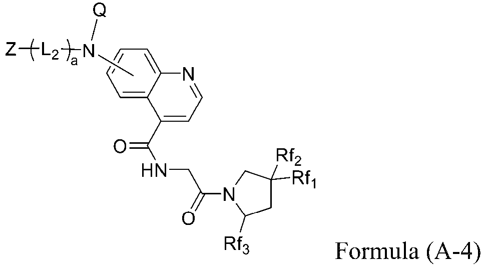
wherein Z is a payload,
Q is selected from the structure represented by any one of Formulas (Q-1) to (Q-21) below: 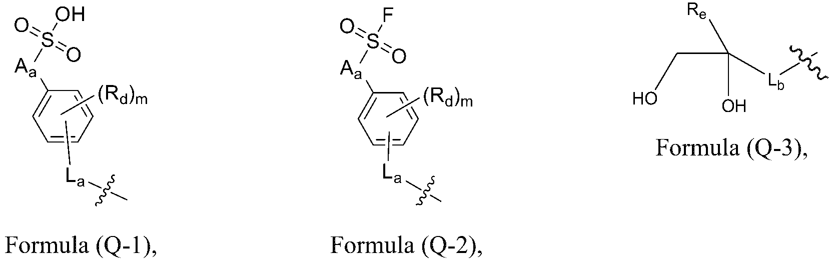 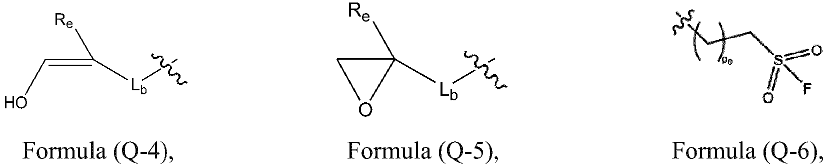 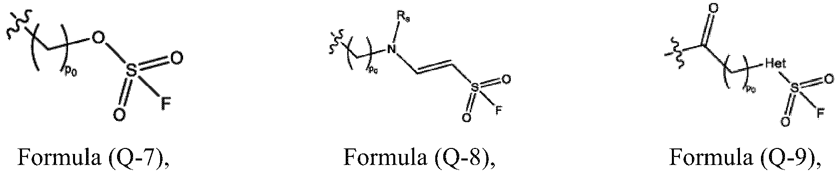 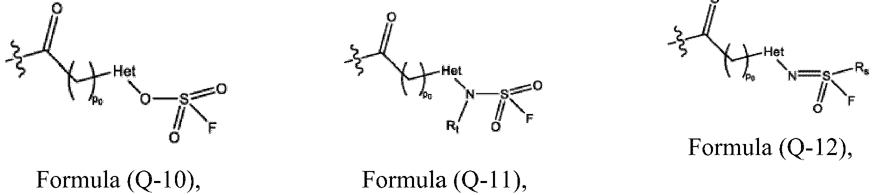 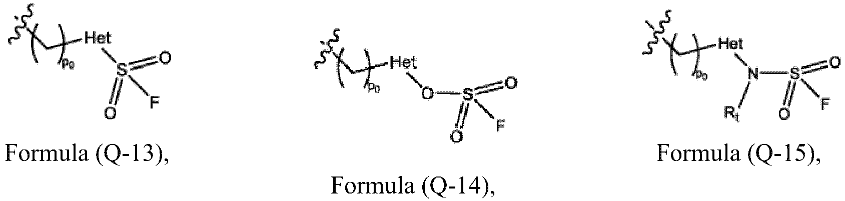 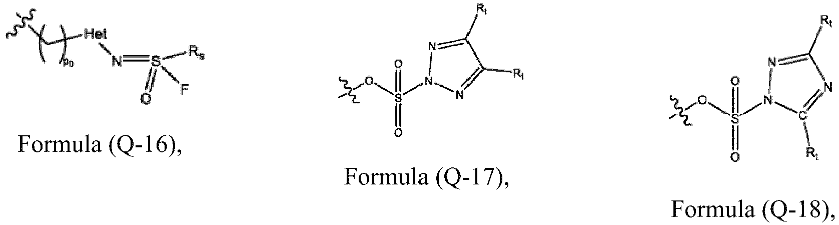 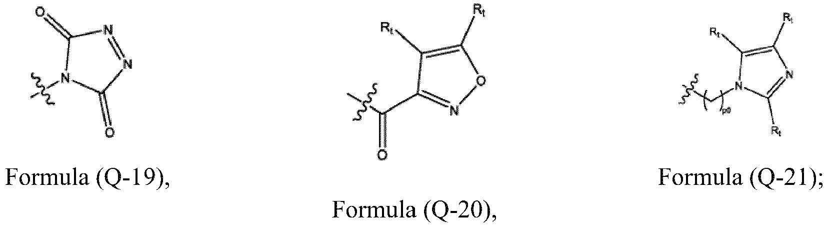
L₂, Aₐ, Lₐ, and L_{b} are the same or different, and are each independently selected from a linking group;
a, m, and p₀ are the same or different, and are each independently selected from an integer of 0 to 6;
R_{d} and Rₑ are the same or different, and are each independently selected from hydrogen, halogen, hydroxy, cyano, nitro, amino, C₁₋₂₀ alkyl, C₁₋₂₀ alkoxy, and halogenated C₁₋₂₀ alkyl;
Rf₁ and Rf₂ are independently selected from hydrogen and halogen, such as fluorine, chlorine, iodine, bromine, preferably fluorine;
Rf₃ is selected from hydrogen and cyano;
Het is an optionally substituted saturated heterocyclylene or heteroarylene;
Rₛ is selected from hydrogen, optionally substituted alkyl, optionally substituted cycloalkyl, optionally substituted heterocycloalkyl, optionally substituted aryl, and optionally substituted heteroaryl;
each Rₜ is independently selected from hydrogen, halogen, nitro, cyano, optionally substituted mercapto, optionally substituted amino, optionally substituted selenyl, optionally substituted C₁₋₂₀ alkyl, optionally substituted C₃₋₂₀ cycloalkyl, optionally substituted heterocycloalkyl, optionally substituted aryl, and optionally substituted heteroaryl;
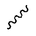 denotes the attachment site of the group.

The present invention further provides a FAP-targeted nuclide probe, wherein the nuclide probe is the compound of Formula (A), (B), (D), or (A-4) described above, labeled with a radionuclide.

According to an embodiment of the present invention, the radionuclide is a diagnostic radionuclide or a therapeutic radionuclide. According to an embodiment of the present invention, the diagnostic radionuclide is at least one of ⁶⁸Ga, ⁶⁷Ga, ⁶⁴Cu, ¹⁸F, ⁸⁶Y, ⁸⁹Zr, ¹¹¹In, ^{99m}Tc, ¹¹C, ²⁰³Pb, ¹²³I, ¹²⁵I, and ¹²⁴I. According to an embodiment of the present invention, the therapeutic radionuclide is at least one of ¹⁷⁷Lu, ⁹⁰Y, ¹²⁵I, ¹³¹I, ²¹¹At, ¹⁵³Sm, ¹⁸⁶Re, ¹⁸⁸Re, ⁶⁷Cu, ²²⁵Ac, ²²⁷Th, ²²³Ra, ²¹³Bi, ²¹²Bi, and ²¹²Pb.

According to an embodiment of the present invention, when the radioisotope is a non-metal ion, it may be chelated by forming a complex with a metal ion such as aluminum, for example, Al¹⁸F.

The present invention further provides a method for preparing the FAP-targeted nuclide probe described above, comprising:
1) providing the compound represented by Formula (A), (B), (D), or (A-4); and
2) chelating the compound with said radionuclide in solution or on a column.

The present invention further provides a preparation kit of the FAP-targeted nuclide probe, comprising:
1) the compound represented by Formula (A), (B), (D), or (A-4); and
2) the radionuclide or a reagent providing said radionuclide.

The present invention further provides use of the compound represented by Formula (A), (B), (D), or (A-4) in the preparation of an FAP Inhibitor.

The present invention further provides a pharmaceutical composition, comprising the compound represented by Formula (A), (B), (D), or (A-4); and at least one pharmaceutically acceptable excipient or carrier.

The present invention further provides use of the above compound, nuclide probe, FAP inhibitor, or pharmaceutical composition in the preparation of a FAP-targeted imaging agent and/or therapeutic agent.

The present invention further provides use of the above compound, nuclide probe, FAP inhibitor, or pharmaceutical composition in the preparation of a medicament for diagnosing and/or treating a disease, wherein the disease is a disease characterized by overexpression of fibroblast activation protein (FAP).

The present invention further provides a method for diagnosing and/or treating a disease, the method comprising administering a diagnostically and/or therapeutically effective amount of the above compound, nuclide probe, FAP inhibitor, or pharmaceutical composition to a subject to be diagnosed and/or treated, wherein the disease is a disease characterized by overexpression of fibroblast activation protein (FAP).

The present invention further provides a kit, comprising the above compound, nuclide probe, FAP inhibitor, or pharmaceutical composition; and instructions for diagnosing and/or treating a disease.

According to an embodiment of the present invention, the disease characterized by overexpression of fibroblast activation protein (FAP) is selected from cancer, chronic inflammation, atherosclerosis, fibrosis (e.g., pulmonary fibrosis), tissue remodeling, scarring disease, rheumatoid arthritis, osteoarthritis, liver cirrhosis, and liver disease.

According to an embodiment of the present invention, the cancer is selected from breast cancer, pancreatic cancer, small intestine cancer, colon cancer, rectal cancer, lung cancer, head and neck cancer, ovarian cancer, hepatocellular carcinoma, esophageal cancer, hypopharyngeal cancer, nasopharyngeal cancer, laryngeal cancer, myeloma cell, bladder cancer, cholangiocarcinoma, clear cell renal carcinoma, neuroendocrine tumor, carcinogenic osteomalacia, sarcoma, CUP (cancer of unknown primary), thymic cancer, glioma, neuroglioma, astrocytoma, cervical cancer, and prostate cancer.

The present invention further provides a compound represented by Formula (M-1): 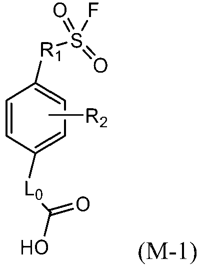
wherein R₁ is selected from a bond and O;
R₂ is selected from hydrogen, C₁₋₁₀ alkyl, and C₁₋₁₀ alkoxy;
L₀ comprises the structure represented by Formula (L-X): 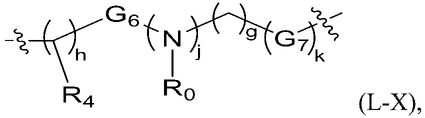
wherein h is selected from 0, 1, and 2; j is 0 or 1; g is selected from 0, 1, 2, 3, and 4; and k is 0, 1, or 2;
G₆ or G₇ is independently -CH₂- or carbonyl, and they are not -CH₂- at the same time;
R₄ is H, -NH₂ or amino group protected by a protecting group.

### Beneficial Effects

The present invention provides an FAP inhibitor compound represented by Formula (A), (B), or (D), which have excellent inhibitory activity against FAP kinase, and can increase the uptake and retention time in target organs, thereby improving the therapeutic or diagnostic effect, and reduce the uptake in non-target organs, thereby improving safety.

### Brief Description of the Drawings

Figure 1: PET-CT image of ⁶⁸Ga-FAPI-46 in U87MG tumor-bearing mice;
Figure 2: Biodistribution results of ⁶⁸Ga-FAPI-46 in U87MG tumor-bearing mice (n=3);
Figure 3: PET-CT image of ⁶⁸Ga-S18 in U87MG tumor-bearing mice;
Figure 4: PET-CT image of ⁶⁸Ga-S19 in U87MG tumor-bearing mice;
Figure 5: PET-CT image of ⁶⁸Ga-S2 in U87MG tumor-bearing mice;
Figure 6: Biodistribution results of ⁶⁸Ga-S2 in U87MG tumor-bearing mice (n=3);
Figure 7: PET-CT image of ⁶⁸Ga-S21 in U87MG tumor-bearing mice;
Figure 8: Biodistribution results of ⁶⁸Ga-S21 in U87MG tumor-bearing mice (n=3);
Figure 9: PET-CT images of ⁶⁸Ga-S22 in U87MG tumor-bearing mice;
Figure 10: Biodistribution results of ⁶⁸Ga-S22 in U87MG tumor-bearing mice (n=3);
Figure 11: PET-CT image of ⁶⁸Ga-S23 in U87MG tumor-bearing mice;
Figure 12: Biodistribution results of ⁶⁸Ga-S23 in U87MG tumor-bearing mice (n=3);
Figure 13: PET-CT image of ⁶⁸Ga-FAPI-04 in U87MG tumor-bearing mice;
Figure 14: Biodistribution results of ⁶⁸Ga-FAPI-04 in U87MG tumor-bearing mice (n=3);
Figure 15: PET-CT image of ⁶⁸Ga-S12 in U87MG tumor-bearing mice;
Figure 16: Biodistribution results of ⁶⁸Ga-S12 in U87MG tumor-bearing mice (n=3);
Figure 17: PET-CT image of ⁶⁸Ga-S15 in U87MG tumor-bearing mice;
Figure 18: Biodistribution results of ⁶⁸Ga-S15 in U87MG tumor-bearing mice (n=3);
Figure 19: PET-CT image of ⁶⁸Ga-C12 in U87MG tumor-bearing mice;
Figure 20: Biodistribution results of ⁶⁸Ga-C12 in U87MG tumor-bearing mice (n=3);
Figure 21: PET-CT image of ⁶⁸Ga-C3-3 in U87MG tumor-bearing mice;
Figure 22: Biodistribution results of ⁶⁸Ga-C3-3 in U87MG tumor-bearing mice (n=3);
Figure 23: PET-CT image of ⁶⁸Ga-C2-1 in U87MG tumor-bearing mice;
Figure 24: Biodistribution results of ⁶⁸Ga-C2-1 in U87MG tumor-bearing mice (n=3)
Figure 25: Biodistribution results of ¹⁷⁷Lu-S23 in HT1080-FAP tumor-bearing mice (n=6);
Figure 26: Biodistribution results of ¹⁷⁷Lu-FAPI-46 in HT1080-FAP tumor-bearing mice (n=6);
Figure 27: Experimental data on tumor treatment efficacy of ¹⁷⁷Lu-S23 in HT1080-FAP tumor-bearing mice: (A) Schematic diagram of the treatment regimen; (B) Body weight change curve of mice during the treatment period; (C) Averaged tumor growth curve during the treatment period;
Figure 28: PET/CT imaging of ⁶⁸Ga-S2 in lung cancer patients;
Figure 29: PET/CT imaging of 68Ga-S23 in a patient with postoperative recurrence mucinous pancreatic cancer of the right lower abdomen.

### Definitions and Explanations of Terms

Unless otherwise specified, the definitions of groups and terms described in the specification and claims of this application, including those defined as examples, illustrative definitions, preferred definitions, definitions listed in tables, definitions of specific compounds in the examples etc., may be combined and incorporated with each other arbitrarily. The resulting group definitions and compound structures should be understood to be within the scope of the description and/or claims of this application.

Unless otherwise specified, the numerical ranges described in this specification and the claims are equivalent to recording at least each specific integer value therein. For example, the numerical range "1-40" is equivalent to recording each integer value in the numerical range "1-10", i.e., 1, 2, 3, 4, 5, 6, 7, 8, 9, 10, and each integer value in the numerical range "11-40", i.e., 11, 12, 13, 14, 15, 16, 17, 18, 19, 20, 21, 22, 23, 24, 25, 26, 27, 28, 29, 30, 31, 32, 33, 34, 35, 36, 37, 38, 39, 40. It should be understood that when "one," "two," or "more" is used when describing substituents herein, "more" refers to an integer of ≥3, such as 3, 4, 5, 6, 7, 8, 9, or 10. When "at least one" is used, it means one, two, or more.

The term "halogen" or "halo" refers to fluorine (F), chlorine (Cl), bromine (Br), or iodine (I).

Generally, the term "substituted" indicates that one or more hydrogen atoms in the given structure are replaced by a specific substituent. Furthermore, when the group is substituted by more than one substituent, each substituent is independent of each other; that is, the more than one substituent may be different from one another or may be the same. Unless otherwise indicated, a substituent may be present at any of the substitutable positions of the group being substituted. When more than one position in the given structural formula can be substituted by one or more substituents selected from specific groups, the substituents may be the same or different at each position. The substituents may include, but are not limited to, =O, deuterium, cyano, nitro, halogen, alkyl, haloalkyl, alkoxy, hydroxy, carboxy, cycloalkyl, cycloalkyloxy, heterocyclyl, heterocyclyl alkyl, aryl, arylalkyl, aryloxy, heteroaryl, heteroarylalkyl, and heteroaryloxy.

Furthermore, it should be noted that, unless otherwise explicitly stated, the description "··· independently selected from" as used in the present invention should be interpreted broadly, meaning that the individual elements described are mutually independent and may be independently selected from the same or different specific groups. More specifically, the description "··· independently selected from" may mean that in different groups, the specific options represented by the same symbol do not influence each another; it may also mean that within the same group, the specific options represented by the same symbol do not influence each another.

Throughout this specification, the substituents of the compounds disclosed in the present invention are disclosed according to types or ranges of groups. It is specifically noted that the present invention encompasses every independent sub-combination of members within these types and ranges of groups. For example, the term "C₁₋₆ alkyl" specifically refers to independently disclosed C₁ alkyl, C₂ alkyl, C₃ alkyl, C₄ alkyl, Cs alkyl, or C₆ alkyl.

Throughout the present invention, linking substituents are described. When the structure clearly requires a linking group, the Markush variables listed for the group should be understood as a linking group. For example, if the structure requires a linking group and the Markush group definition for the variable lists "alkyl" or "aryl", it should be understood that the "alkyl" or "aryl" represents a linked alkylene group or arylene group, respectively.

The linking groups in the present invention may be any divalent (ylene) or trivalent organic group (triyl). The divalent linking group may be selected from a bond, C₁₋₁₀ alkylene, C₂₋₁₀ alkenylene, C₂₋₁₀ alkynylene, C₃₋₁₀ cycloalkylene, C₆₋₁₂ arylene, 5- to 12-membered heteroarylene, 5- to 12-membered heterocyclylene, or a combination of two or more thereof; the C₁₋₁₀ alkylene, C₂₋₁₀ alkenylene, C₂₋₁₀ alkynylene, C₃₋₁₀ cycloalkylene, C₆₋₁₂ arylene, 5- to 12-membered heteroarylene, 5- to 12-membered heterocyclylene, or a combination of two or more thereof may be unseparated or optionally separated by carbonyl, O, S, N atom; the C₁₋₁₀ alkylene, C₂₋₁₀ alkenylene, C₂₋₁₀ alkynylene, C₃₋₁₀ cycloalkylene, C₆₋₁₂ arylene, 5- to 12-membered heteroarylene, 5- to 12-membered heterocyclylene, or a combination of two or more thereof may be optionally substituted with C₁₋₆ alkyl, C₃₋₆ cycloalkyl, halogen atom, halogenated C₁₋₆ alkyl; optionally, the C₁₋₆ alkyl or halogenated C₁₋₆ alkyl may form a C₃₋₆ cycloalkyl with the carbon atom to which it is attached. The trivalent linking group is obtained from the above divalent linking group by deriving a new attachment site. The linking group in the present application can be linked to other structures through any functional group, such as -O-, NH, -COO-, CONH-, etc. Unless otherwise specified, the linking group of the present application is linked to other structures in any direction. When the linking group is a bond, it indicates that the groups at both ends of the linking group are directly linked through a chemical bond, without including other atoms.

The term "C₁₋₂₀ alkyl" refers to a straight or branched saturated monovalent hydrocarbon group having 1 to 20 carbon atoms. For example, "C₁₋₆ alkyl" refers to a straight and branched alkyl group having 1, 2, 3, 4, 5, or 6 carbon atoms. Among them, the alkyl group may optionally be substituted by one or more substituents described in the present application. In some embodiments, the alkyl group contains 1 to 12 carbon atoms; in other embodiments, the alkyl group contains 1 to 6 carbon atoms; and in still other embodiments, the alkyl group contains 1 to 4 carbon atoms. Examples of the alkyl group include, but are not limited to, methyl, ethyl, propyl, butyl, pentyl, hexyl, isopropyl, isobutyl, sec-butyl, tert-butyl, isopentyl, 2-methylbutyl, 1-methylbutyl,1-ethylpropyl, 1,2-dimethylpropyl, neopentyl, 1,1-dimethylpropyl, 4-methylpentyl, 3-methylpentyl, 2-methylpentyl,1-methylpentyl, 2-ethylbutyl, 1-ethylbutyl, 3,3-dimethylbutyl, 2,2-dimethylbutyl, 1,1-dimethylbutyl, 2,3-dimethylbutyl, 1,3-dimethylbutyl, or 1,2-dimethylbutyl, or isomers thereof.

The term "C₃₋₄₀ cycloalkyl" should be understood to mean saturated monovalent monocyclic, bicyclic hydrocarbon ring or bridged cycloalkane having 3 to 40 carbon atoms, preferably "C₃₋₁₀ cycloalkyl". The term "C₃₋₁₀ cycloalkyl" should be understood to mean saturated monovalent monocyclic, bicyclic hydrocarbon ring, or bridged cycloalkane having 3, 4, 5, 6, 7, 8, 9, or 10 carbon atoms. The C₃₋₁₀ cycloalkyl may be monocyclic hydrocarbon group, such as cyclopropyl, cyclobutyl, cyclopentyl, cyclohexyl, cycloheptyl, cyclooctyl, cyclononyl, or cyclodecyl, or a bicyclic hydrocarbon group such as a decahydronaphthalene ring.

The term "3- to 20-membered heterocyclyl" should be understood to refer to a saturated monovalent monocyclic, bicyclic hydrocarbon ring or bridged cycloalkane, which is a non-aromatic cyclic group containing 1-5 heteroatoms independently selected from N, O and S, and having a total number of ring atoms of 3-20 (e.g., 3, 4, 5, 6, 7, 8, 9, 10, etc.), preferably "3- to 10-membered heterocyclyl". The term "3- to 10-membered heterocyclyl" means a saturated monovalent monocyclic, bicyclic hydrocarbon ring or bridged cycloalkane containing 1-5, preferably 1-3 heteroatoms independently selected from N, O and S, for example 1, 2, 3 heteroatoms independently selected from N, O and S. The heterocyclyl may be linked to the rest of the molecule through any one of the carbon atoms or a nitrogen atom (if present). In particular, the heterocyclyl may include, but is not limited to: 4-membered ring, such as azacyclobutyl, oxacyclobutyl; 5-membered ring, such as tetrahydrofuranyl, dioxolenyl, pyrrolidinyl, imidazolidinyl, pyrazolidinyl, pyrrolinyl; or 6-membered ring, such as tetrahydropyranyl, piperidinyl, morpholinyl, dithianyl, thiomorpholinyl, piperazinyl or triazinanyl; or 7-membered ring, such as diazacycloheptanyl. Optionally, the heterocyclyl may be benzo-fused. The heterocyclyl may be bicyclic, such as, but not limited to, 5,5-membered ring, e.g., hexahydrocyclopenta[c]pyrrol-2(1H)-yl ring, or 5,6-membered bicyclic, e.g., hexahydropyrrolo[1,2-a]pyrazin-2(1H)-yl ring. Nitrogen-containing ring may be partially unsaturated, i.e., it may contain one or more double bonds, such as, but not limited to, 2,5-dihydro-1H-pyrrolyl, 4H-[1,3,4]thiadiazinyl, 4,5-dihydrooxazolyl or 4H-[1,4]thiazinyl, or it may be benzo-fused, such as, but not limited to, dihydroisoquinolinyl. According to the present application, the heterocyclyl is non-aromatic. When the 3- to 20-membered heterocyclyl is linked to other groups to form the compound of the present invention, it may be linked via a carbon atom on the 3-20 membered heterocyclyl, or via a heteroatom on the ring of the 3- to 20-membered heterocyclyl. For example, when the 3- to 20-membered heterocyclyl is piperazinyl, it may be linked via a nitrogen atom of the piperazinyl. Or when the 3- to 20-membered heterocyclyl is piperidinyl, it may be liked via the nitrogen atom of the piperidinyl ring and the carbon atom on the ortho position of the nitrogen atom.

The term "C₆₋₂₀ aryl" should be understood to mean a monocyclic, bicyclic, or tricyclic hydrocarbon ring that is monovalent, aromatic or partially aromatic and contains 6 to 20 carbon atoms, preferably "C₆₋₁₄ aryl". The term "C₆₋₁₄ aryl" should be understood as preferably meaning a monovalent aromatic or partially aromatic monocyclic, bicyclic or tricyclic hydrocarbon ring having 6, 7, 8, 9, 10, 11, 12, 13 or 14 carbon atoms ("C₆₋₁₄ aryl"), in particular a ring having 6 carbon atoms ("C₆ aryl"), such as phenyl; or biphenyl, or a ring having 9 carbon atoms ("C₉ aryl"), such as indanyl or indenyl, or a ring having 10 carbon atoms ("C₁₀ aryl"), such as tetrahydronaphthyl, dihydronaphthyl or naphthyl, or a ring having 13 carbon atoms ("C₁₃ aryl"), such as fluorenyl, or a ring having 14 carbon atoms ("C₁₄ aryl"), such as anthracenyl. When the C₆₋₂₀ aryl is substituted, it may be mono- or poly-substituted. Furthermore, there is no limitation on the substitution site, for example, it may be substituted in the ortho, para, or meta site.

The term "5- to 20-membered heteroaryl" should be understood to include monovalent monocyclic, bicyclic or tricyclic aromatic ring system, including aromatic or partially aromatic, which has 5 to 20 ring atoms and contains 1 to 5 heteroatoms independently selected from N, O and S, for example "5- to 14-membered heteroaryl". The term "5- to 14-membered heteroaryl" should be understood to include monovalent monocyclic, bicyclic or tricyclic aromatic ring system, which has 5, 6, 7, 8, 9, 10, 11, 12, 13 or 14 ring atoms, in particular 5, 6, 9 or 10 carbon atoms, and contains 1-5, preferably 1-3 heteroatoms independently selected from N, O and S, and additionally may be benzo-fused in each case. Particularly, the heteroaryl is selected from thienyl, furanyl, pyrrolyl, oxazolyl, thiazolyl, imidazolyl, pyrazolyl, isoxazolyl, isothiazolyl, oxadiazolyl, triazolyl, thiadiazolyl, thiazole-4H-pyrazolyl, etc., and their benzo derivatives, e.g., benzofuranyl, benzothienyl, benzoxazolyl, benzisoxazolyl, benzimidazolyl, benzotriazolyl, indazolyl, indolyl, isoindolyl, etc.; or pyridinyl, pyridazinyl, pyrimidinyl, pyrazinyl, triazinyl, etc., and their benzo derivatives, e.g., quinolinyl, quinazolinyl, isoquinolinyl, etc.; or azocinyl, indolizinyl, purinyl, etc., and their benzo derivatives; or cinnolinyl, phthalazinyl, quinazolinyl, quinoxalinyl, naphthyridinyl, pteridinyl, carbazolyl, acridinyl, phenazinyl, phenothiazinyl, phenoxazinyl, etc. When the 5- to 20-membered heteroaryl is linked to other groups to form the compound of the present invention, it may be attached via a carbon atom on the 5-20 membered heteroaryl ring, or via a heteroatom on the 5-20 membered heteroaryl ring. When the 5-20 membered heteroaryl is substituted, it may be mono- or poly-substituted. Furthermore, there is no limitation on the substitution site, for example, hydrogen attached to a carbon atom on the heteroaryl ring may be substituted, or hydrogen attached to heteroatom on the heteroaryl ring may be substituted.

The stereochemical definitions and rules used in the present disclosure generally follow S.P. Parker, Ed., McGraw-Hill Dictionary of Chemical Terms (1984), McGraw-Hill Book Company, New York; and Eliel, E. and Wilen, S., "Stereochemistry of Organic Compounds," John Wiley & Sons, Inc., New York, 1994.

"Stereoisomers" refer to a compound having the same chemical structure but differing in the spatial arrangement of atoms or groups. Stereoisomer includes enantiomer, diastereomer, conformational isomer (rotational isomer), geometric isomer (cis/trans isomer), and atropisomer, and the like.

"Enantiomers" refer to two isomers of a compound that cannot overlap but are mirror images of each other.

"Diastereomer" refer to stereoisomers that have two or more chiral centers and whose molecules are not mirror images of each other. Diastereomer exhibit different physical properties, such as melting point, boiling point, spectral properties, and reactivity. Mixtures of diastereomer can be separated using high-resolution analytical techniques such as electrophoresis and chromatography, for example, HPLC.

Any asymmetric atom (e.g., carbon) in the compound disclosed herein may exist in a racemic or enantiomerically enriched form, for example in (R)-, (S)-, or (R,S)-configuration. In certain embodiments, each chiral atom has an enantiomeric excess of at least 0%, at least 60%, at least 70%, at least 80%, at least 90%, at least 95%, or at least 99% in (R)- or (S)-configuration.

Any resulting mixture of stereoisomers can be separated into pure or substantially pure geometric isomers, enantiomers, or diastereoisomers based on differences in the physicochemical properties of the components, for example, by chromatography and/or stepwise crystallization.

In the case of racemic amines, the diastereoisomers are prepared from the mixture by reaction with an optically active resolving agent. Examples of suitable resolving agent are optically active acids, such as R and S forms of tartaric acid, diacetyl tartaric acid, dibenzoyl tartaric acid, mandelic acid, malic acid, lactic acid, suitable N-protected amino acid (e.g., N-benzoylproline or N-benzenesulfonylproline), or various optically active camphorsulfonic acids. Chromatographic enantiomeric resolution can also be advantageously carried out using optically active resolving agent (e.g., di-nitrobenzoylphenylglycine, cellulose triacetate or other carbohydrate derivatives immobilized on silica gel, or chirally derivatized methacrylic ester polymers). Suitable eluents for this purpose are aqueous or alcoholic solvent mixtures, such as hexane/isopropanol/acetonitrile.

The term "tautomers" refers to structural isomers that can interconvert via a low energy barrier. If tautomerism is possible (e.g., in solution), a chemical equilibrium of tautomers can be achieved. For example, proton tautomers (also known as prototro pictautomers) involve interconversion via proton migration, such as ketone-enol isomerization and imine-enamine isomerization. Valen cetautomers involve interconversion through recombination of some bonding electrons. A specific example of ketone-enol tautomerism is the interconversion between pentane-2,4-dione and 4-hydroxy-pent-3-en-2-one. Another example of tautomerism is phenol-keto tautomerism. A specific example of phenol-keto tautomerism is the interconversion between pyridine-4-ol and pyridine-4(1H)-one tautomers. Unless otherwise specified, all tautomeric forms of the compounds of the present invention are within the scope of the present invention.

The term "nitroxide" as used herein means that when a compound contains several amine functional groups, one or more than one nitrogen atom can be oxidized to form a N-oxide. Special examples of N-oxides are N-oxides of tertiary amines or N-oxides of nitrogen atoms of nitrogen-containing heterocycles. An oxidizing agent such as hydrogen peroxide or peracid (e.g., peroxycarboxylic acid) may be used to treat the corresponding amines to form N-oxides (see Advanced Organic Chemistry, Wiley Interscience, 4th edition, Jerry March, pages). In particular, N-oxides may be prepared by the method of L.W. Deady (Syn. Comm. 1977, 7, 509-514), in which, for example, in an inert solvent such as dichloromethane, an amine compound is reacted with m-chloroperoxybenzoic acid (MCPBA).

The term "isotopically labeled form" includes, but is not limited to, the compounds of the present invention labeled with isotopes of hydrogen, carbon, nitrogen, oxygen, fluorine, sulfur, and chlorine (e.g., ²H, ³H,¹³C, ¹⁴C, ¹⁵N, ¹⁸O,¹⁷O, ¹⁸F, ³⁵S, and ³⁶Cl). Isotopically labeled compounds of the present invention may be used for determining the tissue distribution of the compounds, their prodrugs, and metabolites; preferred isotopes for such determinations include ³H and ¹⁴C. Furthermore, in certain cases, substitution with a heavier isotope (e.g., deuterium (2H or D)) may provide increased metabolic stability, which offers therapeutic advantages such as an increased *in vivo* half-life or reduced dosage requirements. The isotope-labeled compounds of the present invention may generally be prepared according to the methods described herein by replacing non-isotopically labeled reagent with isotopically labeled reagent.

The term "pharmaceutically acceptable" refers to molecular entities and compositions that are physiologically tolerable and generally do not produce allergic or similar adverse reactions, such as gastrointestinal discomfort, dizziness, etc., when administered to humans.

The term "carrier" refers to diluents, adjuvants, excipients, or matrices administered together with the compound. These drug carriers may be sterile liquids, such as water and oils, including petroleum, animal, plant, or synthetic origins, such as peanut oil, soybean oil, mineral oil, sesame oil, etc. Water and aqueous solutions, brines, and aqueous glucose-glycerol solutions are preferred as carriers, particularly injectable solutions. Suitable drug carriers are described in E.W. Martin's "Remington's Pharmaceutical Sciences."

The term "prodrug" as used herein refers to a compound that is converted *in vivo* into the compound represented by Formula (I). Such conversion is influenced by the hydrolysis of the prodrug in blood or by enzymatic conversion to the parent structure in blood or tissues. The prodrug compounds of the present invention may be esters; in the prior art, esters that may serve as prodrugs include phenyl esters, aliphatic (C₁₋₂₄) esters, acyloxymethyl esters, carbonates, carbamates, and amino acid esters. For example, a compound in the present invention containing a hydroxy group can be acylated to yield a compound in the form of prodrug. Other prodrug forms include phosphates, such as those obtainable by phosphorylation of the hydroxy on the parent compound. For a comprehensive discussion of prodrugs, see the following references: T. Higuchi and V. Stella, Prodrugs as Novel Delivery Systems, Vol. 14 of the A.C.S. Symposium Series, Edward B. Roche, ed., Bioreversible Carriers in Drug Design, American Pharmaceutical Association and Pergamon Press, 1987; J. Rautio et al., Prodrugs: Design and Clinical Applications, Nature Reviews Drug Discovery, 2008, 7, 255-270, and S. J. Hecker et al., Prodrugs of Phosphates and Phosphonates, Journal of Medicinal Chemistry, 2008, 51, 2328-2345.

The term "metabolite" as used herein refers to a product obtained *in vivo* from a specific compound or its salt through metabolic processes. A metabolite of a compound may be identified using techniques known in the art, and its activity may be characterized by experimental methods as described in the present application. Such products may be obtained by subjecting the administered compound to processes such as oxidation, reduction, hydrolysis, amidation, deamidation, esterification, deesterification, enzymatic cleavage, etc. Accordingly, the invention includes metabolites of the compounds, including metabolites obtained by contacting the compound of the invention with a mammal for a period of time.

Pharmaceutically acceptable salts may, for example, be acid addition salts of the compounds containing a nitrogen atom in its chain or ring of the present invention having sufficient basicity, for example, acid addition salts formed with the following inorganic acids: such as hydrochloric acid, hydrofluoric acid, hydrobromic acid, hydroiodic acid, sulfuric acid, pyrosulfuric acid, phosphoric acid, or nitric acid, or bisulfate; or acid addition salts formed with the following organic acids: such as formic acid, acetic acid, acetoacetic acid, pyruvic acid, trifluoroacetic acid, propionic acid, butyric acid, hexanoic acid, heptanoic acid, undecanoic acid, lauric acid, benzoic acid, salicylic acid, 2-(4-hydroxybenzoyl)benzoic acid, camphoric acid, cinnamic acid, cyclopentanepropionic acid, digluconic acid, 3-hydroxy-2-naphthoic acid, nicotinic acid, pamoic acid, pectinic acid, persulfuric acid, 3-phenylpropionic acid, picric acid, pivalic acid, 2-hydroxyethanesulfonic acid, itaconic acid, aminosulfonic acid, trifluoromethanesulfonic acid, dodecylsulfuric acid, ethanesulfonic acid, benzenesulfonic acid, p-toluenesulfonic acid, methanesulfonic acid, 2-naphthalenesulfonic acid, naphthalenedisulfonic acid, camphorsulfonic acid, citric acid, tartaric acid, stearic acid, lactic acid, oxalic acid, malonic acid, succinic acid, malic acid, adipic acid, alginic acid, maleic acid, fumaric acid, D-gluconic acid, mandelic acid, ascorbic acid, glucoheptonic acid, glycerophosphoric acid, aspartic acid, sulfosalicylic acid, hemisulfuric acid or thiocyanic acid.

In addition, another suitable pharmaceutically acceptable salt of the compound of the present invention having sufficient acidity is an alkali metal salt (e.g., sodium or potassium salt), an alkaline earth metal salt (e.g., calcium or magnesium salt), an ammonium salt, or a salt formed with an organic base that provides a physiologically acceptable cation, such as a salt formed with the following substance: sodium ion, potassium ion, N-methylglucosamine, dimethylglucosamine, ethylglucosamine, lysine, dicyclohexylamine, 1,6-hexanediamine, ethanolamine, glucosamine, meglumine, creatine, serine, tris(hydroxymethyl)aminomethane, aminopropanediol, 1-amino-2,3,4-butanetriol. By way of example, the pharmaceutically acceptable salt includes a salt formed by a -COOH group and the following substance: sodium ion, potassium ion, calcium ion, magnesium ion, N-methylglucamine, dimethylglucamine, ethylglucamine, lysine, dicyclohexylamine, 1,6-hexanediamine, ethanolamine, glucamine, meglumine, sarcosine, serinol, tris(hydroxymethyl)aminomethane, aminopropanediol, 1-amino-2,3,4-butanetriol.

In addition, basic nitrogen-containing groups can be quaternized using the following reagents: lower alkyl halides, such as chlorides, bromides, and iodides of methyl, ethyl, propyl, and butyl ; dialkyl sulfates, such as dimethyl sulfate, diethyl sulfate, dibutyl sulfate, and dipentyl sulfate; long-chain halides, such as chlorides, bromides, and iodides of decyl, lauryl, myristyl, and stearyl; and aryl halides, such as bromides of benzyl and phenethyl. By way of example, pharmaceutically acceptable salts include hydrochlorides, sulfates, nitrates, bisulfates, hydrobromides, acetates, oxalates, citrates, methanesulfonates, formates, or meglumine salt, and the like.

Since the compounds of the present invention may have multiple salt-forming sites, the pharmaceutically acceptable salts include not only salts formed at one salt-forming site of the compound, but also salts formed at two, three, or all of the salt-forming sites. Accordingly, the molar ratio of the compound of Formula (I) to the anion of the acid or the cation of the base required for salt formation in the pharmaceutically acceptable salts may vary over a wide range, for example, from 4:1 to 1:4, such as 3:1, 2:1, 1:1, 1:2, 1:3, and so on.

The term "solvate" as used herein refers to an association complex formed between one or more solvent molecules and a compound of the present invention. Solvents that form solvates include, but are not limited to, water, isopropanol, ethanol, methanol, dimethyl sulfoxide, ethyl acetate, acetic acid, and aminoethanol. The term "hydrate" refers to an association complex in which the solvent molecule is water.

The term "ester" as used herein refers to an *in vivo* hydrolyzable ester formed by a compound containing a hydroxy or carboxyl. Such esters are, for example, pharmaceutically acceptable esters that are hydrolyzed in the human or animal body to yield a parent alcohol or acid. The compounds of Formula (I) of the present invention contain a carboxyl group and can form *in vivo* hydrolyzable esters with suitable groups, such as, but not limited to, alkyl, arylalkyl, and the like.

As used herein, the term "treating" any disease or disorder refers, in some embodiments, to ameliorating the disease or disorder (i.e., slowing or arresting or alleviating the development of the disease or at least one of its clinical symptoms). In other embodiments, "treating" refers to alleviating or ameliorating at least one physical parameter, including those that may not be perceptible to the patient. In other embodiments, "treating" refers to modulating the disease or disorder physically (e.g., stabilization of a perceptible symptom) or physiologically (e.g., stabilization of a physical parameter) or both. In other embodiments, "treating" refers to preventing or delaying the onset, occurrence or worsening of the disease or disorder.

The term "effective amount" or "therapeutically effective amount" refers to an amount of the compound of the present invention sufficient to achieve the intended application (including, but not limited to, the treatment of a disease as defined below). The therapeutically effective amount may vary depending on factors such as the intended application (*in vitro* or *in vivo*)*,* or the subject and disease condition being treated, such as the subject's weight and age, the severity of the disease condition, and the mode of administration, which can be readily determined by a person of ordinary skill in the art. The specific dosage will vary depending on factors such as the specific compound selected, the administration regimen employed, whether the compound is administered in combination with other compounds, the timing of administration, the target tissue, and the physical delivery system used.

Unless otherwise specified, abbreviations for any protective groups, amino acids, and other compounds used herein shall conform to their commonly used, recognized abbreviations, or refer to IUPAC-IUB Commission on Biochemical Nomenclature (see Biochem. 1972, 11: 942-944).

The pharmaceutical excipients may be those widely used in the field of drug production. Excipients are primarily used to provide a safe, stable, and functional drug composition, and may also provide a method, allowing the active ingredient to dissolve at a desired rate after administration to a subject, or promoting the effective absorption of the active ingredient after administration of the composition to a subject. The pharmaceutical excipients may be inert fillers or may provide a certain function, such as stabilizing the overall pH of the composition or preventing the degradation of the active ingredients in the composition. The pharmaceutical excipients may include one or more of the following: binders, suspending agents, emulsifiers, diluents, fillers, granulants, adhesives, disintegrants, lubricants, anti-adherents, flow aids, wetting agents, gelling agents, absorption retardants, dissolution inhibitors, enhancers, adsorbents, buffers, chelating agents, preservatives, colorants, flavorings, and sweeteners.

Substances that may be used as pharmaceutically acceptable excipients include, but are not limited to, ion exchangers, aluminum, aluminum stearate, lecithin; serum proteins such as human serum albumin; buffering agents such as phosphates, glycine, sorbic acid, potassium sorbate; mixtures of partial glycerides of saturated vegetable fatty acids, water, salts or electrolytes such as protamine sulfate, disodium hydrogen phosphate, potassium hydrogen phosphate, sodium chloride, zinc salts; colloidal silica, magnesium trisilicate, polyvinylpyrrolidone, polyacrylates, waxes, polyethylene-polyoxypropylene block polymer, lanolin; sugars such as lactose, glucose, and sucrose; starches such as corn starch and potato starch; cellulose and its derivatives such as sodium carboxymethyl cellulose, ethyl cellulose, and cellulose acetate; gum powders; malt; gelatin; talc; excipients such as cocoa butter and suppository wax; oils such as peanut oil, cottonseed oil, safflower oil, sesame oil, olive oil, corn oil, and soybean oil; diol compounds, such as propylene glycol and polyethylene glycol; esters, such as ethyl oleate and ethyl laurate; agar; buffers, such as magnesium hydroxide and aluminum hydroxide; alginic acid; pyrogen-free water; isotonic salts; Ringer's solution; ethanol, phosphate-buffered solution, and other suitable non-toxic lubricants such as sodium lauryl sulfate and magnesium stearate; colorants; release agents; coating materials; sweeteners; flavorings and fragrances; preservatives; and antioxidants.

The pharmaceutical compositions of the present invention may be prepared using any method known to those skilled in the art based on the disclosed content. For example, conventional mixing, dissolution, granulation, emulsification, grinding, encapsulation, embedding, or freeze-drying processes.

The dosage form of the drug of the present invention may be selected based on specific circumstances. The dosage form typically consists of drug, excipients, and a container/sealing system. One or more excipients (also known as inactive ingredients) may be added to the compound of the present invention to improve or facilitate drug manufacturing, stability, administration, and safety, and a method of obtaining the desired drug release profile may be provided. Therefore, the type of excipient added to the drug may depend on various factors, such as the physical and chemical properties of the drug, the route of administration, and the preparation steps. Pharmaceutical excipients exist in this field and include those listed in various pharmacopoeias. (See the U.S. Pharmacopeia (USP), the Japanese Pharmacopoeia (JP), the European Pharmacopoeia (EP), and the British Pharmacopoeia (BP); publications from the Center for Drug Evaluation and Research (CDER) of the U.S. Food and Drug Administration (www.fda.gov), such as the "Inactive Ingredient Guide (1996)"; "Handbook of Pharmaceutical Additives (2002)" by Ash, Synapse Information Resources, Inc., Endicott, NY; etc.

The pharmaceutical compositions of the present application may include one or more than one physiologically acceptable inactive ingredients that facilitate the processing of the active molecule into a formulation for pharmaceutical use.

The appropriate formulation depends on the desired route of administration. Routes of administration include intravenous injection, mucosal or intranasal administration, oral administration etc. For oral administration, the compound may be formulated into liquid or solid dosage forms and may be formulated as immediate-release or controlled-release/sustained-release formulations. Suitable dosage forms for individual via oral administration include tablets, pills, sugar-coated pills, hard- and soft-shell capsules, liquids, gels, syrups, ointments, suspensions, and emulsions. The preferred dosage form of the present invention is an injection.

Therapeutically effective doses may initially be estimated using various methods known in the art. Initial doses for animal studies may be based on effective concentrations established in cell culture assays. The appropriate dosage range for humans may, for example, be determined using data obtained from animal studies and cell culture assays. In certain embodiments, the compounds of the present invention may be prepared as formulations for oral administration.

The appropriate formulation, route of administration, dose, and dosing interval may be selected in accordance with methods known in the art, taking into account the particularity of the individual's condition.

The present invention first provides a compound represented by Formula (A), or a stereoisomer, tautomer, isotopically labeled form, pharmaceutically acceptable salt, or prodrug thereof, 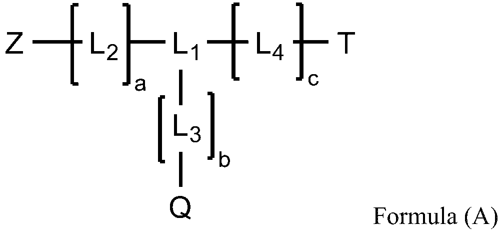
wherein Z is a payload, T is a targeting moiety,
Q is a covalent group and selected from the structure represented by any one of the following Formulas (Q-1), (Q-2), (Q-3), (Q-4), (Q-5), or (Q-6) to (Q-21): 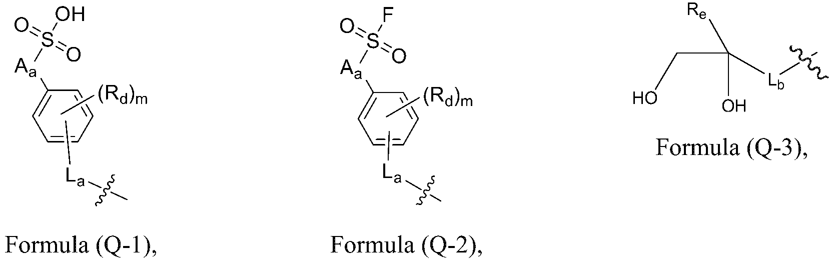 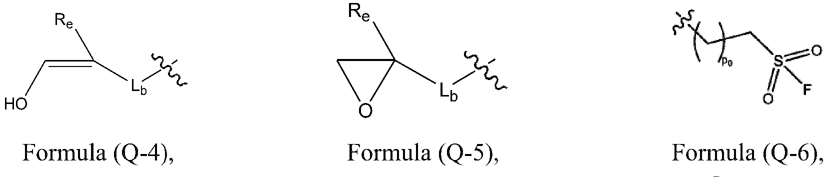 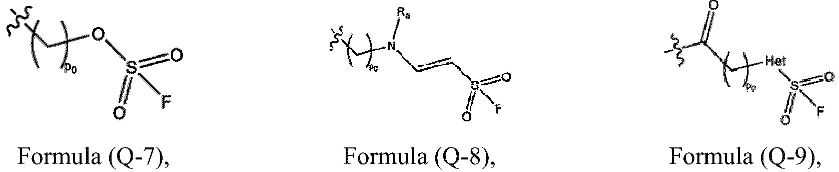 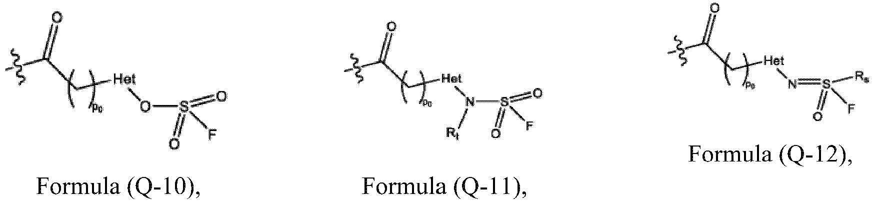 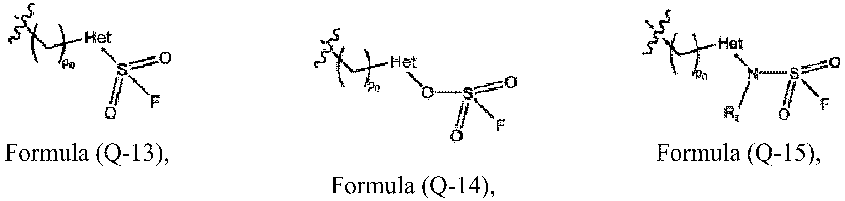 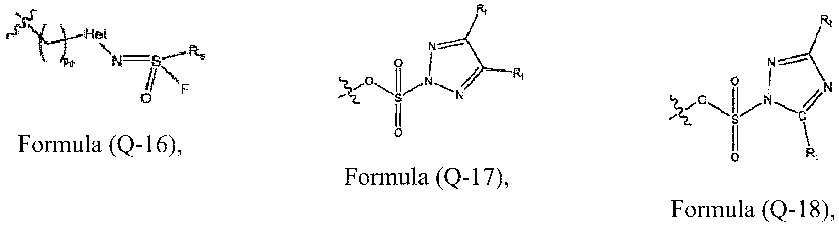 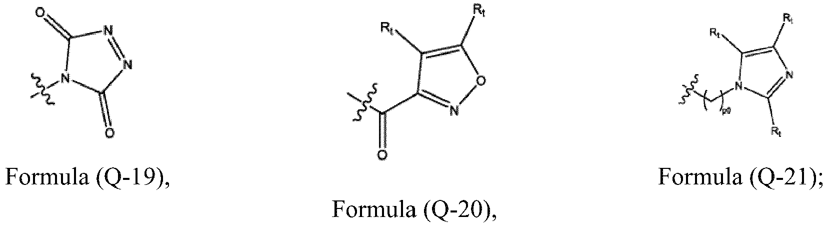
L₁, L₂, L₃, L₄, Aₐ, Lₐ, and L_{b} are the same or different, and are each independently selected from a bond or a linking group,
a, b, c, m, and p₀ are the same or different, and are each independently selected from an integer of 0 to 6 (e.g., 0, 1, 2, 3, 4, 5, 6),
R_{d} and Rₑ are the same or different, and are each independently selected from hydrogen, halogen, hydroxy, cyano, nitro, amino, C₁₋₂₀ alkyl, C₁₋₂₀ alkoxy, and halogenated C₁₋₂₀ alkyl;
Het is an optionally substituted heterocyclylene or heteroarylene;
Rₛ is selected from hydrogen, optionally substituted alkyl, optionally substituted cycloalkyl, optionally substituted heterocycloalkyl, optionally substituted aryl, and optionally substituted heteroaryl;
each Rₜ is independently selected from hydrogen, halogen, nitro, cyano, optionally substituted mercapto, optionally substituted amino, optionally substituted selenyl, optionally substituted C₁₋₂₀ alkyl, optionally substituted C₃₋₂₀ cycloalkyl, optionally substituted heterocycloalkyl, optionally substituted aryl, and optionally substituted heteroaryl;
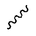 denotes the attachment site of the group.

According to an embodiment of the present invention, Het is a 3- to 20-membered heterocyclylene or 5- to 20-membered heteroarylene optionally substituted by one, two, or more R₃; preferably, Het is a 3- to 10-membered heterocyclylene or 5- to 10-membered heteroarylene optionally substituted by one, two, or more R₃. According to an embodiment of the present invention, R₃ is the same or different and is independently selected from hydrogen, halogen, hydroxy, cyano, nitro, amino, C₁₋₁₀ alkyl, C₁₋₁₀ alkoxy, halogenated C₁₋₁₀ alkyl, and halogenated C₁₋₁₀ alkoxy; preferably, R₃ is the same or different and is independently selected from hydrogen, halogen, hydroxy, cyano, nitro, amino, C₁₋₆ alkyl, C₁₋₆ alkoxy, halogenated C₁₋₆ alkyl, and halogenated C₁₋₆ alkoxy.

According to an embodiment of the present invention, Rₛ is selected from hydrogen; C₁₋₂₀ alkyl optionally substituted with one, two, or more R₄; C₃₋₂₀ cycloalkyl optionally substituted with one, two, or more R₄; 3- to 20- membered heterocyclyl optionally substituted with one, two, or more R₄; C₆₋₂₀ aryl optionally substituted with one, two or more R₄; or 5- to 20- membered heteroaryl optionally substituted with one, two or more R₄; preferably, Rₛ is selected from: hydrogen, C₁₋₁₀ alkyl optionally substituted with one, two, or more R₄; C₃₋₁₀ cycloalkyl optionally substituted with one, two, or more R₄; 3- to 10- membered heterocyclyl optionally substituted with one, two, or more R₄; C₆₋₁₀ aryl optionally substituted with one, two or more R₄; or 5- to 10-membered heteroaryl optionally substituted with one, two or more R₄. According to an embodiment of the present invention, R₄ is the same or different, and is independently selected from hydrogen, halogen, hydroxy, cyano, nitro, amino, C₁₋₁₀ alkyl, C₁₋₁₀ alkoxy, halogenated C₁₋₁₀ alkyl, and halogenated C₁₋₁₀ alkoxy; preferably, R₄ is the same or different, and is independently selected from hydrogen, halogen, hydroxy, cyano, nitro, amino, C₁₋₆ alkyl, C₁₋₆ alkoxy, halogenated C₁₋₆ alkyl, and halogenated C₁₋₆ alkoxy.

According to an embodiment of the present invention, Rₜ is selected from: hydrogen, halogen, nitro, cyano, mercapto optionally substituted with one, two, or more R₅, amino optionally substituted with one, two, or more R₅, selenyl optionally substituted with one, two, or more R₅, C₁₋₂₀ alkyl optionally substituted with one, two, or more R₅, C₃₋₂₀ cycloalkyl optionally substituted with one, two, or more R₅, 3- to 20-membered heterocyclyl optionally substituted with one, two, or more R₅, C₆₋₂₀ aryl optionally substituted with one, two, or more R₅, and 5- to 20-membered heteroaryl optionally substituted with one, two, or more R₅; preferably, Rₜ is selected from: hydrogen, halogen, nitro, cyano, mercapto optionally substituted with one, two, or more R₅, C₁₋₁₀ alkyl optionally substituted with one, two, or more R₅, C₃₋₁₀ cycloalkyl optionally substituted with one, two, or more R₅, 3- to 10-membered heterocyclyl optionally substituted with one, two, or more R₅, C₆₋₁₀ aryl optionally substituted with one, two, or more R₅, and 5- to 10-membered heteroaryl optionally substituted with one, two, or more R₅. According to embodiments of the present invention, R₅ is the same or different and is independently selected from hydrogen, halogen, hydroxy, cyano, nitro, amino, C₁₋₁₀ alkyl, C₁₋₁₀ alkoxy, halogenated C₁₋₁₀ alkyl, halogenated C₁₋₁₀ alkoxy; preferably, R₅ is the same or different and is independently selected from hydrogen, halogen, hydroxy, cyano, nitro, amino, C₁₋₆ alkyl, C₁₋₆ alkoxy, halogenated C₁₋₆ alkyl, halogenated C₁₋₆ alkoxy. In some embodiments, when a compound contains multiple Rₜ, each Rₜ may be the same or different, and each Rₜ is independently selected from the groups described above.

According to an embodiment of the present invention, in Formula (A), L₁ is selected from a trivalent linking group; preferably, L₁ is selected from N, C₁₋₂₀ alkanetriyl optionally substituted with one or more R₀, and any combination thereof; wherein each R₀ is the same or different, and is independently selected from hydrogen, halogen, hydroxy, cyano, nitro, amino, C₁₋₁₀ alkyl, C₁₋₁₀ alkoxy, halogenated C₁₋₁₀ alkyl, and halogenated C₁₋₁₀ alkoxy; preferably, each R₀ is the same or different, and is independently selected from hydrogen, halogen, hydroxy, cyano, nitro, amino, C₁₋₆ alkyl, C₁₋₆ alkoxy, halogenated C₁₋₆ alkyl, and halogenated C₁₋₆ alkoxy; further preferably, each R₀ is the same or different, and is independently selected from hydrogen, halogen, hydroxy, cyano, nitro, amino, C₁₋₄ alkyl, C₁₋₄ alkoxy, halogenated C₁₋₄ alkyl, and halogenated C₁₋₄ alkoxy.

According to an embodiment of the present invention, L₂, L₃, L₄, Aₐ, Lₐ, and L_{b} are the same or different, and are each independently selected from a divalent linking group; preferably, L₂, L₃, L₄, Aₐ, Lₐ, and L_{b} are each independently selected from a bond, -O-, -S-, -P-, -O-(HO-P=O)-O-, - N(R₀)-, -CO-, C₁₋₂₀ alkylene optionally substituted with one or more R₀, 3- to 20-membered heterocyclylene optionally substituted with one or more R₀, and any combination thereof; further preferably, L₂, L₃, L₄, Aₐ, Lₐ, and L_{b} are each independently selected from a bond, -O-, -S-, -P-, - O-(HO-P=O)-O-, -N(R₀)-, -CO-, C₁₋₁₀ alkylene (C₁₋₆ alkylene, C₁₋₄ alkylene) optionally substituted with one or more R₀, 3- to 20-membered heterocyclylene optionally substituted with one or more R₀ (3- to 10-membered heterocyclylene, 3- to 6-membered heterocyclylene), and any combination thereof.

According to an embodiment of the present invention, each R₀ is the same or different, and is independently selected from hydrogen, halogen, hydroxy, cyano, nitro, amino, C₁₋₁₀ alkyl, C₁₋₁₀ alkoxy, halogenated C₁₋₁₀ alkyl, and halogenated C₁₋₁₀ alkoxy; preferably, each R₀ is the same or different, and is independently selected from hydrogen, halogen, hydroxy, cyano, nitro, amino, C₁₋₆ alkyl, C₁₋₆ alkoxy, halogenated C₁₋₆ alkyl, and halogenated C₁₋₆ alkoxy; further preferably, each R₀ is the same or different, and is independently selected from hydrogen, halogen, hydroxy, cyano, nitro, amino, C₁₋₄ alkyl, C₁₋₄ alkoxy, halogenated C₁₋₄ alkyl, and halogenated C₁₋₄ alkoxy.

According to an embodiment of the present invention, the heterocyclylene comprises at least one N (e.g., 1, 2, 3, or 4 N); preferably, the heterocyclylene is piperazinylene.

According to an embodiment of the present invention, at least one of L₂ and L₄ comprises 3-to 20-membered heterocyclylene (3- to 10-membered heterocyclylene, 3- to 6-membered heterocyclylene); preferably, at least one of L₂ and L₄ comprises heterocyclylene having one nitrogen atom or two nitrogen atoms; preferably, at least one of L₂ and L₄ comprises piperazinylene.

According to an embodiment of the present invention, R_{d} and Re are the same or different, and are each independently selected from hydrogen, halogen, hydroxy, cyano, nitro, amino, C₁₋₁₀ alkyl, C₁₋₁₀ alkoxy, halogenated C₁₋₁₀ alkyl, and halogenated C₁₋₁₀ alkoxy; preferably, each independently selected from hydrogen, halogen, hydroxy, cyano, nitro, amino, C₁₋₆ alkyl, C₁₋₆ alkoxy, halogenated C₁₋₆ alkyl, and halogenated C₁₋₆ alkoxy; further preferably, each independently selected from hydrogen, halogen, hydroxy, cyano, nitro, amino, C₁₋₄ alkyl, C₁₋₄ alkoxy, halogenated C₁₋₄ alkyl, and halogenated C₁₋₄ alkoxy.

According to an embodiment of the present invention, the compound represented by Formula (A) is a multifunctional compound comprising at least three functional groups: a targeting moiety T, a covalent group Q, and a payload Z.

The various embodiments of the compounds of the present invention are described below.

### [Targeting moiety T]

T is selected from a targeting moiety that can target fibroblast activation protein-α, and may be a small molecule compound, a polypeptide, an antibody, etc.

In some embodiments, T is selected from a structure targeting fibroblast activation protein-α represented by the following Formula (T-0): 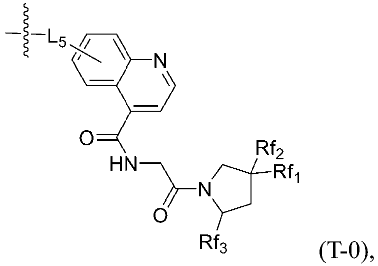
in Formula (T-0), L₅ is selected from a divalent linking group, preferably is S, O, and a bond;
Rf₁ and Rf₂ are independently selected from hydrogen and halogen, such as fluorine, chlorine, iodine, bromine, preferably fluorine;
Rf₃ is selected from hydrogen and cyano.
By way of example, T may be selected from the structure targeting fibroblast-activating protein-α represented by Formula (T-1) below, 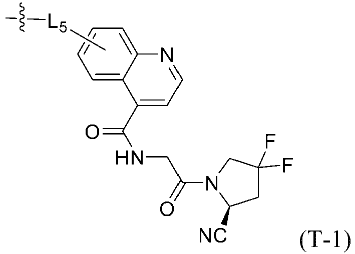
wherein L₅ is selected from a divalent linking group, preferably is S, O, or a bond, and preferably is a bond; 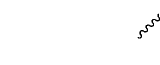 denotes the attachment site of the group.

As an example, T is selected from the structure represented by the following Formula (T-1-1): 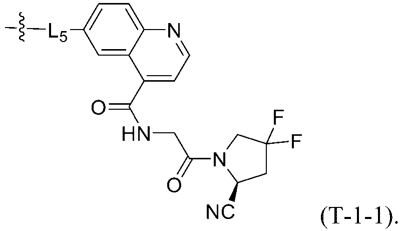
wherein L₅ is selected from a divalent linking group, preferably is S, O, or a bond, and preferably is a bond;
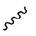 denotes the attachment site of the group.

### [Payload Z]

The payload Z may be a luminescent group, a fluorescent group, a cytotoxin, a radionuclide chelating group, etc.

According to an embodiment of the present invention, Z is selected from a nuclide chelating group.

According to an embodiment of the present invention, Z is selected from a group formed by a bifunctional chelating agent; preferably, the bifunctional chelating agent is selected from DOTA, NOTA, NODA, NODAGA, DOTP, TETA, ATSM, PTSM, EDTA, EC, HBEDCC, DTPA, SBAD, BAPEN, Df, DFO, TACN, NO₂A/NOTAM, CB-DO₂A, Cyclen, NOTA-AA, DO₃A, DO₃AP, HYNIC, MAS₃, MAG₃, DOTAGA, NOTAGA, and isonitrile.

According to an embodiment of the present invention, Z is preferably selected from

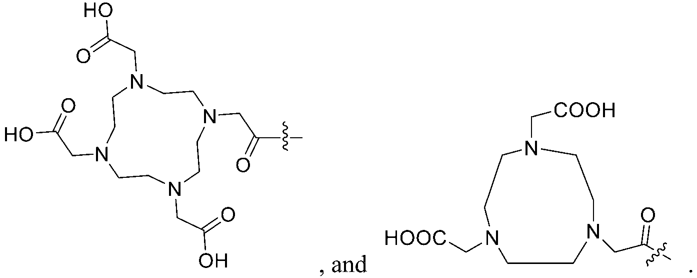

### [Covalent group Q]

The covalent group Q has a role of covalently binding to fibroblast activation protein α.

In some embodiments, Q is selected from the following groups:

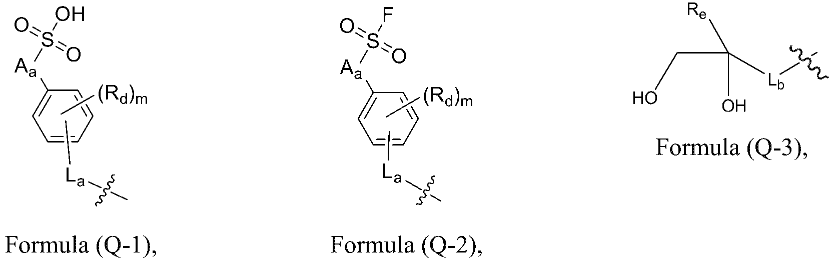

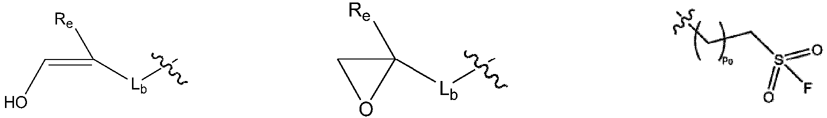

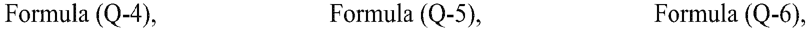

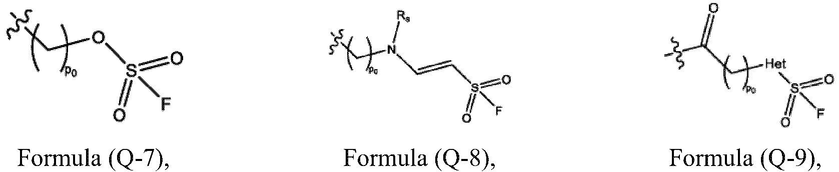

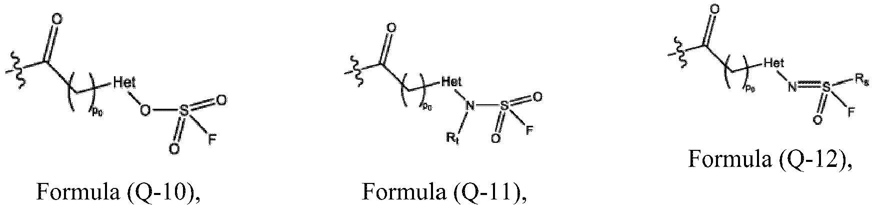

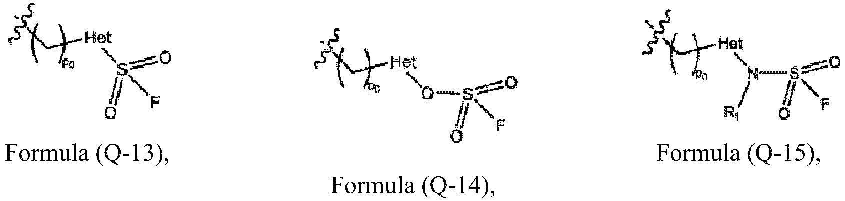

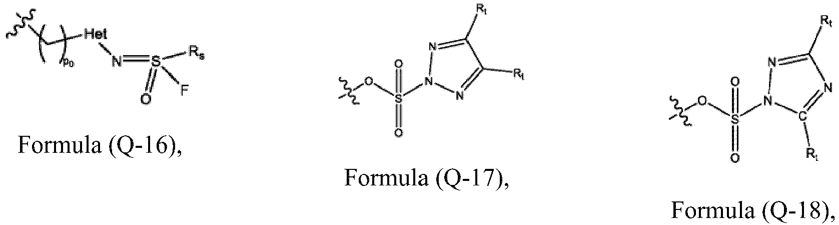

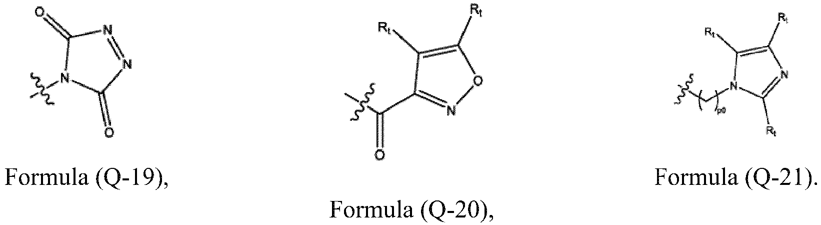

Further, in the compound represented by Formula (A), Q is selected from the structures represented by the following formulas (Q-1-1), (Q-2-1), (Q-3), (Q-4) and (Q-5):

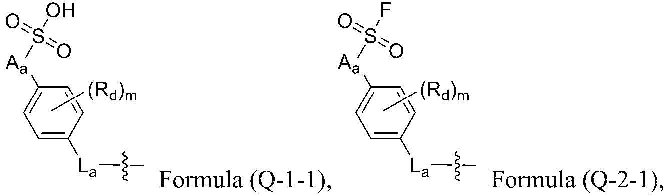

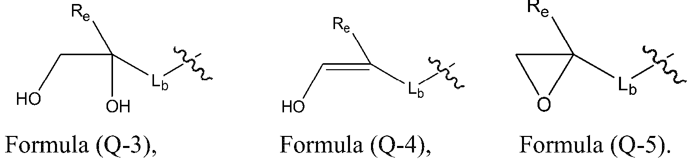

The inventors have found that when Q is selected from the para-structures of Formulae (Q-1-1) and (Q-2-1), it has a better covalent binding effect than the ortho- and meta-structures.

In other embodiments, Q is selected from the group represented by any one of Formulae (Q-6) to (Q-16), wherein Het is a 3- to 20-membered heterocyclylene or 5- to 20-membered heteroarylene optionally substituted with one, two or more R₃; preferably, Het is a 3- to 12-membered (e.g., 4- to 12-membered, 3- to 10-membered) heterocyclylene or 5- to 12-membered (e.g., 5- to 10-membered) heteroarylene optionally substituted with one, two or more R₃.

According to an embodiment of the present invention, Aₐ, Lₐ, and L_{b} are a divalent linking group, and may be independently selected from a bond, -O-, -S-, -P-, -O-(HO-P=O)-O- ((phosphate group), -N(R₀)-, -CO-, C₁₋₂₀ alkylene optionally substituted with one or more R₀, 3- to 20-membered heterocyclylene optionally substituted with one or more R₀, and any combination thereof; further preferably, Aₐ, Lₐ, and L_{b} are each independently selected from a bond, -O-, -S-, - P-, -O-(HO-P=O)-O-, -N(R₀)-, -CO-, C₁₋₁₀ alkylene (C₁₋₆ alkylene, C₁₋₄ alkylene) optionally substituted with one or more R₀, 3- to 20-membered heterocyclylene optionally substituted with one or more R₀ (3- to 10-membered heterocyclylene, 3- to 6-membered heterocyclylene), and any combination thereof;
wherein, each R₀ is the same or different, and is independently selected from hydrogen, halogen, hydroxy, cyano, nitro, amino, C₁₋₁₀ alkyl, C₁₋₁₀ alkoxy, halogenated C₁₋₁₀ alkyl, and halogenated C₁₋₁₀ alkoxy; preferably, each R₀ is the same or different, and is independently selected from hydrogen, halogen, hydroxy, cyano, nitro, amino, C₁₋₆ alkyl, C₁₋₆ alkoxy, halogenated C₁₋₆ alkyl, and halogenated C₁₋₆ alkoxy; further preferably, each R₀ is the same or different, and is independently selected from hydrogen, halogen, hydroxy, cyano, nitro, amino, C₁₋₄ alkyl, C₁₋₄ alkoxy, halogenated C₁₋₄ alkyl, and halogenated C₁₋₄ alkoxy.

According to an embodiment of the present invention, R_{d} and Re are each selected from hydrogen, halogen, hydroxy, cyano, nitro, amino, C₁₋₁₀ alkyl, C₁₋₁₀ alkoxy, halogenated C₁₋₁₀ alkyl, and halogenated C₁₋₁₉ alkoxy; preferably, R_{d} and Re are each selected from hydrogen, halogen, hydroxy, cyano, nitro, amino, C₁₋₆ alkyl, C₁₋₆ alkoxy, halogenated C₁₋₆ alkyl, and halogenated C₁₋₆ alkoxy; further preferably, R_{d} and Re each selected from hydrogen, halogen, hydroxy, cyano, nitro, amino, C₁₋₄ alkyl, C₁₋₄ alkoxy, halogenated C₁₋₄ alkyl, and halogenated C₁₋₄ alkoxy.

Preferably, when m is not 0, each R_{d} is independently selected from hydrogen, halogen, hydroxy, cyano, nitro, amino, C₁₋₁₀ alkyl, C₁₋₁₀ alkoxy, halogenated C₁₋₁₀ alkyl, and halogenated C₁₋₁₀ alkoxy.

In some embodiments, Aₐ is oxygen; in other embodiments, Aₐ is a bond.

In some embodiments, Lₐ and L_{b} are independently selected from the structure represented by Formula (L-2) below: 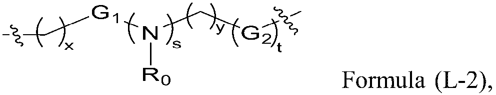 x is selected from 0, 1, and 2; s is 0 or 1; y is selected from 0, 1, 2, 3, and 4; t is 0 or 1; G₁ or G₂ is independently -CH₂- or carbonyl, and they are not -CH₂- at the same time; R₀ is each independently selected from hydrogen, halogen, hydroxy, cyano, nitro, amino, C₁₋₁₀ alkyl, C₁₋₁₀ alkoxy, halogenated C₁₋₁₀ alkyl, and halogenated C₁₋₁₀ alkoxy; 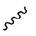 denotes the attachment site of the group.

In some embodiments, Lₐ and L_{b} are independently selected from the groups represented by Formula (L-3) or Formula (L-4) below: 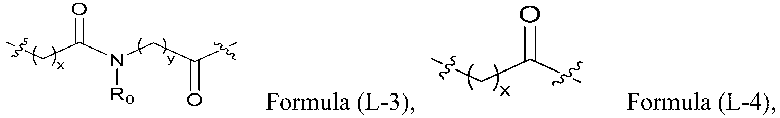 x is selected from 0, 1, and 2; y is selected from 0, 1, 2, 3, and 4; and R₀ is H or C₁₋₃ alkyl.

As an example, Q is selected from the groups represented by Formula (L-5) and Formula (L-6) below: 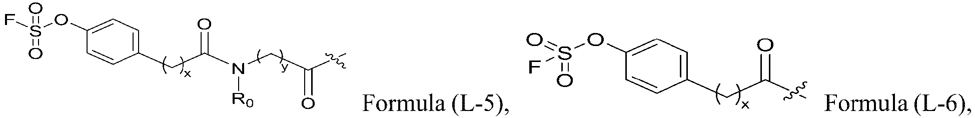 x is selected from 0, 1, and 2; y is selected from 0, 1, 2, 3, and 4; and R₀ is H or C₁₋₃ alkyl.

In some embodiments, Lₐ and L_{b} are independently selected from: 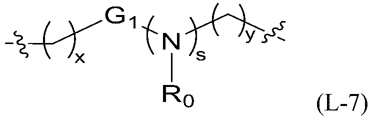
wherein x is selected from 0, 1, and 2; s is 0 or 1; and y is selected from 0, 1, 2, 3, and 4;
G₁ is -CH₂- or carbonyl group;
R₀ is each independently selected from hydrogen, halogen, hydroxy, cyano, nitro, amino, C₁₋₁₀ alkyl, C₁₋₁₀ alkoxy, halogenated C₁₋₁₀ alkyl, halogenated C₁₋₁₀ alkoxy; 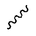 denotes the attachment site of the group.

As some specific embodiments, Q may be selected from any one of the structures listed in the table below:

| No. | Covalent Group Q | No. | Covalent Group Q |
|---|---|---|---|
| 1. | 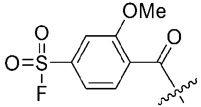 | 22. | 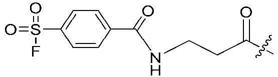 |
| 2. | 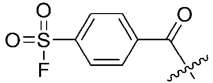 | 23. | 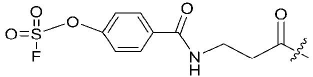 |
| 3. | 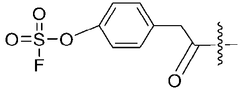 | 24. | 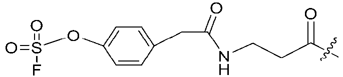 |
| 4. | 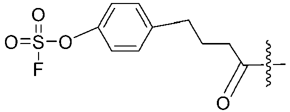 | 25. | 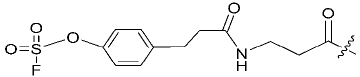 |
| 5. | 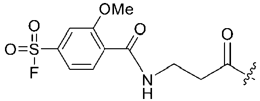 | 26. | 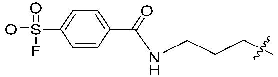 |
| 6. | 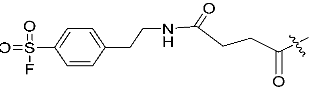 | 27. | 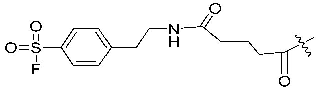 |
| 7. | 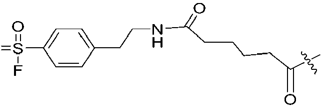 | 28. | 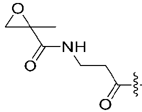 |
| 8. | 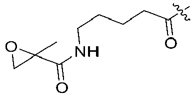 | 29. | 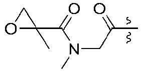 |
| 9. | 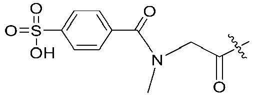 | 30. | 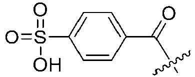 |
| 10. | 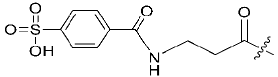 | 31. | 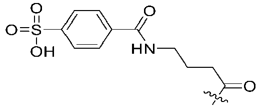 |
| 11. | 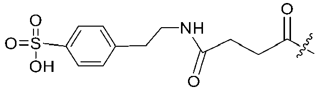 | 32. | 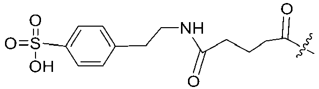 |
| 12. | 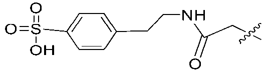 | 33. | 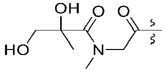 |
| 13. | 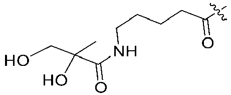 | 34. | 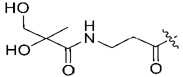 |
| 14. | 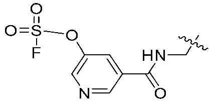 | 35. | 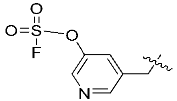 |
| 15. | 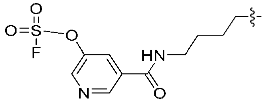 | 36. | 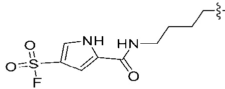 |
| 16. | 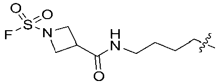 | 37. | 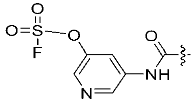 |
| 17. | 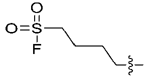 | 38. | 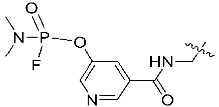 |
| 18. | 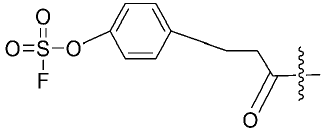 | 39. | 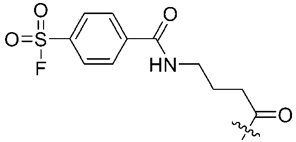 |
| 19. | 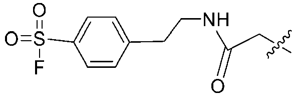 | 40. | 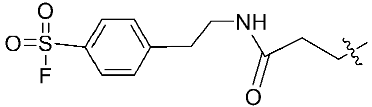 |
| 20. | 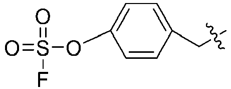 | 41. | 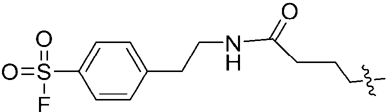 |
| 21. | 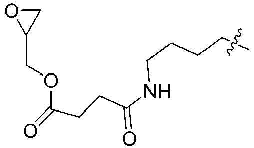 | | |

### [Linking Group]

In the compounds represented by Formula (A), the linking group shown below is included: 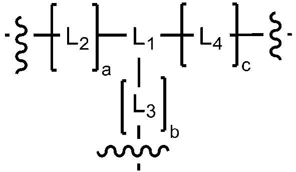
wherein a, b, and c may be independently selected from an integer of 0 to 6, and L₂, L₃, and L₄ are the same or different, and are each independently selected from a divalent linking group; preferably, L₂, L₃, and L₄ are each independently selected from a bond, -O-, -S-, -N(R₀)-, -CO-, C₁₋₂₀ alkylene optionally substituted with one or more R₀, 3- to 20-membered heterocyclylene optionally substituted with one or more R₀, and any combination thereof; further preferably, L₂, L₃, and L₄ are each independently selected from a bond, -O-, -S-, -N(R₀)-, -CO-, C₁₋₁₀ alkylene (C₁₋₆ alkylene, C₁₋₄ alkylene) optionally substituted with one or more R₀, 3- to 20-membered heterocyclylene (3- to 10-membered heterocyclylene, 3- to 6-membered heterocyclylene) optionally substituted with one or more R₀ , and any combination thereof;
L₁ is selected from a trivalent linking group; preferably, L₁ is selected from N, C₁₋₂₀ alkanetriyl optionally substituted with one or more R₀, and any combination thereof; preferably, L₁ is selected from N, C₁₋₁₀ alkanetriyl (C₁₋₆ alkanetriyl, C₁₋₄ alkanetriyl) optionally substituted with one or more R₀, and any combination thereof;
wherein, each R₀ is the same or different, and is independently selected from hydrogen, halogen, hydroxy, cyano, nitro, amino, C₁₋₁₀ alkyl, C₁₋₁₀ alkoxy, halogenated C₁₋₁₀ alkyl, and halogenated C₁₋₁₀ alkoxy; preferably, each R₀ is the same or different, and is independently selected from hydrogen, halogen, hydroxy, cyano, nitro, amino, C₁₋₆ alkyl, C₁₋₆ alkoxy, halogenated C₁₋₆ alkyl, and halogenated C₁₋₆ alkoxy; further preferably, each R₀ is the same or different, and is independently selected from hydrogen, halogen, hydroxy, cyano, nitro, amino, C₁₋₄ alkyl, C₁₋₄ alkoxy, halogenated C₁₋₄ alkyl, and halogenated C₁₋₄ alkoxy.

In some embodiments, L₂, L₃, and L₄ each comprise the structure represented by Formula (L-1) below: 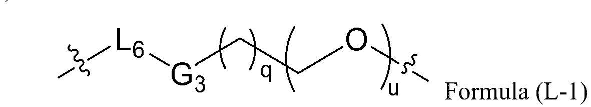
wherein q is an integer of 1 to 20, and u is an integer of 0 to 10;
G₃ is C₃₋₁₀ cycloalkyl, 3- to 10-membered heterocycloalkyl, 5- to 10-membered heteroaryl, and C₆₋₁₀ aryl; preferably, C₃₋₆ cycloalkyl, 3- to 6-membered heterocycloalkyl, 5- to 10-membered heteroaryl, and C₆₋₁₉ aryl;
L₆ is a bond or -CO-C₁₋₃ alkylene-NH-.

In some embodiments, in Formula (L-1), G₃ is selected from at least one of the following groups:

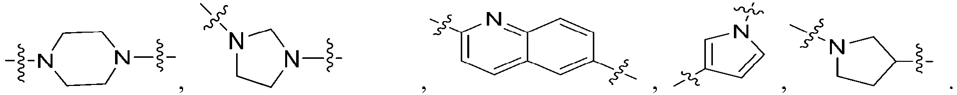

In some embodiments, q is an integer of 1 to 5, and u is 0.

In some embodiments, L₂, L₃, and L₄ each comprise -N(R₀) or the structure represented by Formula (L-1): 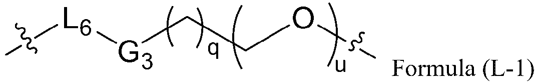
wherein q is an integer of 1 to 20, and u is an integer of 0 to 10;
G₃ is C₃₋₁₀ cycloalkyl, 3- to 10-membered heterocycloalkyl, 5- to 10-membered heteroaryl, or C₆₋₁₀ aryl; preferably, C₃₋₆ cycloalkyl, 3- to 6-membered heterocycloalkyl, 5- to 10-membered heteroaryl, or C₆₋₁₀ aryl.

L₆ is a bond or -CO-C₁₋₃ alkylene-NH-.

In some embodiments, L₂, L₃, and L₄ may be independently selected from the following linking structure:

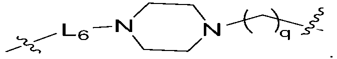

### [Compound A]

The compound represented by Formula (A), or a stereoisomer, tautomer, isotopically labeled form, pharmaceutically acceptable salt, or prodrug thereof, 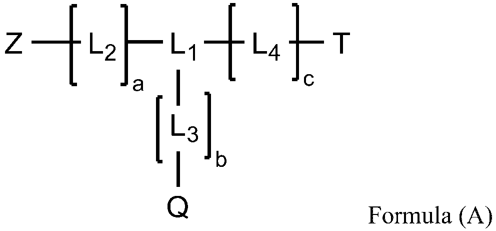
wherein Z is a payload, T is a targeting moiety,
Q is selected from the structure represented by the following Formulas (Q-1), (Q-2), (Q-3), (Q-4), or (Q-5), or the structure represented by any one of (Q-6) to (Q-21): 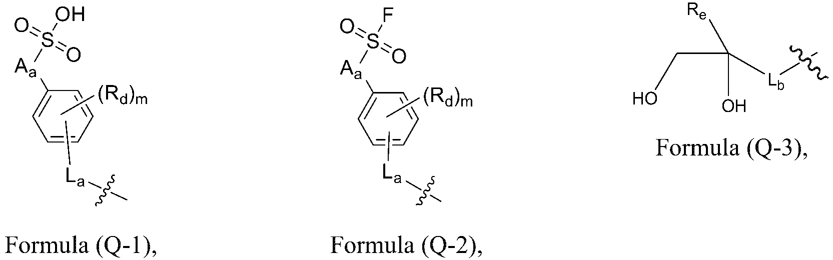 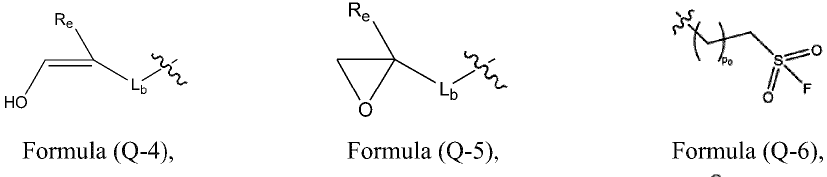 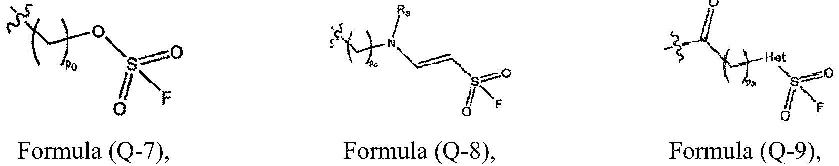 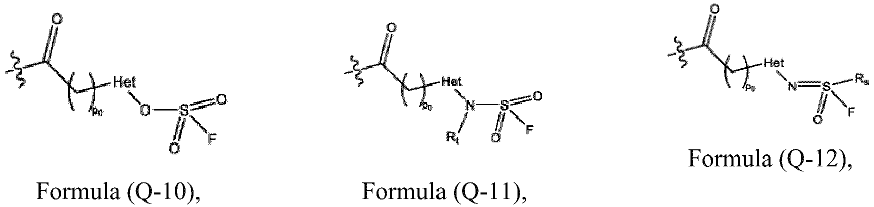 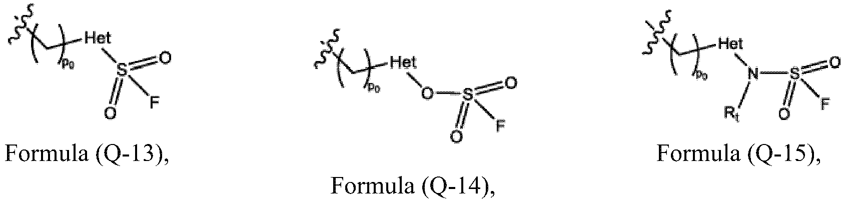 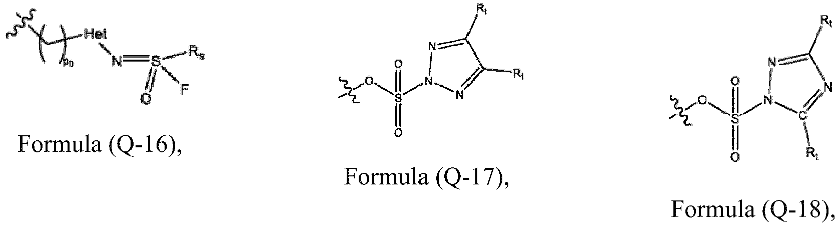 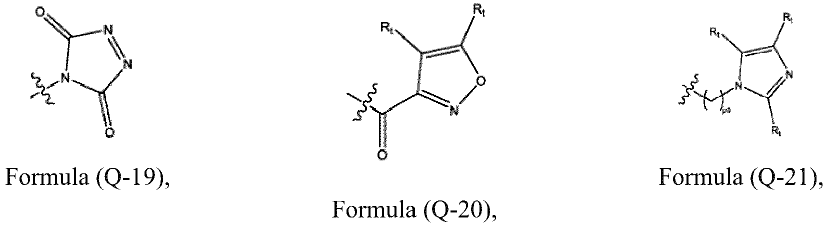
L₁, L₂, L₃, L₄, Aₐ, Lₐ, and L_{b} are the same or different, and are each independently selected from a bond or a linking group,
a, b, c, m, and p₀ are the same or different, and are each independently selected from an integer of 0 to 6 (e.g., 0, 1, 2, 3, 4, 5, 6),
R_{d} and Rₑ are the same or different, and are each independently selected from hydrogen, halogen, hydroxy, cyano, nitro, amino, C₁₋₂₀ alkyl, C₁₋₂₀ alkoxy, and halogenated C₁₋₂₀ alkyl;
Het is an optionally substituted saturated heterocyclylene or heteroarylene;
Rₛ is selected from hydrogen, optionally substituted alkyl, optionally substituted cycloalkyl, optionally substituted heterocycloalkyl, optionally substituted aryl, and optionally substituted heteroaryl;
each Rₜ is independently selected from hydrogen, halogen, nitro, cyano, optionally substituted mercapto, optionally substituted amino, optionally substituted selenyl, optionally substituted C₁₋₂₀ alkyl, optionally substituted C₃₋₂₀ cycloalkyl, optionally substituted heterocycloalkyl, optionally substituted aryl, and optionally substituted heteroaryl;
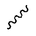 denotes the attachment site of the group.

In the compound represented by Formula (A), Q is selected from the structures represented by the following formulas (Q-1-1), (Q-2-1), (Q-3), (Q-4) and (Q-5):

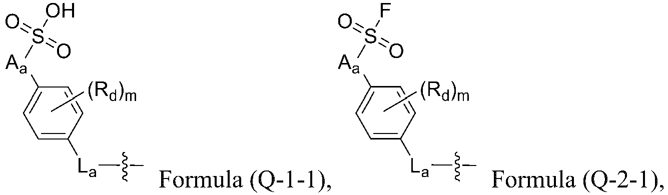

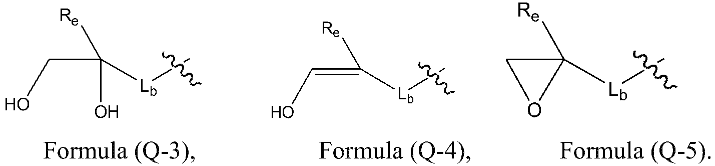

According to an embodiment of the present invention, the compound represented by Formula (A) has the structure represented by the following Formula (A-1), 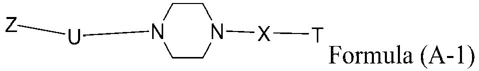 wherein,
U is selected from -U₁-, -U₂-U₃(Rₐ)-U₄-;
X is selected from X₁-, -X₂-X₃(R_{b})-X₄-;
when U is selected from -U₁-, X is not -X₁-;
U₁, U₂, U₃, U₄, X₁, X₂, X₃ and X₄ are the same or different, and are each independently selected from a linking group;
Rₐ and R_{b} are the same or different, and are each independently selected from the structure represented by Formulas (Q-1), (Q-2), (Q-3), (Q-4), or (Q-5).

In some embodiments, in the compound represented by Formula (A-1), U is selected from - U₂-U₃(Rₐ)-U₄-, and X is selected from -X₁-; or in the compound represented by Formula (A-1), U is selected from -U₁-, and X is selected from -X₂-X₃(R_{b})-X₄-.

In some embodiments, the compound represented by Formula (A) is selected from the compound represented by Formula (A-2) below: 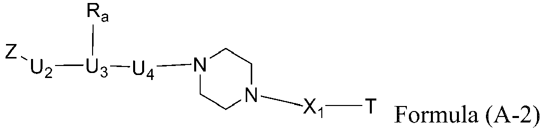
wherein Rₐ is selected from the structure represented by Formulas (Q-1), (Q-2), (Q-3), (Q-4), or (Q-5);
Z, T, U₂, U₃, U₄, X₁, Aₐ, Lₐ, L_{b}, R_{d}, Rₑ, and m are as defined above.

In some embodiments, the compound represented by Formula (A) is selected from the compound represented by Formula (A-3) below: 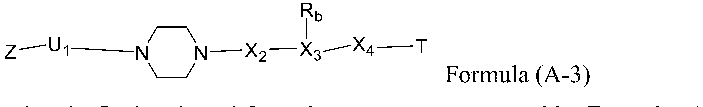
wherein, R_{b} is selected from the structure represented by Formulae (Q-1), (Q-2), (Q-3), (Q-4), or (Q-5);
Z, T, U₁, X₂, X₃, X₄, Aₐ, Lₐ, L_{b}, R_{d}, Rₑ, and m are as defined above.

According to an embodiment of the present invention, U₃ and X₃ are the same or different, and are each independently selected from a trivalent linking group; preferably, U₃ and X₃ are each independently selected from N, C₁₋₂₀ alkanetriyl (C₁₋₁₀ alkanetriyl, C₁₋₆ alkanetriyl, C₁₋₄ alkanetriyl) optionally substituted with one or more R₀, and any combination thereof.

According to an embodiment of the present invention, U₁, U₂, U₄, X₁, X₂, X₄, Lₐ, L_{b}, and Aₐ are the same or different, and are each independently selected from a divalent linking group; preferably, U₁, U₂, U₄, X₁, X₂, X₄, Lₐ, L_{b}, and Aₐ are each independently selected from a bond, - O-, -S-, -N(R₀)-, -CO-, C₁₋₂₀ alkylene (C₁₋₁₉ alkylene, C₁₋₆ alkylene, C₁₋₄ alkylene) optionally substituted with one or more R₀, and any combination thereof.

According to an embodiment of the present invention, R_{d} and Rₑ are the same or different, and are each independently selected from hydrogen, halogen, hydroxy, cyano, nitro, amino, C₁₋₁₀ alkyl, C₁₋₁₀ alkoxy, halogenated C₁₋₁₀ alkyl, and halogenated C₁₋₁₀ alkoxy; preferably, R_{d} and Rₑ are each independently selected from hydrogen, halogen, hydroxy, cyano, nitro, amino, C₁₋₆ alkyl, C₁₋₆ alkoxy, halogenated C₁₋₆ alkyl, and halogenated C₁₋₆ alkoxy; further preferably, R_{d} and Rₑ are each independently selected from hydrogen, halogen, hydroxy, cyano, nitro, amino, C₁₋₄ alkyl, C₁₋₄ alkoxy, halogenated C₁₋₄ alkyl, and halogenated C₁₋₄ alkoxy.

According to an embodiment of the present invention, R₀ is the same or different, and is each independently selected from hydrogen, halogen, hydroxy, cyano, nitro, amino, C₁₋₁₀ alkyl, C₁₋₁₀ alkoxy, halogenated C₁₋₁₀ alkyl, and halogenated C₁₋₁₀ alkoxy; preferably, R₀ is the same or different, and is each independently selected from hydrogen, halogen, hydroxy, cyano, nitro, amino, C₁₋₆ alkyl, C₁₋₆ alkoxy, halogenated C₁₋₆ alkyl, and halogenated C₁₋₆ alkoxy; further preferably, R₀ is the same or different, and is each independently selected from hydrogen, halogen, hydroxy, cyano, nitro, amino, C₁₋₄ alkyl, C₁₋₄ alkoxy, halogenated C₁₋₄ alkyl, and halogenated C₁₋₄ alkoxy.

According to an embodiment of the present invention, U₁ is selected from a bond. According to an embodiment of the present invention, Aₐ is selected from a bond. According to an embodiment of the present invention, X₄ is selected from a bond. According to an embodiment of the present invention, X₃ is selected from N. According to an embodiment of the present invention, X₂ is selected from C₁₋₁₀ alkylene (preferably, C₁₋₆ alkylene, C₁₋₄ alkylene, C₃ alkylene). According to an embodiment of the present invention, Lₐ is selected from -CO-, -CO-C₁₋₃ alkylene-, -CO-C₁₋₃ alkylene-NH-CO-, -CO-C₁₋₃ alkylene-NH-CO-C₁₋₃ alkylene-, -C₁₋₃ alkylene-, C₁₋₃ alkylene-NH-CO-C₁₋₃ alkylene-, -CO-C₁₋₄ alkylene-N(CH₃)-CO-, -CO-C₁₋₆ alkylene-CO-NH-C₁₋₃ alkylene-; according to an embodiment of the present invention, Lₐ is selected from -CO-C₁₋₆ alkylene-N(R₀)-CO-; preferably, Lₐ is selected from -CO-C₁₋₄ alkylene-N(R₀)-CO-, such as -CO-methylene-N(R₀)-CO-; preferably, R₀ is selected from C₁₋₄ alkyl, such as methyl.

According to an embodiment of the present invention, in the compound represented by Formula (A), Q is selected from the structure represented by Formula (Q-1), Formula (Q-3), or Formula (Q-4).

According to an embodiment of the present invention, the compound represented by Formula (A) is selected from any one of the following compounds:

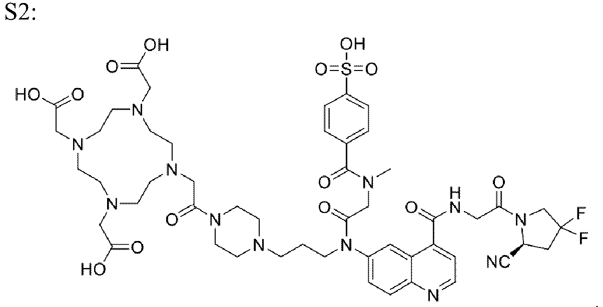

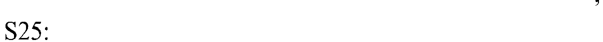

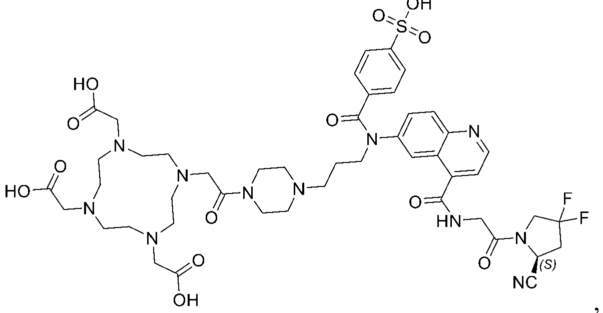

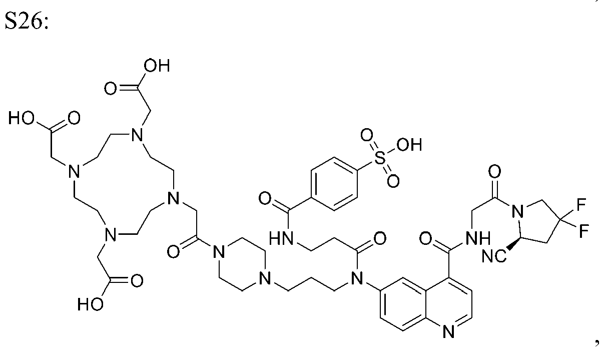

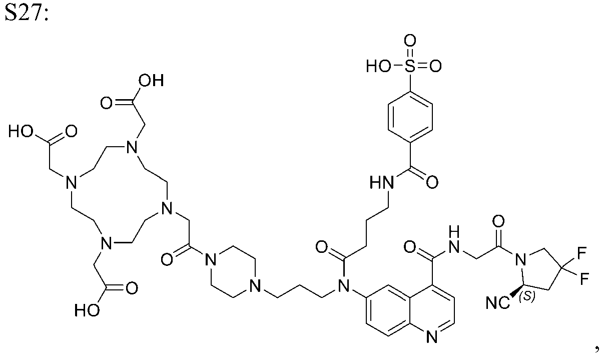

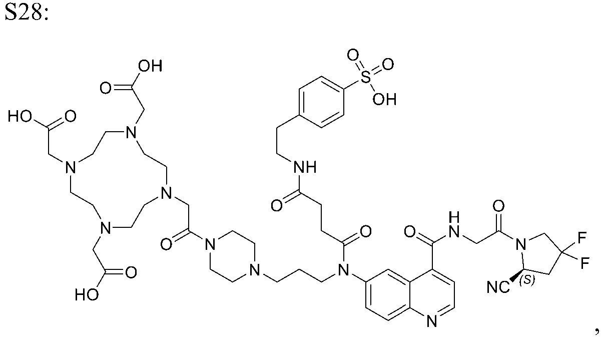

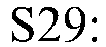

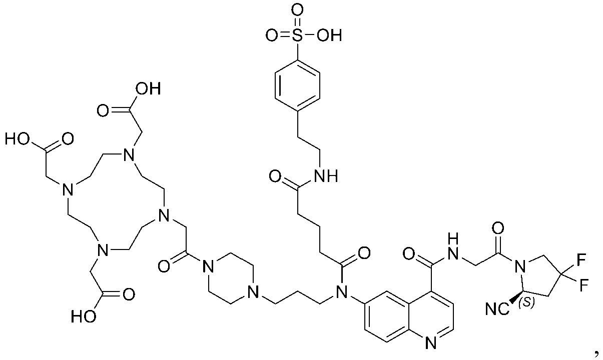

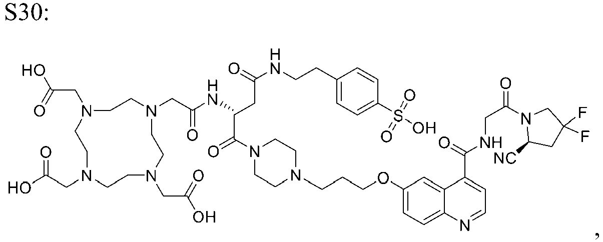

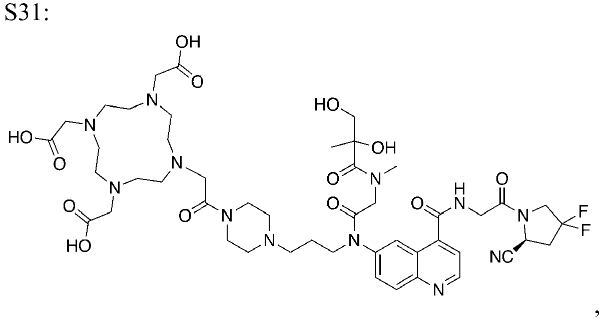

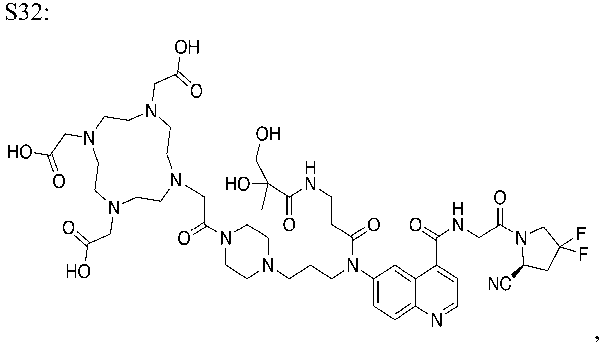

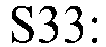

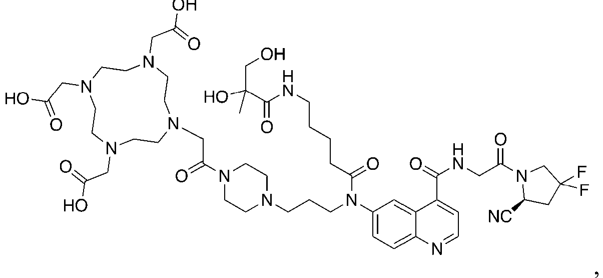

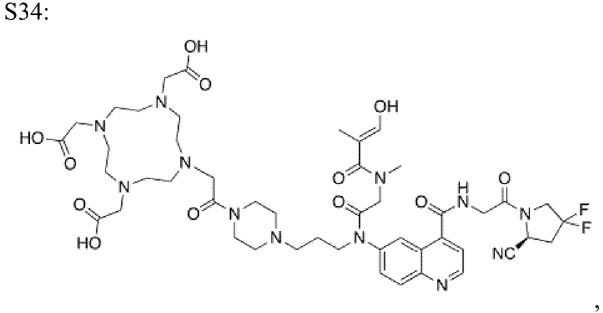

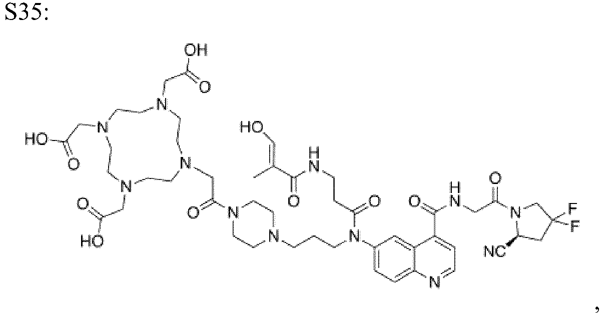

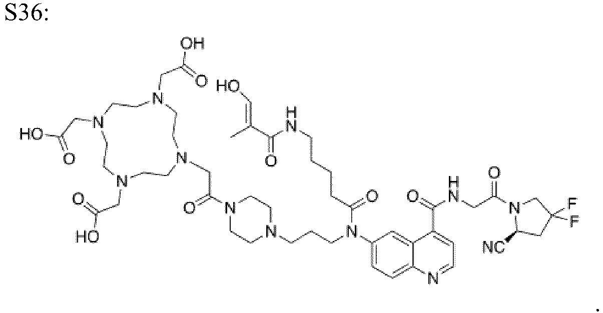

In some embodiments, the compound represented by Formula (A) is the compound represented by the following Formula (B):
The compound represented by Formula (B), or a stereoisomer, tautomer, isotopically labeled form, pharmaceutically acceptable salt, or prodrug thereof, characterized in that, 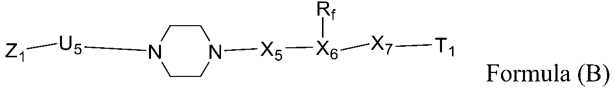
wherein Z₁ is a payload, T₁ is a targeting moiety,
U₅, X₅, X₆, and X₇ are the same or different, and are each independently selected from a linking group;
R_{f} is selected from the structure represented by Formula (Q-2-2) or Formula (Q-5-1) below: 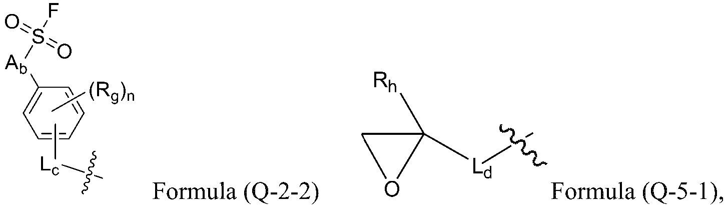
L_{c}, L_{d}, and A_{b} are the same or different, and are each independently selected from a linking group,
n is the same or different, and is each independently selected from an integer of 0 to 6 (e.g., 0, 1, 2, 3, 4, 5, 6),
R_{g} and Rₕ are the same or different, and are each independently selected from hydrogen, halogen, -OH, cyano, nitro, amino, C₁₋₂₀ alkyl, C₁₋₂₀ alkoxy, and halogenated C₁₋₂₀ alkyl;
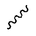 denotes the attachment site of the group.

Preferably, R_{f} is selected from the structure represented by the Formula (Q-2-3) below:

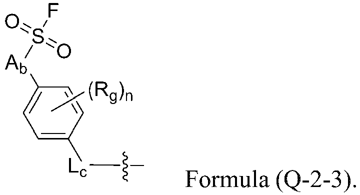

According to an embodiment of the present invention, Z₁, T₁, U₅, X₅, X₆, X₇, L_{c}, L_{d}, A_{b}, R_{g}, and Rₕ are as defined above for Z, T, U₁, X₂, X₃, X₄, Lₐ, L_{b}, Aₐ, R_{d}, and Rₑ, respectively.

According to an embodiment of the present invention, U₅ is selected from a bond, and -C₀₋₄ alkyl CO-;

According to an embodiment of the present invention, Aₐ is selected from a bond and -O-.

According to an embodiment of the present invention, X₇ is selected from a bond.

According to an embodiment of the present invention, X₆ is selected from N.

According to an embodiment of the present invention, X₅ is selected from C₁₋₁₀ alkylene (preferably C₁₋₆ alkylene, C₁₋₄ alkylene, and C₃ alkylene).

According to an embodiment of the present invention, L_{c} and L_{d} are the same or different, and are each independently selected from -CO-, -C₁₋₆ alkylene-, -CO-C₁₋₆ alkylene-, -C₁₋₆ alkylene-CON(R₀)-, -C₁₋₆ alkylene-N(R₀)-CO-, -CO-C₁₋₆ alkylene-N(R₀)-CO-C₁₋₆ alkylene-, -CO-C₁₋₆ alkylene-CO-N(R₀)-C₁₋₆ alkylene-, -C₁₋₆ alkylene-CO-N(R₀)-C₁₋₆ alkylene-, -C₁₋₆ alkylene-N(R₀)-CO-C₁₋₆ alkylene-, -C₁₋₆ alkylene-N(R₀)-CO-C₁₋₆ alkylene-COO-C₁₋₆ alkylene-, -C₁₋₆ alkylene-CO-N(R₀)-C₁₋₆ alkylene-COO-C₁₋₆ alkylene-, -CO-C₁₋₆ alkylene-N(R₀)-CO-C₁₋₆ alkylene-COO-C₁₋₆ alkylene-, -CO-C₁₋₆ alkylene-CO-N(R₀)-C₁₋₆ alkylene-COO-C₁₋₆ alkylene-, and the like; preferably, L_{c} and L_{d} are each independently selected from -C₁₋₃ alkylene-, -CO-C₁₋₃ alkylene-, -C₁₋₃ alkylene-CON(R₀)-, -C₁₋₃ alkylene-N(R₀)-CO-, -CO-C₁₋₃ alkylene-N(R₀)-CO-C₁₋₃ alkylene-, -CO-C₁₋₃ alkylene-CO-N(R₀)-C₁₋₃ alkylene-, -C₁₋₃ alkylene-CO-N(R₀)-C₁₋₃ alkylene-, -C₁₋₃ alkylene-N(R₀)-CO-C₁₋₃ alkylene-, -C₁₋₃ alkylene-N(R₀)-CO-C₁₋₃ alkylene-COO-C₁₋₃ alkylene-, -C₁₋₃ alkylene-CO-N(R₀)-C₁₋₃ alkylene-COO-C₁₋₃ alkylene-, -CO-C₁₋₃ alkylene-N(R₀)-CO-C₁₋₃ alkylene-COO-C₁₋₃ alkylene-, -CO-C₁₋₃ alkylene-CO-N(R₀)-C₁₋₃ alkylene-COO-C₁₋₃ alkylene-, and the like.

According to an embodiment of the present invention, R₀ is selected from C₁₋₄ alkyl, such as methyl;
According to an embodiment of the present invention, L_{c} is selected from -CO-, -CO-C₁₋₃ alkylene-, -CO-C₁₋₃ alkylene-NH-CO-, -CO-C₁₋₃ alkylene-NH-CO-C₁₋₃ alkylene-, C₁₋₃ alkylene - NH-CO-C₁₋₃ alkylene, -CO-C₁₋₆ alkylene-CO-NH-C₁₋₃ alkylene-, -CO-C₁₋₆ alkylene-N(R₀)-CO-, and -CO-C₁₋₄ alkylene-N(CH₃)-CO-.

According to an embodiment of the present invention, R_{g} is selected from hydrogen, halogen, C₁₋₆ alkyl, and C₁₋₆ alkoxy.

According to an embodiment of the present invention, L_{d} is selected from -CO-, CO-C₁₋₄ alkylene-, -CO-C₁₋₄ alkylene-N(CH₃)-CO-, and -CO-C₁₋₄ alkylene-NH-CO-.

According to an embodiment of the present invention, Rₕ is selected from hydrogen, C₁₋₆ alkyl, and C₁₋₆ alkoxy.

According to an embodiment of the present invention, A_{b} is selected from a bond and oxygen. According to an embodiment of the present invention, R_{g} is selected from H and methoxy.

In some embodiments, the compound represented by Formula (A) has the structure represented by Formula (A-4): 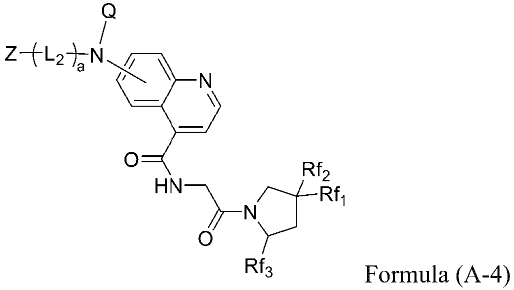
wherein Z is a payload,
Q is selected from the structures represented by the following Formulas (Q-1), (Q-2), (Q-3), (Q-4), and (Q-5): 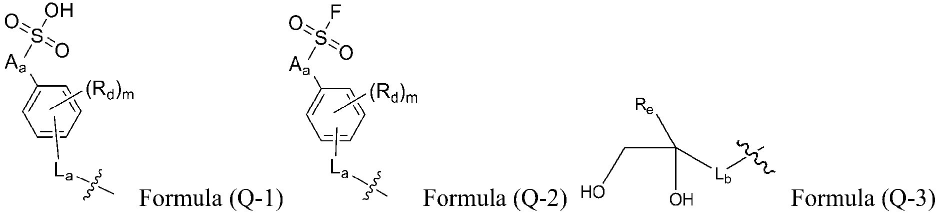 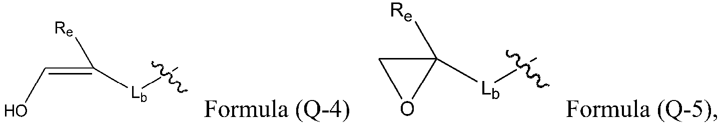
L₂, Aₐ, Lₐ, and L_{b} are the same or different, and are each independently selected from a linking group;
a and m are the same or different, and are each independently selected from an integer of 0 to 6;
R_{d} and Rₑ are the same or different, and are each independently selected from hydrogen, halogen, hydroxy, cyano, nitro, amino, C₁₋₂₀ alkyl, C₁₋₂₀ alkoxy, and halogenated C₁₋₂₀ alkyl;
Rf₁ and Rf₂ are independently selected from hydrogen and halogen, such as fluorine, chlorine, iodine, bromine, preferably fluorine;
Rf₃ is selected from hydrogen and cyano;
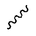 denotes the attachment site of the group.

According to an embodiment of the present invention, in Formula (A-4), L₂, Aₐ, Lₐ, and L_{b} have the same meanings as the linking groups and covalent groups described above.

According to an embodiment of the present invention, in Formula (A-4), L₂ comprises the structure represented by Formula (L-1) below: 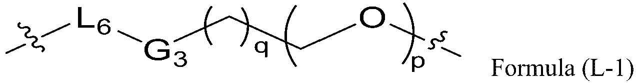
wherein q is an integer of 1 to 20, and p is an integer of 0 to 10;
G₃ is C₃₋₁₀ cycloalkyl, 3- to 10-membered heterocycloalkyl, 5- to 10-membered heteroaryl, or C₆₋₁₀ aryl; preferably, C₃₋₆ cycloalkyl, 3- to 6-membered heterocycloalkyl, 5- to 10-membered heteroaryl, or C₆₋₁₀ aryl;
L₆ is a bond or -CO-C₁₋₃ alkylene-NH-; and/or,
Lₐ and L_{b} are independently selected from: 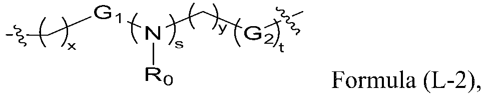
x is selected from 0, 1, and 2; s is 0 or 1; y is selected from 0, 1, 2, 3, and 4; t is 0 or 1;
G₁ or G₂ is independently -CH₂- or carbonyl, and they are not -CH₂- at the same time;
R₀ is each independently selected from hydrogen, halogen, OH, cyano, nitro, amino, C₁₋₁₀ alkyl, C₁₋₁₀ alkoxy, halogenated C₁₋₁₀ alkyl, and halogenated C₁₋₁₉ alkoxy;
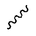 denotes the attachment site of the group.

According to an embodiment of the present invention, in Formula (L-1), G₃ is selected from at least one of the following groups: 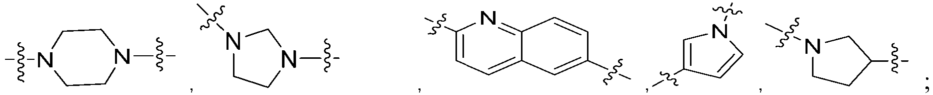 q is an integer of 1 to 5, and u is 0.

In some embodiments, Lₐ and L_{b} are independently selected from the groups represented by Formula (L-3) and Formula (L-4) below: 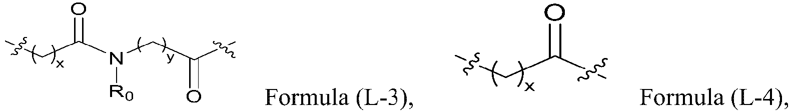 x is selected from 0, 1, and 2; y is selected from 0, 1, 2, 3, and 4; and R₀ is H or C₁₋₃ alkyl.

In some embodiments, in Formula (A-4), Q is selected from the groups represented by Formula (L-5) and Formula (L-6) below: 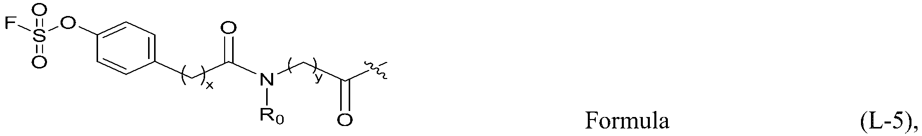 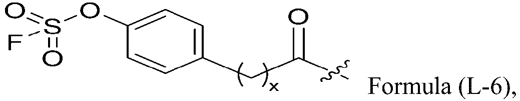 x is selected from 0, 1, and 2; y is selected from 0, 1, 2, 3, and 4; and R₀ is H or C₁₋₃ alkyl.

In the present invention, the inventors unexpectedly discovered that the compounds represented by Formula (A-4) exhibit superior tumor-targeting performance than when Q is attached closer to the Z side.

In some embodiments, when Q is Formula (L-5), the compound provides better tumor targeting performance and tumor retention capacity.

According to an embodiment of the present invention, the examples of the present application provide the following compounds:

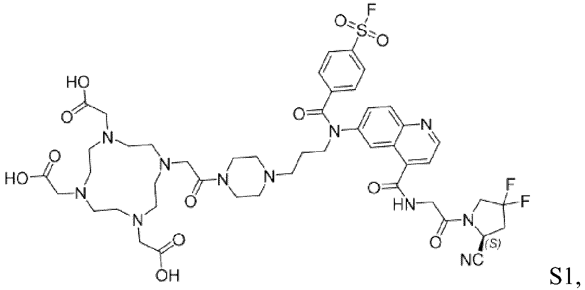

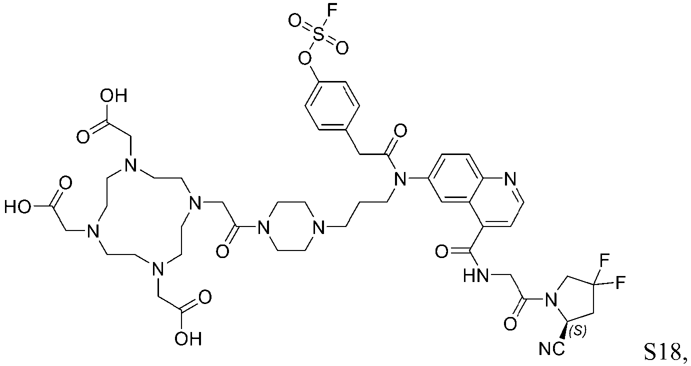

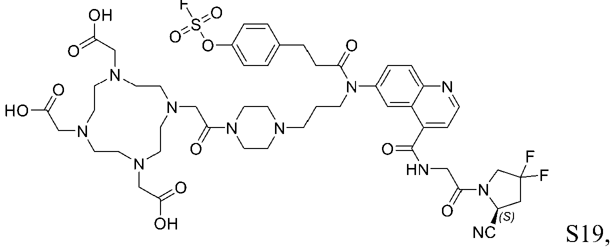

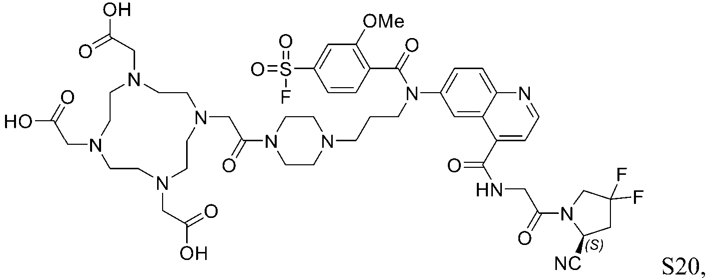

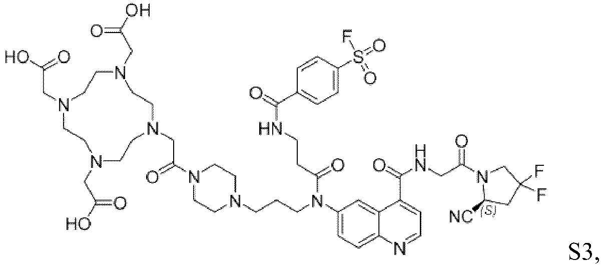

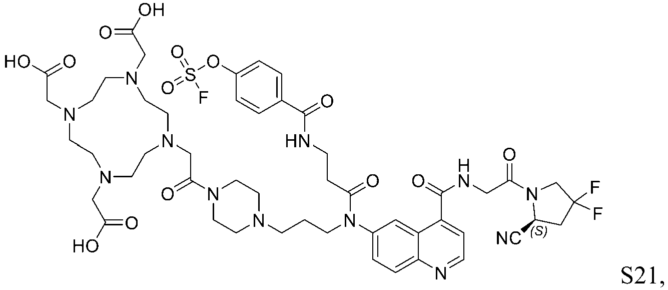

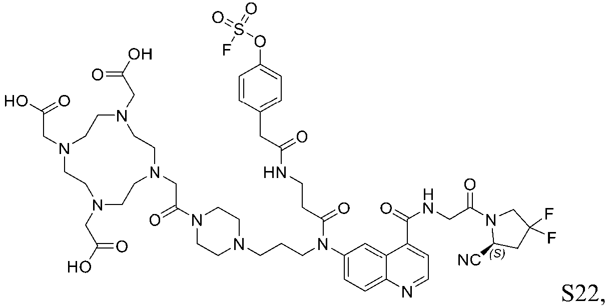

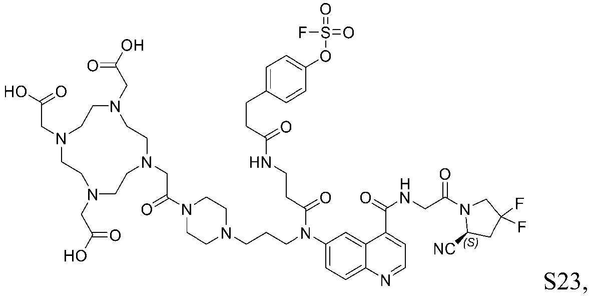

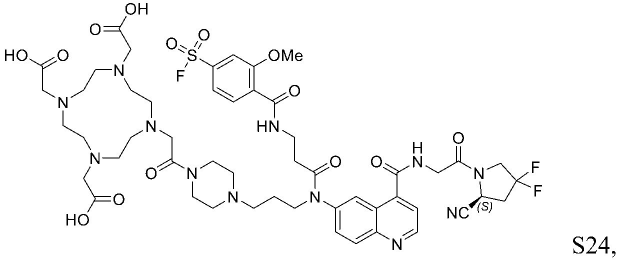

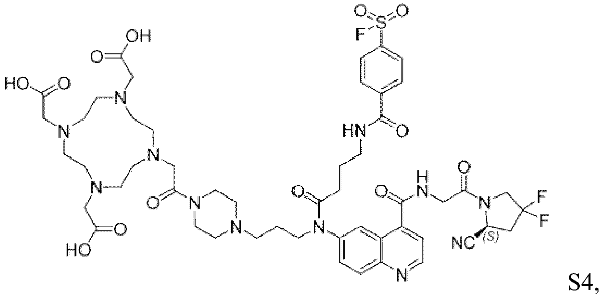

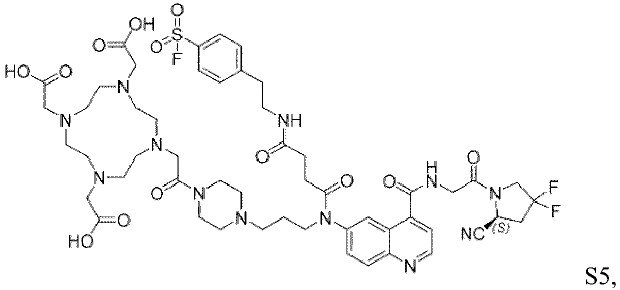

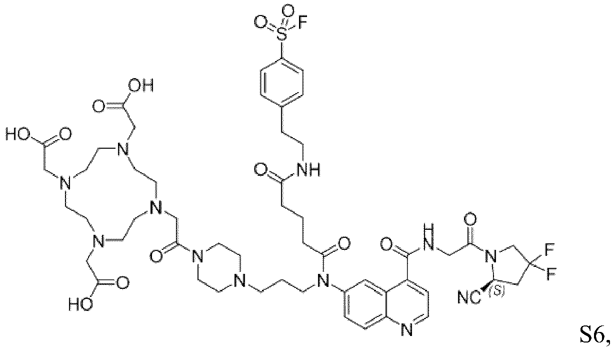

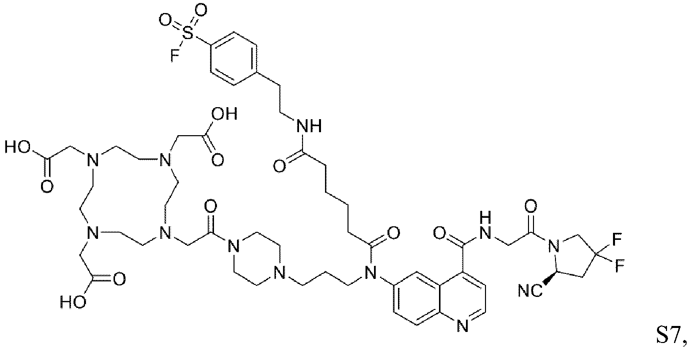

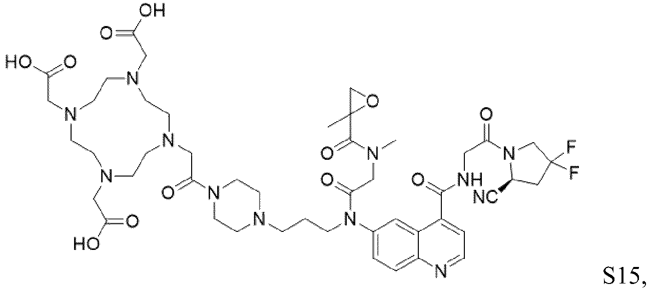

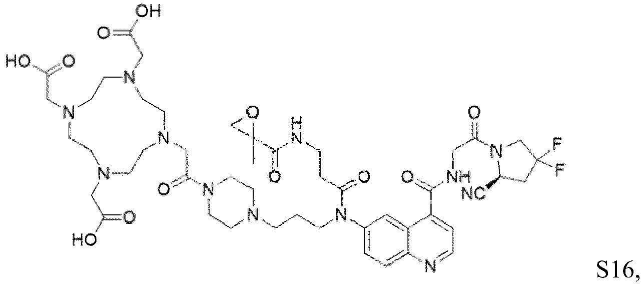

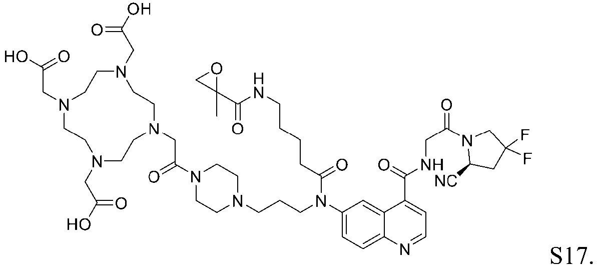

In some embodiments, the compound of Formula (A) has the structure represented by Formula (D) below: 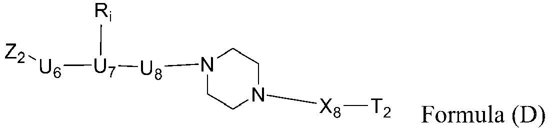
wherein Z₂ is a payload, T₂ is a targeting moiety,
U₆, U₇, U₈, and X₈ are the same or different, and are each independent selected from a linking group;
Rᵢ is selected from the structure represented by Formula (Q-2-4) or Formula (Q-5-2) below: 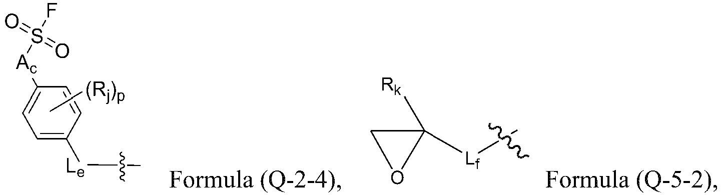
Lₑ, A_{c}, and L_{f} are the same or different, and are each independently selected from a linking group,
p is selected from an integer of 0 to 6 (e.g., 0, 1, 2, 3, 4, 5, 6),
Rⱼ and Rₖ are selected from hydrogen, halogen, -OH, cyano, nitro, amino, C₁₋₂₀ alkyl, C₁₋₂₀ alkoxy, and halogenated C₁₋₂₀ alkyl;
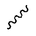 denotes the attachment site of the group.

Preferably, Rᵢ is selected from the structure represented by the following Formula (Q-2-5):

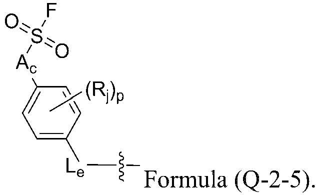

According to an embodiment of the present invention, Z₂, T₂, U₆, U₇, U₈, X₈, Lₑ, L_{f}, A_{c}, Rⱼ, and Rₖ are as defined above for Z, T, U₂, U₃, U₄, X₁, Lₐ, L_{b}, Aₐ, R_{d}, and Rₑ, respectively.

According to an embodiment of the present invention, U₆ is selected from -NH-, -CONH-, and -C₀₋₄ alkyl CONH-.

According to an embodiment of the present invention, U₇ is selected from C₁₋₄ straight or branched alkanetriyl preferably methanetriyl;

According to an embodiment of the present invention, U₈ is selected from -CO-.

According to an embodiment of the present invention, X₈ is selected from #-O-C₁₋₁₀ alkylene-*, preferably #-O-C₁₋₆ alkylene-*, #-O-C₁₋₄ alkylene-*, and #-O-C₁₋₃ alkylene-*, wherein # denotes the attachment site to the quinoline ring, and * denotes the attachment site to the piperazine ring.

According to an embodiment of the present invention, Lₑ is selected from -C₁₋₃ alkylene -, - CO-, -O-, -NH-, and a combination thereof, such as: -C₁₋₃ alkylene-, -C₁₋₃ alkylene-NH-CO-C₁₋₃ alkylene-, -CO-, -CO-C₁₋₃ alkylene-, -CO-C₁₋₃ alkylene-NH-CO-, -CO-C₁₋₃ alkylene-NH-CO-C₁₋₃ alkylene-. According to an embodiment of the present invention, R_{g} is selected from hydrogen, halogen, C₁₋₆ alkyl, and C₁₋₆ alkoxy.

According to an embodiment of the present invention, L_{f} is selected from -C₁₋₄ alkylene-, - CO-, -O-, -NH-, and a combination thereof, such as: -C₁₋₄ alkylene-, -C₁₋₄ alkylene-CO-NH-C₁₋₄ alkylene-, or -C₁₋₄ alkylene-CO-NH-C₁₋₄ alkylene- CO-O-C₁₋₄ alkylene-.

According to an embodiment of the present invention, Lₑ has a length of 1, 2, or 3 carbon atoms.

In some specific embodiments, the structure of Formula (D) is selected from the following compounds:

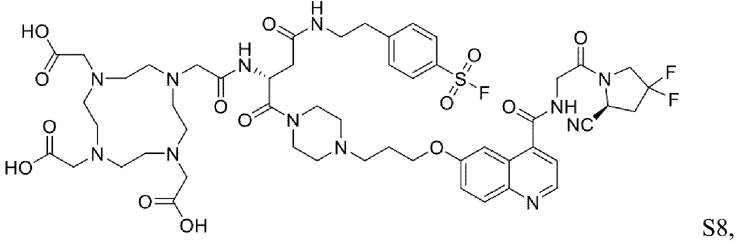

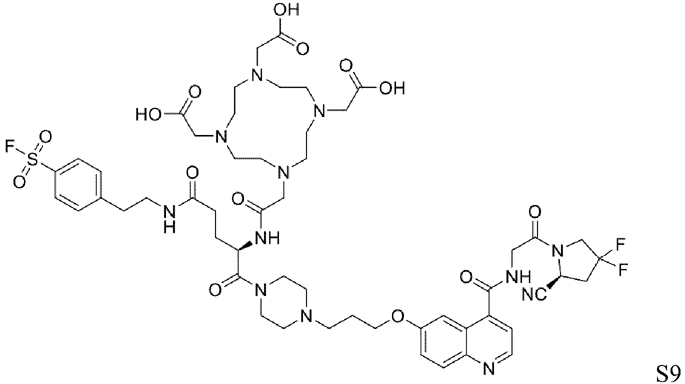

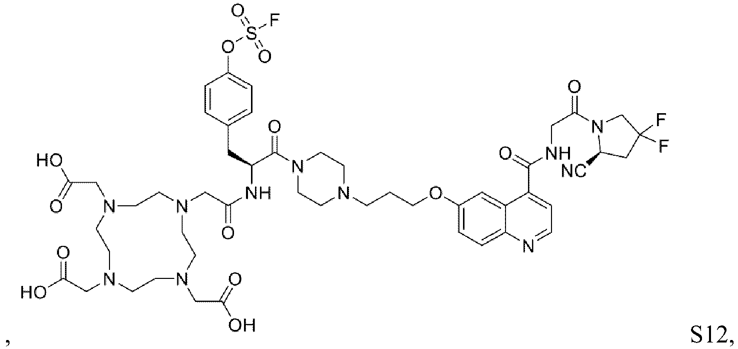

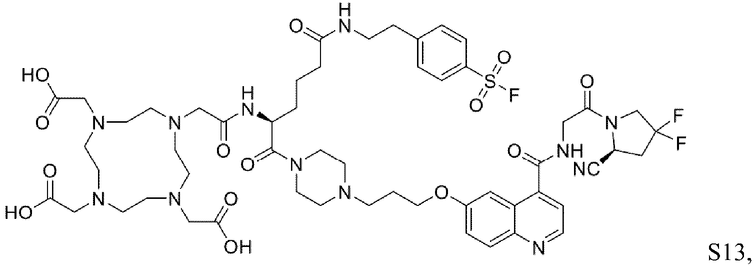

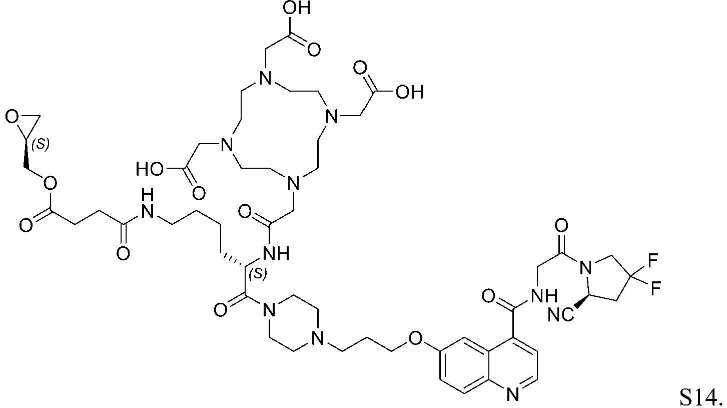

The present invention also provides a compound represented by Formula (A-5), or a stereoisomer, tautomer, isotopically labeled form, pharmaceutically acceptable salt, or prodrug thereof: 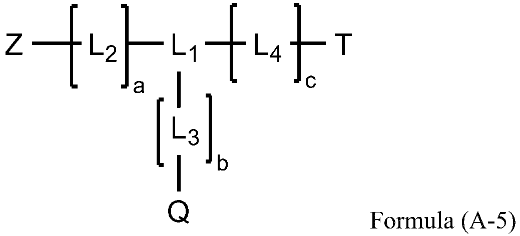 wherein Z is a payload, T is a targeting moiety, Q is selected from the structures represented by Formulas (Q-1), (Q-2), (Q-3), (Q-4), and (Q-5) below: 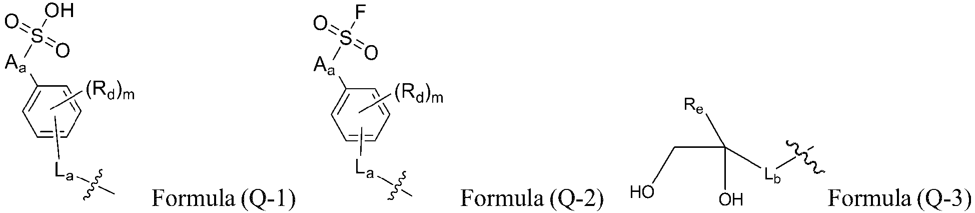 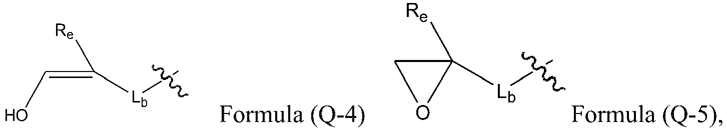
L₁, L₂, L₃, L₄, Aₐ, Lₐ, and L_{b} are the same or different, and are each independently selected from a bond and a linking group,
a, b, c, and m are the same or different, and are each independently selected from an integer of 0 to 6 (e.g., 0, 1, 2, 3, 4, 5, 6),
R_{d} and Rₑ are the same or different, and are each independently selected from hydrogen, halogen, hydroxy, cyano, nitro, amino, C₁₋₂₀ alkyl, C₁₋₂₀ alkoxy, and halogenated C₁₋₂₀ alkyl;
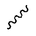 denotes the attachment site of the group;
wherein Lₐ and L_{b} are independently selected from the structure represented by Formula (L-2): 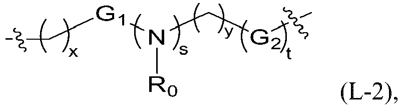
x is selected from 0, 1, and 2; s is 0 or 1; y is selected from 0, 1, 2, 3, and 4; and t is 0 or 1;
G₁ or G₂ is independently -CH₂- or carbonyl, and they are not -CH₂-at the same time;
R₀ is independently selected from hydrogen, halogen, hydroxy, cyano, nitro, amino, C₁₋₁₀ alkyl, C₁₋₁₀ alkoxy, halogenated C₁₋₁₀ alkyl, and halogenated C₁₋₁₀ alkoxy,
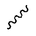 denotes the attachment site of the group.

It is understood that each of the groups in Formula (A-5) are as defined in Formula (A).

According to an embodiment of the present invention, L₁ is selected from a trivalent linking group; preferably, L₁ is selected from N, C₁₋₂₀ alkanetriyl optionally substituted with one or more R₀, and any combination thereof; further preferably, L₁ is selected from N, C₁₋₁₀ alkanetriyl (C₁₋₆ alkanetriyl, C₁₋₄ alkanetriyl), and any combination thereof; and/or
Aₐ is selected from a divalent linking group; preferably, Aₐ is selected from a bond, -O-, -S-, -N(R₀)-, -CO-, C₁₋₂₀ alkylene (C₁₋₁₀ alkylene, C₁₋₆ alkylene, C₁₋₄ alkylene) optionally substituted with one or more R₀, and any combination thereof; preferably, Aₐ is selected from a bond; and/or
L₂, L₃, L₄, and Aₐ are the same or different, and are each independently selected from a divalent linking group; preferably, L₂, L₃, L₄, and Aₐ are each independently selected from a bond, -O-, -S-, -N(R₀)-, -CO-, C₁₋₂₀ alkylene optionally substituted with one or more R₀, 3- to 20-membered heterocyclylene optionally substituted with one or more R₀, and any combination thereof; further preferably, L₂, L₃, L₄, and Aₐ are each independently selected from a bond, -O-, - S-, -N(R₀)-, -CO-, C₁₋₁₀ alkylene (C₁₋₆ alkylene, C₁₋₄ alkylene) optionally substituted with one or more R₀, 3- to 20-membered heterocyclylene (3- to 10-membered heterocyclylene, 3- to 6-membered heterocyclylene) optionally substituted with one or more R₀, and any combination thereof;
preferably, the heterocyclylene contains at least one N; preferably, the heterocyclylene is piperazinylene;
preferably, at least one of L₂ and L₄ comprises 3- to 20-membered heterocyclylene; preferably, at least one of L₂ and L₄ comprises heterocyclylene having one nitrogen atom or two nitrogen atoms; preferably, at least one of L₂ and L₄ comprises piperazinylene.

According to an embodiment of the present invention, the compound has the structure represented by Formula (A-4).

According to an embodiment of the present invention, the compound has the structure represented by Formula (A-6): 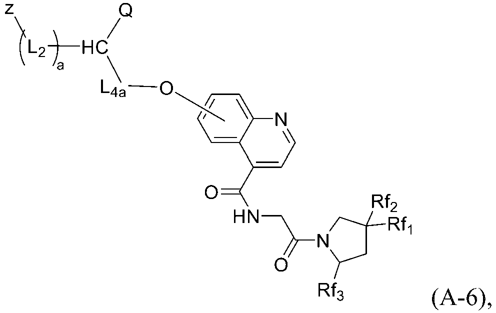
Z is a payload, Q and L₂ are as defined above,
a is an integer of 0 to 6;
Rf₁ and Rf₂ are independently selected from hydrogen and halogen, such as fluorine, chlorine, iodine, bromine, preferably fluorine;
Rf₃ is selected from hydrogen and cyano;
L₄ₐ is selected from a bond, -O-, -S-, -N(R₀)-, -CO-, C₁₋₂₀ alkylene optionally substituted with one or more R₀, 3- to 20-membered heterocyclylene optionally substituted with one or more R₀, and any combination thereof.

In some embodiments, Lₐ and L_{b} are independently selected from: 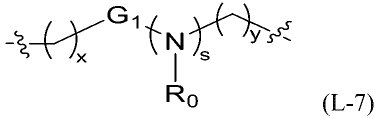
wherein x is selected from 0, 1, and 2; s is 0 or 1; and y is selected from 0, 1, 2, 3, and 4;
G₁ is -CH₂- or carbonyl.

In some embodiments, Lₐ and L_{b} are independently selected from -CH₂-, and the group shown below: 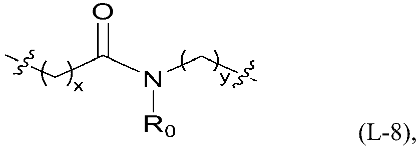 wherein x is selected from 0, 1, and 2, and y is selected from 0, 1, 2, 3, and 4.

In some embodiments, L₄ₐ comprises the structure represented by Formula (L-9) below: 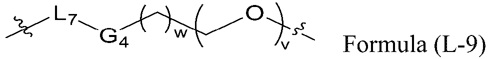
wherein w is an integer of 1 to 20, and v is an integer of 0 to 10;
G₄ is as defined for G₃ above;
L₇ is carbonyl or C₁₋₁₀ alkylene group.

In some embodiments, L₂ comprises -N(R₀)- or the structure represented by Formula (L-1): 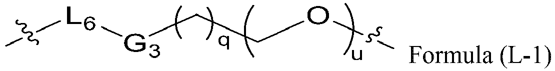
wherein q is an integer of 1 to 20, and u is an integer of 0 to 10;
G₃ is as defined above, L₆ is a bond or -CO-C₁₋₃ alkylene-NH-.

The present invention also provides a FAP-targeted nuclide probe, wherein the nuclide probe is the compound represented by Formulas (A), (B), (D), (A-1), (A-2), (A-3), (A-4), (A-5), or (A-6) above, or derivative thereof labeled with a radionuclide.

According to an embodiment of the present invention, the radionuclide is a diagnostic radionuclide or a therapeutic radionuclide. According to an embodiment of the present invention, the diagnostic radionuclide is at least one of ⁶⁸Ga, ⁶⁷Ga, ⁶⁴Cu, ¹⁸F, ⁸⁶Y, ⁸⁹Zr, ¹¹¹In, ^{99m}Tc, ¹¹C, ²⁰³Pb, ¹²³I, ¹²⁵I, and ¹²⁴I. According to an embodiment of the present invention, the therapeutic radionuclide is at least one of ¹⁷⁷Lu, ⁹⁰Y, ¹²⁵I, ¹³¹I, ²¹¹At, ¹⁵³Sm, ¹⁸⁶Re, ¹⁸⁸Re, ⁶⁷Cu, ²²⁵Ac, ²²⁷Th, ²²³Ra, ²¹³Bi, ²¹²Bi, and ²¹²Pb.

According to an embodiment of the present invention, when the radioisotope is a non-metal ion, it may be chelated by forming a complex with a metal ion such as aluminum, for example, Al¹⁸F.

The present invention further provides a method for preparing the FAP-targeted nuclide probe described above, comprising:
1) providing the compound represented by Formula (A), (B), or (D) above; and
2) chelating the compound with said radionuclide in solution or on a column.

The present invention further provides a preparation kit of the FAP-targeted nuclide probe, comprising:
1) the compound represented by Formula (A), (B), or (D); and
2) the radionuclide or a reagent providing said radionuclide.

The present invention further provides use of the compound represented by Formula (A), (B), or (D) in the preparation of a FAP Inhibitor.

The present invention further provides a pharmaceutical composition, comprising the compound represented by Formula (A), (B), or (D); and at least one pharmaceutically acceptable excipient or carrier.

The present invention further provides use of the above compound, nuclide probe, FAP inhibitor, or pharmaceutical composition in the preparation of a FAP-targeted imaging agent and/or therapeutic agent.

The present invention further provides use of the above compound, nuclide probe, FAP inhibitor, or pharmaceutical composition in the preparation of a medicament for diagnosing and/or treating a disease, wherein the disease is a disease characterized by overexpression of fibroblast activation protein (FAP).

The present invention further provides a method for diagnosing and/or treating a disease, the method comprising administering a diagnostically and/or therapeutically effective amount of the above compound, nuclide probe, FAP inhibitor, or pharmaceutical composition to a subject to be diagnosed and/or treated, wherein the disease is a disease characterized by overexpression of fibroblast activation protein (FAP).

The present invention further provides a kit, comprising the above compound, nuclide probe, FAP inhibitor, or pharmaceutical composition; and instructions for diagnosing and/or treating a disease.

According to an embodiment of the present invention, the disease characterized by overexpression of fibroblast activation protein (FAP) is selected from cancer, chronic inflammation, atherosclerosis, fibrosis (e.g., pulmonary fibrosis), tissue remodeling, scarring disease, rheumatoid arthritis, osteoarthritis, liver cirrhosis, and liver disease.

According to an embodiment of the present invention, the cancer is selected from breast cancer, pancreatic cancer, small intestine cancer, colon cancer, rectal cancer, lung cancer, head and neck cancer, ovarian cancer, hepatocellular carcinoma, esophageal cancer, hypopharyngeal cancer, nasopharyngeal cancer, laryngeal cancer, myeloma cell, bladder cancer, cholangiocarcinoma, clear cell renal carcinoma, neuroendocrine tumor, carcinogenic osteomalacia, sarcoma, CUP (cancer of unknown primary), thymic cancer, glioma, neuroglioma, astrocytoma, cervical cancer, and prostate cancer.

The present invention also provides the use of a compound represented by Formula (A-0) in the preparation of the compound represented by Formula (A) wherein Q is Formula (Q-1), Formula (Q-3), or Formula (Q-4), 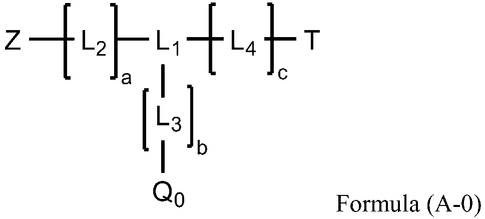 wherein Q₀ is selected from the structure represented by Formula (Q-2) or (Q-5) below: 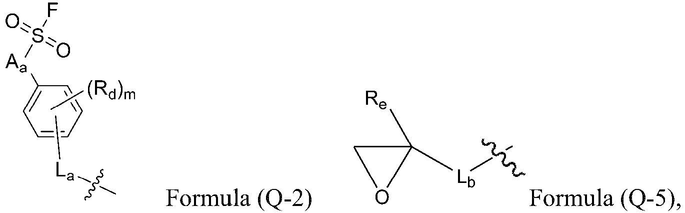 in Formula (A-0), Z, T, L₁, L₂, L₃, L₄, Aₐ, Lₐ, a, b, c, m, R_{d}, and Rₑ are as defined in Formula (A) above.

The present invention further provides some novel compounds, comprising the structure represented by Formula (M-1): 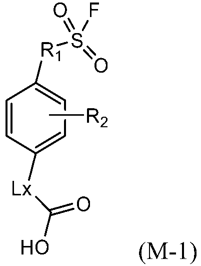
wherein R₁ is selected from a bond and O;
R₂ is selected from hydrogen, C₁₋₁₀ alkyl, and C₁₋₁₀ alkoxy;
Lx comprises the structure represented by Formula (L-X): 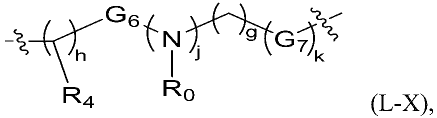
wherein h is selected from 0, 1, and 2; j is 0 or 1; g is selected from 0, 1, 2, 3, and 4; and k is 0, 1, or 2;
G₆ or G₇ is independently -CH₂- or carbonyl, and they are not -CH₂- at the same time;
R₄ is H, -NH₂ or amino protected by a protecting group.

According to an embodiment of the present invention, the compound of Formula (M-1) is selected from the compound represented by Formula (M-2) or Formula (M-3): 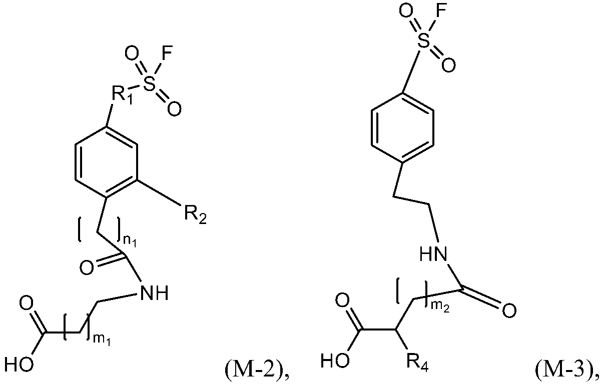
wherein R₂ is hydrogen or methoxy,
R₁ is a bond or O,
n₁ is an integer of 0 to 2 (e.g., 0, 1, 2),
m₁ is an integer of 1 to 3 (e.g., 1, 2, 3),
m₂ is an integer of 1 to 3 (e.g., 1, 2, 3).

According to an embodiment of the present invention, the compound represented by Formula (M-1) is selected from any one of the compounds shown below:

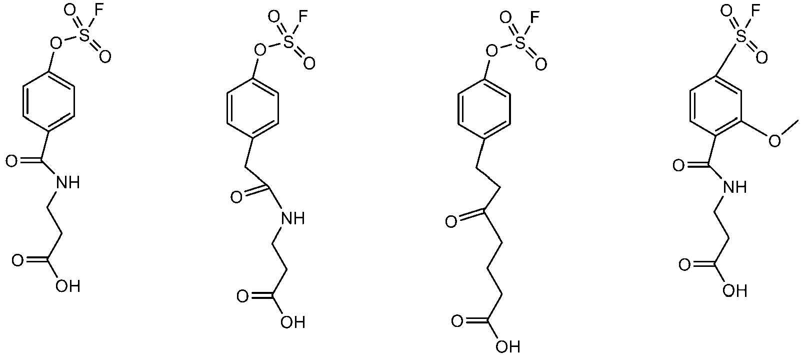

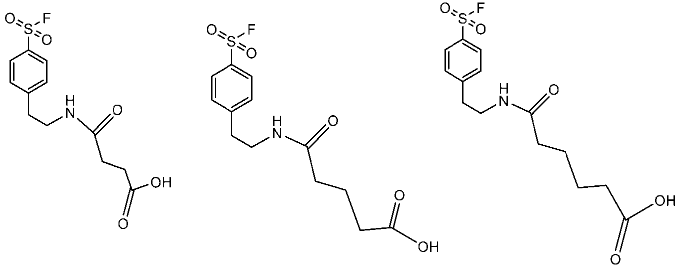

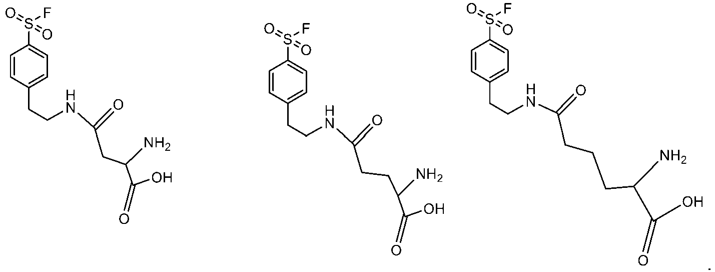

The present invention also provides the use of the compound represented by the above Formula (M-1) in the preparation of a radiopharmaceutical.

In an embodiment of the present invention, the radiopharmaceutical refers to a nuclide-conjugated drug, such as a nuclide-conjugated small molecule compound drug, a nuclide-conjugated polypeptide drug, a nuclide-conjugated antibody drug, or a nuclide-conjugated aptamer drug. The compound of Formula (M-1) above reacts with a compound containing a targeting moiety to produce a covalent warhead-substituted conjugated compound.

In an embodiment of the present invention, the compound represented by Formula (M-1) is used to prepare compounds provided in sections such as the first and second aspects of this application.

In an embodiment of the present invention, the radiopharmaceutical comprises the compound described above and is a drug having diagnostic and/or therapeutic effects formed by radiolabeling compound A.

In the description of this specification, reference to terms such as "an embodiment", "some embodiments", "example", "specific example" or "some examples" means that specific features, structures, materials, or characteristics described in connection with the embodiment or example is included in at least one embodiment or example of the present application. In this specification, the schematic representations of the above terms do not necessarily refer to the same embodiment or example. Furthermore, the described specific features, structures, materials, or characteristics may be combined in any suitable manner in any one or more embodiments or examples. Additionally, those skilled in the art may incorporate and combine different embodiments or examples and features of different embodiments or examples described in this specification without conflicting with each other.

Although the embodiments of the present invention have been shown and described above, it is understood that the above embodiments are exemplary and are not to be construed as limiting the present invention. Those skilled in the art may change, modify, substitute, and vary the above embodiments within the scope of the present invention.

### Detailed Description of the Embodiments

The technical solution of the present invention will be further described in detail below with reference to specific examples. It should be understood that the following examples are only illustrative and explanatory of the present invention and should not be construed as limiting the protection scope of the present invention. All technologies implemented based on the above content of the present invention are covered within the protection scope intended by the present invention.

Unless otherwise specified, the raw materials and reagents used in the following examples are commercially available or can be prepared by known methods.

### I. Instruments and Materials

### 1 Experimental Materials

### 1.1 Compound Synthesis and Characterization Materials

| Materials | Manufacturer | Grade |
|---|---|---|
| Chromatography-Grade Acetonitrile | Shanghai CINC High-Purity Solvents Co., Ltd. | HPLC Grade |
| Chromatography-grade methanol | Shanghai CINC High-Purity Solvents Co., Ltd. | HPLC Grade |
| Organic Solvents for Synthesis (Petroleum Ether, Ethyl Acetate, Methanol, Ethanol, Dichloromethane, DMF, Tetrahydrofuran, Dioxane, etc.) | Shenzhen Donggangtian Chemical Co., Ltd. | AR Grade |
| Raw materials and reagents for synthesis | Shenzhen Dieckmann Technology Development Co., Ltd. | NA |
| | Shanghai Haohong Biomedical Technology Co., Ltd. | |
| | Anhui Zesheng Technology Co., Ltd. | |
| | Shanghai Titan Technology Co., Ltd. | |
| | WuXi Lianbo (Wuhan) Chemical Technology Co., Ltd. | |
| | Jiangsu Aikang Biopharmaceutical R&D Co., Ltd. | |
| | Shanghai Bide Pharmaceutical Technology Co., Ltd. | |

### 1.2 Materials for Enzyme Inhibition Assay

(1) Fibroblast activation protein (i.e., FAP, source: MCE, HY-P72659)
(2) Fibroblast Activation Protein Substrate (Z-Gly-Pro-7-AMC, Source: MCE, HY-D0027)
(3) Fibroblast Activation Buffer (i.e., FAB; 50 mM Tris, 100 mM NaCl, 1 mM EDTA, pH = 7.4)

### 1.3 Materials for Small Animal Imaging Experiments

(1) Experimental Animals
   Species/Strain: BALB/c Nude mice
   Sex/Quantity: Female, 30
   Body weight/Age: 4-6 weeks, 15-18 g
   Grade: SPF
(2) Tumor Cell Lines

FAP-positive cell line U87MG: astrocytic glioblastoma cells of the human brain, culture medium condition: DMEM medium containing 10% fetal bovine serum and 1% penicillin-streptomycin. After cell expansion, the cells were inoculated into the right lower limb near the back of nude mice, at an injection dose of 1×10⁶ cells per mouse. The mice were ready for imaging experiments 2-3 weeks after tumor cell inoculation.

FAP-positive cell line HT1080: human fibrosarcoma cell line, culture medium condition: DMEM medium containing 10% fetal bovine serum and 1% penicillin-streptomycin. After cell expansion, the cells were inoculated into the right lower limb near the back of nude mice, at an injection dose of 1×10⁶ cells per mouse. The mice were ready for imaging experiments 2-3 weeks after tumor cell inoculation.

### 2. Major Instruments

### 2.1 Compound Synthesis and Characterization

| Instruments | Manufacturer | Model |
|---|---|---|
| High-Performance Liquid Chromatograph | Agilent | Agilent 1200 series |
| High-Performance Liquid Chromatography-Tandem Mass Spectrometry | Waters | LC: Waters 2695 |
| | | DAD: Waters 2487 |
| | | MS: Micromass Quattro Micro |
| Semi-preparative HPLC purifier | Welch | Welch |
| NMR Spectrometer | Bruker | Avance III 400 |
| High-resolution mass spectrometer | Bruker | Bruker maXis II QTOF Mass Spectrometer |
| High-resolution mass spectrometer | Thermo | Q Exactive Hybrid Quadrupole-Orbitrap Mass Spectrometer |

### 2.2 Small Animal Imaging Experiments

| Name | Purpose | Model | No. |
|---|---|---|---|
| ⁶⁸Ge/⁶⁸Ga generator | Production of nuclide | Isotope Technologies Garching GmbH, Germany | 002 |
| High-Performance Liquid Chromatograph | Purity testing | SHIMADZU, Japan | - |
| InliView-3000B Animal PET/SPECT/CT Preclinical Molecular Imaging System | Small Animal Imaging | InliView-3000B, China | B3-DSW-5005 |

### 2.3 Compound Characterization Methods and Chromatographic Conditions

HRMS characterization method: Dissolve an appropriate amount of sample in 0.1% formic acid aqueous solution to prepare a sample solution with a concentration at ppm level, then inject the sample, analyze, and process the experimental data.

NMR characterization method: Weigh 3 to 5 mg of the sample, dissolve in 500 µL of deuterated solvent (D₂O, DMSO-d₆, or CD₃OD), detect, and analyze experimental results.

### Chromatographic conditions:

### Chromatographic Conditions (HPLC)

| Chemical Purity Determination | |
|---|---|
| Chromatographic Column | SHIMADZU Shim-Pack GIST, 5 µm, C18, 4.6 × 250 mm |
| Mobile phase | A: Purified water containing 0.1% TFA |
| | B: Acetonitrile containing 0.1% TFA |
| Elution Program | 0-15 min, 10-60% B; |
| | 15-17 min, 60-95% B; |
| | 17-20 min, 95%-10% B |
| | Flow rate: 0.6 mL/min |

### Prep HPLC Conditions (Semi-Prep HPLC)

| Semi-preparative HPLC purification | |
|---|---|
| Instrumentation | Welch |
| Column Model | Welch Ultimate XB-C18 30×250 mm, 10 µm |
| Mobile Phase | A: Purified water containing 0.1% TFA |
| | B: Acetonitrile containing 0.1% TFA |
| Conditions | 0-15 min, 0%B; |
| | 15-50 min, 0-30%B; |
| | 50-55 min, 30-95%B; |
| | 55-65 min, 95% B |
| | Flow rate: 15 mL/min |

### LCMS:

| | |
|---|---|
| Column | Synergi 4 µm Fusion-RP 80 Å, LC column 50 × 2 mm |
| Mobile phase | A: Purified water containing 0.1% TFA |
| | B: Acetonitrile containing 0.1% TFA |
| Elution Program | 5-95% B 4 min |
| | 0-3 min, 5-95% B; |
| | 3-4 min, 95% B |
| | Flow rate: 0.6 mL/min |

### II. Preparation Example

### Exemplary synthetic routes are shown below:

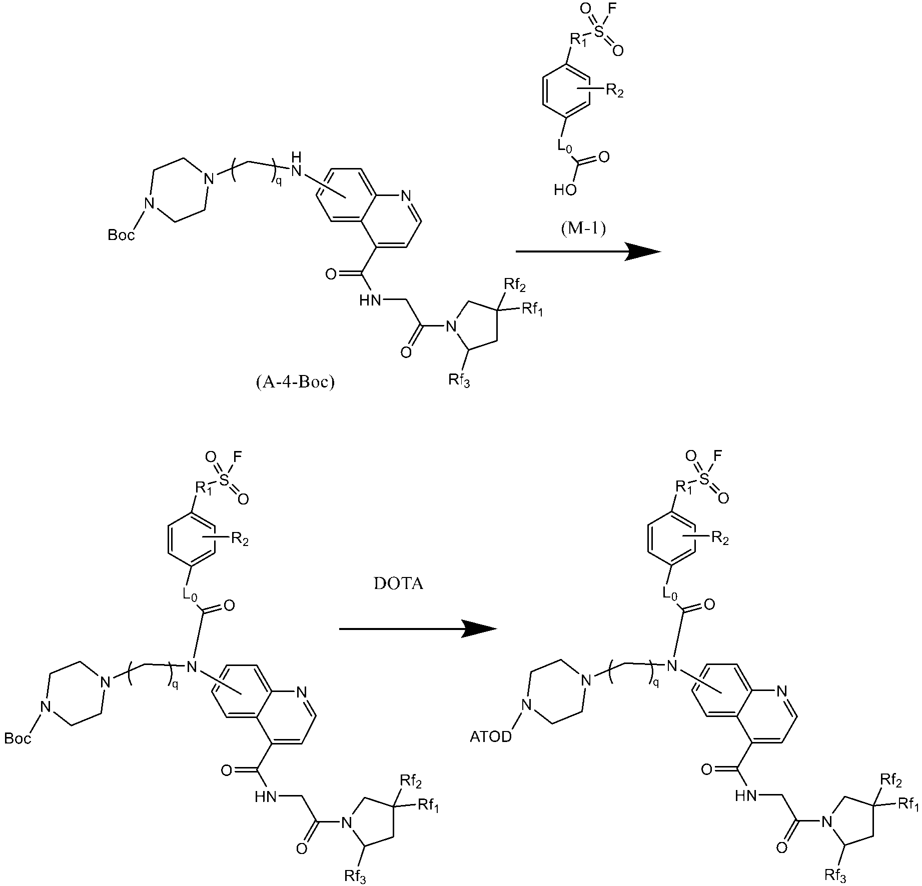

### 1. Preparation Example 1: Synthesis Method and Synthesis Route of compound S1

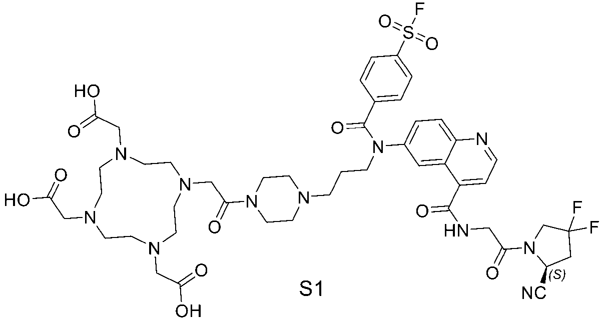

The specific synthetic route for S1 is as follows:

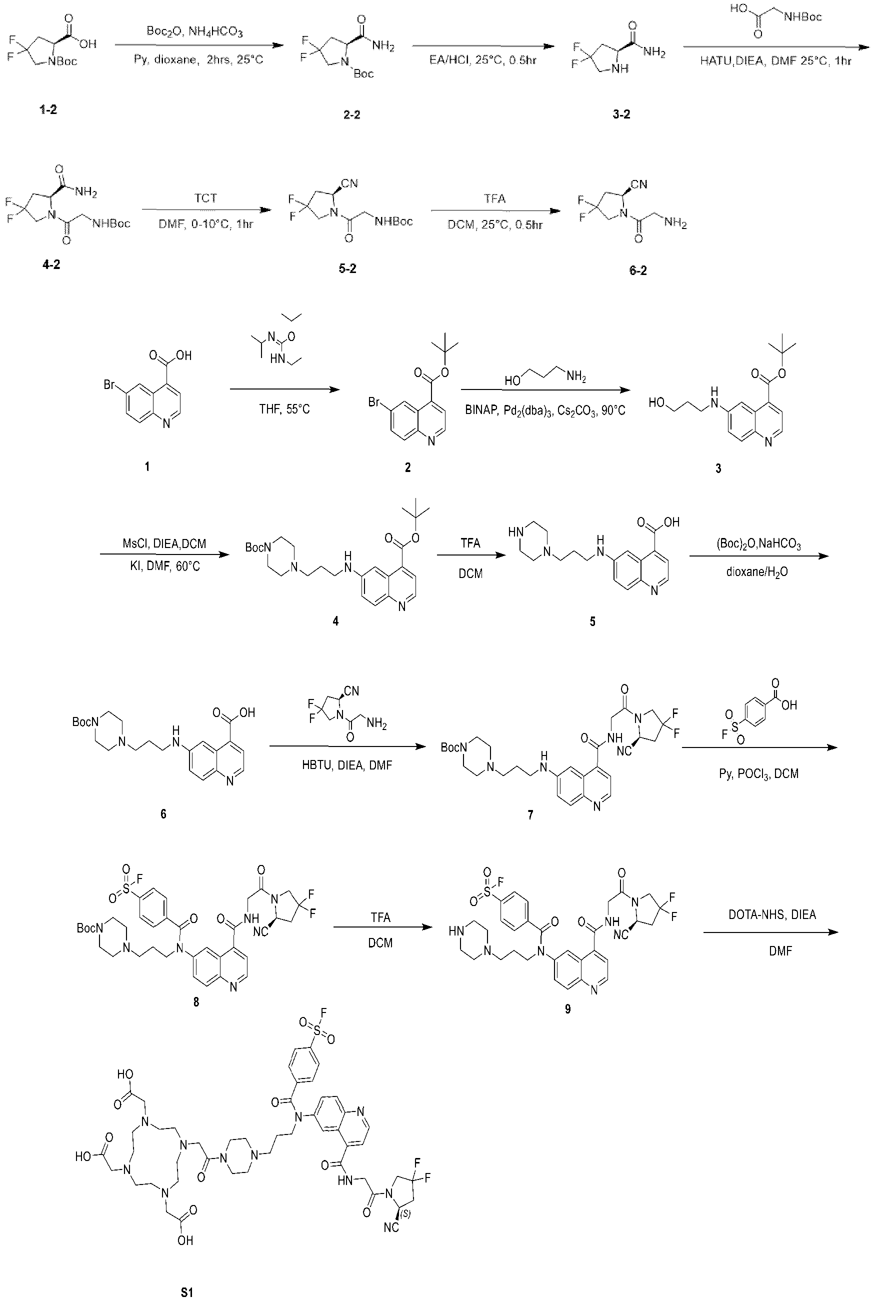

### 1.1 Preparation of Compound 6-2

### Step 1: Synthesis of Compound 2-2

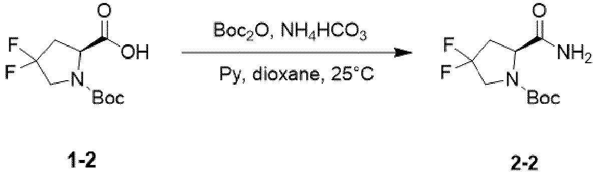

At room temperature (25°C), Compound **1-2** (10.0 g, 39.8 mmol) was dissolved in a solution of dioxane (50.0 mL), then pyridine (9.5 g, 120.2 mmol), NH₄HCO₃ (6.0 g, 71.5 mmol) and Boc₂O (15.6 g, 71.5 mmol) were added. The resulting reaction solution was stirred at room temperature for 2 hours. After completion of the reaction, the reaction solution was diluted with ethyl acetate (250 mL) and washed with 1 M HCl (100 mL × 2) and saturated brine (100 mL × 2), and the organic phase was dried over anhydrous sodium sulfate and concentrated under reduced pressure. The resulting residue was purified by silica gel column chromatography (eluent: petroleum ether/ethyl acetate = 10/1) to afford Compound **2-2** as a white solid (10.0 g, 100% yield).

### Step 2: Synthesis of Compound 3-2

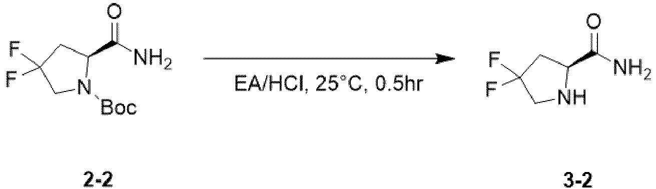

At room temperature, Compound **2-2** (10.0 g, 39.9 mmol) was dissolved in a solution of HCl/ethyl acetate (100 mL, 4.0 M). The resulting reaction solution was stirred at room temperature for 0.5 hours. After completion of the reaction, the reaction solution was concentrated under reduced pressure to afford Compound **3-2** as a white solid (7.5 g, 100% yield), which was used directly in the next step without further purification.

### Step 3: Synthesis of compound 4-2

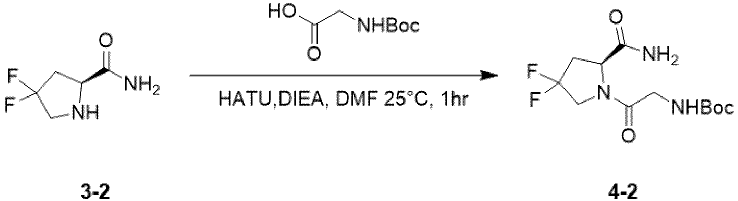

At room temperature, Compound **3-2** (7.5 g, 49.9 mmol) and Boc-Glycine (10.5 g, 59.9 mmol) were dissolved in anhydrous DMF (10.0 mL), then DIEA (N,N-diisopropylethylamine) (19.3 g, 1496 mmol) and HATU (2-(7-azabenzotriazole)-N,N,N',N'-tetramethyluronium hexafluorophosphate) (22.8 g, 59.9 mmol) were added. The reaction solution was stirred at room temperature for 1 hour. After completion of the reaction, the reaction solution was diluted with ethyl acetate (250 mL), washed with 1M HCl (100 mL × 2) and saturated brine (100 mL × 2) respectively, and dried over anhydrous sodium sulfate. The organic phase was concentrated and the resulting residue was purified by silica gel column chromatography (eluent: petroleum ether/ethyl acetate = 1/1) to give Compound **4-2** as a white solid (8.6 g, 70.4% yield).

### Step 4: Synthesis of Compound 5-2

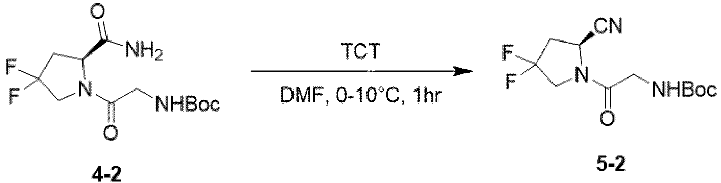

At 0°C, Compound **4-2** (4.8 g, 15.6 mmol) was dissolved in anhydrous DMF (20 mL), then cyanuric chloride (TCT) (2.0 g, 10.8 mmol) was added. The resulting reaction solution was stirred at 0 to 10°C for 1 hour. After completion of the reaction, the reaction solution was diluted with ethyl acetate (150 mL), washed with saturated NaHCO₃ aqueous solution (50 mL × 2) and saturated brine (50 mL × 2) respectively, and dried over anhydrous sodium sulfate. The organic phase was concentrated and dried, and the resulting residue was purified by silica gel column chromatography (eluent: petroleum ether/ethyl acetate = 1/1) to give Compound **5-2** as a white solid (4.2 g, 93.1% yield).

### Step 5: Synthesis of compound 6-2

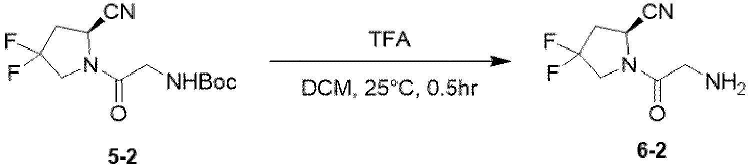

At room temperature, Compound **5-2** (1.0 g, 3.4 mmol) was dissolved in anhydrous dichloromethane (DCM) (5.0 mL), and trifluoroacetic acid (TFA) (1.0 mL) was added. The resulting reaction solution was stirred at room temperature for 0.5 hours. After completion of the reaction, the reaction solution was diluted with dichloromethane (25 mL) and concentrated under reduced pressure to yield Compound **6-2** as a colorless oil (653.9 mg, 100% yield), which was used directly in the next step without further purification.

### 1.2 Preparation of Compound S1

### Step 6: Synthesis of Compound 2

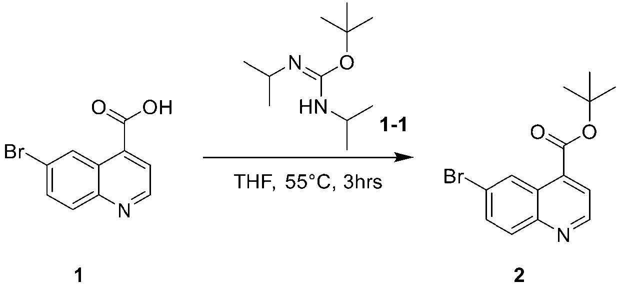

At room temperature, Compound **1** (5.0 g, 19.8 mmol) and Compound **1-1** (19.8 g, 98.8 mmol) were dissolved in anhydrous tetrahydrofuran (100 mL) and reacted for 3 hours at 55°C. After completion of the reaction, the reaction solution was diluted by adding ethyl acetate (150 mL), washed with water (50 mL ×2) and saturated brine (50 mL ×1) respectively, then dried over anhydrous sodium sulfate, and finally concentrated under reduced pressure. The resulting residue was purified by silica gel column chromatography (elution: petroleum ether/ethyl acetate = 10/1) to give Compound **2** (4.8 g, 78.5% yield). LCMS: [M+H-C(CH₃)₃]⁺= 254.2/256.2.

### Step 7: Synthesis of Compound 3

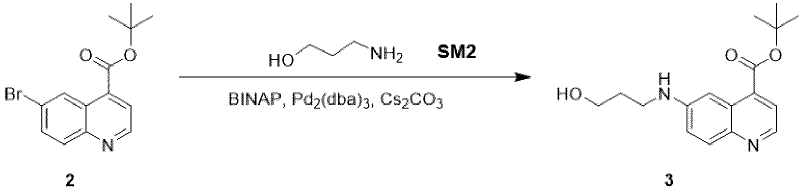

At room temperature, Compound **2** (3.7 g, 12.0 mmol), Compound SM2 (3.14 g, 41.8 mmol), cesium carbonate (22.0 g, 67.5 mmol), BINAP (1,1'-binaphthyl-2,2'-bis(diphenylphosphine)) (1.5 g, 3.1 mmol) were dissolved in anhydrous toluene (100 mL). The reaction solution was purged three times with a nitrogen balloon, and then Pd₂(dba)₃ (883.0 mg, 2.4 mmol) was added, followed by three additional purges with a nitrogen balloon. The reaction solution was then reacted overnight at 90°C for 12 hours. After the reaction was complete, the reaction solution was diluted by adding 100 mL of ethyl acetate, then washed with water (30 mL × 2) and saturated brine (50 mL × 1) respectively, then dried over anhydrous sodium sulfate, and finally concentrated under reduced pressure. The resulting residue was purified by silica gel column chromatography (elution: dichloromethane/methanol = 30/1) to give Compound **3** as a brownish-red solid (2.1 g, 57.8% yield). LCMS: [M+H- C(CH₃)₃]⁺ = 247.1.

### Step 8: Synthesis of Compound 4

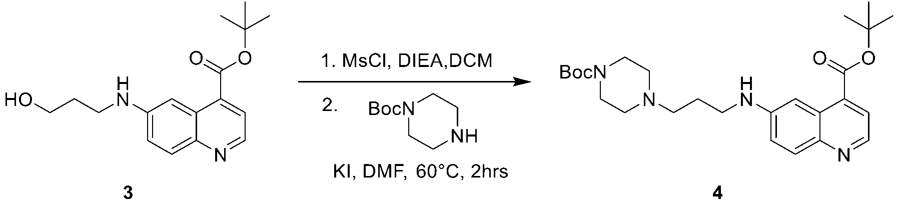

At room temperature, Compound **3** (1.5 g, 4.9 mmol) and DIEA (3.2 g, 24.8 mmol) were dissolved in anhydrous dichloromethane (20.0 mL), and MsCl (910.0 mg, 7.9 mmol) was added dropwise. The reaction solution was stirred at room temperature for 0.5 hours. After the reaction was complete, the solvent was removed by rotary evaporation. Then, DMF (20.0 mL) and N*-tert-*butoxycarbonylpiperazine (3.3 g, 17.7 mmol) were added to the reaction flask. After stirring and heating to 50°C, KI (12.0 g, 72.2 mmol) was added, followed by heating to 60°C for 2hours of reaction. After the reaction was complete, the reaction solution was diluted by adding ethyl acetate (100 mL), washed with saline (30 mL × 2), then dried over anhydrous sodium sulfate, and finally concentrated under reduced pressure. The resulting residue was purified by silica gel column chromatography (elution: petroleum ether/ethyl acetate = 1/2) to give Compound **4** (1.9 g, 81.5% yield) as a yellow solid. LCMS: [M+H]⁺ = 471.3.

### Step 9: Synthesis of Compound 5

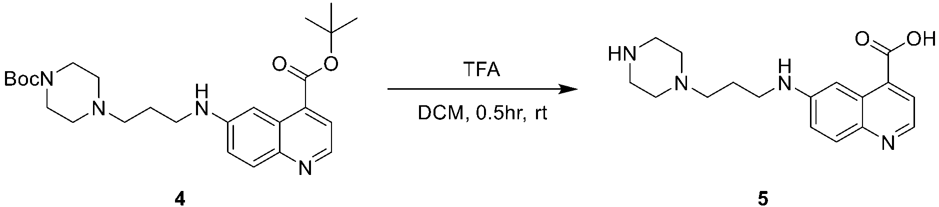

At room temperature, Compound **4** (2.0 g, 4.0 mmol) was dissolved in anhydrous dichloromethane (2.0 mL), followed by addition of trifluoroacetic acid (8.0 mL). The resulting reaction solution was stirred at room temperature for 0.5 hours. After the reaction was complete, the reaction solution was diluted with dichloromethane (25.0 mL), and the solvent was removed by rotary evaporation to give Compound **5** as a brown oil (1.3 g, 100% yield), which was used directly in the next step without further purification. LCMS: [M+H]⁺= 315.3.

### Step 10: Synthesis of Compound 6

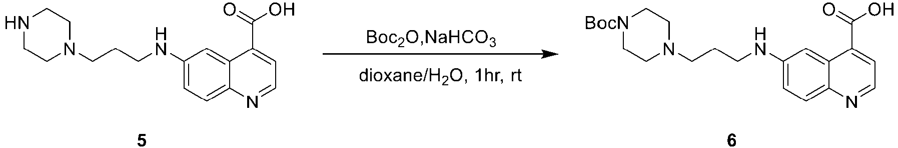

At room temperature, Compound **5** (1.3 g, 4.1 mmol) was dissolved in dioxane/water (1:1, v/v, 20 mL), followed by addition of sodium bicarbonate (1.04 g, 12.3 mmol) and Boc₂O (1.8 g, 8.2 mmol). The resulting reaction solution was stirred at room temperature for 1 hour. After completion of the reaction, a yellow solid precipitated from the reaction solution. The solution was filtered to obtain the yellow solid, which was washed with water. The aqueous phase was passed through a high-performance liquid chromatography column (0.1% acetic acid in water, acetonitrile), and the resulting fraction was lyophilized to give Compound **6** as a yellow solid (1.5 g, 87.8% yield). LCMS: [M+H]⁺= 415.3.

### Step 11: Synthesis of Compound 7

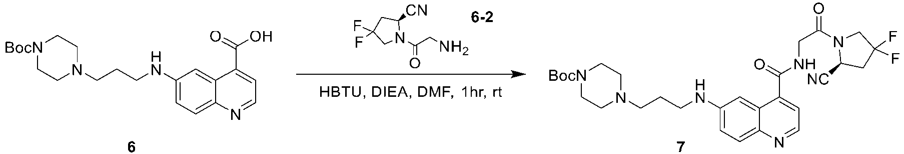

At room temperature, Compound **6** (880.0 mg, 2.1 mmol), Compound **6-2** (582.1 mg, 3.0 mmol), and DIEA (1.37 g, 10.6 mmol) were dissolved in anhydrous DMF (10.0 mL), followed by addition of HBTU (O-benzotriazole-tetramethyluronium hexafluorophosphate) (1.0 g, 2.6 mmol). The resulting reaction solution was stirred at room temperature for 1 hour. After the reaction was complete, the reaction solution was diluted with ethyl acetate (100 mL), washed with saturated brine (50 mL × 2 times), and the organic phase was rotary evaporated to dryness. The resulting residue was purified by silica gel column chromatography (eluent: dichloromethane/methanol = 10/1) to afford Compound **7** (700.0 mg, 56.4% yield). LCMS: [M+H]⁺ = 587.6.

### Step 12: Synthesis of Compound 8

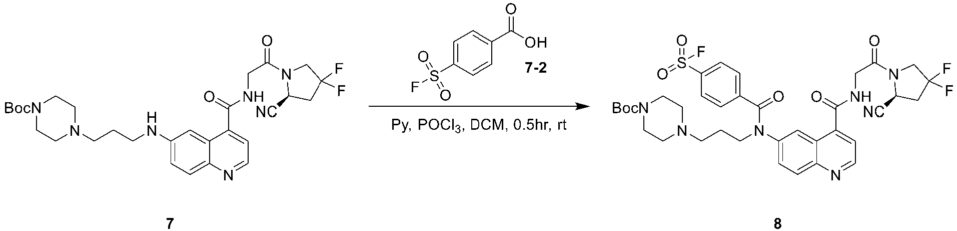

At room temperature, Compound **7** (150.0 mg, 0.19 mmol) and Compound **7-2** (59.4 mg, 0.29 mmol) were dissolved in anhydrous dichloromethane (10 mL), and pyridine (767.6 mg, 9.7 mmol) and POCl₃(148.6 mg, 0.97 mmol) were added. The resulting reaction solution was stirred at room temperature for 0.5 hours. After the reaction was complete, the reaction solution was diluted with dichloromethane (50 mL), washed with water (15 mL × 2) and saturated brine (15 mL × 2), respectively, and the organic phase was rotary evaporated to dryness. The resulting residue was purified by semi-preparative high-performance liquid chromatography, and the resulting fraction was lyophilized to give Compound **8** as a yellow solid powder (80.0 mg, 40.5% yield). LCMS: [M+H]⁺= 773.2.

### Step 13: Synthesis of Compound 9

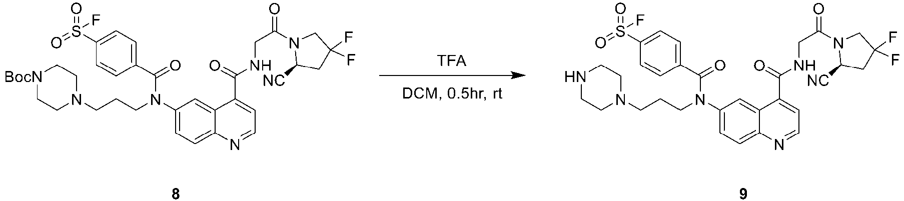

At room temperature, compound **8** (60.0 mg, 3.2 mmol) was dissolved in anhydrous dichloromethane (5.0 mL), followed by addition of trifluoroacetic acid (1.0 mL), and the resulting reaction solution was stirred at room temperature for 0.5 hours. After the reaction was complete, the reaction solution was diluted with dichloromethane (25 mL), and the solvent was removed by rotary evaporation, to yield Compound **9** as a reddish-brown oil (52.2 mg, 100% yield). The compound was used directly in the next step without further purification. LCMS: [M+H]⁺= 673.2.

### Step 14: Synthesis of Compound S1

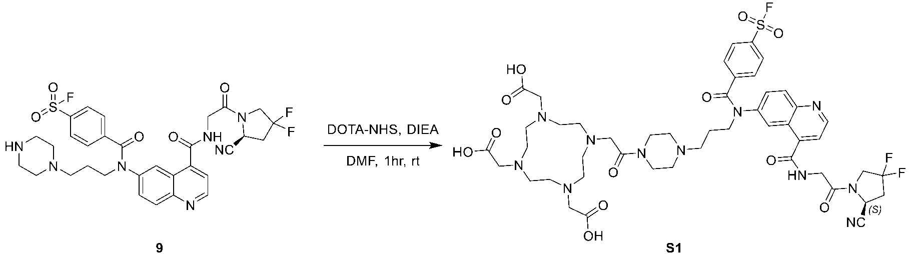

At room temperature, Compound **9** (80.0 mg, 119.0 µmol), DOTA-NHS (CAS No. 170908) (120.0 mg, 239.5 µmol) in anhydrous DMF (3.0 mL), followed by addition of DIEA (46.0 mg, 356.5 µmol). The resulting reaction solution was stirred at room temperature for 1 hour. After the reaction was complete, the reaction solution was subjected to rotatory evaporation to dryness, and the residue was separated and purified by semi-preparative high-performance liquid chromatography (0.1% acetic acid in water, acetonitrile), to yield Compound **S1** (29.1 mg, 96.3% purity, 26.5% yield). LCMS: [M+H]⁺= 1059.2.

The ¹H NMR data for compound **S1** are as follows: ¹H NMR (400 MHz, Deuterium Oxide) δ 8.92 (d, *J* = 4.3 Hz, 1H), 8.22 - 8.03 (m, 1H), 7.94 (s, 1H), 7.87 (t, *J* = 9.5 Hz, 3H), 7.69 - 7.60 (m, 3H), 7.58 - 7.40 (m, 3H), 5.28 - 5.19 (m, 1H), 4.38 - 4.10 (m, 6H), 3.89 - 3.68 (m, 6H), 3.62 - 3.46 (m, 6H), 3.45 - 3.28 (m, 10H), 3.22 - 2.93 (m, 12H), 2.24 - 1.99 (m, 3H).

Meanwhile, referring to step 5 of the synthesis of Compound S2, Compound S25 was further prepared from compound S1:

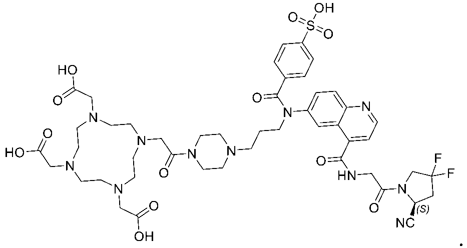

### 2. Preparation Example 2: Synthesis Method and Synthesis Route of Compound S3

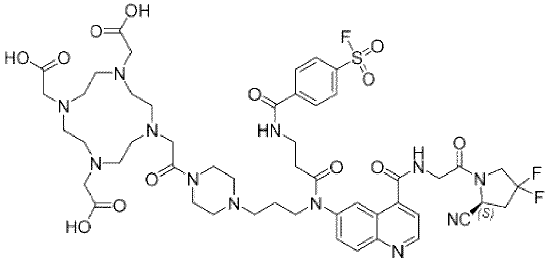

### Detailed synthetic route for Compound S3:

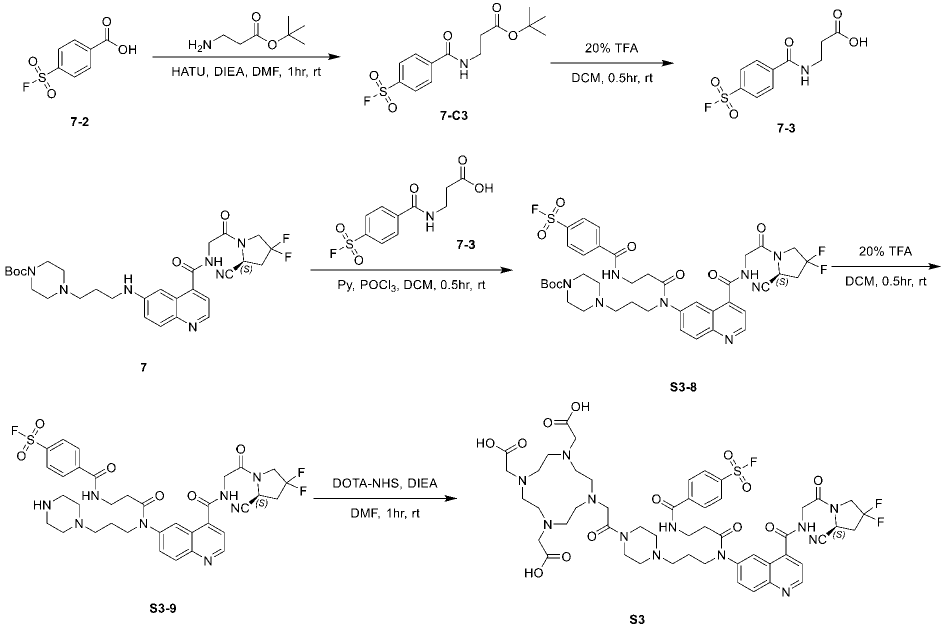

### Step 1: Synthesis of Compound 7-C3

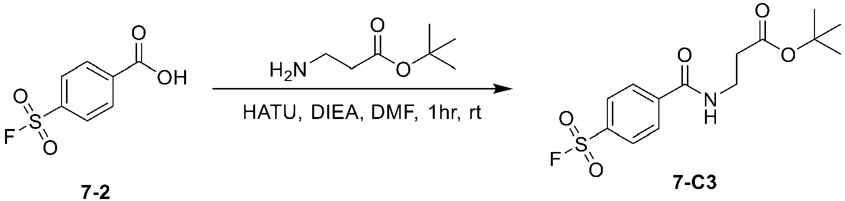

At room temperature, Compound **7-2** (250 mg, 122.4 µmol) and tert-butyl 3-aminopropionate (213.4 mg, 146.9 µmol) were dissolved in anhydrous DMF (10.0 mL), followed by addition of DIEA (474.0 mg, 367.4 µmol) and HATU (558.5 mg, 146.9 µmol). The resulting reaction solution was stirred at room temperature for 1 hour. After the reaction was complete, the reaction solution was diluted with ethyl acetate (100 mL), washed with saturated brine (50 mL × 2), and the organic phase was rotary evaporated to dryness. The resulting residue was purified by silica gel column chromatography (eluent: petroleum ether/ethyl acetate = 2/1) to give Compound **7-C3** as a white solid (355.2 mg, 87.5% yield).

### Step 2: Synthesis of Compound 7-3

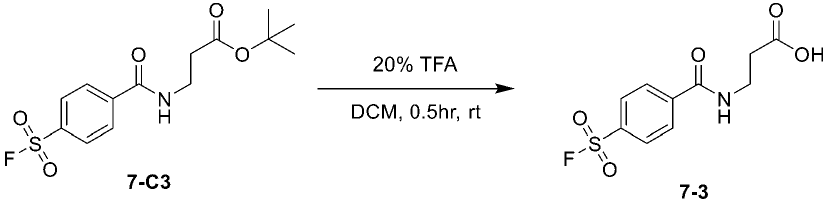

At room temperature, Compound **7-C3** (355.2 mg, 107.2 µmol) was dissolved in anhydrous dichloromethane (5.0 mL), followed by addition of trifluoroacetic acid (1.0 mL). The resulting reaction solution was stirred at room temperature for 0.5 hours. After the reaction was complete, the reaction solution was diluted with dichloromethane (25 mL), and the solvent was removed by rotary evaporation. The residue was dissolved and diluted with ethyl acetate (100 mL), washed with saturated brine (50 mL × 2), and the organic phase was subjected to rotary evaporation to dryness, and was subjected to co-rotary evaporated to dryness 2 to 3 times with petroleum ether to afford Compound **7-3** as a white solid powder (140.2 mg, 48.3% yield). The compound was used directly in the next step without further purification.

### Step 3: Synthesis of Compound S3-8

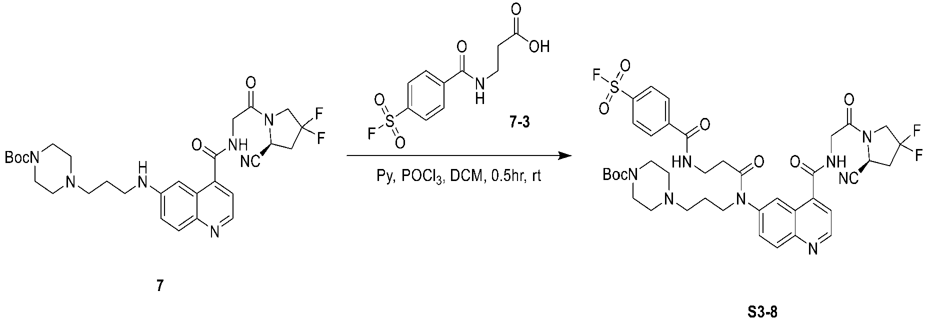

At room temperature, Compound **7** (200 mg, 0.3 mmol) (see Preparation Example 1, Step 11) and Compound **7-3** (59.4 mg, 0.5 mmol) were dissolved in anhydrous dichloromethane (10 mL), followed by addition of pyridine (1.34 g, 16.9 mmol) and POCl₃ (260.8 mg, 1.6 mmol) were added, and the reaction solution was stirred at room temperature for 0.5 hours. After completion of the reaction, the reaction solution was diluted with dichloromethane (50 mL), washed with water (15 mL × 2) and saturated brine (15 mL × 2) respectively, and the organic phase was rotary evaporated to dryness. The resulting residue was purified by semi-preparative high-performance liquid chromatography, and the resulting fraction was lyophilized to afford Compound **S3-8** as a yellow solid powder (75.2 mg, 26.1% yield). LCMS: [M+H]⁺= 844.2.

### Step 4: Synthesis of Compound S3-9

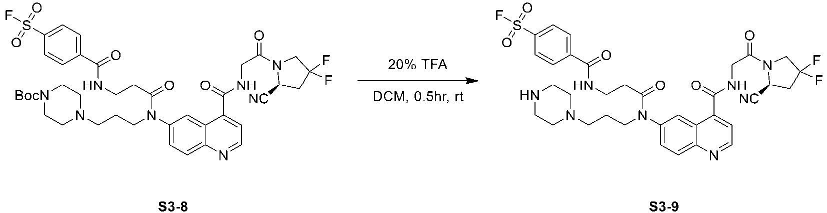

At room temperature, Compound **S3-8** (75.2 mg, 89.1 µmol) was dissolved in anhydrous dichloromethane (5.0 mL), and trifluoroacetic acid (1.0 mL) was added. The reaction solution was stirred at room temperature for 0.5 hours. After the reaction was complete, the reaction solution was diluted with dichloromethane (25 mL), and the solvent was removed by rotary evaporation to afford Compound **S3-9** as a reddish-brown oil (66.2 mg, 100% yield). The compound was used directly in the next step without further purification was required. LCMS: [M+H]⁺= 744.2.

### Step 5: Synthesis of Compound S3

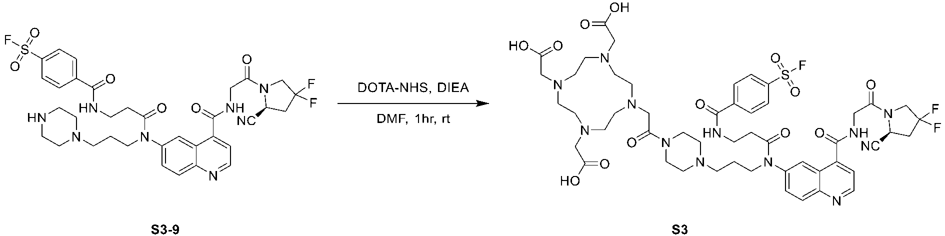

At room temperature, Compounds **S3-9** (66.2 mg, 89.0 µmol) and DOTA-NHS (89.1 mg, 178.0 µmol) were dissolved in anhydrous DMF (3.0 mL), followed by addition of DIEA (57.4 mg, 445.0 µmol). The reaction solution was stirred at room temperature for 1 hour. After the reaction was complete, the reaction solution was rotary evaporated to dryness. Separation and purification were conducted by semi-preparative high-performance liquid chromatography (0.1% acetic acid in water, acetonitrile), to yield Compound **S3** as a white solid (21.8 mg, 94.4% purity, 17.0% yield). LCMS: [M+H]⁺= 1130.31.

The ¹H-NMR data for Compound **S3** are as follows: ¹H NMR (400 MHz, Deuterium Oxide) δ 9.02 (d, *J =* 4.5 Hz, 1H), 8.23 (s, 1H), 8.20 - 8.09 (m, 2H), 7.86 (t, *J* = 8.5 Hz, 2H), 7.80 - 7.73 (m, 2H), 7.73 - 7.66 (m, 1H), 5.12 - 5.07 (m, 1H), 5.02 - 4.95 (m, 1H), 4.43 - 4.22 (m, 3H), 4.20 - 4.06 (m, 2H), 3.87 - 3.71 (m, 7H), 3.64 (s, 2H), 3.61 - 3.46 (m, 7H), 3.41 (s, 7H), 3.32 - 3.20 (m, 3H), 3.19 - 2.97 (m, 12H), 2.94 - 2.81 (m, 2H), 2.63 (s, 2H), 2.03 (d, *J =* 16.6 Hz, 2H).

Meanwhile, referring to step 5 of the synthesis of Compound S2, Compound S26 was prepared from Compound S3: 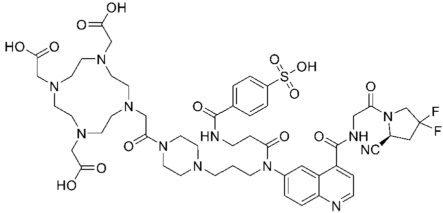 (LCMS: [M+2H]²⁺/2: 564.63).

### 3. Preparation Example 3: Synthesis Method and Synthesis Route for Compound S4

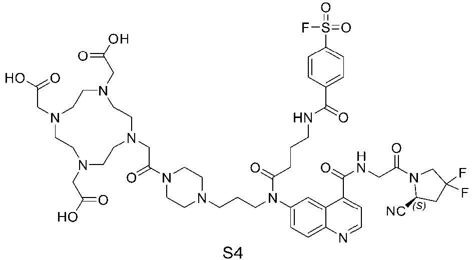

### Synthesis route for Compound S4:

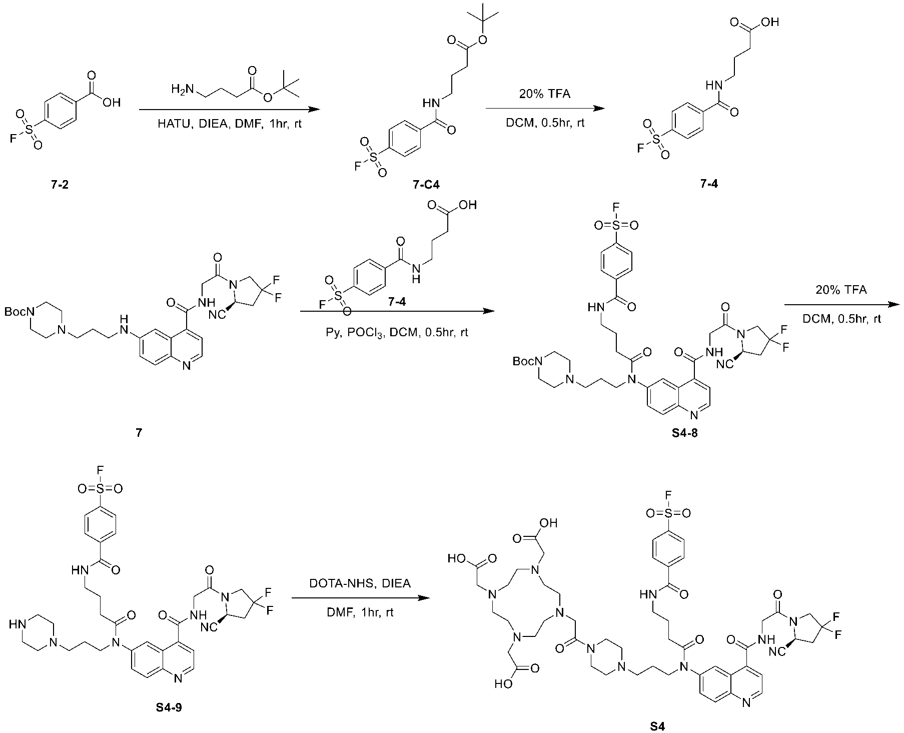

### Step 1: Synthesis of Compound 7-C4

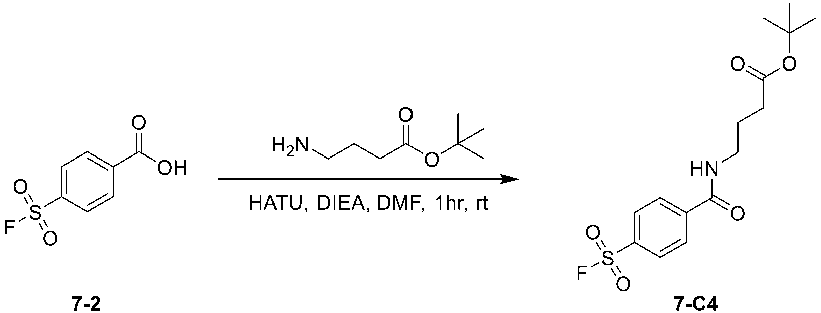

At room temperature, Compound **7-2** (250.0 mg, 122.4 µmol) and tert-butyl 3-aminobutyrate (234.0 mg, 146.9 µmol) were dissolved in anhydrous DMF (10.0 mL), followed by addition of DIEA (474.0 mg, 367.4 µmol) and HATU (558.5 mg, 146.9 µmol). The reaction solution was stirred at room temperature for 1 hour. After the reaction was complete, the reaction solution was diluted with ethyl acetate (100 mL), washed with saturated brine (50 mL × 2 times), and the organic phase was rotary evaporated to dryness. The resulting residue was purified by silica gel column chromatography (eluent: petroleum ether/ethyl acetate = 2/1) to afford Compound **7-C4** as a white solid (390.1 mg, 92.2% yield).

### Step 2: Synthesis of Compound 7-4

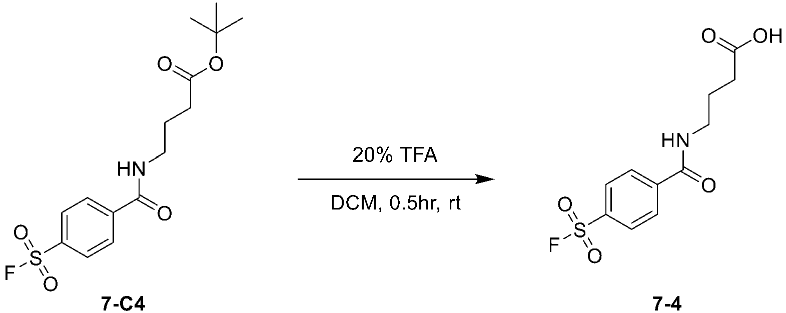

At room temperature, Compound **7-C4** (390.1 mg, 113.5 µmol) was dissolved in anhydrous dichloromethane (5.0 mL), followed by addition of trifluoroacetic acid (1.0 mL). The reaction solution was stirred at room temperature for 0.5 hours. After the reaction was complete, the reaction solution was diluted with dichloromethane (25 mL), and the solvent was removed by rotary evaporation. The residue was dissolved and diluted with ethyl acetate (100 mL), washed with saturated brine (50 mL × 2 times). The organic phase was rotary evaporated to dryness, followed by co-rotary evaporation with petroleum ether, repeated twice, to afford Compound **7-4** as a white solid powder (245.2 mg, yield 75.1%). The product was used directly in the next step without further purification.

### Step 3: Synthesis of Compound S4-8

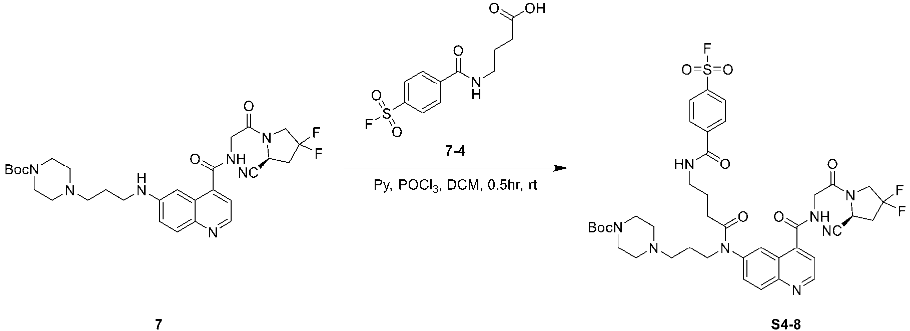

At room temperature, Compound **7** (see Preparation Example 1, Step 11) (200 mg, 0.3 mmol) and Compound **7-4** (148.4 mg, 0.5 mmol) were dissolved in anhydrous dichloromethane (10 mL), followed by addition of pyridine (1.3 g, 16.9 mmol) and POCl₃ (260.8 mg, 1.6 mmol), and the reaction solution was stirred at room temperature for 0.5 hours. After the reaction was complete, the reaction solution was diluted with dichloromethane (50 mL), washed with water (15 mL × 2) and saturated saline (15 mL × 2), respectively, and the organic phase was rotary evaporated to dryness. The resulting residue was purified by semi-preparative high-performance liquid chromatography, and the resulting fraction lyophilized to yield Compound **S4-8** as a yellow solid powder (65.2 mg, 22.3% yield). LCMS: [M+H]⁺= 859.3.

### Step 4: Synthesis of Compound S4-9

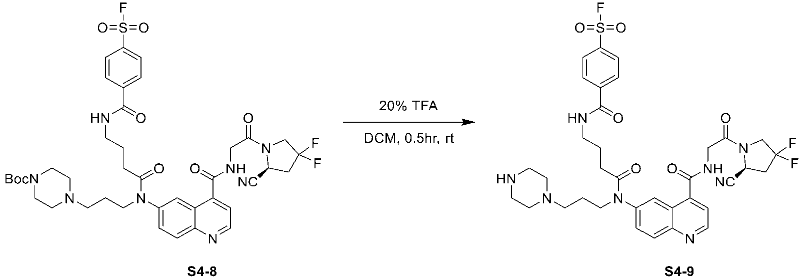

At room temperature, Compound **S4-8** (65.2 mg, 75.9 µmol) was dissolved in anhydrous dichloromethane (5.0 mL), followed by addition of trifluoroacetic acid (1.0 mL). The reaction solution was stirred at room temperature for 0.5 hours. After the reaction was complete, the reaction solution was diluted with dichloromethane (25 mL), and the solvent was removed by rotary evaporation to yield Compound **S4-9** as a reddish-brown oil (57.5 mg, 100% yield). The compound was used directly in the next step without further purification. LCMS: [M+H]⁺ =758.7.

### Step 5: Synthesis of Compound S4

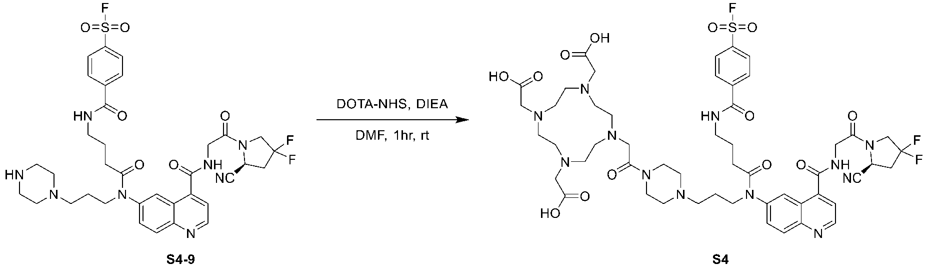

At room temperature, Compound **S4-9** (57.5 mg, 75.8 µmol) and DOTA-NHS (76.0 mg, 151.7 µmol) were dissolved in anhydrous DMF (3.0 mL), and DIEA (48.9 mg, 379.0 µmol) was added. The reaction solution was stirred at room temperature for 1 hour. After the reaction was complete, the reaction solution was rotary evaporated to dryness, and the residue was separated and purified by semi-preparative HPLC (0.1% acetic acid in water, acetonitrile), to yield Compound **S4** as a white solid (26.3 mg, 97.0% purity, 30.2% yield). LCMS: [M+H]⁺= 1145.47.

The ¹H-NMR data for Compound **S4** are as follows: ¹H NMR (400 MHz, Deuterium Oxide) δ 8.77 (dd, J = 7.8, 4.5 Hz, 1H), 8.19 (s, 1H), 8.09 - 7.89 (m, 2H), 7.82 - 7.74 (m, 1H), 7.65 - 7.54 (m, 2H), 7.42 (d, J = 8.3 Hz, 1H), 7.24 (d, J = 8.0 Hz, 1H), 5.20 - 5.11 (m, 1H), 4.45 - 4.25 (m, 3H), 4.24 - 4.10 (m, 1H), 3.88 - 3.68 (m, 8H), 3.54 (d, J = 32.5 Hz, 7H), 3.41 (s, 7H), 3.27 (d, J = 7.9 Hz, 5H), 3.20 - 2.88 (m, 13H), 2.36 - 2.24 (m, 2H), 2.03 - 1.94 (m, 2H), 1.94 - 1.82 (m, 2H).

Meanwhile, referring to step 5 of the synthesis of compound S2, S27 was further prepared from compound S4: 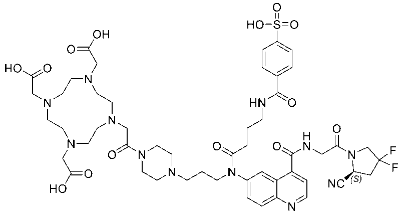 .4. Preparation Example 4: Synthesis Method and Synthesis Route of

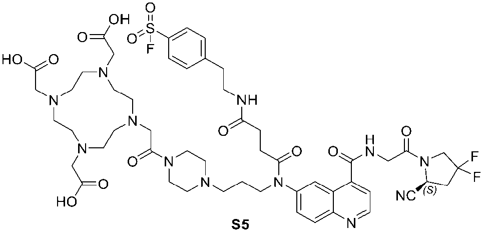

### Synthesis Route of Compound S5:

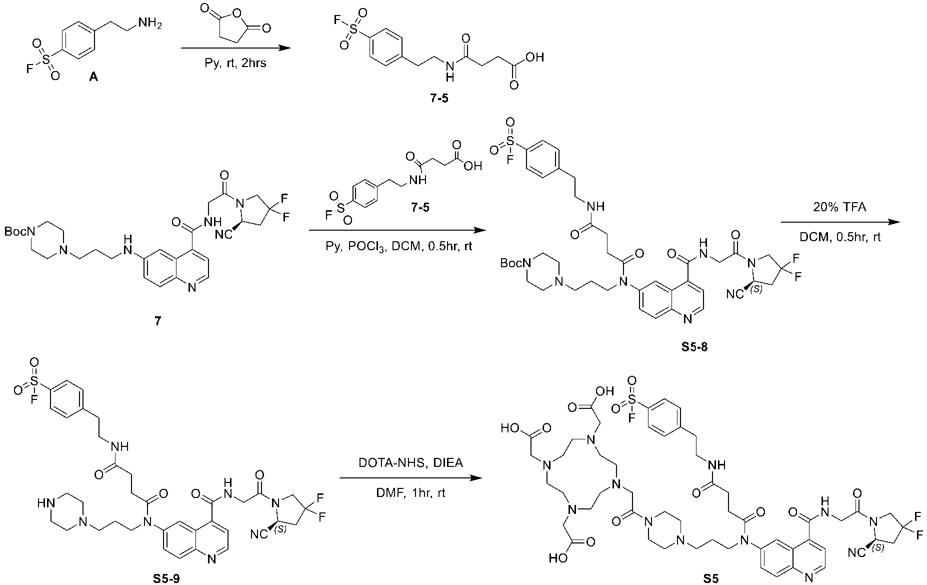

### Step 1: Synthesis of Compound 7-5

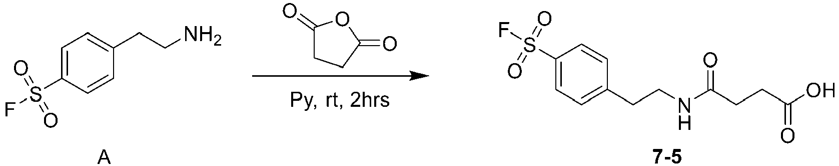

At room temperature, Compound **A** (200.0 mg, 122.4 µmol) was dissolved in anhydrous pyridine (5.0 mL), followed by addition of succinic anhydride (98.4 mg, 122.4 µmol). The reaction solution was stirred at room temperature for 2 hours. After the reaction was complete, the reaction solution was concentrated under reduced pressure. The resulting residue was purified by silica gel column chromatography (eluent: petroleum ether/ethyl acetate = 1/1) to afford Compound **7-5** as a colorless oil (200.0 mg, 67.0% yield).

¹H NMR data for Compound **7-5:** ¹H NMR (400 MHz, Chloroform-d) δ 7.99 (d, J = 8.2 Hz, 2H), 7.51 (d, J = 8.0 Hz, 2H), 3.62 (q, J = 6.8 Hz, 3H), 3.03 (t, J = 7.0 Hz, 2H), 2.81 - 2.67 (m, 2H), 2.58 - 2.43 (m, 2H).

### Step 2: Synthesis of Compounds S5-8

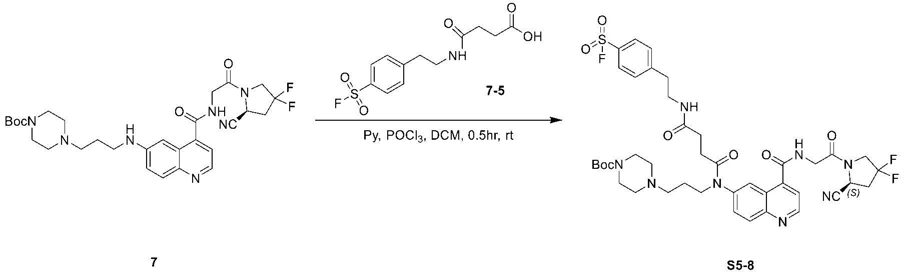

At room temperature, Compound 7 (see Preparation Example 1, Step 11) (200 mg, 0.3 mmol) and Compound **7-5** (155.1 mg, 0.5 mmol) were dissolved in anhydrous dichloromethane (10 mL), then pyridine (1.34 g, 16.9 mmol) and POCl₃ (260.8 mg, 1.6 mmol) were added. The reaction solution was stirred at room temperature for 0.5 hours. After completion of the reaction, the reaction solution was diluted with dichloromethane (50 mL), washed with water (15 mL × 2) and saturated brine (15 mL × 2) respectively, and dried over anhydrous sodium sulfate, and the organic phase was rotary evaporated to dryness. The resulting residue was purified by semi-preparative high-performance liquid chromatography, and the resulting fraction was lyophilized to afford Compound **S5-8** as a yellow solid powder (65.2 mg, 22.3% yield). LCMS: [M+H]⁺= 872.9.

### Step 3: Synthesis of Compound S5-9

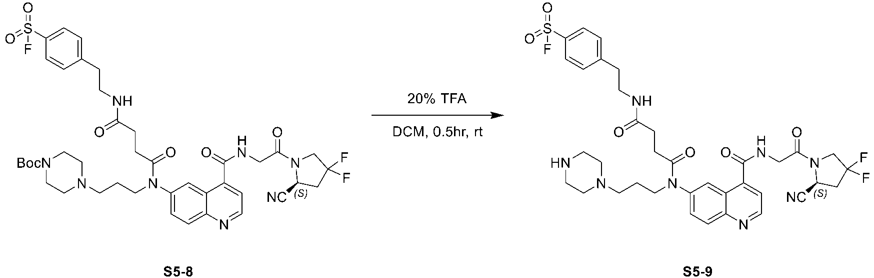

At room temperature, Compound S5-8 (65.2 mg, 75.9 µmol) was dissolved in anhydrous dichloromethane (5.0 mL), followed by addition of trifluoroacetic acid (1.0 mL). The reaction solution was stirred at room temperature for 0.5 hours. After the reaction was complete, the reaction solution was diluted with dichloromethane (25 mL), and the solvent was removed by rotary evaporation to afford Compound **S5-9** as a reddish-brown oil (57.9 mg, 100% yield). The compound was used directly in the next step without further purification. LCMS: [M+H]⁺= 772.9.

### Step 4: Synthesis of Compound S5

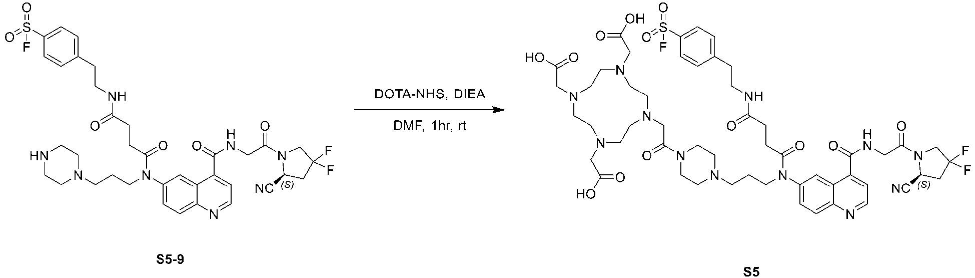

At room temperature, Compound **S5-9** (57.9 mg, 75.1 µmol) and DOTA-NHS (75.2 mg, 150.0 µmol) were dissolved in anhydrous DMF (3.0 mL), followed by addition of DIEA (48.9 mg, 379.0 µmol). The reaction solution was stirred at room temperature for 1 hour. After the reaction was complete, the reaction solution was rotary evaporated to dryness, and the residue was separated and purified by semi-preparative HPLC (0.1% acetic acid in water, acetonitrile), to yield Compound **S5** as a white solid (25.2 mg, 97.9% purity, 28.9% yield). LCMS: [M+H]⁺= 1158.71.

The ¹H-NMR data for Compound **S5** are as follows: ¹H NMR (400 MHz, Methanol-*d*₄) δ 9.02 (d, *J =* 4.4 Hz, 1H), 8.58 (s, 1H), 8.22 (d, *J* = 8.9 Hz, 1H), 7.92 (d, *J* = 8.1 Hz, 2H), 7.86 (d, *J* = 9.0 Hz, 1H), 7.72 (d, *J* = 4.5 Hz, 1H), 7.54 (d, *J* = 8.1 Hz, 2H), 5.18 (dd, *J* = 9.3, 2.8 Hz, 1H), 4.43 - 4.09 (m, 5H), 3.71 - 3.51 (m, 5H), 3.48 (p, *J =* 1.6 Hz, 1H), 3.41 (t, *J =* 7.0 Hz, 4H), 3.35 - 3.27 (m, 16H), 3.21 - 3.01 (m, 8H), 2.91 (q, *J =* 8.5, 6.9 Hz, 6H), 2.42 (s, 5H), 2.01 (d, *J* = 20.7 Hz, 3H).

Meanwhile, referring to step 5 of the synthesis of Compound **S2,** Compound **S28** was further prepared from Compound **S5:**

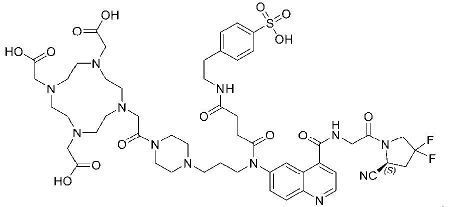

### 5. Preparation Example 5: Synthesis Method and Synthesis Route of Compound S6

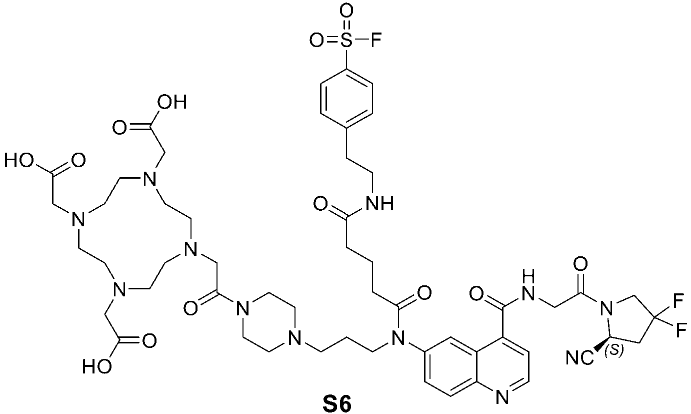

### Synthesis of Compound S6:

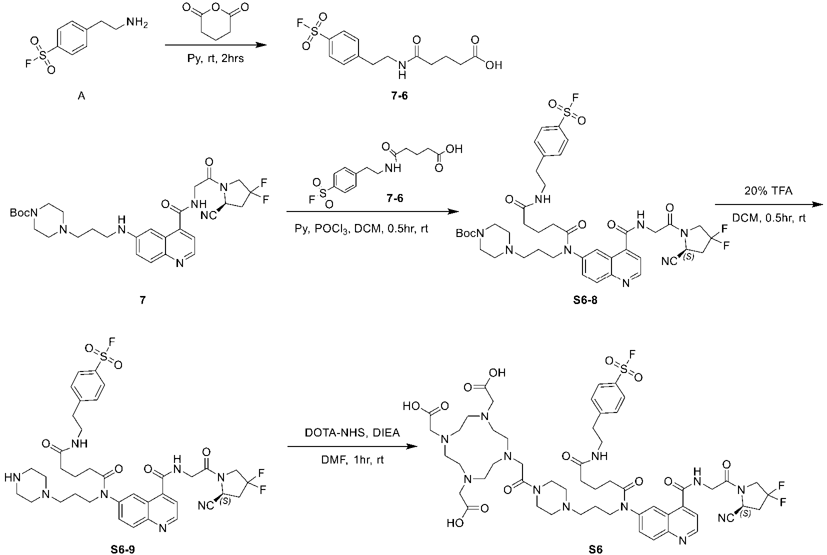

### Step 1: Synthesis of Compound 7-6

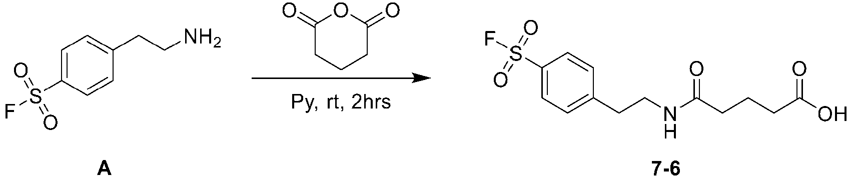

At room temperature, Compound **A** (200.0 mg, 122.4 µmol) was dissolved in anhydrous pyridine (5.0 mL), then glutaric anhydride (112.3 mg, 122.4 µmol) was added. The reaction solution was stirred at room temperature for 2 hours. After the reaction was complete, the reaction solution was concentrated under reduced pressure. The resulting residue was purified by silica gel column chromatography (eluent: petroleum ether/ethyl acetate = 1/1) to afford Compound **7-6** as a colorless liquid oil (220.1 mg, 70.4 % yield).

### Step 2: Synthesis of Compound S6-8

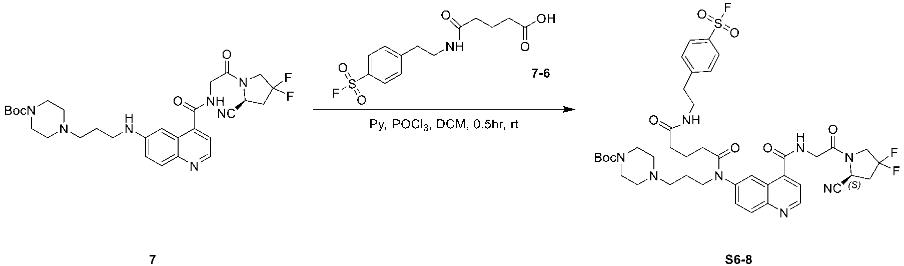

At room temperature, Compound **7** (200 mg, 0.3 mmol) and Compound **7-6** (108.1 mg, 0.5 mmol) were dissolved in anhydrous dichloromethane (10 mL), followed by addition of pyridine (1.34 g, 16.9 mmol) and POCl₃(260.8 mg, 1.6 mmol), and the reaction solution was stirred at room temperature for 0.5 hours. After completion of the reaction, the reaction solution was diluted with dichloromethane (50 mL), washed with water (15 mL × 2) and saturated brine (15 mL × 2), respectively, and dried over anhydrous sodium sulfate. The organic phase was rotary evaporated to dryness, and the resulting residue was purified by semi-preparative high-performance liquid chromatography. The resulting fraction was lyophilized to give Compound **S6-8** as a yellow solid powder (66.5 mg, 22.3% yield). LCMS: [M+H]⁺= 886.5.

### Step 3: Synthesis of Compound S6-9

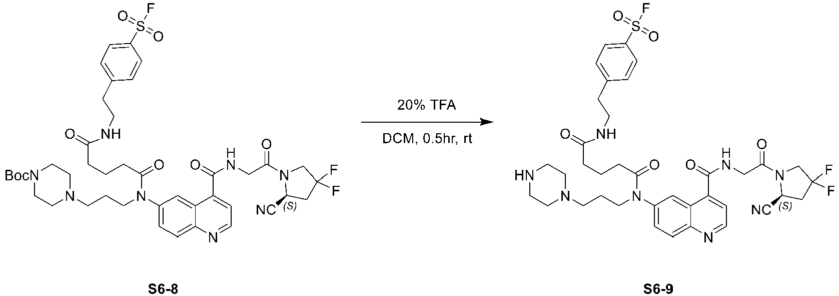

At room temperature, Compound **S6-8** (66.5 mg, 75.1 µmol) was dissolved in anhydrous dichloromethane (5.0 mL), followed by addition of trifluoroacetic acid (1.0 mL). The reaction solution was stirred at room temperature for 0.5 hours. After the reaction was complete, the reaction solution was diluted with dichloromethane (25 mL), and the solvent was removed by rotary evaporation, to yield Compound **S6-9** as a brownish-red oil (58.9 mg, 100% yield). The compound was used directly in the next step without further purification. LCMS: [M+H]⁺= 786.6.

### Step 4: Synthesis of Compound S6

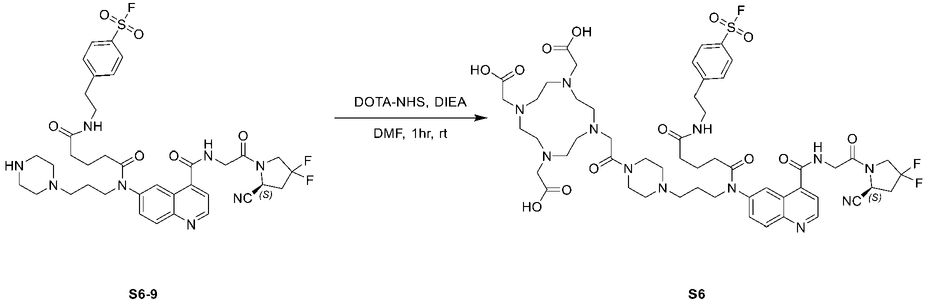

At room temperature, Compound **S6-9** (58.9 mg, 75.1 µmol) and DOTA-NHS (75.2 mg, 150.0 µmol) were dissolved in anhydrous DMF (3.0 mL), then DIEA (48.9 mg, 379.0 µmol) was added. The reaction solution was stirred at room temperature for 1 hour. After the reaction was complete, the reaction solution was rotary evaporated to dryness, and the residue was separated and purified by semi-preparative HPLC (0.1% acetic acid in water, acetonitrile), to yield Compound **S6** as a white solid (23.4 mg, 95.6% purity, 26.6% yield). LCMS: [M+H]⁺= 1172.85.

The ¹H NMR data for Compound **S6** are as follows: ¹H NMR (400 MHz, Methanol-*d*₄) δ 8.98 (d, *J* = 4.4 Hz, 1H), 8.63 - 8.46 (m, 1H), 8.20 (d, *J =* 8.9 Hz, 1H), 7.94 (d, *J =* 8.2 Hz, 2H), 7.82 (d, *J* = 8.4 Hz, 1H), 7.69 (d, *J =* 4.4 Hz, 1H), 7.46 (d, *J =* 8.0 Hz, 2H), 5.18 (dd, *J* = 9.4, 2.9 Hz, 1H), 4.46 - 4.07 (m, 6H), 3.35 - 3.29 (m, 24H), 3.18 - 3.00 (m, 9H), 3.00 - 2.68 (m, 10H), 2.17 (s, 3H), 2.11 - 1.93 (m, 5H), 1.91 - 1.77 (m, 2H).

Meanwhile, referring to step 5 of the synthesis of Compound **S2,** Compound **S29** was further prepared from compound S6:

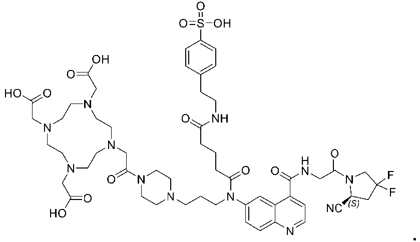

Referring to the synthesis of Compound **S6,** Compound **S7** was prepared. The structural formula of Compound **S7** is as follows:

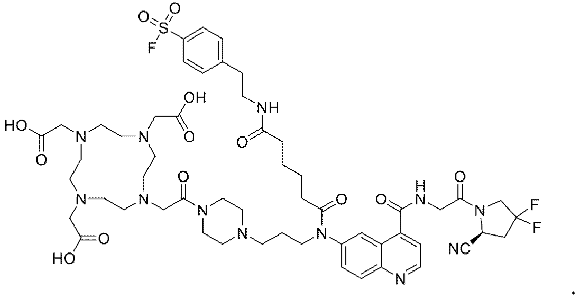

The mass spectrometry and ¹H-NMR data for Compound **S7** are as follows:
LC-MS: [M+H]⁺ : 1187.12
HR MS (ESI): Calculated value of [M+2H]²⁺/2: 593.25409, Measured value: 593.2546
¹H NMR (400 MHz, Deuterium Oxide) δ 9.00 (d, *J* = 4.4 Hz, 1H), 8.27 (d, *J* = 8.9 Hz, 1H), 8.20 (d, *J* = 2.3 Hz, 1H), 7.85 (d, *J =* 8.3 Hz, 2H), 7.80 (dd, *J =* 8.9, 2.5 Hz, 1H), 7.77 (d, *J =* 4.5 Hz, 1H), 7.42 (d, *J* = 8.1 Hz, 2H), 5.15 (dd, *J* = 8.5, 4.1 Hz, 1H), 4.44 - 4.26 (m, 4H), 4.23 - 4.11 (m, 2H), 3.80 (d, *J =* 13.0 Hz, 3H), 3.74 (s, 2H), 3.56 (s, 3H), 3.53 - 3.47 (m, 3H), 3.47 - 3.34 (m, 8H), 3.32 - 3.21 (m, 4H), 3.21 - 3.08 (m, 6H), 3.07 - 2.90 (m, 5H), 2.85 (t, *J =* 6.4 Hz, 2H), 2.08 (t, *J =* 6.9 Hz, 2H), 2.06 - 1.96 (m, 4H), 1.34 (d, *J =* 7.0 Hz, 1H), 1.30 - 1.17 (m, 4H).

### 6. Preparation Example 6: Synthesis Method and Synthesis Route for Compound S2

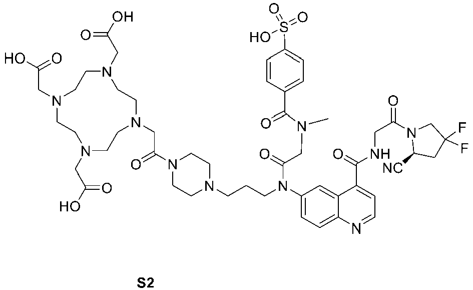

The synthesis of Compound **S2** is as follows:

### Step 1: Synthesis of Compound S2-a

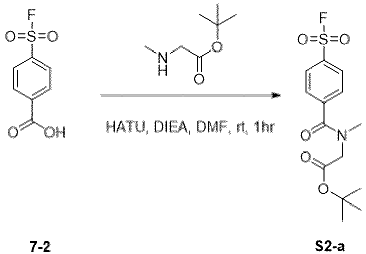

At room temperature, Compound **7-2** (250 mg, 1.2 mmol) was dissolved in anhydrous DMF (10 mL), then sarcosine tert-butyl ester (296.2 mg, 1.3 mmol), DIEA (774.0 mg, 6.0 mmol), and HATU (532.0 mg, 1.4 mmol) were added. The resulting reaction solution was stirred at room temperature for 1 hour. After the reaction was complete, the reaction solution was diluted with ethyl acetate (50 mL), washed with aqueous solution (50 mL × 2) and saturated brine (50 mL × 2) respectively, dried, and the residue obtained by concentrating and drying the organic phase was purified by silica gel column chromatography (eluent: petroleum ether/ethyl acetate = 3/1) to afford Compound **S2-a** as a white solid (230 mg, 86.4% yield).

### Step 2: Synthesis of Compound S2-b

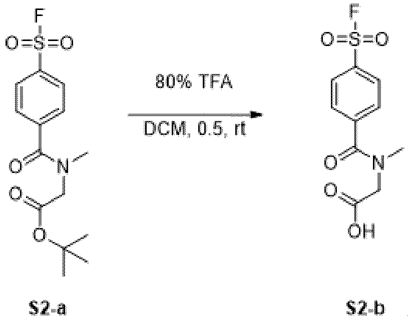

At room temperature, Compound **S2-a** (230 mg, 0.7 mmol) was dissolved in anhydrous DCM (3 mL), followed by addition of trifluoroacetic acid (5.0 mL). The resulting reaction solution was stirred at room temperature for 0.5 hours. After the reaction was complete, the reaction solution was diluted with dichloromethane (25 mL), concentrated, diluted with ethyl acetate (50 mL), and then washed with water (50 mL × 2) and saturated brine (50 mL × 2), respectively. After drying, Compound **S2-b** as a white solid (135 mg, 70.6% yield) was obtained, which was used directly in the next step without further purification.

### Step 3: Synthesis of Compound 8-2

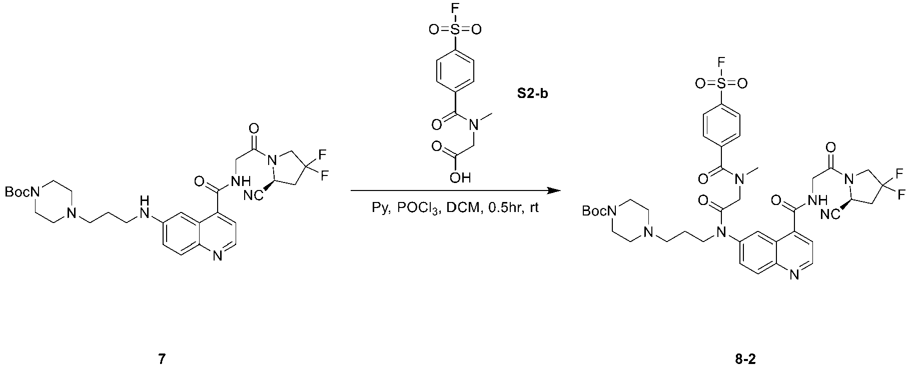

At room temperature, Compound **7** (200.0 mg, 341.0 µmol) and Compound **S2-b** (140.0 mg, 511.5 µmol) were dissolved in anhydrous dichloromethane (10 mL), then pyridine (1.34 g, 17.0 mmol) and POCl₃(260 mg, 1705.0 µmol) were added. The resulting reaction solution was stirred at room temperature for 0.5 hours. After the reaction was complete, the solution was diluted with dichloromethane (50 mL), washed with water (15 mL × 2) and saturated brine (15 mL × 2), respectively. The organic phase was rotary evaporated to dryness, and the resulting residue was purified by semi-preparative liquid chromatography. The resulting fraction was lyophilized to yield Compound **8-2** as a yellow solid powder (90.1 mg, 31.3% yield). LCMS: [M+H]⁺= 844.4.

### Step 4: Synthesis of Compound 9-2

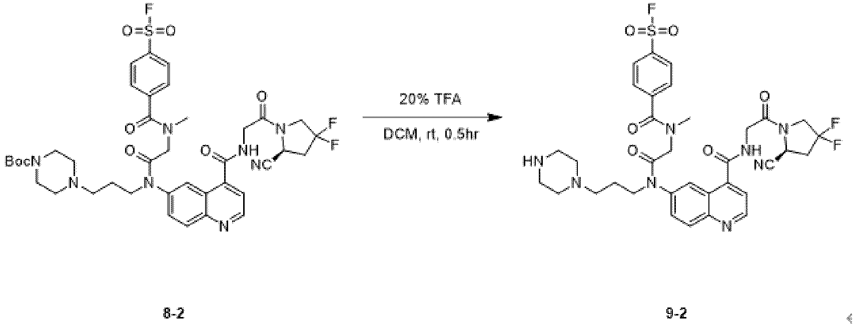

At room temperature, Compound **8-2** (90.1 mg, 106.9 µmol) was dissolved in anhydrous dichloromethane (5.0 mL), followed by addition of trifluoroacetic acid (1.0 mL). The resulting reaction solution was stirred at room temperature for 0.5 hours. After the reaction was complete, the solution was diluted with dichloromethane (25 mL), rotary evaporated to dryness to afford Compound **9-2** as a reddish-brown oil (27.0 mg, 100% yield). The compound was used directly in the next step without further purification. LCMS: [M+H]⁺= 744.1.

### Step 5: Synthesis of Compound S2

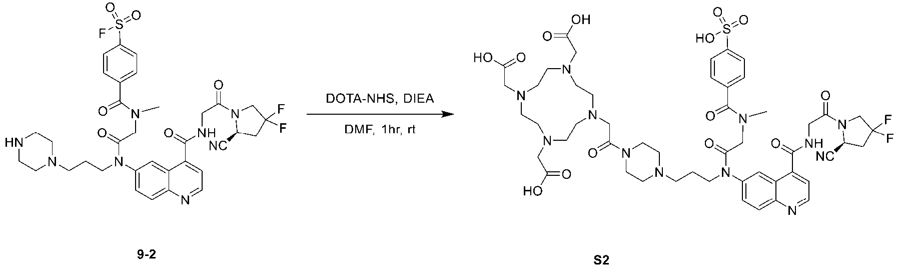

At room temperature, Compound **9-2** (79.4 mg, 106.8 µmol) and DOTA-NHS (107.1 mg, 213.6 µmol) were dissolved in anhydrous DMF (3.0 mL), then DIEA (41.3 mg, 320.4 µmol) was added. The resulting reaction solution was stirred at room temperature for 1 hour. After the reaction was complete, the reaction solution was rotary evaporated to dryness, and the residue was purified by semi-preparative liquid chromatography to afford Compound **S2** as a white solid (3.6 mg, 96.8% purity). LCMS: [M+H] = 1128.

The ¹H-NMR data for Compound **S2** are as follows: ¹H NMR (400 MHz, Deuterium Oxide) δ 9.11 - 8.99 (m, 1H), 8.43 - 8.30 (m, 1H), 8.19 (d, *J =* 9.0 Hz, 1H), 7.98 - 7.92 (m, 1H), 7.88 (d, *J =* 8.2 Hz, 1H), 7.82 (d, *J =* 4.5 Hz, 1H), 7.78 - 7.73 (m, 2H), 7.54 (d, *J =* 8.2 Hz, 1H), 7.34 - 7.24 (m, 2H), 5.17 - 5.09 (m, 1H), 4.44 - 4.23 (m, 5H), 4.23 - 4.12 (m, 3H), 4.12 - 3.91 (m, 5H), 3.88 - 3.70 (m, 11H), 3.62 (s, 5H), 3.57 - 3.48 (m, 5H), 3.47 - 3.37 (m, 10H), 3.26 - 3.00 (m, 21H), 3.00 - 2.87 (m, 5H), 2.16 - 2.01 (m, 1H), 1.98 - 1.86 (m, 2H).

### 7. Preparation Example 7: Synthesis Method and Synthesis Route of Compound S17

### Synthesis of S17

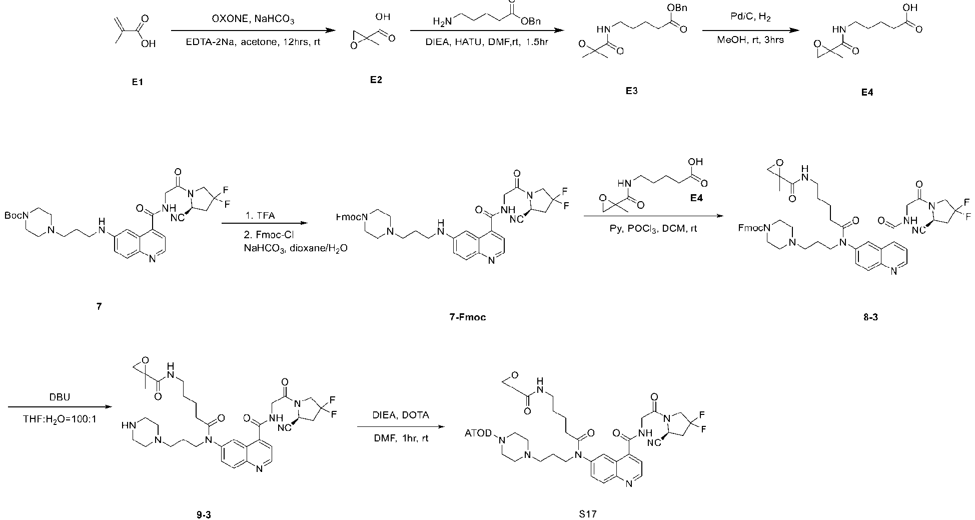

The synthesis steps of S17 were as follows:

### Step 1: Synthesis of Compound E2

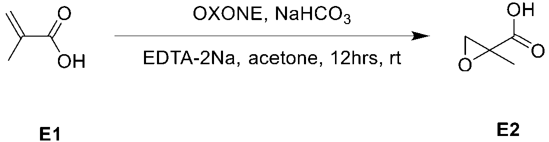

At room temperature, Compound **E1** (5 g, 58.1 mmol) and EDTA-2Na (600 mg, 1.6 mmol) were dissolved in anhydrous acetone (250 mL). The resulting reaction solution was stirred at room temperature for 2 hours, then added dropwise to 250 mL of mixed aqueous solution (prepared by dissolving potassium peroxysulfate (OXONE) (50 g, 87.1 mmol) and NaHCO₃ (22 g, 261.4 mmol) in 250 mL of water). The resulting reaction solution was stirred at room temperature for 12 hours. After the reaction was complete, the pH of the reaction solution was adjusted to 5.0, followed by washing with ethyl acetate (50 mL), water (15 mL × 2), and saturated brine (15 mL × 2), respectively. The organic phase was rotary evaporated to dryness, and the residue was purified by semi-preparative liquid chromatography. The resulting fraction was lyophilized to afford Compound **E2** as a colorless oil (2.3 g, 38.7% yield).

### Step 2: Synthesis of Compound E3

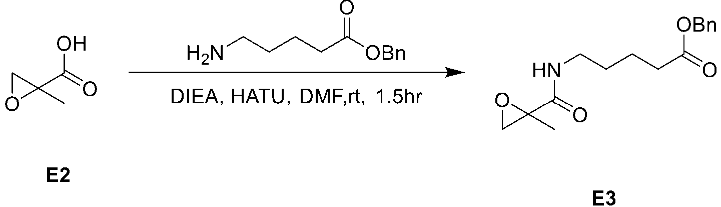

At room temperature, Compound **E2** (350 mg, 3.4 mmol) was dissolved in anhydrous DMF (10 mL), followed by addition of benzyl 5-aminovalerate (296.2 mg, 1.3 mmol), DIEA (774.0 mg, 6.0 mmol), and HATU (532.0 mg, 1.4 mmol). The resulting reaction solution was stirred at room temperature for 1.5 hours. After the reaction was complete, the reaction solution was diluted with ethyl acetate (50 mL), washed with water (50 mL × 2) and saturated saline (50 mL × 2), respectively, dried over anhydrous sodium sulfate, and the organic phase was dried and concentrated. The resulting residue was purified by silica gel column chromatography (eluent: petroleum ether/ethyl acetate =3/1) to obtain Compound **E3** as a white solid (790 mg, 79% yield).

### Step 3: Synthesis of Compound E4

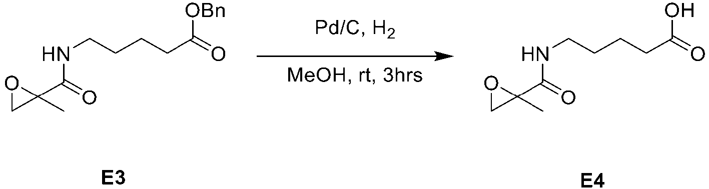

At room temperature, Compound **E3** (790 mg, 58.1 mmol) and Pd/C (79 mg, 5.8 mmol) were dissolved in anhydrous methanol (10 mL), and the solution was purged three times with nitrogen and three times with hydrogen. The resulting reaction solution was stirred at room temperature for 3 hours. After the reaction was complete, the reaction solution was filtered, and the organic phase was concentrated and dried. The resulting residue was purified by silica gel column chromatography (eluent: petroleum ether/ethyl acetate = 1/1) to afford Compound **E4** as a colorless oil (375.1 mg, 68.7% yield).

### Step 4: Synthesis of Compound 7-Fmoc

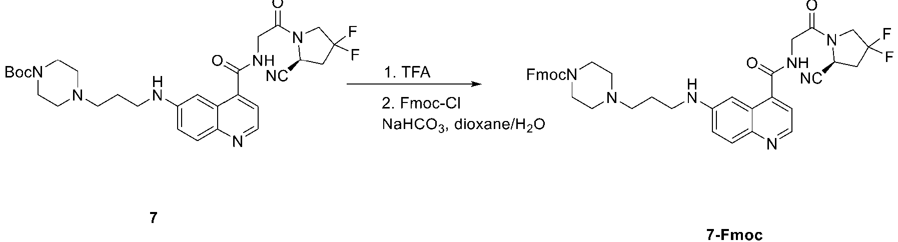

At room temperature, Compound **7** (500 mg, 853.8 µmol) was dissolved in anhydrous dichloromethane (10 mL), followed by addition of trifluoroacetic acid (2 mL). The resulting reaction solution was stirred at room temperature for 0.5 hours. After the reaction was complete, the reaction solution was diluted with dichloromethane (10 mL), and the solvent was removed by rotary evaporation to yield a brownish-red oily compound (414 mg, 852.5 µmol, 100% yield). The brownish-red oily compound was dissolved in 10 mL of a mixed solution (dioxane/H₂O = 1:1), and sodium bicarbonate (218.4 mg, 2.5 mmol) was added. At 0°C, Fmoc-Cl (220.8 mg, 852.5 µmol) was dissolved in 5 mL of dioxane, and added dropwise to the above reaction solution. The resulting solution was stirred at 0 to 10°C for 1 hour. After the reaction was complete, the solution was diluted with ethyl acetate (50 mL), washed with water (15 mL × 2) and saturated brine (15 mL × 2), respectively, and the organic phase was concentrated. The resulting residue was purified by silica gel column chromatography (eluent: dichloromethane/methanol = 10/1 to 5/1) to obtain Compound **7-Fmoc** as a yellow solid (230 mg, 38.1% yield).

### Step 5: Synthesis of Compound 8-3

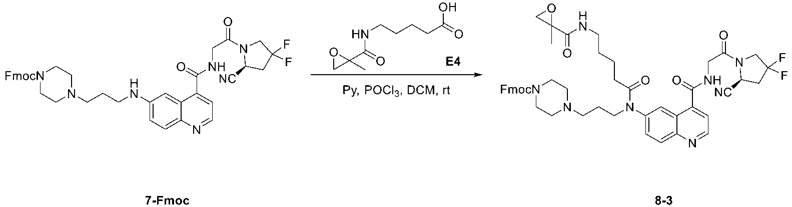

At room temperature, Compound **7-Fmoc** (100 mg, 141.3 µmol) and Compound **E4** (42.6 mg, 211.9 µmol) was dissolved in anhydrous dichloromethane (10 mL), then pyridine (553 mg, 7.0 mmol) and POCl₃ (108.0 mg, 706.5 µmol) were added. The resulting reaction solution was stirred at room temperature for 0.5 hours. After the reaction was complete, the reaction solution was diluted with dichloromethane (50 mL), washed with water (15 mL × 2) and saturated saline (15 mL × 2), respectively, and the organic phase was rotary evaporated to dryness. The resulting residue was purified by semi-preparative liquid chromatography, and the resulting fraction was lyophilized to yield compound 8-3 as a yellow solid powder (33.5 mg, 25.3% yield). LCMS: [M+H]⁺ = 892.2.

### Step 6: Synthesis of Compound 9-3

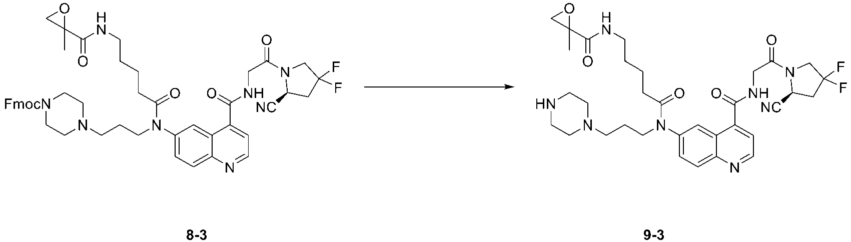

At room temperature, Compound **8-3** (33.5 mg, 37.5 µmol) was dissolved in 2 mL of a mixed solvent (tetrahydrofuran/DBU aqueous solution = 100:1). The resulting reaction solution was stirred at room temperature for 2 min, then purified by semi-preparative liquid chromatography, and the resulting fraction was lyophilized to afford Compound **9-3** as a colorless oil (18 mg, 71.7% yield).

### Step 7: Synthesis of Compound S17

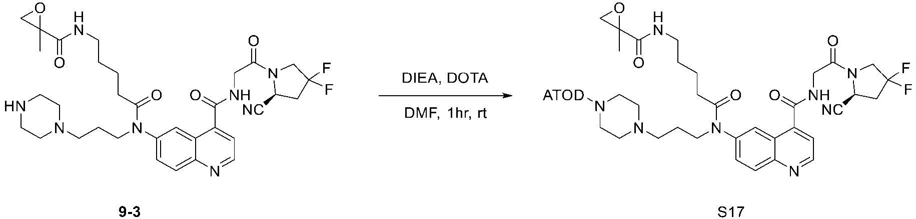

At room temperature, Compound **9-3** (18 mg, 26.9 µmol) and DOTA-NHS (26.9 mg, 53.8 µmol) were dissolved in anhydrous DMF (3.0 mL), followed by addition of DIEA (10.4 mg, 80.7 µmol). The resulting reaction solution was stirred at room temperature for 1 hour. After the reaction was complete, the reaction solution was rotary evaporated to dryness, and the residue was purified by semi-preparative HPLC to afford Compound **S17** as a white solid (7.5 mg, 96.7% purity, 26.4% yield). LCMS: [M+H]⁺= 1056.4; HR MS (ESI): Calculated value of [M+H]⁺: 1055.51205, measured value: 1055.51245.

The¹H-NMR data for Compound **S17** are as follows:
¹H NMR (400 MHz, Deuterium Oxide) δ 9.03 (d, J = 4.5 Hz, 1H), 8.27 (d, J = 9.0 Hz, 1H), 8.23 (s, 1H), 7.83 (dd, J = 9.0, 2.3 Hz, 1H), 7.78 (d, J = 4.5 Hz, 1H), 5.17 (dd, J = 8.5, 4.2 Hz, 1H), 4.45 - 4.25 (m, 4H), 4.25 - 4.11 (m, 2H), 3.79 (q, J = 16.3 Hz, 7H), 3.70 (s, 2H), 3.57 - 3.32 (m, 14H), 3.31 - 3.22 (m, 4H), 3.14 (d, J = 6.1 Hz, 6H), 3.02 (t, J = 6.9 Hz, 4H), 2.98 - 2.95 (m, 1H), 2.95 - 2.91 (m, 1H), 2.86 (d, J = 4.8 Hz, 1H), 2.78 (d, J = 4.8 Hz, 1H), 2.23 (t, J = 7.5 Hz, 2H), 2.07 - 1.96 (m, 3H), 1.55 - 1.43 (m, 3H), 1.42 (s, 3H), 1.35 (dt, J = 13.7, 6.2 Hz, 3H).

Compounds **S15** and **S16** were prepared by referring to the synthetic method of Compound **S17.**

The structural formula of Compound **S15** is as follows:

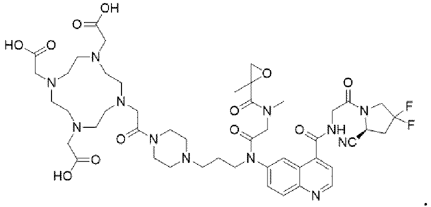

The mass spectrometry and ¹H-NMR data for Compound **S15** are as follows:
LC-MS: [M+H]⁺: 1028.57
HR MS (ESI): calculated value of [M+H]⁺: 1027.48075, measured value: 1027.48022.
¹H NMR (600 MHz, Deuterium Oxide) δ 9.07 - 8.96 (m, 1H), 8.33 - 8.22 (m, 2H), 7.90 - 7.83 (m, 1H), 7.81 - 7.72 (m, 1H), 5.20 - 5.08 (m, 1H), 4.43 - 4.35 (m, 1H), 4.35 - 4.24 (m, 3H), 4.20 - 4.09 (m, 1H), 4.08 - 3.86 (m, 4H), 3.86 - 3.69 (m, 5H), 3.66 (s, 3H), 3.53 - 3.29 (m, 13H), 3.24 (s, 4H), 3.11 (s, 6H), 3.08 (s, 4H), 3.04 - 2.96 (m, 5H), 2.96 - 2.89 (m, 2H), 2.88 (d, *J =* 6.5 Hz, 2H), 2.00 (s, 2H), 1.50 (d, *J =* 2.5 Hz, 2H), 1.39 (d, *J =* 5.0 Hz, 1H).

The structural formula of Compound **S16** is as follows:

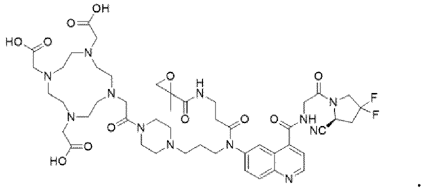

The mass spectrometry and ¹H-NMR data for Compound **S16** are as follows:
LC-MS: [M+H]⁺: 1028.30
HR MS (ESI): Calculated value of [M+H]⁺: 1027.48075, Measured value: 1027.48047.
¹H NMR (400 MHz, Deuterium Oxide) δ 9.03 (d, *J* = 4.5 Hz, 1H), 8.27 (d, *J* = 9.0 Hz, 1H), 8.22 (s, 1H), 7.81 (d, *J* = 2.3 Hz, 1H), 7.78 (d, *J =* 4.5 Hz, 1H), 5.17 (dd, *J* = 8.4, 4.2 Hz, 1H), 4.44 - 4.28 (m, 4H), 4.23 - 4.11 (m, 2H), 3.89 - 3.72 (m, 7H), 3.70 (s, 2H), 3.50 - 3.32 (m, 14H), 3.14 (s, 8H), 3.08 - 2.95 (m, 7H), 2.92 (d, *J =* 4.5 Hz, 2H), 2.84 (d, *J =* 4.8 Hz, 1H), 2.48 - 2.41 (m, 2H), 1.95 (s, 2H), 1.45 (s, 3H).

### 8. Preparation Example 8: Synthesis Method and Synthesis Route of Compound S12

### Synthesis Route of S12

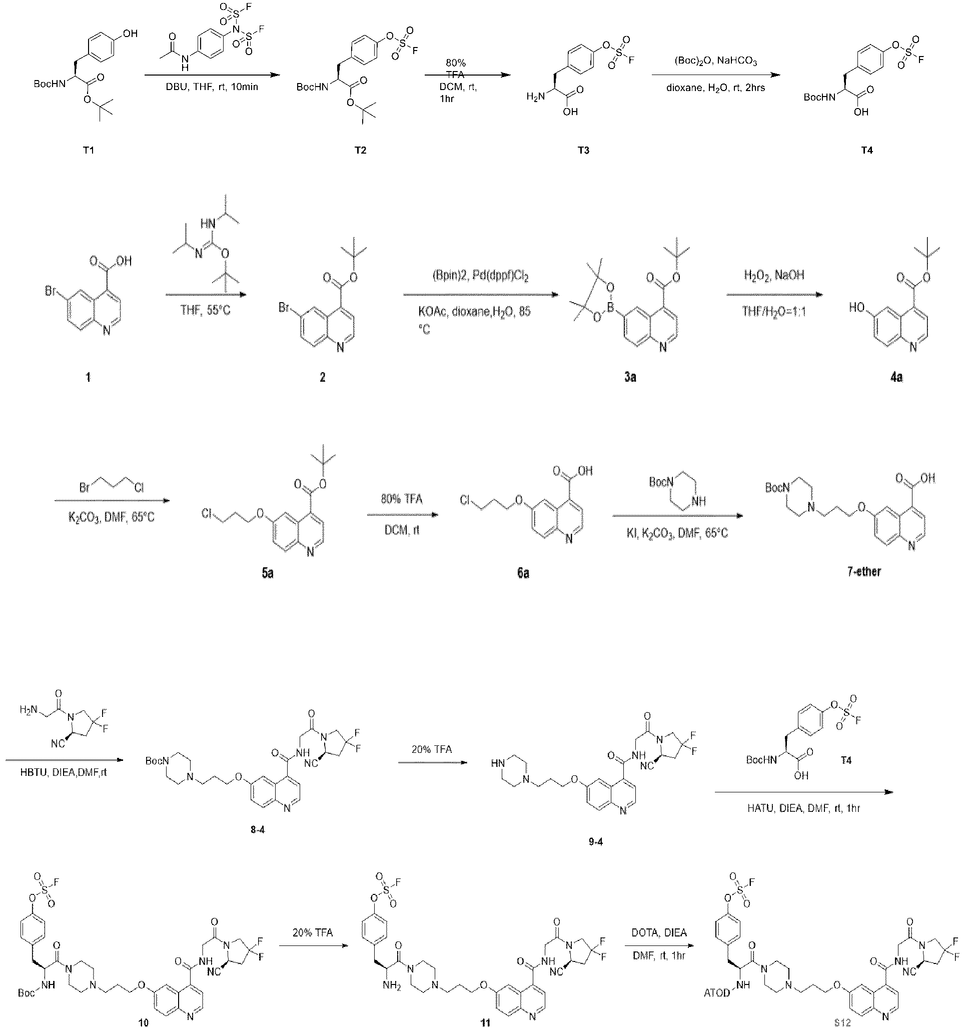

The synthesis steps of S12 were as follows:

### Step 1: Synthesis of Compound T2

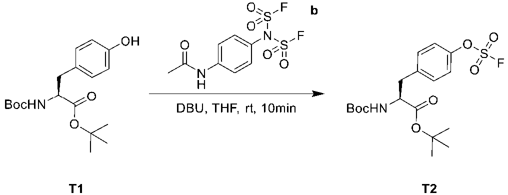

At room temperature, Compound **T1** (1.3 g, 3.8 mmol) and Compound **b** (1.4 g, 4.56 mmol) were dissolved in anhydrous tetrahydrofuran, and DBU (1.2 g, 8.3 mmol) was added dropwise. The resulting reaction solution was stirred at room temperature for 10 min. After the reaction was complete, the reaction solution was diluted with ethyl acetate (50 mL), washed with dilute hydrochloric acid (50 mL×2), and the organic phase was concentrated. The resulting residue was purified by silica gel column chromatography (eluent: petroleum ether/ethyl acetate = 10/1) to afford Compound **T2** as a white solid (1.55 g, 96% yield).

### Step 2: Synthesis of Compound T3

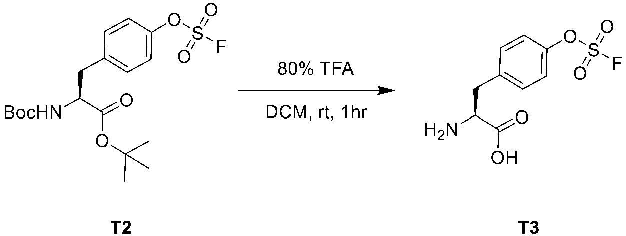

At room temperature, Compound **T2** (1.0 g, 2.38 mmol) was dissolved in anhydrous dichloromethane (2.0 mL), followed by addition of trifluoroacetic acid (8 mL). The resulting reaction solution was stirred at room temperature for 1 hour. After the reaction was complete, the reaction solution was diluted with dichloromethane (25 mL), and the solvent was removed by rotatory evaporation to yield Compound **T3** as a reddish-brown oil (627.6 mg, 100% yield). The compound was used directly in the next step without further purification.

### Step 3: Synthesis of Compound T4

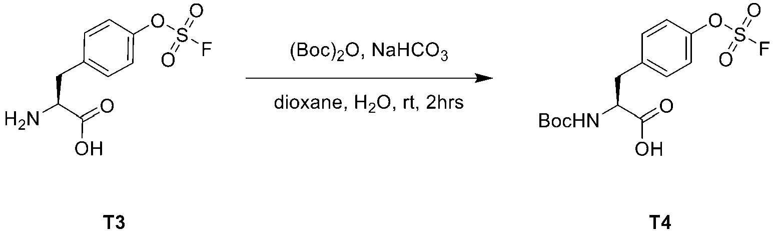

At room temperature, Compound **T3** (627 mg, 2.3 mmol) was dissolved in 10 mL of a mixed solution (dioxane/H₂O = 1:1), and sodium bicarbonate (601 mg, 6.9 mmol) and (Boc)₂O (1.0 g, 4.6 mmol) were added, respectively. The resulting reaction solution was stirred at room temperature for 2 hours. After the reaction was complete, the reaction solution was adjusted to pH of 5.0, then diluted with ethyl acetate (50 mL), washed with water (15 mL × 2) and saturated brine (15 mL × 2), respectively. After concentrating the organic phase, the residue obtained was purified by silica gel column chromatography (eluent: petroleum ether/ethyl acetate = 10/1) to afford Compound **T4** as a white solid (630 mg, 72.7% yield).

### Step 4: Synthesis of Compound 3a

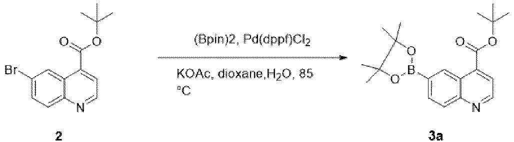

At room temperature, Compound **2** (3.0 g, 9.7 mmol), (Bpin)₂ (4.9 g, 19.4 mmol), KOAc (3.0 g, 9.7 mmol), and Pd(dppf)Cl₂ (731.7 mg, 0.2 mmol) were dissolved in 60 mL of dioxolane. The resulting reaction solution was purged with nitrogen three times at room temperature, then stirred at 85°C for 12 hours. After the reaction was complete, the reaction solution was diluted with ethyl acetate (50 mL), washed with water (15 mL × 2) and saturated saline (15 mL × 2), respectively, and the organic phase was concentrated. The resulting residue was purified by silica gel column chromatography (eluent: petroleum ether/ethyl acetate = 5/1) to afford Compound **3a** as a white solid (3.45 g, 100% yield).

### Step 5: Synthesis of Compound 4a

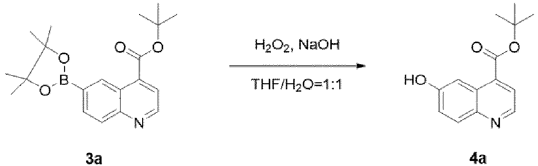

At room temperature, Compound **3a** (3.45 g, 9.7 mmol) was dissolved in tetrahydrofuran (30 mL), sodium hydroxide solution (1.16 g dissolved in 30 mL water) was added, then 30% H₂O₂ (1.98 g, 58.2 mmol) was added dropwise. The resulting reaction solution was stirred at room temperature for 2 hours. After the reaction was complete, the reaction solution was adjusted to a pH of 6 to 7 with dilute hydrochloric acid, diluted with ethyl acetate (50 mL), and washed with water (15 mL × 2) and saturated brine (15 mL × 2), respectively. The organic phase was concentrated, and the resulting residue was purified by silica gel column chromatography (eluent: petroleum ether/ethyl acetate = 3/1) to afford Compound **4a** as a white solid (2.38 g, 100% yield).

### Step 6: Synthesis of Compound 5a

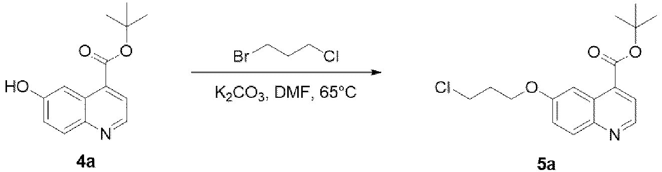

At room temperature, Compound **4a** (2.38 g, 9.7 mmol), 1-bromo-3-chloropropane (2.75 g, 17.4 mmol), and potassium carbonate (4.0 g, 29.1 mmol) were dissolved in anhydrous DMF (25 mL), and the resulting reaction solution was stirred at 65°C for 12 hours. After the reaction was complete, the solution was diluted with ethyl acetate (50 mL), washed with water (15 mL × 2) and saturated brine (15 mL × 2), respectively, and the organic phase was rotary evaporated to dryness. The residue obtained after concentrating the organic phase was purified by silica gel column chromatography (eluent: petroleum ether/ethyl acetate = 15/1:10/1) to afford Compound **5a** as a white solid (2.3 g, 73.9% yield).

### Step 7: Synthesis of Compound 6a

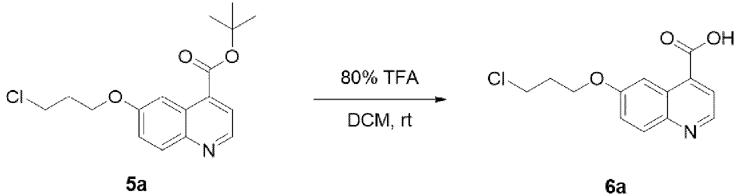

At room temperature, Compound **5a** (1.3 g) was dissolved in anhydrous dichloromethane (2.0 mL), followed by addition of trifluoroacetic acid (8 mL). The resulting reaction solution was stirred at room temperature for 0.5 hours. After the reaction was complete, the reaction solution was diluted with dichloromethane (25 mL), and the solvent was removed by rotary evaporation to yield Compound **6a** as a reddish-brown oil (1.07 g, 100% yield). The compound was used directly in the next step without further purification.

### Step 8: Synthesis of Compound 7-ether

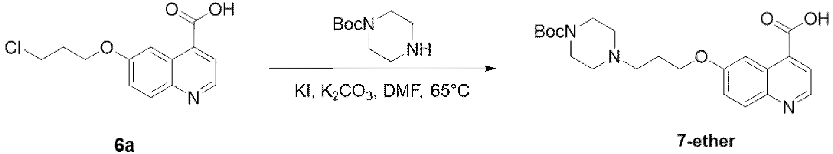

At room temperature, Compound **6a** (850 mg, 3.2 mmol) and N-Boc-piperazine (985 mg, 6.4 mmol) were dissolved in DMF (20 mL), and KI (880 mg, 6.4 mmol) was added. The resulting reaction solution was stirred at 80°C for 12 hours. The solution was diluted with ethyl acetate (50 mL), then washed with water (15 mL × 2) and saturated brine (15 mL × 2), respectively. The organic phase was concentrated, and the residue was purified by silica gel column chromatography (eluent: petroleum ether/ethyl acetate = 6/1) to afford Compound **7-ether** as a white solid (550 mg, 37.8% yield).

### Step 9: Synthesis of Compound 8-4

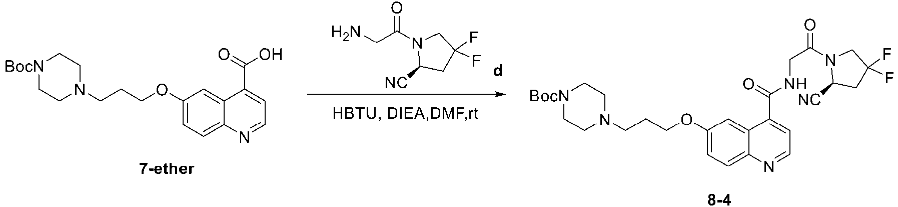

At room temperature, Compound **7-ether** (1.15 g, 2.7 mmol) and Compound **d** (757 mg, 3.9 mmol) were dissolved in anhydrous DMF (10 mL), and DIEA (1.78 g, 13.5 mmol) and HBTU (1.3 g, 3.4 mmol) were added. The resulting reaction solution was stirred at room temperature for 1 hour. After the reaction was complete, the reaction solution was diluted with ethyl acetate (50 mL), washed with water (50 mL × 2) and saturated brine (50 mL × 2), respectively, and dried over anhydrous sodium sulfate, and the organic phase was concentrated and dried. The resulting residue was purified by silica gel column chromatography (eluent: dichloromethane/methanol = 10/1) to afford Compound **8-4** as a white solid (780 mg, 48.1% yield).

### Step 10: Synthesis of Compound 9-4

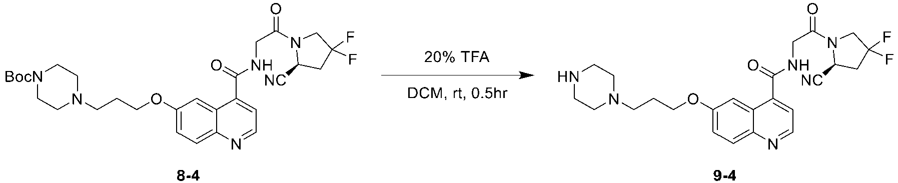

At room temperature, Compound **8-4** (240 mg) was dissolved in anhydrous dichloromethane (5.0 mL), followed by addition of trifluoroacetic acid (1 mL). The resulting reaction solution was stirred at room temperature for 0.5 hours. After the reaction was complete, the reaction solution was diluted with dichloromethane (25 mL), and the solvent was removed by rotary evaporation to yield Compound **9-4** as a reddish-brown oil (199 mg, 100% yield), which was directly used in the next step without further purification.

### Step 11: Synthesis of Compound 10

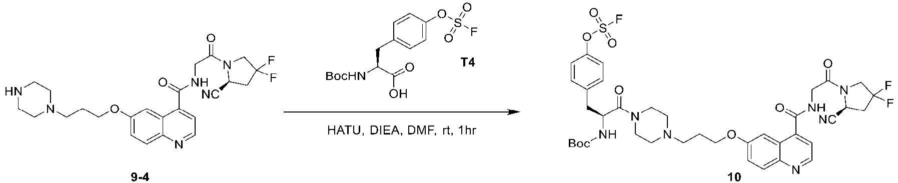

At room temperature, Compound **9-4** (199 mg, 40.9 µmol) and Compound **T4** (178.3 mg, 49.1 µmol) were dissolved in anhydrous DMF (10 mL), then DIEA (158.2 mg, 122.7 µmol) and HBTU (186.1 mg, 49.1 mmol) were added. The resulting reaction solution was stirred at room temperature for 1 hour. After the reaction was complete, the reaction solution was diluted with ethyl acetate (50 mL), washed with water (50 mL × 2) and saturated brine (50 mL × 2), respectively, and dried over anhydrous sodium sulfate, and the organic phase was concentrated and dried. The resulting residue was purified by silica gel column chromatography (eluent: dichloromethane/methanol = 10/1) to afford Compound **10** as a white solid (231 mg, 67.9% yield).

### Step 12: Synthesis of Compound 11

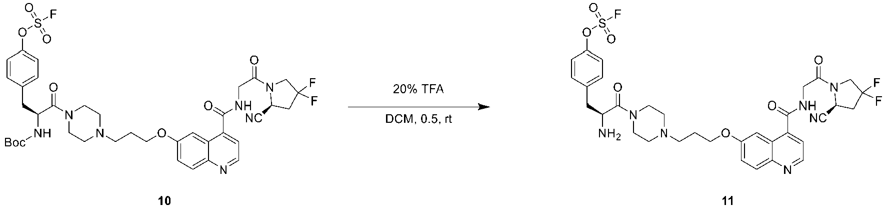

At room temperature, Compound **10** (130 mg, 156.2 µmol) was dissolved in anhydrous dichloromethane (5.0 mL), followed by addition of trifluoroacetic acid (1 mL). The resulting reaction solution was stirred at room temperature for 0.5 hours. After the reaction was complete, the reaction solution was diluted with dichloromethane (25 mL), and the solvent was removed by rotary evaporation to afford Compound 11 as a reddish-brown oil (114.3 mg, 100% yield). The compound was used directly in the next step without further purification.

### Step 13: Synthesis of Compound S12

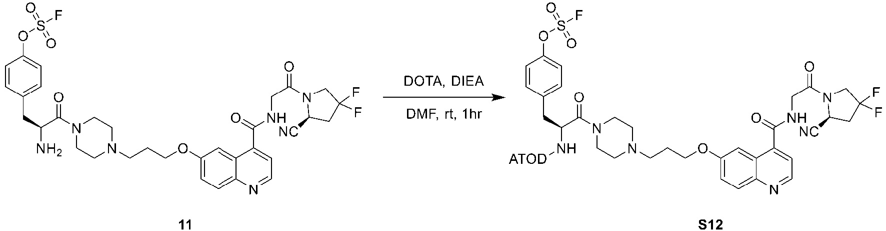

At room temperature, Compound **11** (114.3 mg, 156.2 µmol) and DOTA-NHS (156.5 mg, 312.4 µmol) were dissolved in anhydrous DMF (3.0 mL), and DIEA (60.2 mg, 459.6 µmol) was added. The resulting reaction solution was stirred at room temperature for 1 hour. After the reaction was complete, the reaction solution was dried by rotary evaporated to dryness, and the residue was purified by semi-preparative liquid chromatography, to yield Compound **S12** as a white solid (31.1 mg, 98.0% purity, 17.8% yield). LCMS: [M+H]⁺= 1119.1.

The ¹H-NMR data for Compound S12 are as follows: ¹H NMR (400 MHz, Deuterium Oxide) δ 8.77 (d, *J* = 4.5 Hz, 1H), 8.01 (d, *J* = 9.3 Hz, 1H), 7.63 (d, *J* = 4.5 Hz, 1H), 7.61 (d, *J* = 2.9 Hz, 1H), 7.50 (dd, *J* = 9.3*,* 2.7 Hz, 1H), 7.46 (s, 3H), 5.17 (dd, *J* = 8.7, 4.2 Hz, 1H), 5.05 (t, *J=* 8.1 Hz, 1H), 4.35 (d, *J =* 4.2 Hz, 2H), 4.32 - 4.09 (m, 4H), 3.87 - 3.62 (m, 6H), 3.53 - 3.27 (m, 12H), 3.27 - 3.16 (m, 3H), 3.10 (s, 4H), 3.06 - 2.92 (m, 5H), 2.81 - 2.70 (m, 2H), 2.31 - 2.17 (m, 2H).

Compounds **S8, S9, S13,** and **S14** were prepared by referring the synthetic method for Compound **S12.**

The structural formula of Compound **S8** is as follows:

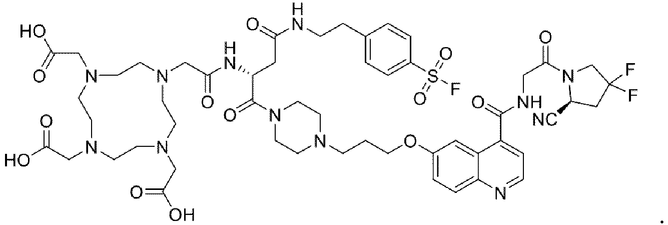

The mass spectrometry and ¹H-NMR data for Compound **S8** are as follows:
LC-MS: [M+H]⁺ : 1174.49
HR MS (ESI): [M+2H]²⁺/2: Calculated value: 587.23590, Measured value: 587.2357.
¹H NMR (400 MHz, Deuterium Oxide) δ 8.78 (d, *J =* 4.5 Hz, 1H), 8.00 (d, *J =* 9.2 Hz, 1H), 7.95 (d, *J* = 8.2 Hz, 2H), 7.64 (dd, *J =* 4.1, 2.4 Hz, 1H), 7.62 (d, *J =* 2.8 Hz, 1H), 7.57 - 7.48 (m, 3H), 5.17 (dd, *J =* 8.8, 4.1 Hz, 1H), 5.03 - 4.96 (m, 1H), 4.36 (d, *J* = 4.2 Hz, 2H), 4.34 - 4.25 (m, 3H), 4.24 - 4.11 (m, 2H), 3.89 - 3.71 (m, 5H), 3.59 - 3.30 (m, 18H), 3.16 - 2.98 (m, 7H), 2.98 - 2.87 (m, 4H), 2.75 - 2.61 (m, 2H), 2.42 - 2.28 (m, 2H).

Meanwhile, referring to step 5 of the synthesis of Compound **S2,** S30 was further prepared from Compound **S8:**

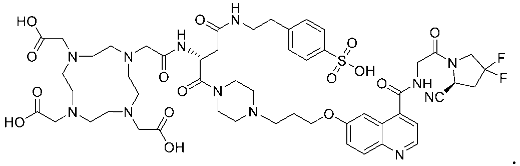

The structural formula of Compound **S9** is as follows:

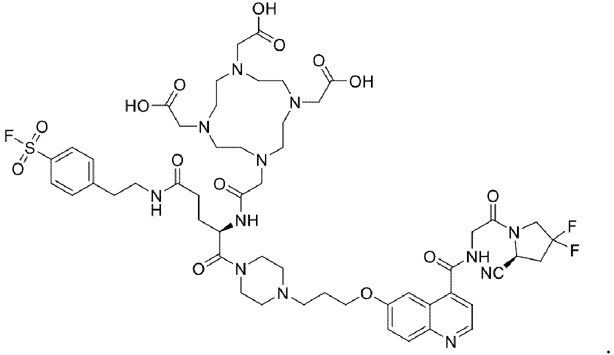

The mass spectrometry and ¹H-NMR data for Compound **S9** are as follows:
LC-MS: [M+H]⁺ : 1188.42
HR MS (ESI): [M+H+K]²⁺:Calculated value: 613.22167, Measured value: 613.2185.
¹H NMR (400 MHz, Deuterium Oxide) δ 8.78 (d, *J =* 4.5 Hz, 1H), 8.01 (d, *J =* 9.3 Hz, 1H), 7.96 (d, *J =* 8.5 Hz, 2H), 7.63 (t, *J =* 3.6 Hz, 2H), 7.56 - 7.48 (m, 3H), 5.18 (dd, *J =* 8.6, 4.3 Hz, 1H), 4.68 - 4.59 (m, 1H), 4.39 - 4.25 (m, 5H), 4.25 - 4.08 (m, 2H), 3.90 - 3.71 (m, 5H), 3.60 - 3.30 (m, 18H), 3.18 - 2.99 (m, 7H), 2.99 - 2.88 (m, 4H), 2.42 - 2.33 (m, 2H), 2.29 (t, *J* = 7.5 Hz, 2H), 1.95 - 1.73 (m, 2H).

The structural formula of Compound **S13** is as follows:

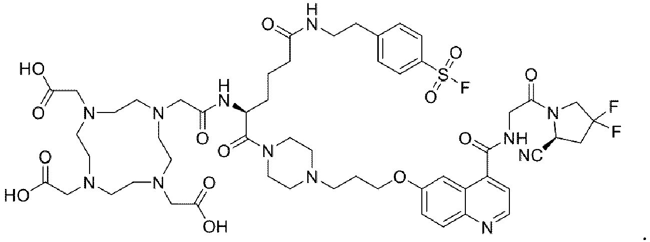

The mass spectrometry and ¹H-NMR data for Compound **S13** are as follows:
LC-MS: [M+H]⁺: 1202.62
HR MS (ESI): Calculated value of [M+H]⁺: 1201.49583, Measured value: 1201.49708.
¹H NMR (400 MHz, Deuterium Oxide) δ 8.76 (d, *J =* 4.5 Hz, 1H), 7.96 (d, *J =* 9.3 Hz, 1H), 7.89 (d, *J =* 8.1 Hz, 2H), 7.62 (d, *J =* 4.5 Hz, 1H), 7.58 (d, *J =* 2.8 Hz, 1H), 7.50 (d, *J =* 8.3 Hz, 2H), 7.46 (dd, *J =* 9.3, 2.7 Hz, 1H), 5.17 (dd, *J =* 8.7, 4.1 Hz, 1H), 4.74 - 4.68 (m, 1H), 4.35 (d, *J* = 5.0 Hz, 2H), 4.33 - 4.25 (m, 3H), 4.25 - 4.11 (m, 2H), 3.88 - 3.71 (m, 5H), 3.63 - 3.39 (m, 15H), 3.39 - 3.29 (m, 4H), 3.21 - 3.03 (m, 7H), 3.03 - 2.86 (m, 5H), 2.41 - 2.28 (m, 2H), 2.24 - 2.12 (m, 2H), 1.65 - 1.44 (m, 4H).

The structural formula of Compound **S14** is as follows:

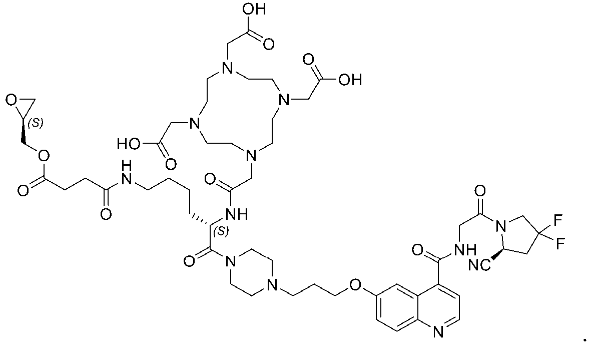

The mass spectrometry and ¹H-NMR data for Compound **S14** are as follows:
LC-MS: [M+H]⁺: 1158.71
¹H NMR (400 MHz, Deuterium Oxide) δ 8.97 (d, *J =* 5.2 Hz, 1H), 8.19 (d, *J =* 9.3 Hz, 1H), 8.00 (d, *J* = 5.2 Hz, 1H), 7.80 - 7.70 (m, 2H), 4.65 (dd, *J =* 9.4, 5.6 Hz, 2H), 4.44 (s, 1H), 4.41 - 4.33 (m, 3H), 4.27 - 4.19 (m, 2H), 4.18 - 4.09 (m, 2H), 4.09 - 4.03 (m, 1H), 3.88 - 3.65 (m, 8H), 3.62 - 3.30 (m, 13H), 3.25 - 2.94 (m, 10H), 2.91 - 2.80 (m, 2H), 2.75 (s, 3H), 2.60 - 2.49 (m, 2H), 2.43 - 2.35 (m, 2H), 1.77 - 1.67 (m, 3H), 1.60 - 1.52 (m, 2H), 1.38 (s, 2H), 1.33 (s, 4H), 1.31 - 1.23 (m, 2H).

### 9. Preparation Example 9: Synthesis Method and Synthesis Route of Compound S18

### Synthesis Route of S18

### Step 1: Synthesis of Compound M2-1

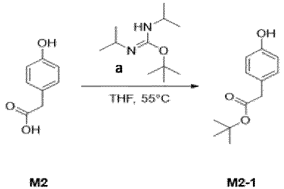

At room temperature, Compound **M2** (3.0 g, 19.7 mmol) was dissolved in anhydrous tetrahydrofuran (30.0 mL), followed by addition of Compound **a** (11.8 g, 59.1 mmol). The resulting reaction solution was stirred at 55°C for 3 hours. After the reaction was complete, the reaction solution was diluted with ethyl acetate (50 mL),and the organic phase was washed with saturated brine (100 mL×2), and dried by rotary evaporated to dryness. The resulting residue was purified by silica gel column chromatography (eluent: petroleum ether/ethyl acetate = 5/1) to afford Compound **M2-1** as a white solid (3.28 g, 80% yield).

### Step 2: Synthesis of Compound M2-2

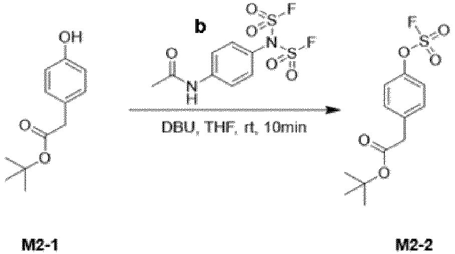

At room temperature, Compound **M2-1** (600 mg, 2.8 mmol) and Compound **b** (1.05 g, 3.3 mmol) were dissolved in anhydrous tetrahydrofuran, then DBU (936.3 mg, 6.1 mmol) was added dropwise. The resulting reaction solution was stirred at room temperature for 10 min. After the reaction was complete, the reaction solution was diluted with ethyl acetate (50 mL), washed with 1 M dilute hydrochloric acid (50 mL × 2), and the organic phase was concentrated. The resulting residue was purified by silica gel column chromatography (eluent: petroleum ether/ethyl acetate = 5/1) to afford Compound **M2-2** as a white solid (850.2 mg, 100% yield).

### Step 3: Synthesis of Compound M2-3

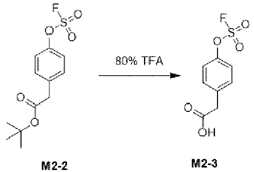

At room temperature, Compound **M2-2** (850.2 mg, 2.9 mmol) was dissolved in anhydrous DCM (10.0 mL), and trifluoroacetic acid (2.0 mL) was added. The resulting reaction solution was stirred at room temperature for 0.5 hours. After the reaction was complete, the solution was diluted with dichloromethane (25 mL) and concentrated under reduced pressure to yield Compound **M2-3** as a brown oil (673.5 mg, 100% yield), which was used directly in the next step without further purification.

### Step 4: Synthesis of Compound 8

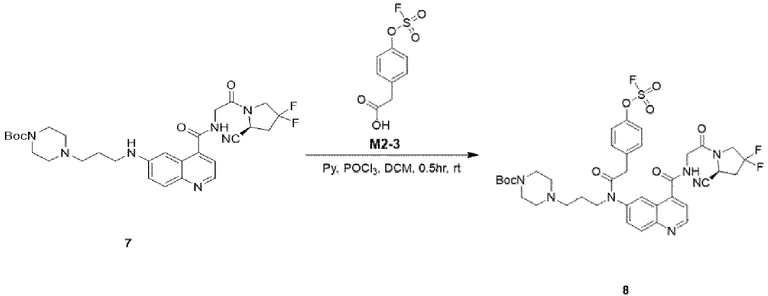

At room temperature, Compound **7** (100.0 mg, 170.4 µmol) and Compound **M2-3** (77.9 mg, 255.9 µmol) were dissolved in anhydrous dichloromethane (10 mL), then pyridine (672.9 mg, 8.5 mmol) and POCl₃(130.0 mg, 853.0 µmol) were added. The resulting reaction solution was stirred at room temperature for 0.5 hours. After the reaction was complete, the solution was diluted with dichloromethane (50 mL), washed with water (15 mL × 2) and saturated brine (15 mL × 2), respectively, and the organic phase was dried by rotary evaporated to dryness. The resulting residue was purified by semi-preparative liquid chromatography, and the resulting fraction was lyophilized to yield Compound **8** as a yellow solid powder (35 mg, 25.6% yield). LCMS: [M+H]⁺= 874.4.

### Step 5: Synthesis of Compound 9

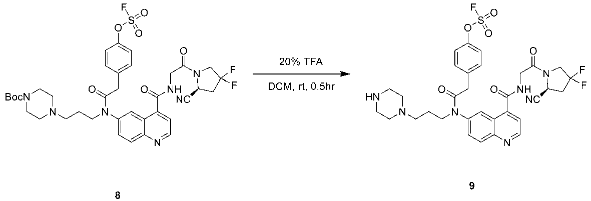

At room temperature, Compound **8** (35 mg, 59.6 µmol) was dissolved in anhydrous dichloromethane (5.0 mL), followed by addition of trifluoroacetic acid (1.0 mL). The resulting reaction solution was stirred at room temperature for 0.5 hours. After the reaction was complete, the reaction solution was diluted with dichloromethane (25 mL), and rotary evaporated to dryness to afford Compound **9** as a reddish-brown oil (27.0 mg, 100% yield). The compound was used directly in the next step without further purification. LCMS: 20-95% B 4 min, RT = 2.56 min, [M+H]⁺ = 703.2.

### Step 6: Synthesis of Compound S18

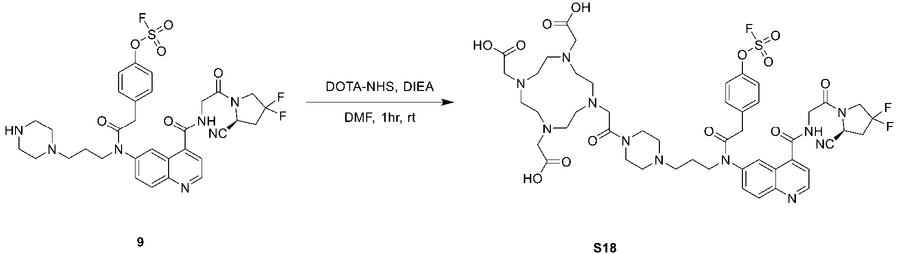

At room temperature, Compound **9** (30.7 mg, 43.7 µmol) and DOTA-NHS (43.7 mg, 87.4 µmol) were dissolved in anhydrous DMF (3.0 mL), and DIEA (16.9 mg, 131.1 µmol) was added. The resulting reaction solution was stirred at room temperature for 1 hour. After the reaction was complete, the reaction solution was rotary evaporated to dryness, and the residue was purified by semi-preparative liquid chromatography to afford Compound **S18** as a white solid (19.1 mg, 98.9% purity, 47.2% yield). LCMS: [M+H]⁺ = 1089.2.

The ¹H-NMR data for Compound **S18** are as follows: ¹H NMR (400 MHz, Deuterium Oxide) δ 9.04 (d, *J* = 4.5 Hz, 1H), 8.17 (d, *J* = 9.0 Hz, 1H), 8.09 (s, 1H), 7.79 (d, *J =* 4.5 Hz, 1H), 7.73 - 7.68 (m, 1H), 7.24 (d, *J =* 8.3 Hz, 2H), 7.05 (d, *J =* 8.3 Hz, 2H), 5.17 (dd, *J =* 8.5, 4.1 Hz, 1H), 4.41 - 4.28 (m, 3H), 4.27 - 4.12 (m, 2H), 4.08 - 3.96 (m, 2H), 3.87 - 3.72 (m, 7H), 3.69 (s, 2H), 3.65 (s, 2H), 3.57 - 3.48 (m, 4H), 3.46 - 3.36 (m, 6H), 3.32 - 3.21 (m, 3H), 3.19 - 2.93 (m, 13H), 2.10 - 2.00 (m, 2H).

Compounds **S19** and **S20** were prepared by referring to the synthetic method for Compound **S18.**

The structural formula of Compound **S19** is as follows:

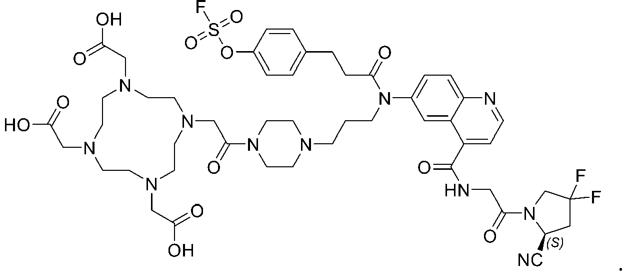

The mass spectrometry and ¹H-NMR data for Compound **S19** are as follows:
LC-MS: [M+H]⁺: 1103.63
HR MS (ESI): calculated value of [M+H]⁺: 1102.42741, measured value: 1102.42822.
¹H NMR (400 MHz, Deuterium Oxide) δ 9.01 (d, *J* = 4.5 Hz, 1H), 8.16 (d, *J* = 8.9 Hz, 1H), 7.95 (s, 1H), 7.76 (d, *J* = 4.5 Hz, 1H), 7.40 (dd, *J* = 8.8, 2.3 Hz, 1H), 7.33 (d, *J* = 8.5 Hz, 2H), 7.15 (d, *J* = 8.7 Hz, 2H), 5.15 (dd, *J =* 8.3, 4.3 Hz, 1H), 4.45 - 4.38 (m, 1H), 4.35 - 4.26 (m, 2H), 4.23 - 4.13 (m, 1H), 4.08 - 3.95 (m, 1H), 3.94 - 3.62 (m, 10H), 3.58 - 3.33 (m, 11H), 3.30 - 3.09 (m, 10H), 3.09 - 2.90 (m, 6H), 2.87 (t, *J =* 6.9 Hz, 2H), 2.60 - 2.52 (m, 2H), 1.97 - 1.86 (m, 2H).

The structural formula of Compound S20 is as follows:

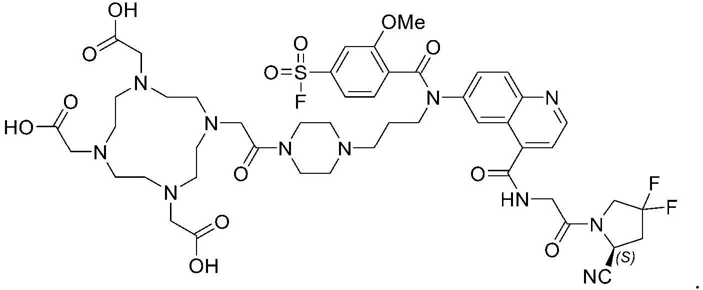

The mass spectrometry and ¹H-NMR data for Compound **S20** are as follows:
LC-MS: [M+H]⁺: 1089.56
HR MS (ESI): Calculated value of [M+H]⁺: 1088.41176; Measured value: 1088.41237.
¹H NMR (400 MHz, Deuterium Oxide) δ 9.04 (d, *J =* 4.5 Hz, 1H), 8.17 (d, *J =* 9.0 Hz, 1H), 8.09 (s, 1H), 7.79 (d, *J* = 4.5 Hz, 1H), 7.70 (d, *J =* 7.6 Hz, 1H), 7.24 (d, *J =* 8.3 Hz, 2H), 7.05 (d, *J* = 8.3 Hz, 2H), 5.17 (dd, *J* = 8.5, 4.1 Hz, 1H), 4.41 - 4.28 (m, 3H), 4.27 - 4.12 (m, 2H), 4.08 - 3.96 (m, 2H), 3.87 - 3.72 (m, 7H), 3.69 (s, 2H), 3.65 (s, 2H), 3.57 - 3.48 (m, 4H), 3.46 - 3.36 (m, 6H), 3.32 - 3.21 (m, 3H), 3.19 - 2.93 (m, 13H), 2.10 - 2.00 (m, 2H).

### 10. Preparation Example 10

### Synthesis Method and Synthesis Route of Compound S22

### Synthesis steps of S22:

### Step 1: Synthesis of Compound M2-4

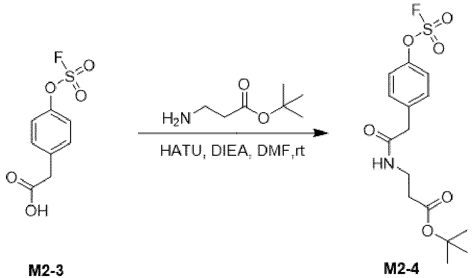

At room temperature, Compounds **M2-3** (400 mg, 1.7 mmol) was dissolved in anhydrous DMF (10 mL), and tert-butyl 3-aminopropionate (296.2 mg, 2.0 mmol), DIEA (657.9 mg, 5.1 mmol), and HATU (760 mg, 2.0 mmol) were added in sequence. The resulting reaction solution was stirred at room temperature for 1 hour. After the reaction was complete, the solution was diluted with ethyl acetate (50 mL), washed with water (50 mL × 2) and saturated brine (50 mL × 2), respectively, and dried, and the organic phase was concentrated. The resulting residue was purified by silica gel column chromatography (eluent: petroleum ether/ethyl acetate = 3/1) to afford Compound **M2-4** as a white solid (550.2 mg, 89% yield).

### Step 2: Synthesis of Compound M2-5

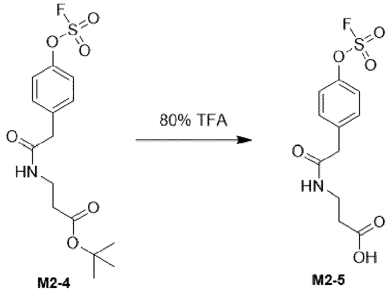

At room temperature, Compound **M2-4** (550.2 mg, 1.5 mmol) was dissolved in anhydrous DCM (2 mL), and trifluoroacetic acid (8.0 mL) was added. The resulting reaction solution was stirred at room temperature for 0.5 hours. After the reaction was complete, the solution was diluted with dichloromethane (25 mL), concentrated under reduced pressure, diluted with ethyl acetate (50 mL), and washed with water (50 mL × 2) and saturated brine (50 mL × 2), respectively, and dried to afford, Compound **M2-5** as a colorless oil (350 mg, 75.3% yield), which was used directly in the next step without further purification.

Compound **M2-5:** ¹H NMR (400 MHz, Chloroform-*d*) δ 7.43 (d, *J =* 8.7 Hz, 2H), 7.37 (d, *J* = 8.6 Hz, 2H), 6.19 (s, 1H), 3.63 (s, 2H), 3.57 (q, *J* = 5.9 Hz, 2H), 2.64 (dd, *J* = 11.4, 2.9 Hz, 2H).

### Step 6: Synthesis of Compound 8-1

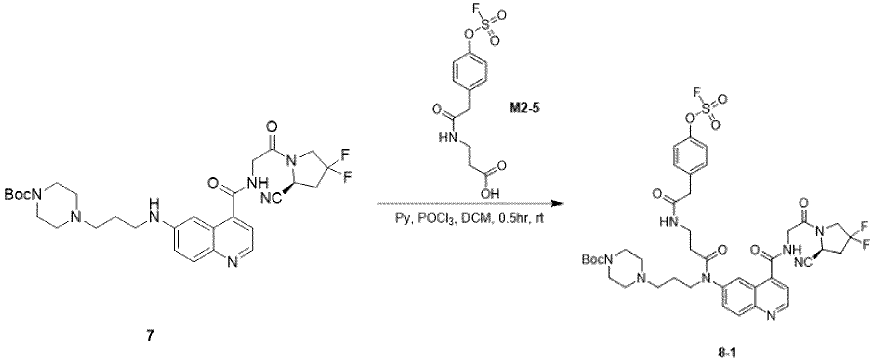

At room temperature, Compound **7** (100.0 mg, 170.6 µmol) and Compound **M2-5** (77.9 mg, 255.9 µmol) were dissolved in anhydrous dichloromethane (10 mL), then pyridine (672.9 mg, 8.5 mmol) and POCl₃(130.0 mg, 853.0 µmol) were added. The resulting reaction solution was stirred at room temperature for 0.5 hours. After the reaction was complete, the solution was diluted with dichloromethane (50 mL), washed with water (15 mL × 2) and saturated saline (15 mL × 2), respectively, and the organic phase was rotary evaporated to dryness. The resulting residue was purified by semi-preparative liquid chromatography, and the resulting fraction was lyophilized to yield Compound **8-1** as a yellow solid powder (35 mg, 23.5% yield). LCMS: [M+H]⁺= 874.4.

### Step 7: Synthesis of Compound 9-1

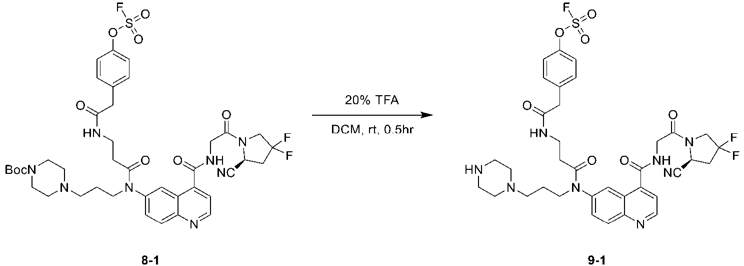

At room temperature, Compound **8-1** (30.0 mg, 34.3 µmol) was dissolved in anhydrous dichloromethane (5.0 mL), followed by addition of trifluoroacetic acid (1.0 mL). The resulting reaction solution was stirred at room temperature for 0.5 hours. After the reaction was complete, the reaction solution was diluted with dichloromethane (25 mL), and the solvent was removed by rotary evaporation to afford Compound **9-1** as a reddish-brown oil (27.0 mg, 100% yield). The compound was used directly in the next step without further purification. LCMS: 20-95% B for 4 min, RT = 2.56 min, [M+H]⁺= 774.1.

### Step 8: Synthesis of Compound S22

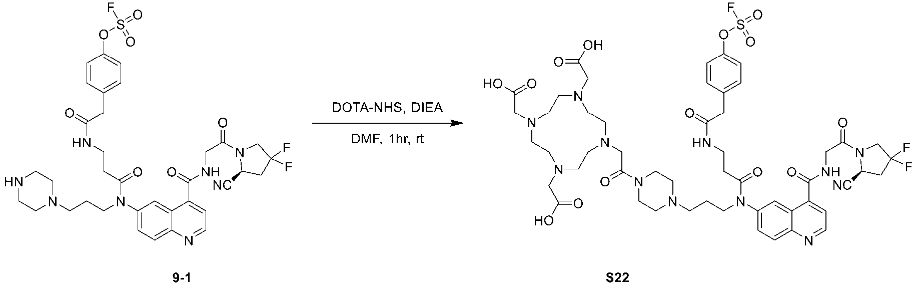

At room temperature, Compound **9-1** (27.0 mg, 30.9 µmol) and DOTA-NHS (30.9 mg, 61.8 µmol) were dissolved in anhydrous DMF (3.0 mL), and DIEA (13 mg, 92.7 µmol) was added. The resulting reaction solution was stirred at room temperature for 1 hour. After the reaction was complete, the reaction solution was dried by rotary evaporated to dryness, and the residue was purified by semi-preparative liquid chromatography to afford Compound **S22** as a white solid (19.1 mg, 98.9% purity, 47.2% yield). LCMS: [M+H]⁺= 1160.2.

The ¹H-NMR data for Compound **S22** are as follows: ¹H NMR (400 MHz, Deuterium Oxide) δ 9.02 (d, *J* = 4.5 Hz, 1H), 8.21 - 8.16 (m, 2H), 7.77 (d, *J* = 4.5 Hz, 1H), 7.49 - 7.43 (m, 3H), 7.42 - 7.37 (m, 2H), 5.15 - 5.08 (m, 1H), 4.40 - 4.23 (m, 4H), 4.20 - 4.07 (m, 2H), 3.88 - 3.69 (m, 9H), 3.54 (s, 2H), 3.52 - 3.34 (m, 14H), 3.24 - 3.09 (m, 10H), 3.08 - 2.97 (m, 3H), 2.96 - 2.88 (m, 2H), 2.41 (t, *J* = 6.6 Hz, 2H), 2.02 - 1.92 (m, 2H).

Compounds **S21, S23,** and **S24** were prepared by referring to the synthetic method for Compound **S22.**

The structural formula of Compound **S21** is as follows:

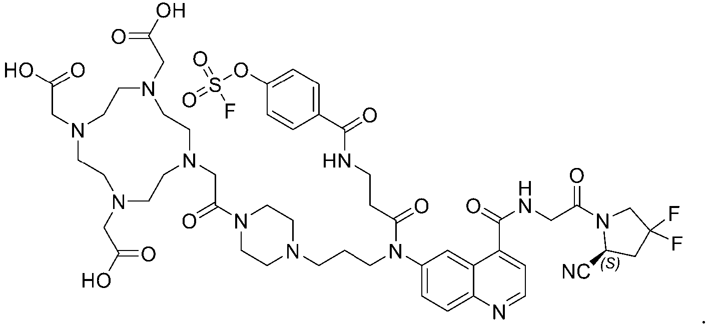

The mass spectrometry and ¹H-NMR data for Compound **S21** are as follows:
LC-MS: [M+H]⁺: 1146.71
HR MS (ESI): calculated value of [M+H]⁺: 1145.43323, measured value: 1145.43469.
¹H NMR (400 MHz, Deuterium Oxide) δ 9.01 (d, *J =* 4.5 Hz, 1H), 8.24 (d, *J =* 2.2 Hz, 1H), 8.16 (d, *J* = 8.9 Hz, 1H), 7.80 - 7.70 (m, 4H), 7.56 (d, *J =* 8.6 Hz, 2H), 5.07 (dd, *J =* 8.6, 4.0 Hz, 1H), 4.39 - 4.21 (m, 4H), 4.20 - 4.07 (m, 2H), 4.06 - 3.87 (m, 3H), 3.79 (q, *J =* 16.3 Hz, 6H), 3.69 (s, 2H), 3.56 (t, *J =* 6.3 Hz, 2H), 3.51 - 3.36 (m, 10H), 3.27 - 3.10 (m, 10H), 3.07 - 2.97 (m, 3H), 2.95 - 2.85 (m, 2H), 2.61 (t, *J* = 6.1 Hz, 2H), 2.05 - 1.93 (m, 2H).

Compound **S23** was prepared by referring to the synthetic steps for Compound **S22.**

The structural formula of Compound **S23** is as follows:

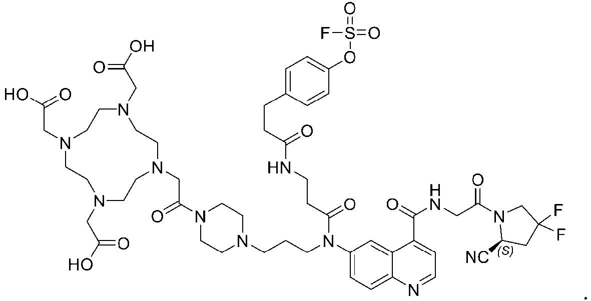

The mass spectrometry and ¹H-NMR data for Compound **S23** are as follows:
LC-MS: [M+H]⁺ : 1174.91
HR MS (ESI): Calculated value for [M+H]⁺: 1173.46453, Measured value: 1173.46594.
¹H NMR (400 MHz, Deuterium Oxide) δ 9.07 (d, *J* = 4.5 Hz, 1H), 8.30 (d, *J* = 9.0 Hz, 1H), 8.22 (d, *J =* 2.3 Hz, 1H), 7.82 (d, *J* = 4.5 Hz, 1H), 7.74 (dd, *J =* 9.0, 2.3 Hz, 1H), 7.16 (d, *J =* 8.7 Hz, 2H), 7.09 (d, *J =* 8.5 Hz, 2H), 5.14 (dd, *J =* 8.3, 4.3 Hz, 1H), 4.41 - 4.23 (m, 4H), 4.21 - 4.05 (m, 2H), 3.80 (q, *J =* 16.3 Hz, 7H), 3.70 (s, 2H), 3.55 - 3.36 (m, 12H), 3.26 - 3.11 (m, 12H), 3.08 - 2.98 (m, 3H), 2.98 - 2.89 (m, 2H), 2.83 (t, *J* = 7.0 Hz, 2H), 2.43 (t, *J =* 7.0 Hz, 2H), 2.13 - 2.05 (m, 2H), 2.04 - 1.94 (m, 2H).

The structural formula of Compound **S24** is as follows:

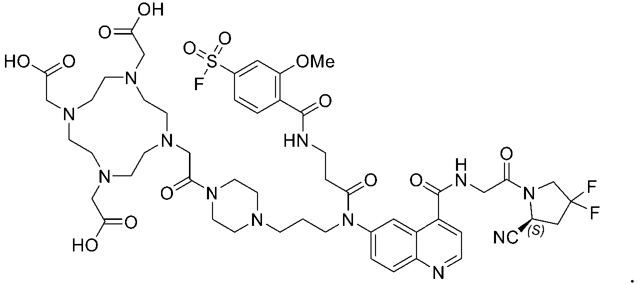

The mass spectrometry and ¹H-NMR data for Compound **S24** are as follows:
LC-MS: [M+H]⁺: 1160.70
HR MS (ESI): Calculated value of [M+H]⁺: 1159.44888, Measured value: 1159.44874
¹H NMR (400 MHz, Deuterium Oxide) δ 8.99 (d, *J =* 4.5 Hz, 1H), 8.26 - 8.17 (m, 3H), 8.12 (d, *J* = 8.9 Hz, 1H), 7.75 (dd, *J =* 9.4, 3.2 Hz, 2H), 7.41 (d, *J =* 9.7 Hz, 1H), 5.05 (d, *J =* 8.8 Hz, 1H), 4.37 - 4.19 (m, 4H), 4.18 - 4.07 (m, 2H), 4.00 (s, 3H), 3.80 (q, *J=* 16.3 Hz, 6H), 3.69 (s, 2H), 3.61 - 3.53 (m, 3H), 3.52 - 3.33 (m, 11H), 3.31 - 3.09 (m, 11H), 3.08 - 2.96 (m, 3H), 2.92 - 2.81 (m, 2H), 2.67 - 2.59 (m, 2H), 2.04 - 1.95 (m, 2H).

### 11. Preparation Example 11: Synthesis of Compound C12:

Compound C12 was prepared by referring to the synthesis steps for S12 in Preparation Example 8,

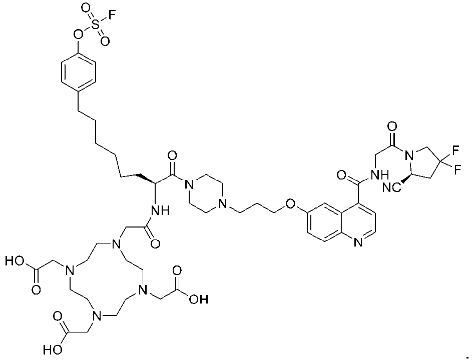

### 12. Preparation Example 12: Synthesis of Compound C3-3:

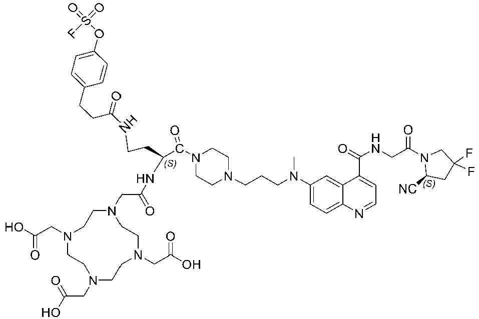

The synthetic route for Compound **C3-3** is as follows:

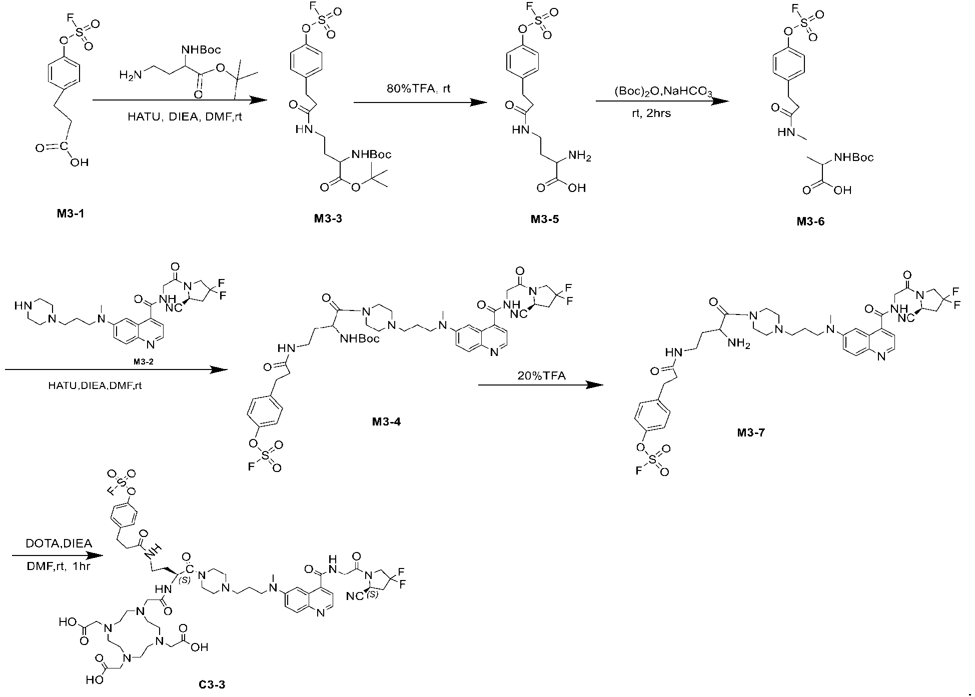

### 13. Preparation Example 13: Synthesis of Compound C2-1

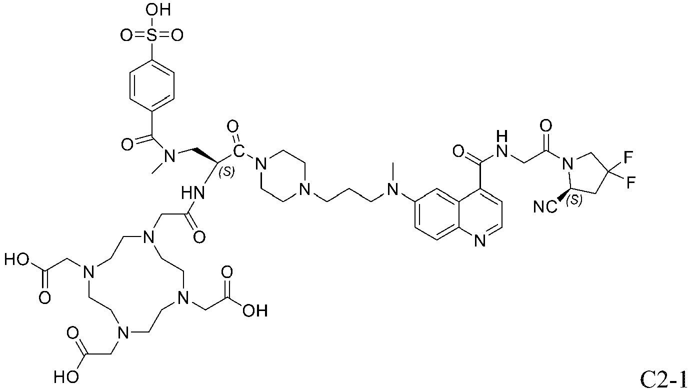

Compound **C2-1** was prepared by referring to the synthetic route for Compound **C3-3**.

### III. Efficacy Examples

### 1. Enzyme Inhibition Test

### 1.1 Enzyme Inhibition Test Method

1) The compounds were dissolved in DMSO to prepare a stock concentration and then diluted to 300 nM with fibroblast activation buffer FAB.
2) 2 µL of each compound (300 nM) was added to 38 µL of FAB and diluted to 15 nM. Each compound was diluted from 15 nM to 3.662 pM using a two-fold serial dilution method in 384-well plate, with 20 µL of compound per well.
3) 20 µL of FAP (2 ng) was added to the wells described in 2), and incubated at room temperature for 30 minutes.
4) 20 µL of FAP substrate (60 µM) was added to the wells in 3), and incubated at 37°C for 25 hours.
5) Inhibitor-free control (NIC) was used as positive control.
6) The following negative controls were added to each group of compounds:
   Protein-free control (NPC): 20 µL compound +20 µL FAB +20 µL FAP substrate;
   Protein pure control (POC): 20 µL FAP +40 µL FAB.
7) Measurements were repeated.
8) The emission spectrum was read at 465 nm (excitation wavelength of 360 nm) for 10 ms using a Molecular Devices SpectraMax iDS microplate reader.

### 1.2 Enzyme Inhibition Test Results

**Table 1: IC₅₀ values of compounds against kinase at 24 h**

| No. | Compound | IC₅₀ values of compounds against kinase at 24 h (nM) |
|---|---|---|
| 1 | S1 | 0.3286 |
| 2 | S18 | 0.1532 |
| 3 | S19 | 0.8283 |
| 4 | S20 | 0.2222 |
| 5 | S2 | 0.3328 |
| 6 | S3 | 0.1955 |
| 7 | S21 | 0.3672 |
| 8 | S22 | 0.1398 |
| 9 | S23 | 0.1572 |
| 10 | S24 | 0.2258 |
| 11 | S4 | 0.2333 |
| 12 | S5 | 0.3687 |
| 13 | S6 | 0.3468 |
| 14 | S7 | 0.5995 |
| 15 | S15 | 0.4030 |
| 16 | S16 | 0.4753 |
| 17 | S17 | 0.5769 |
| 19 | S25 | 0.4149 |
| 20 | S26 | 0.3013 |
| 21 | S27 | 0.3820 |
| 22 | S28 | 0.4239 |
| 23 | S29 | 0.4652 |
| 24 | S0 (FAPI46) | 0.4056 |

**Table 2: IC₅₀ values of compounds against kinase at 24 h**

| No. | Compound | IC₅₀ values of compounds against kinase at 24 h (nM) |
|---|---|---|
| 25 | S8 | 0.3711 |
| 26 | S30 | 0.4858 |
| 27 | S9 | 0.5736 |
| 28 | S12 | 0.8211 |
| 29 | S13 | 0.4661 |
| 30 | S14 | 640.7 |
| 31 | S11 (FAPI04) | 0.3666 |

The structural formula of S0 is as follows:

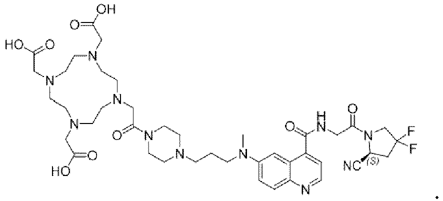

The structural formula of S11 is as follows:

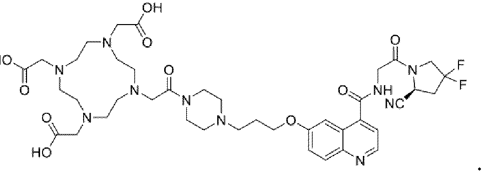

### 2. Imaging Experiment

### 2.1 Method for Labeling with Radioactive ⁶⁸Ga

1) Preparation of the precursor compound solution: an appropriate amount of the precursor compound was dissolved in DMSO to a concentration of 3 nmol/10 µL, and stored at -20°C for later use.
2) Preparation of 0.25 M ammonium acetate solution: 205.08 mg of demetallized ammonium acetate (82.03 g/mol) was dissolved in 10 mL of deionized water to obtain a 0.25 M ammonium acetate solution, and stored at 4°C for later use.
3) Activation of C18 cartridges: C18 cartridges were activated within 30 minutes prior to use by slowly injecting 5 mL of absolute ethanol and 10 mL of deionized water in sequence, then draining the liquid.
4) Elution of radioactive ⁶⁸GaCl₃: 1 mL of radioactive ⁶⁸GaCl₃ in hydrochloric acid (0.05 M), with an activity of 2 to 5 mCi, was obtained by using an automated program segmented elution.
5) Labeling and purification: 270 µL of ammonium acetate solution was added to the above ⁶⁸GaCl₃ in hydrochloric acid (0.05 M), and the pH was adjusted to 3.5 to 4.5. One tube of the prepared precursor solution was added, and the mixed solution was reacted at 95°C to 105°C for 10 to 15 minutes, and the reaction was terminated. After the reaction solution was loaded on the C18 Cartridges, free ⁶⁸Ga was removed by rinsing with 3 mL of deionized water, and then the product was gradient eluted with 500 µL of absolute ethanol. The eluate was collected and its radioactivity was measured. The absolute ethanol was volatilized and the residue was redissolved with physiological saline. Pyrogen was removed by 0.22 µm filter membrane, and an injection solution with a radioactivity concentration of 100-200 µCi/100 µL was prepared for analysis and subsequent experiments.
6) Determination of radiochemical purity: 10 µCi of the above radioactive injection solution was taken and analyzed for radiochemical purity using HPLC. The threshold of the radioactivity detector was set to 200 K. Chromatographic conditions: elute with a 5% to 95% mobile phase B gradient from 0 to 12 min, maintain 95% mobile phase B from 12 to 15 min (mobile phase A is an aqueous solution containing 0.1% TFA; mobile phase B is an acetonitrile solution containing 0.1% TFA), flow rate 1 mL/min.

The radiochemical purity of each compound labelled with ⁶⁸Ga is shown in Table 3.

**Table 3. Radioactive chemical purity after ⁶⁸Ga labeling**

| Compound | Labeling Yield | Radioactive chemical purity |
|---|---|---|
| S0 (FAPI46) | 54.30% | 97.89% |
| S18 | >95% | 98.42% |
| S19 | >95% | 97.97% |
| S2 | >95% | 94.79% |
| S3 | 89.36% | 94.79% |
| S21 | >95% | 95.60% |
| S22 | >95% | 95.22% |
| S23 | >95% | 97.04% |
| S11 (FAPI04) | >95% | 96.61% |
| S12 | >95% | 93.99% |
| S14 | >95% | 97% |
| S15 | 83% | 96% |
| S16 | 85% | 95.30% |
| S17 | 90% | 97.08% |

### 2.3 Experimental Animals and Cell Line

Species/Strain: BALB/c Nude mice
Age/Weight: 4 to 6 weeks, 15 to 18 g
FAP-transfected HT1080 cell line: Human fibrosarcoma cell line; culture medium condition: DMEM medium containing 10% fetal bovine serum and 1% penicillin-streptomycin. After cell expansion, the cells were inoculated into the right lower limb near the back of nude mice, an a dose of 1 × 10⁶ cells per mouse. The mice were ready for imaging experiments 2 to 3 weeks after tumor cell inoculation.
U87MG: Glioblastoma cell line; culture medium condition: MEM medium containing 10% fetal bovine serum and 1% penicillin-streptomycin. After cell expansion, the cells were inoculated into the right lower limb near the back of nude mice, at a dose of 1 × 10⁶ cells per mouse. The mice were ready for imaging experiments 2 to 3 weeks after tumor cell inoculation.

### 2.4 Small Animal Imaging Experimental Methods

### 2.4.1 Instrument Parameters

CT Scan and Reconstruction Protocol: CT_Conventional_Scan/CT_FDK_Recon
PET Scan and Reconstruction Protocol: PET_Conventional_Scan/PET_OSEM_Recon
Image Processing Workstation: Inveon Research Workplace

2.4.2 Drug Administration: 100-200 µCi of radioactive injection solution (100-150 µL in volume) was administered via the tail vein using a 1 mL disposable sterile syringe.

2.4.3 Anesthesia Method: Portable Anesthesia System (a multifunctional desktop anesthesia machine for small animals) was used with isoflurane gas anesthesia. (Isoflurane, specification: 100 mL; storage condition: room temperature; manufacturer: Reward Life Science Co., Ltd.). Prior to imaging, the animals were placed in an anesthesia induction box. Once anesthetized, the animals were transferred to the PET/CT animal chamber and maintained a prone position. Continuous anesthesia was maintained throughout the PET/CT scan and imaging, lasting approximately 10 to 20 minutes. The isoflurane flow rate was set to 1.5 LPM (liters per minute).

### 2.4.4 PET-CT Imaging Method

Mice bearing with subcutaneous transplanted tumor (U87 MG cells) were taken, and the tumor size was about 150 mm³. Approximately 7.4 MBq of ⁶⁸Ga-labeled compounds were injected through tail vein, respectively, and PET/CT imaging was performed at 30 min, 60 min, and 120 min after injection. The tumor imaging in each group was observed.

### 2.4.5 Biodistribution

After completing the small-animal PET scan, image reconstruction was performed. The images and data were processed using the device's built-in software, and the tumor, heart (including its contents), liver, kidneys, and muscles were delineated as regions of interest. The percentage of injected dose per gram of tissue (abbreviated as %ID/g) was obtained for each region of interest.

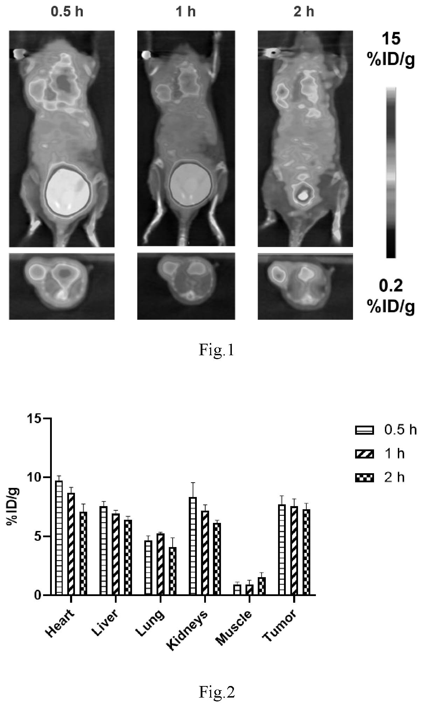

Figures 1 to 24 illustrate the PET-CT imaging results and biodistribution of each ⁶⁸Ga-labeled compound in U87MG tumor-bearing mice. The results demonstrate that the compounds provided in the examples of the present application, which incorporate a covalently attached warhead, exhibit higher tumor uptake and higher target-to-background ratio compared to ^{6g}Ga-FAPI-46 and ⁶⁸Ga-FAPI-04, thereby demonstrating superior tumor targeting performance.

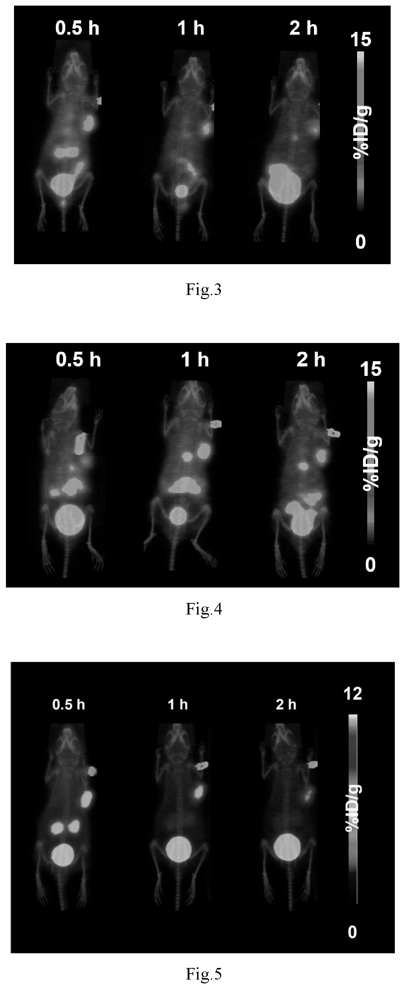

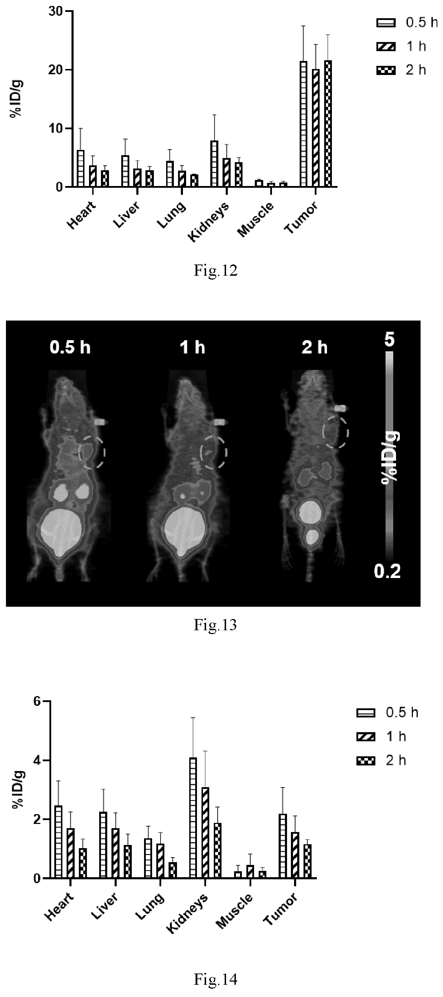

Specifically, as shown in Figure 3 vs. Figures 1 and 13, ⁶⁸Ga-S18 exhibits higher uptake in tumors and lower uptake in kidney and other organs. PET-CT images show that the tumor uptake of ⁶⁸Ga-S18 is significantly higher than that of ⁶⁸Ga-FAPI-46 and ⁶⁸Ga-FAPI-04, with an uptake value of approximately 13% ID/g at 0.5 h, demonstrating that ⁶⁸Ga-S18 possesses good tumor targeting and can be used for tumor radiodiagnosis.

Based on Figure 4 vs. Figures 1 and 13, ⁶⁸Ga-S19 follows a renal metabolic pathway and exhibits higher tumor uptake, with an uptake value of approximately 7% ID/g at 0.5 h, comparable to that of ⁶⁸Ga-S18. This indicates that ⁶⁸Ga-S19 also possesses good tumor targeting and can be used for tumor radiodiagnosis.

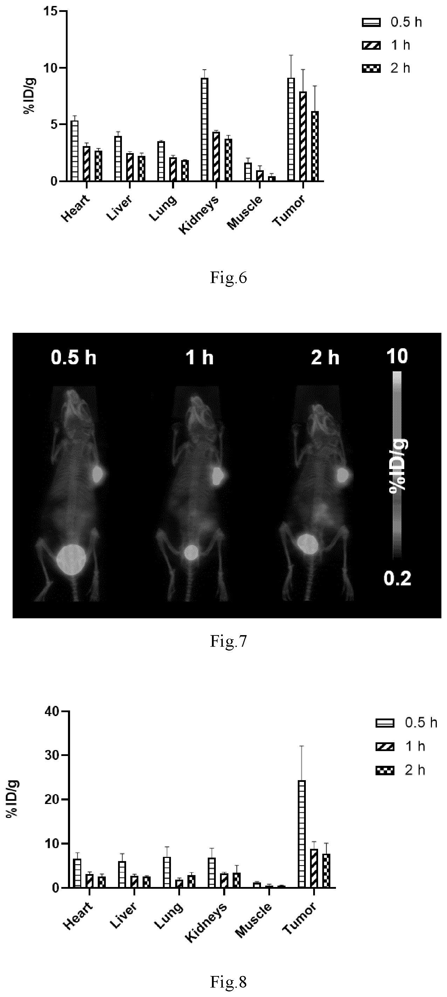

Based on Figure 5 vs. Figures 1 and 13, ⁶⁸Ga-S2 follows a renal metabolic pathway, exhibiting higher uptake in tumors and very low uptake in non-target organs. As shown in the PET-CT images, the tumor uptake of ⁶⁸Ga-S2 is significantly higher than that of ⁶⁸Ga-FAPI-46 and ⁶⁸Ga-FAPI-04, demonstrating excellent tumor-targeting performance and providing superior imaging effects. A comparison of Figure 6 with Figures 2 and 14 reveals that the tumor uptake of ⁶⁸Ga-S2 at 0.5 h was approximately 9% ID/g, comparable to that of ⁶⁸Ga-FAPI-46 (7% DccMean), and significantly higher than that of ⁶⁸Ga-FAPI-04 (2% ID/g). At the same time, ⁶⁸Ga-S2 exhibits significantly lower uptake in non-target organs compared to ⁶⁸Ga-FAPI-46. Therefore, ⁶⁸Ga-S2 provides superior PET-CT imaging quality compared to ⁶⁸Ga-FAPI-46 and ⁶⁸Ga-FAPI-04, and demonstrates excellent effect as a tumor diagnostic agent.

Based on Figures 7 to 12 vs. Figures 1, 2, 13, and 14, ⁶⁸Ga-S21, ⁶⁸Ga-S22, and ⁶⁸Ga-S23 all exhibit excellent tumor-targeting performance. As shown in the PET-CT images, all three compounds demonstrate high uptake in tumors and very low uptake in non-target organs. Tumor uptake is significantly higher than that of ⁶⁸Ga-FAPI-46 and ⁶⁸Ga-FAPI-04, presenting excellent imaging quality. According to the corresponding biodistribution data, ⁶⁸Ga-S21 has an uptake value of approximately 23% ID/g at 0.5 h, ⁶⁸Ga-S22 has an uptake value of approximately 12% ID/g at 0.5 h, and ⁶⁸Ga-S23 has an uptake value of approximately 20% ID/g at 0.5 h; all are significantly higher than those of ⁶⁸Ga-FAPI-46 and ⁶⁸Ga-FAPI-04. Furthermore, the uptake of these three compounds in non-tumor organs is very low. Additionally, PET-CT images and biodistribution data show that the retention time of these three compounds in the tumor site could reach over 2 h; ⁶⁸Ga-S22 could remain in the tumor for 2 h and maintain an uptake of 11% ID/g, while ⁶⁸Ga-S23 could remain in the tumor for 2 h and maintain an uptake of 20% ID/g, demonstrating significantly improved tumor retention capacity. By replacing ⁶⁸Ga with a therapeutic radionuclide, the radionuclide-labeled S21, S22, and S23 will be able to provide excellent tumor targeting and retention capabilities, while exhibiting extremely low toxicity to non-target organs. As anticancer agents, these three radionuclide-labeled compounds all demonstrate excellent therapeutic efficacy.

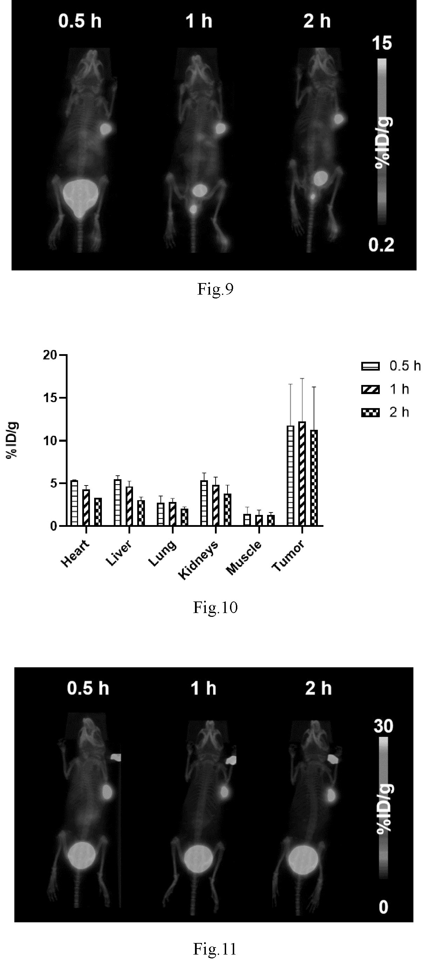

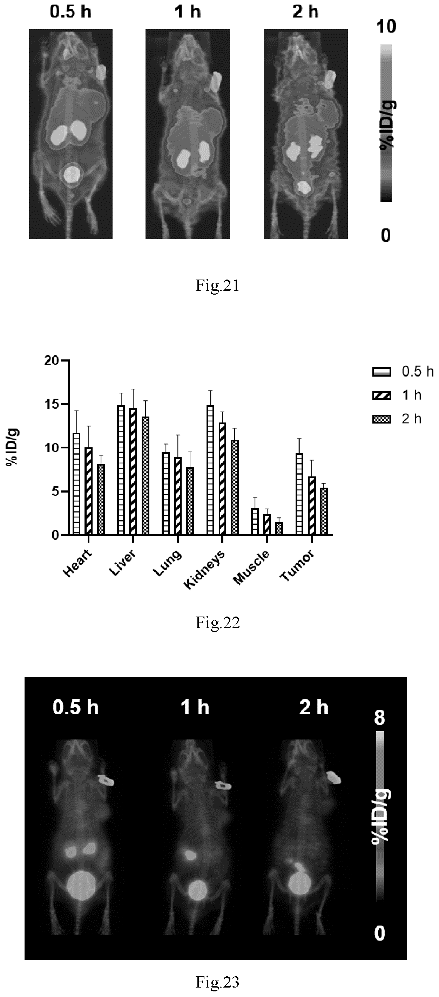

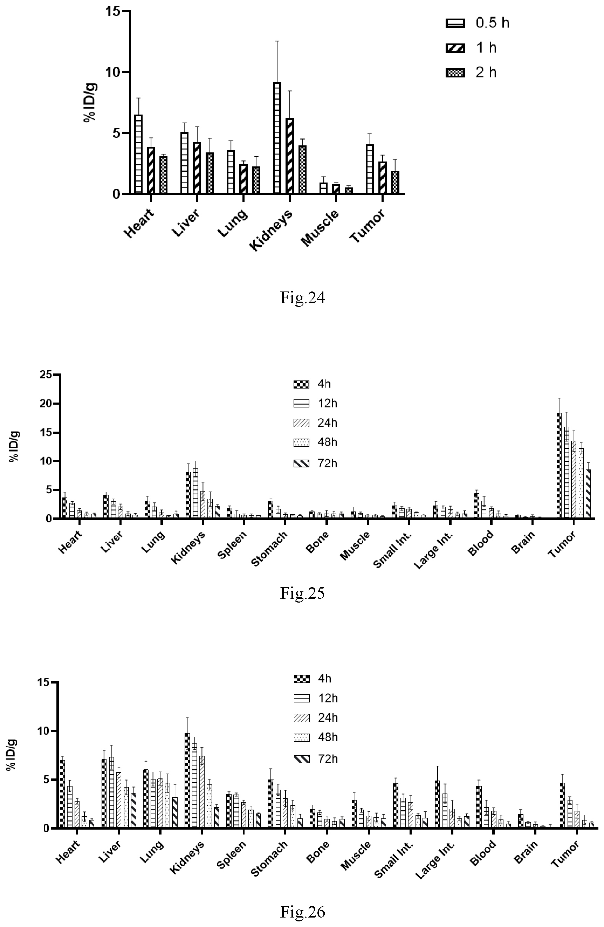

Furthermore, comparing Figures 11 and 12 with Figures 21 and 22, the tumor-targeting and tumor retention capacity of ⁶⁸Ga-S23 are significantly superior to those of ⁶⁸Ga-C3-3. Moreover, ⁶⁸Ga-C3-3 exhibits high uptake in non-target organs (such as heart, liver, and lungs), and the target-to-non-target ratio of ⁶⁸Ga-C3-3 is much higher than that of ⁶⁸Ga-S23. Comparing Figures 5 and 6 with Figures 23 and 24, the tumor uptake value of ⁶⁸Ga-S2 (with an uptake value of approximately 9% ID/g at 0.5 h) is higher than ⁶⁸Ga-C2-1 (with an uptake value of approximately 4% ID/g at 0.5 h), while the uptake value in non-target organs is lower than that of ⁶⁸Ga-C2-1. The tumor-targeting of ⁶⁸Ga-S2 is significantly superior to ⁶⁸Ga-C2-1, indicating that linking the covalent warhead to the side near the FAPI targeting group, rather than to the side near the chelating group, confers superior targeting on the compound. This significantly improves the target-to-non-target ratio and enhances tumor uptake capacity.

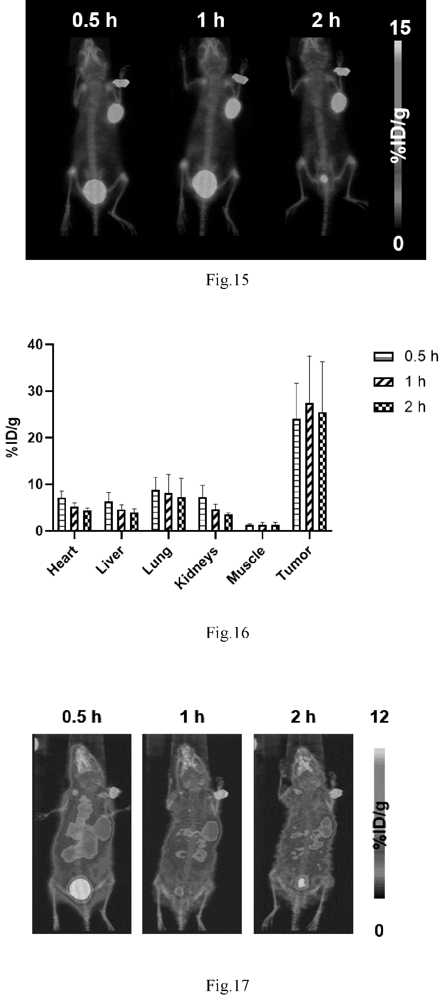

Based on Figures 15 and 16 vs. Figures 1, 2, 13, and 14, ⁶⁸Ga-S12 follows a renal metabolic pathway, exhibiting high uptake in tumors and very low uptake in non-target organs; the tumor uptake of ⁶⁸Ga-S12 at 0.5 h is approximately 21% ID/g. PET-CT images and biodistribution data show that the tumor uptake of ⁶⁸Ga-S12 is significantly higher than that of ⁶⁸Ga-FAPI46 and ⁶⁸Ga-FAPI04, indicating that ⁶⁸Ga-S 12 possesses superior tumor-targeting performance. Furthermore, PET-CT images and biodistribution data also indicate that the retention time of ⁶⁸Ga-S12 can reach 2 hours at the tumor site, showing that tumor retention capacity significantly superior to that of ⁶⁸Ga-FAPI46 and 68Ga-FAPI04. This suggests that S12 labeled with therapeutic radionuclides can provide excellent tumor targeting and tumor retention capability and is usable as a therapeutic agent.

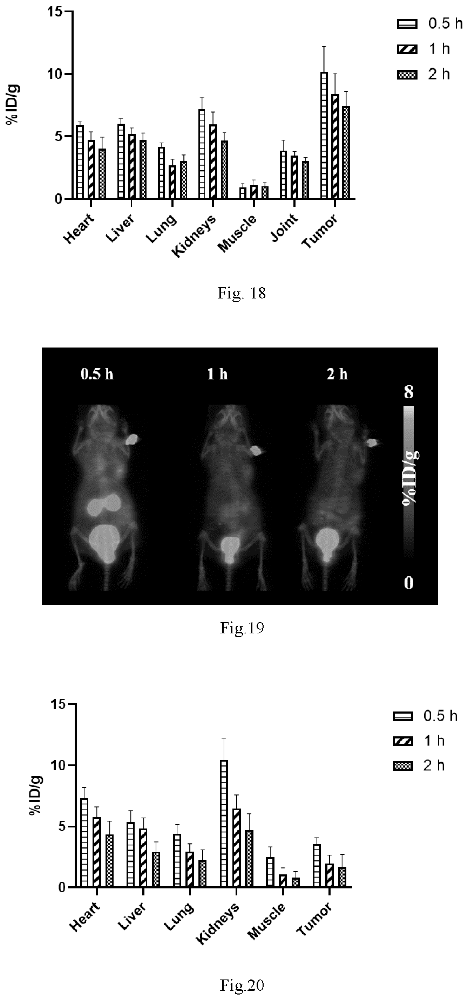

Further, it can be seen from a comparison of Figures 15 and 16 with Figures 19 and 20, ⁶⁸GaS12 and ⁶⁸Ga-C12 have different chain lengths for their covalent warhead groups, the tumor uptake value of ⁶⁸Ga-S12 (approximately 21% ID/g at 0.5 h) is higher than that of ⁶⁸Ga-C12 (approximately 4% ID/g at 0.5 h), and tumor/heart, tumor/liver, and tumor/lung ratios for ⁶⁸Ga-C12 at various time points are all less than 1. ⁶⁸Ga-S12 exhibits superior tumor targeting compared to 68Ga-C12, indicating that a shorter chain length of the linking group provides the compound with better tumor targeting performance. Preferably, the number of carbon atoms in the linking group of the covalent warhead is ≤3, allowing the compound to achieve superior tumor targeting performance and better tumor uptake capacity.

Figures 17 and 18 show the PET-CT imaging results and biodistribution results of ⁶⁸Ga-S15 in U87MG tumor-bearing mice, respectively. It can be seen that ⁶⁸Ga-S15 exhibits significant uptake in tumor, with the uptake in non-target organs being significantly lower than that of ⁶⁸Ga-FAPI46, indicating a lower tumor-to-background ratio. ⁶⁸Ga-S15 can provide superior PET-CT image quality than ^{6g}Ga-FAPI46 and can be used as a tumor diagnostic agent. This also demonstrates that epoxy-based covalent warhead can significantly improve the specific targeting performance of the compound.

### 3. Animal experiment evaluation of the tumor treatment efficacy of ¹⁷⁷Lu-labeled S23:

### 3.1 ¹⁷⁷Lu radiolabeling method

1) Preparation of the precursor compound solution: an appropriate amount of the precursor compound was dissolved in DMSO to a concentration of 3 nmol/10 µL, and stored at -20°C for later use.
2) Preparation of 0.01 M ammonium acetate solution: 8.2 mg of demetallized ammonium acetate (82.03 g/mol) was dissolved in 10 mL of deionized water to obtain a 0.01 M ammonium acetate solution, and acetic acid was added to adjust the pH to 4.0, and stored at 4°C in refrigerator for later use.
3) Activation of C18 cartridges: C18 cartridges were activated within 30 minutes prior to use by slowly injecting 5 mL of absolute ethanol and 10 mL of deionized water in sequence, then draining the liquid.
4) Labeling and purification: 300 µL of ammonium acetate solution (0.01M, pH=4.0) was added to the ¹⁷⁷LuCl₃ solution, to adjust pH to 4.0. 3nM precursor solution was added, and the mixed solution was reacted at 95 to 105°C for 10 to 15 minutes, and the reaction was terminated. After the reaction solution was loaded on the C18 Cartridges, free ¹⁷⁷Lu was removed by rinsing with 3 mL of deionized water, and then the product was gradient eluted with 500 µL of absolute ethanol. The eluate was collected and its radioactivity was measured. The absolute ethanol was volatilized by heating and the residue was redissolved with physiological saline. Pyrogen was removed by 0.22 µm filter membrane, and an injection solution with a radioactivity concentration of 1mCi/100 µL was prepared for analysis and subsequent experiments.
5) Determination of labeling rate and radiochemical purity: 10 µCi of the radioactive injection solution, obtained after the reaction and purification, was applied to SG paper, developed with 500 µL of 0.01 M citric acid/sodium citrate, and placed in an iTLC for the determination of labeling ratio and radiochemical purity. The labeling ratio of S23 labeled with ¹⁷⁷Lu was 95.89%, and the radiochemical purity was 99.00%.

### 3.2 Biodistribution experiment of ¹⁷⁷Lu-S23:

### 1) Experimental Grouping

Twelve HT1080-FAP tumor-bearing mice were randomly divided into two groups (¹⁷⁷Lu-S23 group and ¹⁷⁷Lu-FAPI46 group).

### 2) Organ Sampling and Data Collection Protocol

¹⁷⁷Lu-S23 and ¹⁷⁷Lu-FAPI46 were administered via tail vein injection, respectively. Mice were sacrificed by neck amputation at 4, 12, 24, 48 and 72 hours after administration, respectively, and dissected. Tissue samples were collected, including tumors, blood, heart, lung, liver, spleen, kidney, stomach, intestine, bone, flesh, brain, etc. Heart was squeezed, stomach and intestine contained contents, the skeletal muscle was taken from the hind leg muscle, the bone was taken from the hind leg, and all organs were weighed. Radioactivity counts were performed on the harvested tissues using a gamma-counter. At the same time, the injected sample was accurately diluted 100-fold, 1% of which was taken into a counter tube as a standard 1% ID (i.e. one percent of the administered dose), and the 1% ID standard and the radioactivity count of the biological sample were simultaneously measured on a γ-counter.

### 3) Parameters and Data Analysis

Data for biodistribution was expressed as a percentage of radioactivity count per gram of tissue or organ to the total dose administered (radioactivity count) (% ID/g).

The specific calculation formula is: $%ID / g = \frac{{Counts}_{tissue or organ}}{{Counts}_{total dose administered} \times {W}_{tissue or organ}} \times 100 %$

Data for each sampling point was expressed as the mean ± standard deviation (mean ± SD) of tumor-bearing mice.

### 3.3 Tumor Treatment Efficacy Evaluation for ¹⁷⁷Lu-S23

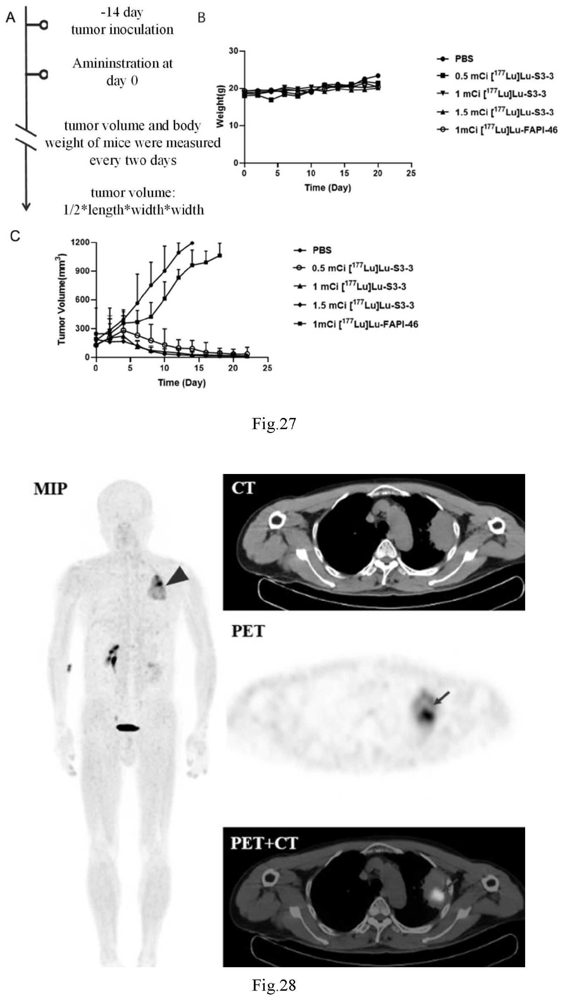

1) HT1080-FAP cells were suspended in PBS at a density of 1 × 10⁸ cells/mL and inoculated into the right axillary region of 35 nude mice from the same batch. When tumor volume reached 50 to 200 mm³, the 35 tumor-bearing mice were randomly divided into 5 groups (n = 7): PBS group, ¹⁷⁷Lu-S23 (0.5 mCi) group, ¹⁷⁷Lu-S23 (1 mCi) group, ¹⁷⁷Lu-S23 (1.5 mCi) group, ¹⁷⁷Lu-FAPI-46 (1 mCi) group. The PBS group received an injection of 0.1 mL of PBS, while the treatment groups received injections of the corresponding doses of ¹⁷⁷Lu-S23 or ¹⁷⁷Lu-FAPI-46;
2) Tumor volume (measurement method for tumor volume: measuring longest diameter and shortest diameter of tumor, calculated as: Volume = 0.5 × longest diameter × shortest diameter²) and body weight of mice were measured before dosing and on the second day post-dosing. Thereafter, body weight and tumor size of mice were monitored and recorded every two days. The study was terminated when body weight decreased by 5% or tumor volume>1200 mm³. The treatment regimen is shown in Figure 27(A).

Experimental Results: Figure 25 shows the biodistribution results of ¹⁷⁷Lu-S23 in HT1080-FAP-bearing mice (n=6), and Figure 26 shows the biodistribution results of ¹⁷⁷Lu-FAPI46 in HT1080-FAP-bearing mice (n=6). As shown in Figures 25 and 26, the tumor uptake value of ¹⁷⁷Lu-S23 remained at approximately 18% ID/g at 4 h and was still maintained at 8% ID/g at 72 h, while the uptake in non-tumor tissues remained low--below 5% ID/g in all organs except kidney. This indicates that ¹⁷⁷Lu-S23 possesses excellent tumor-targeting performance and tumor retention capacity. In contrast, ¹⁷⁷Lu-FAPI46 exhibits tumor uptake of only approximately 5% ID/g at 4 h, and had been almost completely metabolized by 72 h, indicating poor tumor-targeting and retention capabilities. Figure 27 further presents experimental data of the tumor treatment efficacy of ¹⁷⁷Lu-S23 in HT1080-FAP tumor-bearing mice, including (A) a schematic diagram of the treatment regimen, (B) body weight change curves of mice during the treatment period, and (C) averaged tumor growth curves during the treatment period. Figure 27 (C) shows that each dose of ¹⁷⁷Lu-S23 can all significantly inhibit tumor growth, and all have significantly superior tumor inhibitory effects, compared to PBS blank control group and ¹⁷⁷Lu-FAPI46. Even if a low dose, 0.5mCi of ¹⁷⁷Lu-S23 is used, it has better tumor inhibitory effect than 1mCi ¹⁷⁷Lu-FAPI46, and the increased dose, 1mCi or 1.5mCi of ¹⁷⁷Lu-S23 has better tumor inhibitory effect. At the same time, mice have no obvious weight loss, no abnormalities in eating and movement, indicating that the increased dose is safe. The results show that ¹⁷⁷Lu-S23 has excellent tumor treatment efficacy and can be used as a therapeutic drug for tumor.

### 4. Clinical Trial Study

### 4.1 PET/CT Imaging of ⁶⁸Ga-S2 in Lung Cancer Patients

Lung cancer patients received an intravenous injection of ⁶⁸Ga-S2 (144 MBq). Using Ingenuity TF PET/CT (Philips, Amsterdam, Netherlands), a static three-dimensional PET scan was acquired 1 hour after injection at a rate of 2 minutes per bed positions, with a total of 10 to 12 bed positions covering the whole body. The PET/CT images are shown in Figure 28. Regions of interest were delineated, and tumor uptake was measured using PMOD software to calculate the maximum SUV (SUVmax). The results are shown in Table 4.

**Table 4. SUVmax values of the ⁶⁸Ga-S2 probe in muscle, kidney, and tumor of patients with FAP-positive tumors**

| Tissue | Tumor | Kidney | Muscle |
|---|---|---|---|
| SUVmax (% ID/g) | 12.2 | 13.4 | 1.0 |

As shown in Table 4, there is marked ⁶⁸Ga-52 uptake at the tumor site of patients. The drug is primarily metabolized by kidney, and the uptake in the lesion area is significantly higher than the muscle background.

As shown in Figure 28, this patient exhibits significant ⁶⁸Ga-S2 uptake at the tumor site. ⁶⁸GaS2 is primarily metabolized by kidney; furthermore, the uptake values in liver, muscle blood pool, and other sites are low, demonstrating excellent targeting. This indicates that ⁶⁸Ga-S2 exhibits high-specific uptake in lung cancer tumors in vivo with a high tumor-to-background ratio, demonstrating potential as a diagnostic probe, while also providing strong evidence for the application of ⁶⁸Ga-S2 in internal targeted radiotherapy.

### 4.2 PET/CT Imaging of ⁶⁸Ga-S23 in Patients with Postoperative Recurrence of Mucinous Adenocarcinoma of the Intestinal Tract in the Right Lower Abdomen

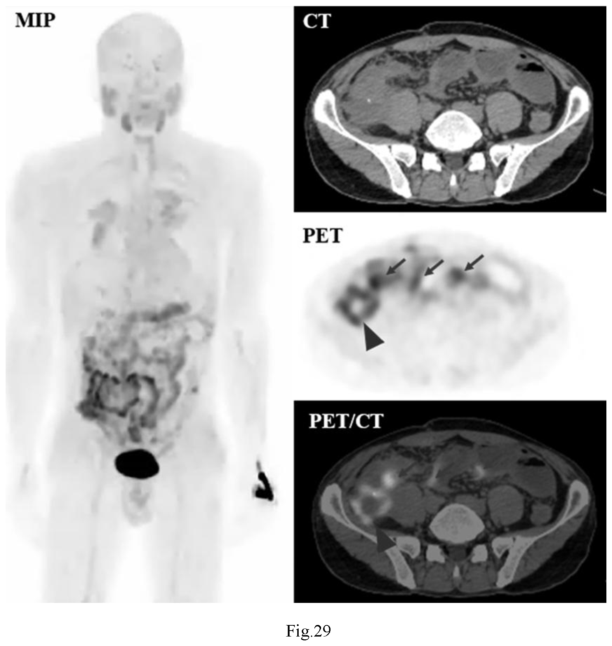

In a patient with postoperative recurrence of mucinous adenocarcinoma of the intestinal tract in the right lower abdomen, ⁶⁸Ga-S23 (148 MBq) was intravenously injected. At 1 hour post-injection, a static three-dimensional PET scan was acquired using an Ingenuity TF PET/CT scanner (Philips, Amsterdam, Netherlands), with an acquisition time of 2 minutes per bed position and a total of 10 to 12 bed positions covering the whole body. The PET/CT images are shown in Figure 29. Regions of interest were delineated using PMOD software to measure tumor uptake, and the maximum SUV (SUVmax) was calculated. The results are presented in Table 5

**Table 5. SUVmax values of the ⁶⁸Ga-S23 probe in muscle, kidney, primary tumor, and the largest metastatic lesion in patients with FAP-positive tumors**

| Tissue | Tumor (Primary) | Tumor (Metastasis) | Kidney | Muscle |
|---|---|---|---|---|
| SUVmax (% ID/g) | 9.0 | 9.4 | 3.7 | 0.8 |

As shown in Table 5, both the primary tumor and metastatic lesions in the patient exhibited significant radioactive uptake of ⁶⁸Ga-S23, and the uptake levels were markedly higher than those in the pancreas and liver.

As shown in Figure 29, the patient had postoperative recurrence of mucinous adenocarcinoma of the intestinal tract in the right lower abdomen. There was intense ⁶⁸Ga-S23 uptake in the intestinal tract in the right lower abdomen (at the site of the primary lesion), with numerous small visible lesion areas. The drug was primarily metabolized via kidney, while uptake in liver and other organs was relatively low. These findings indicate that this probe can accurately detect the lesion areas in patients with intestinal mucinous adenocarcinoma, with a low backgroundvalue, thereby demonstrating its potential as a diagnostic probe.

The above describes the embodiments of the present invention. However, the present invention is not limited to the above embodiments. Any modifications, equivalent substitutions, improvements, etc., made within the spirit and principles of the present invention shall be included within the scope of protection of the present invention.

## Claims

1. A compound represented by Formula (A), or a stereoisomer, tautomer, isotopically labeled form, pharmaceutically acceptable salt, or prodrug thereof, 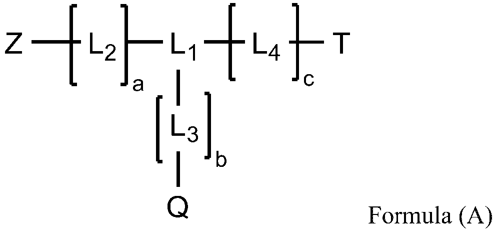
wherein Z is a payload, T is a targeting moiety,
Q is a covalent group selected from the structures represented by the following Formulas (Q-1) to (Q-21): 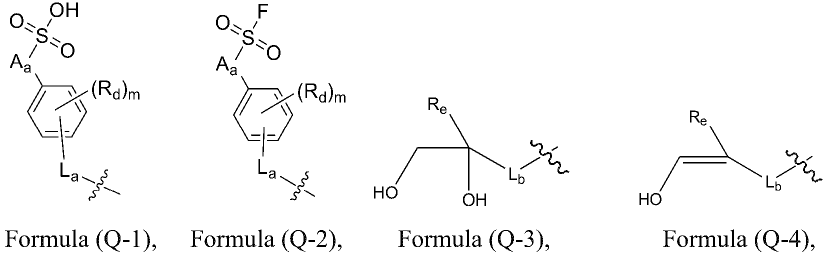 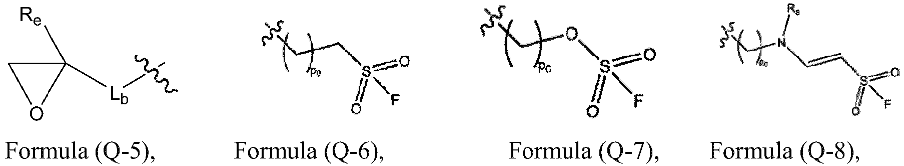 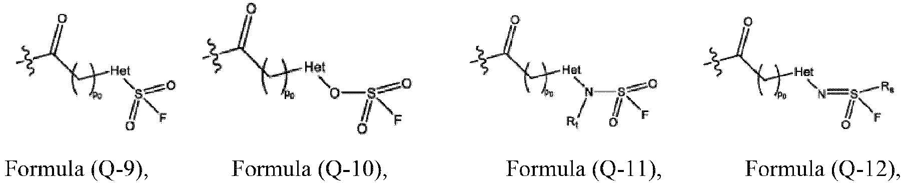 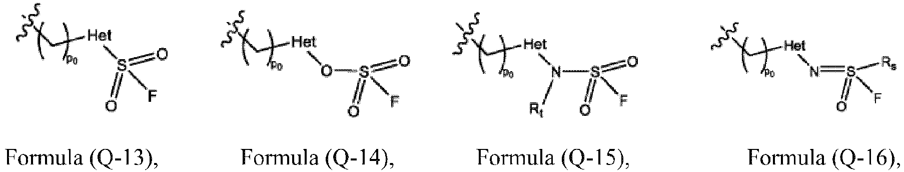 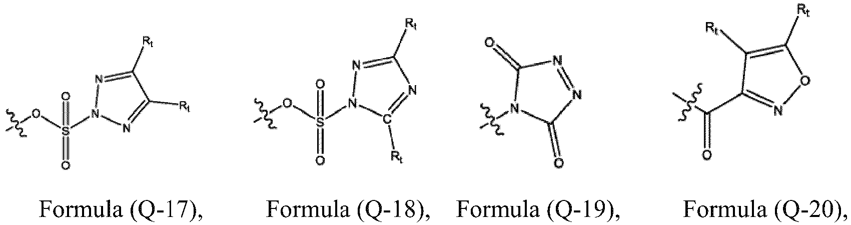 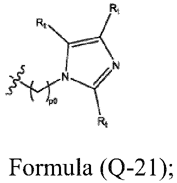
L₁, L₂, L₃, L₄, Aₐ, Lₐ, and L_{b} are the same or different, and are each independently selected from a bond or a linking group,
a, b, c, m, and p₀ are the same or different, and are each independently selected from an integer of 0 to 6 (e.g., 0, 1, 2, 3, 4, 5, 6),
R_{d} and Rₑ are the same or different, and are each independently selected from hydrogen, halogen, hydroxy, cyano, nitro, amino, C₁₋₂₀ alkyl, C₁₋₂₀ alkoxy, and halogenated C₁₋₂₀ alkyl;
Het is an optionally substituted saturated heterocyclylene or heteroarylene;
Rₛ is selected from hydrogen, optionally substituted alkyl, optionally substituted cycloalkyl, optionally substituted heterocycloalkyl, optionally substituted aryl, and optionally substituted heteroaryl;
each Rₜ is independently selected from hydrogen, halogen, nitro, cyano, optionally substituted mercapto, optionally substituted amino, optionally substituted selenyl, optionally substituted C₁₋₂₀ alkyl, optionally substituted C₃₋₂₀ cycloalkyl, optionally substituted heterocycloalkyl, optionally substituted aryl, and optionally substituted heteroaryl;
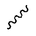 denotes the linking site of the group.

2. The compound, or the stereoisomer, tautomer, isotopically labeled form, pharmaceutically acceptable salt, or prodrug thereof according to claim 1, **characterized in that**, in the compound represented by Formula (A), Q is selected from the structures represented by Formulas (Q-1-1), (Q-2-1), (Q-3), (Q-4), or (Q-5) below:

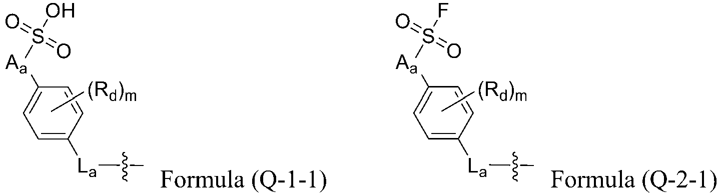

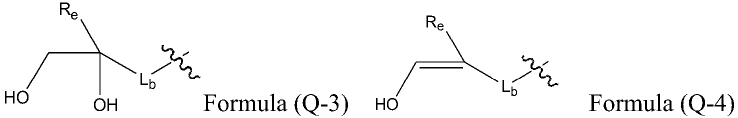

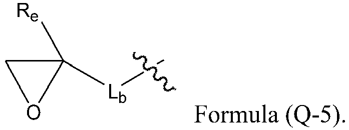

3. The compound, or the stereoisomer, tautomer, isotopically labeled form, pharmaceutically acceptable salt, or prodrug thereof according to claim 1 or 2, **characterized in that**, in Formula (A),
L₁ is selected from a trivalent linking group; preferably, L₁ is selected from N, C₁₋₂₀ alkanetriyl optionally substituted with one or more R₀, and any combination thereof;
wherein each R₀ is the same or different, and is independently selected from hydrogen, halogen, hydroxy, cyano, nitro, amino, C₁₋₁₀ alkyl, C₁₋₁₀ alkoxy, halogenated C₁₋₁₀ alkyl, and halogenated C₁₋₁₀ alkoxy; preferably, each R₀ is the same or different, and is independently selected from hydrogen, halogen, hydroxy, cyano, nitro, amino, C₁₋₆ alkyl, C₁₋₆ alkoxy, halogenated C₁₋₆ alkyl, and halogenated C₁₋₆ alkoxy; further preferably, each R₀ is the same or different, and is independently selected from hydrogen, halogen, hydroxy, cyano, nitro, amino, C₁₋₄ alkyl, C₁₋₄ alkoxy, halogenated C₁₋₄ alkyl, and halogenated C₁₋₄ alkoxy;
preferably, L₁ is selected from N, C₁₋₁₀ alkanetriyl (C₁₋₆ alkanetriyl, C₁₋₄ alkanetriyl) optionally substituted with one or more R₀, and any combination thereof.

4. The compound, or the stereoisomer, tautomer, isotopically labeled form, pharmaceutically acceptable salt, or prodrug thereof according to claim 1 or 2, **characterized in that**, in Formula (A),
L₂, L₃, L₄, Aₐ, Lₐ, and L_{b} are the same or different, and are each independently selected from a divalent linking group; preferably, L₂, L₃, L₄, Aₐ, Lₐ, and L_{b} are each independently selected from a bond, -O-, -S-, -P-, -O-(HO-P=O)-O-, -N(R₀)-, -CO-, C₁₋₂₀ alkylene optionally substituted with one or more R₀, 3- to 20-membered heterocyclylene optionally substituted with one or more R₀, and any combination thereof; further preferably, L₂, L₃, L₄, Aₐ, Lₐ, and L_{b} are each independently selected from a bond, -O-, -S-, -P-, -O-(HO-P=O)-O-, -N(R₀)-, -CO-, C₁₋₁₀ alkylene (C₁₋₆ alkylene, C₁₋₄ alkylene) optionally substituted with one or more R₀, 3- to 20-membered heterocyclylene (3-to 10-membered heterocyclylene, 3- to 6-membered heterocyclylene) optionally substituted with one or more R₀ , and any combination thereof;
preferably, each R₀ is the same or different, and is independently selected from hydrogen, halogen, hydroxy, cyano, nitro, amino, C₁₋₁₀ alkyl, C₁₋₁₀ alkoxy, halogenated C₁₋₁₀ alkyl, and halogenated C₁₋₁₀ alkoxy;
preferably, the heterocyclylene comprises at least one N; preferably, the heterocyclylene is piperazinylene;
preferably, a and c are not 0 at the same time;
preferably, at least one of L₂ and L₄ comprises 3- to 20-membered heterocyclylene; preferably, at least one of L₂ and L₄ comprises heterocyclylene having one nitrogen atom or two nitrogen atoms; preferably, at least one of L₂ and L₄ comprises piperazinylene;
preferably, Aₐ is selected from a bond.

5. The compound, or the stereoisomer, tautomer, isotopically labeled form, pharmaceutically acceptable salt, or prodrug thereof according to claim 1 or 2, **characterized in that**,
R_{d} is selected from hydrogen, halogen, hydroxy, cyano, nitro, amino, C₁₋₁₀ alkyl, C₁₋₁₀ alkoxy, halogenated C₁₋₁₀ alkyl, and halogenated C₁₋₁₀ alkoxy; preferably, R_{d} is selected from hydrogen, halogen, hydroxy, cyano, nitro, amino, C₁₋₆ alkyl, C₁₋₆ alkoxy, halogenated C₁₋₆ alkyl, and halogenated C₁₋₆ alkoxy; further preferably, R_{d} is selected from hydrogen, halogen, hydroxy, cyano, nitro, amino, C₁₋₄ alkyl, C₁₋₄ alkoxy, halogenated C₁₋₄ alkyl, and halogenated C₁₋₄ alkoxy;
preferably, when m is not 0, each R_{d} is independently selected from hydrogen, halogen, hydroxy, cyano, nitro, amino, C₁₋₁₀ alkyl, C₁₋₁₀ alkoxy, halogenated C₁₋₁₀ alkyl, and halogenated C₁₋₁₀ alkoxy.

6. The compound, or the stereoisomer, tautomer, isotopically labeled form, pharmaceutically acceptable salt, or prodrug thereof according to claim 1, **characterized in that**:
Z is selected from a nuclide-chelating group;
preferably, Z is selected from a group formed by a bifunctional chelating agent; preferably, the bifunctional chelating agent is selected from DOTA, NOTA, NODA, NODAGA, DOTP, TETA, ATSM, PTSM, EDTA, EC, HBEDCC, DTPA, SBAD, BAPEN, Df, DFO, TACN, NO2A/NOTAM, CB-DO2A, Cyclen, NOTA-AA, DO3A, DO3AP, HYNIC, MAS3, MAG3, and isonitrile;
preferably, Z is selected from 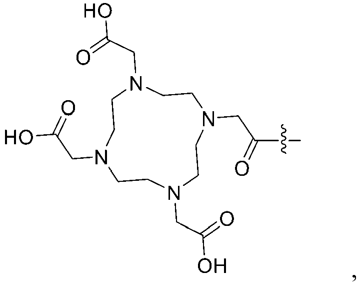 and

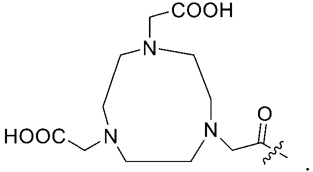

7. The compound, or the stereoisomer, tautomer, isotopically labeled form, pharmaceutically acceptable salt, or prodrug thereof according to claim 1, **characterized in that**:
T is selected from a structure targeting fibroblast activation protein-α represented by the following Formula (T-1), 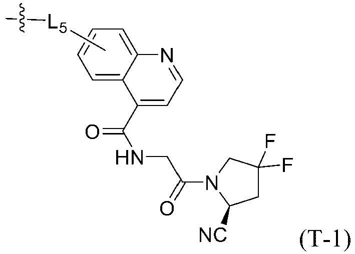
wherein L₅ is a divalent linking group, preferably S, O, or a bond, and preferably L₅ is a bond; 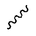 denotes the attachment site of the group;
preferably, T is selected from the structure represented by the following Formula (T-1-1):

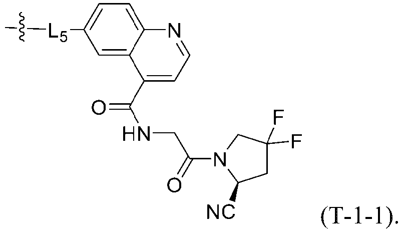

8. The compound, or the stereoisomer, tautomer, isotopically labeled form, pharmaceutically acceptable salt, or prodrug thereof according to claim 1, **characterized in that**:
the compound represented by Formula (A) has the structure represented by the following 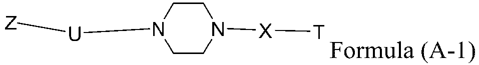
wherein,
U is selected from -U₁-, -U₂-U₃(Rₐ)-U₄-;
X is selected from X₁-, -X₂-X₃(R_{b})-X₄-;
when U is selected from -U₁-, X is not -X₁-;
U₁, U₂, U₃, U₄, X₁, X₂, X₃ and X₄ are the same or different, and are each independently selected from a linking group;
Rₐ and R_{b} are the same or different, and are each independently selected from the structures represented by Formulas (Q-1), (Q-2), (Q-3), (Q-4), and (Q-5).

9. The compound, or the stereoisomer, tautomer, isotopically labeled form, pharmaceutically acceptable salt, or prodrug thereof according to claim 8, **characterized in that**:
in the compound represented by Formula (A-1), U is selected from -U₂-U₃(Rₐ)-U₄-, and X is selected from -X₁-; or in the compound represented by Formula (A-1), U is selected from -U₁-, and X is selected from -X₂-X₃(Rₕ)-X₄-.

10. The compound, or the stereoisomer, tautomer, isotopically labeled form, pharmaceutically acceptable salt, or prodrug thereof according to claim 8 or 9, **characterized in that**:
U₃ and X₃ are the same or different, and are each independently selected from a trivalent linking group; preferably, U₃ and X₃ are each independently selected from N, C₁₋₂₀ alkanetriyl (C₁₋₁₀ alkanetriyl, C₁₋₆ alkanetriyl, C₁₋₄ alkanetriyl) optionally substituted with one or more R₀, and any combination thereof; and/or
U₁, U₂, U₄, X₁, X₂, X₄, Lₐ, L_{b}, and Aₐ are the same or different, and are each independently selected from a divalent linking group; preferably, U₁, U₂, U₄, X₁, X₂, X₄, Lₐ, L_{b}, and Aₐ are each independently selected from a bond, -O-, -S-, -P-, -O-(HO-P=O)-O-, -N(R₀)-, -CO-, C₁₋₂₀ alkylene (C₁₋₁₀ alkylene, C₁₋₆ alkylene, C₁₋₄ alkylene) optionally substituted with one or more R₀, and any combination thereof; preferably, Aₐ is selected from a bond;
wherein each R₀ is the same or different, and is independently selected from hydrogen, halogen, hydroxy, cyano, nitro, amino, C₁₋₁₀ alkyl, C₁₋₁₀ alkoxy, halogenated C₁₋₁₀ alkyl, and halogenated C₁₋₁₀ alkoxy; preferably, each R₀ is the same or different, and is independently selected from hydrogen, halogen, hydroxy, cyano, nitro, amino, C₁₋₆ alkyl, C₁₋₆ alkoxy, halogenated C₁₋₆ alkyl, and halogenated C₁₋₆ alkoxy; further preferably, each R₀ is the same or different, and is independently selected from hydrogen, halogen, hydroxy, cyano, nitro, amino, C₁₋₄ alkyl, C₁₋₄ alkoxy, halogenated C₁₋₄ alkyl, and halogenated C₁₋₄ alkoxy.

11. The compound, or the stereoisomer, tautomer, isotopically labeled form, pharmaceutically acceptable salt, or prodrug thereof according to claim 10, **characterized in that**, in the compound represented by Formula (A-1), U is selected from -U₁-, X is selected from -X₂-X₃(R_{b})-X₄-, and
U₁ is selected from a bond;
X₄ is selected from a bond;
X₃ is selected from N;
X₂ is selected from C₁₋₁₀ alkylene (preferably C₁₋₆ alkylene, C₁₋₄ alkylene, C₃ alkylene);
Q is selected from the structures represented by Formula (Q-1) or (Q-2), and Aₐ is selected from a bond, Lₐ is selected from -CO-, -CO-C₁₋₃ alkylene-, -CO-C₁₋₃ alkylene-NH-CO-, -CO-C₁₋₃ alkylene-NH-CO-C₁₋₃ alkylene-, -C₁₋₃ alkylene-, C₁₋₃ alkylene-NH-CO-C₁₋₃ alkylene-, -CO-C₁₋₆ alkylene-N(R₀)-CO-, -CO-C₁₋₄ alkylene-N(CH₃)-CO-, -CO-C₁₋₆ alkylene-CO-NH-C₁₋₃ alkylene-; preferably Q is selected from -CO-C₁₋₄ alkylene-N(R₀)-CO-, such as -CO-methylene-N(R₀)-CO-; preferably, R₀ is selected from C₁₋₄ alkyl.

12. The compound, or the stereoisomer, tautomer, isotopically labeled form, pharmaceutically acceptable salt, or prodrug thereof according to claim 1, **characterized in that**:
Q is selected from the structure represented by Formula (Q-1);
preferably, the compound represented by Formula (A) is selected from:

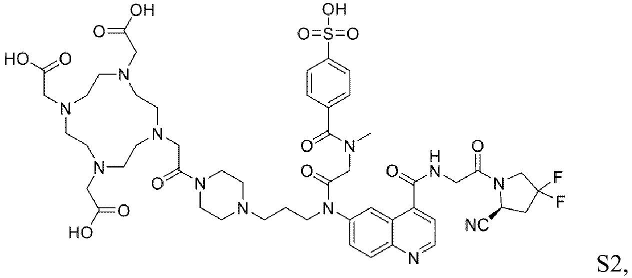

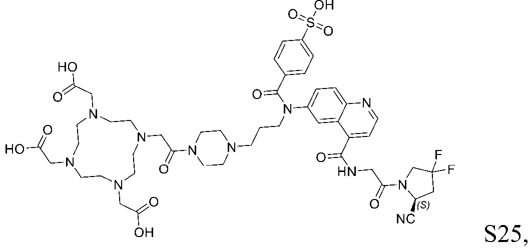

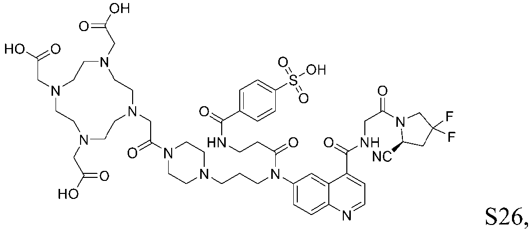

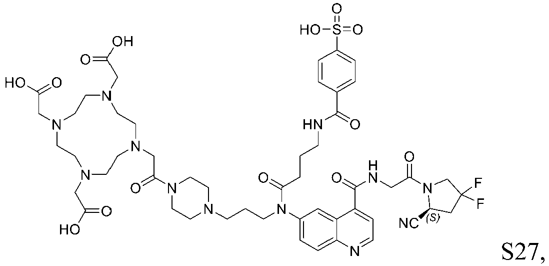

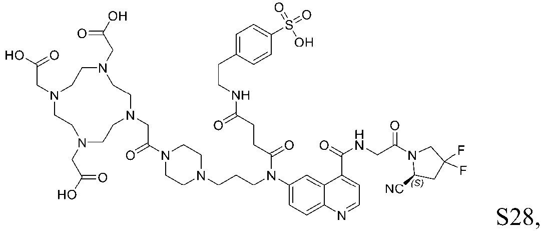

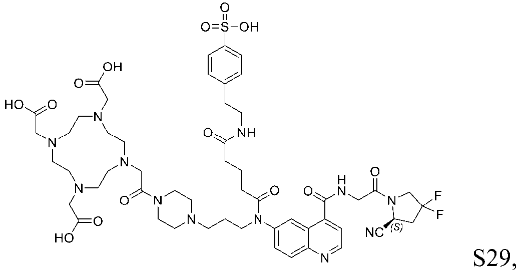

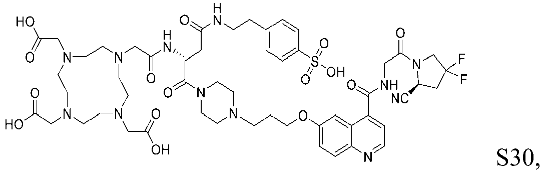

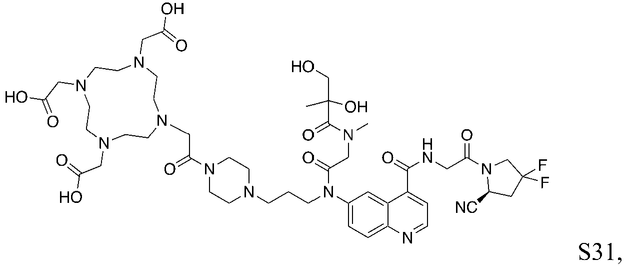

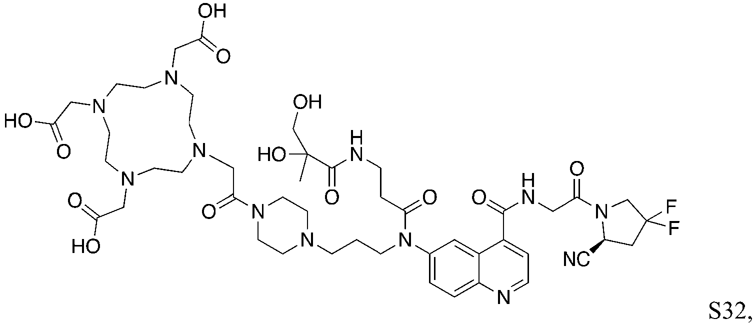

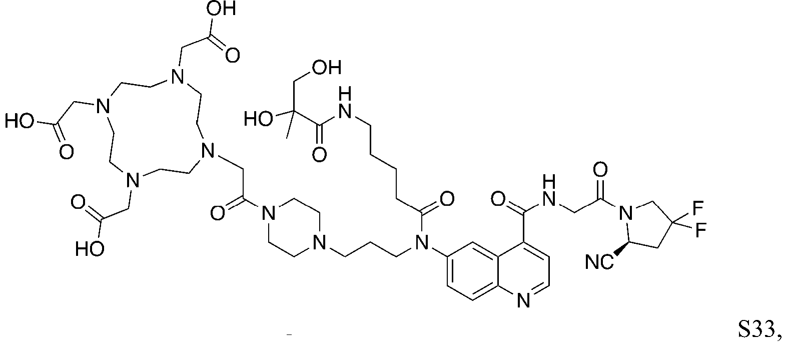

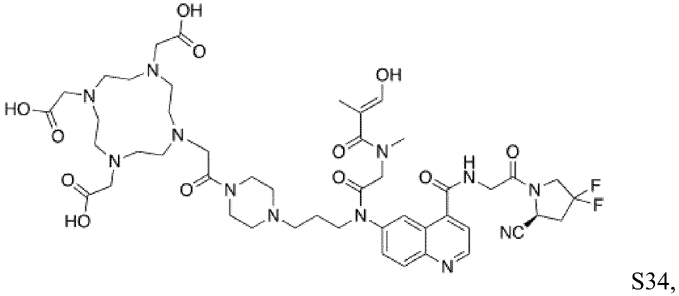

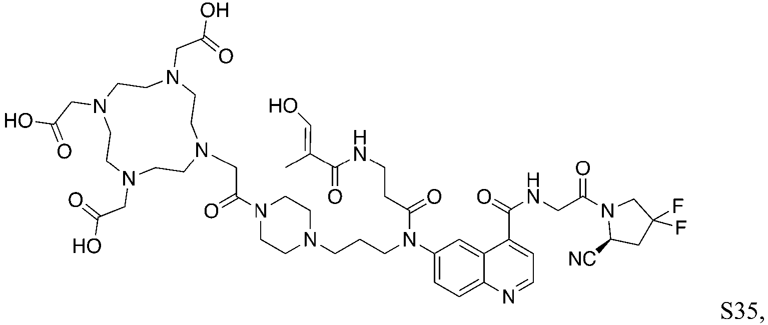

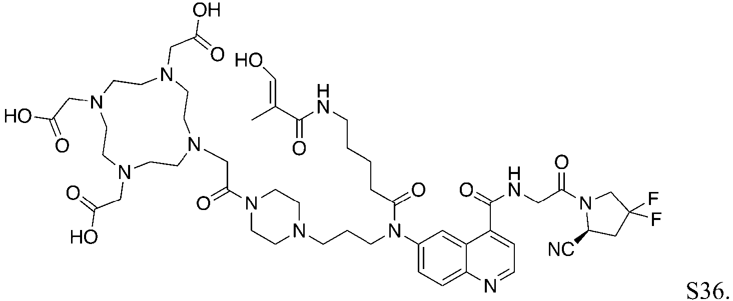

13. Use of a compound represented by Formula (A-0) in the preparation of the compound represented by Formula (A) of claim 1, wherein, in the compound represented by Formula (A), Q has the structure represented by Formula (Q-1), Formula (Q-3), or Formula (Q-4): 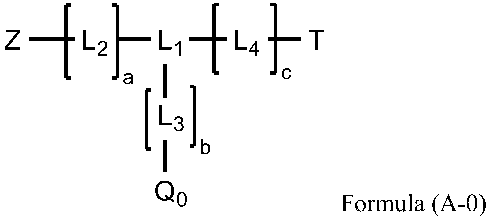 wherein Q₀ is selected from the structure represented by Formula (Q-2) or (Q-5) below: 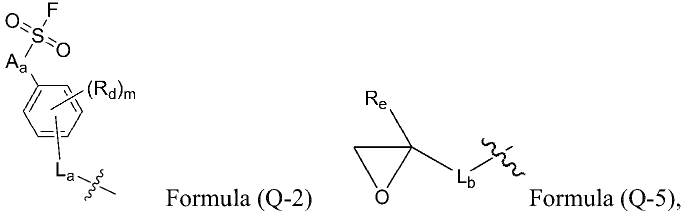
L₁, L₂, L₃, L₄, Aₐ, Lₐ, and L_{b} are the same or different, and are each independently selected from a bond or a linking group,
a, b, c, and m are the same or different, and are each independently selected from an integer of 0 to 6 (e.g., 0, 1, 2, 3, 4, 5, 6),
R_{d} and Rₑ are the same or different, and are each independently selected from hydrogen, halogen, hydroxy, cyano, nitro, amino, C₁₋₂₀ alkyl, C₁₋₂₀ alkoxy, and halogenated C₁₋₂₀ alkyl;
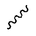 denotes the attachment site of the group.

14. A compound represented by Formula (B), or a stereoisomer, tautomer, isotopically labeled form, pharmaceutically acceptable salt, or prodrug thereof, **characterized in that**: 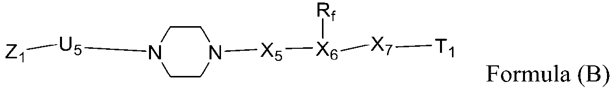
wherein Z₁ is a payload, T₁ is a targeting moiety,
U₅, X₅, X₆, and X₇ are the same or different, and are each independently selected from a linking group;
R_{f} is selected from the structure represented by Formula (Q-2-2) or Formula (Q-5-1) below: 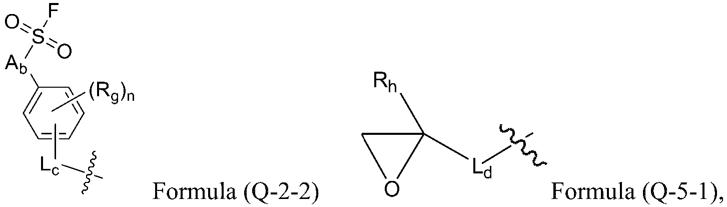
L_{c}, L_{d}, and A_{b} are the same or different, and are each independently selected from a linking group,
n is the same or different, and is each independently selected from an integer of 0 to 6 (e.g., 0, 1, 2, 3, 4, 5, 6),
R_{g} and Rₕ are the same or different, and are each independently selected from hydrogen, halogen, -OH, cyano, nitro, amino, C₁₋₂₀ alkyl, C₁₋₂₀ alkoxy, and halogenated C₁₋₂₀ alkyl;
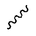 denotes the attachment site of the group.

15. The compound, or the stereoisomer, tautomer, isotopically labeled form, pharmaceutically acceptable salt, or prodrug thereof according to claim 14, **characterized in that**:
R_{f} is selected from the structure represented by the Formula (Q-2-3) below:

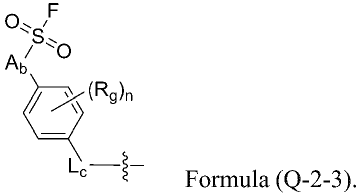

16. The compound, or the stereoisomer, tautomer, isotopically labeled form, pharmaceutically acceptable salt, or prodrug thereof according to claim 14 or 15, **characterized in that**:
X₆ is selected from a trivalent linking group; preferably, X₆ is each independently selected from N, C₁₋₂₀ alkanetriyl (C₁₋₁₀ alkanetriyl, C₁₋₆ alkanetriyl, C₁₋₄ alkanetriyl) optionally substituted with one or more R₀, and any combination thereof;
preferably, U₅, X₅, X₇, L_{c}, L_{d}, and A_{b} are the same or different, and are each independently selected from a divalent linking group; preferably, U₅, X₅, X₇, L_{c}, L_{d}, and A_{b} are each independently selected from a bond, -O-, -S-, -P-, -O-(HO-P=O)-O-, -N(R₀)-, -CO-, C₁₋₂₀ alkylene (C₁₋₁₀ alkylene, C₁₋₆ alkylene, C₁₋₄ alkylene) optionally substituted with one or more R₀, and any combination thereof;
preferably, Aₐ is selected from a bond;
preferably, R_{g} and Rₕ are the same or different, and are each independently selected from hydrogen, halogen, hydroxy, cyano, nitro, amino, C₁₋₂₀ alkyl, C₁₋₂₀ alkoxy, and halogenated C₁₋₂₀ alkyl;
preferably, each R₀ is the same or different, and is each independently selected from hydrogen, halogen, hydroxy, cyano, nitro, amino, C₁₋₁₀ alkyl, C₁₋₁₀ alkoxy, halogenated C₁₋₁₀ alkyl, and halogenated C₁₋₁₀ alkoxy; preferably, each R₀ is the same or different, and is each independently selected from hydrogen, halogen, hydroxy, cyano, nitro, amino, C₁₋₆ alkyl, C₁₋₆ alkoxy, halogenated C₁₋₆ alkyl, and halogenated C₁₋₆ alkoxy; further preferably, each R₀ is the same or different, and is each independently selected from hydrogen, halogen, hydroxy, cyano, nitro, amino, C₁₋₄ alkyl, C₁₋₄ alkoxy, halogenated C₁₋₄ alkyl, and halogenated C₁₋₄ alkoxy.

17. The compound, or the stereoisomer, tautomer, isotopically labeled form, pharmaceutically acceptable salt, or prodrug thereof according to claim 16, **characterized in that**:
U₅ is selected from a bond, -CO-, and -C₁₋₄ alkyl CO-;
X₇ is selected from a bond;
X₆ is selected from N;
X₅ is selected from C₁₋₁₀ alkylene, preferably C₁₋₆ alkylene, C₁₋₄ alkylene, and C₃ alkylene;
L_{c} and L_{d} are the same or different, and are each independently selected from -CO-, -C₁₋₆ alkylene-, -CO-C₁₋₆ alkylene-, -C₁₋₆ alkylene-CO-N(R₀)-, -C₁₋₆ alkylene-N(R₀)-CO-, -CO-C₁₋₆ alkylene-N(R₀)-CO-C₁₋₆ alkylene-, -CO-C₁₋₆ alkylene-CO-N(R₀)-C₁₋₆ alkylene-, -C₁₋₆ alkylene-CO-N(R₀)-C₁₋₆ alkylene-, -C₁₋₆ alkylene-N(R₀)-CO-C₁₋₆ alkylene-, -C₁₋₆ alkylene-N(R₀)-CO-C₁₋₆ alkylene-COO-C₁₋₆ alkylene-, -C₁₋₆ alkylene-CO-N(R₀)-C₁₋₆ alkylene-COO-C₁₋₆ alkylene-, -CO-C₁₋₆ alkylene-N(R₀ )-CO-C₁₋₆ alkylene-COO-C₁₋₆ alkylene-, -CO-C₁₋₆ alkylene-CO-N(R₀)-C₁₋₆ alkylene-COO-C₁₋₆ alkylene-, etc; preferably, L_{c} and L_{d} are each independently selected from -C₁₋₃ alkylene-, -CO-C₁₋₃ alkylene-, -C₁₋₃ alkylene-CO-N(R₀)-, -C₁₋₃ alkylene-N(R₀)-CO-, -CO-C₁₋₃ alkylene-N(R₀)-CO-C₁₋₃ alkylene-, -CO-C₁₋₃ alkylene-CO-N(R₀)-C₁₋₃ alkylene-, -C₁₋₃ alkylene-CO-N(R₀)-C₁₋₃ alkylene-, -C₁₋₃ alkylene-N(R₀)-CO-C₁₋₃ alkylene-, -C₁₋₃ alkylene-N(R₀)-CO-C₁₋₃ alkylene-COO-C₁₋₃ alkylene-, -C₁₋₃ alkylene-CO-N(R₀)-C₁₋₃ alkylene-COO-C₁₋₃ alkylene-, -CO-C₁₋₃ alkylene-N(R₀)-CO-C₁₋₃ alkylene-COO-C₁₋₃ alkylene-, -CO-C₁₋₃ alkylene-CO-N(R₀)-C₁₋₃ alkylene-COO-C₁₋₃ alkylene-;
R₀ is selected from C₁₋₄ alkyl, such as methyl;
L_{c} is selected from -CO-, -CO-C₁₋₃ alkylene-, CO-C₁₋₃ alkylene-NH-CO-, and -CO-C₁₋₃ alkylene-NH-CO-C₁₋₃ alkylene-; R_{g} is selected from hydrogen, halogen, C₁₋₆ alkyl, C₁₋₆ alkoxy, C₁₋₃ alkylene -NH-CO-C₁₋₃ alkylene, -CO-C₁₋₆ alkylene-N(R₀)-CO-, -CO-C₁₋₄ alkylene-N(CH₃)-CO-, and -CO-C₁₋₆ alkylene-CO-NH-C₁₋₃ alkylene-;
L_{d} is selected from -CO-, CO-C₁₋₄ alkylene-, -CO-C₁₋₄ alkylene-N(CH₃)-CO-, and -CO-C₁₋₄ alkylene-NH-CO-;
Rₕ is selected from hydrogen, C₁₋₆ alkyl, and C₁₋₆ alkoxy;
A_{b} is selected from a bond and oxygen;
R_{g} is selected from H and methoxy.

18. The compound, or the stereoisomer, tautomer, isotopically labeled form, pharmaceutically acceptable salt, or prodrug thereof according to claim 16, **characterized in that**:
the compound represented by formula (B) is selected from the following compounds:

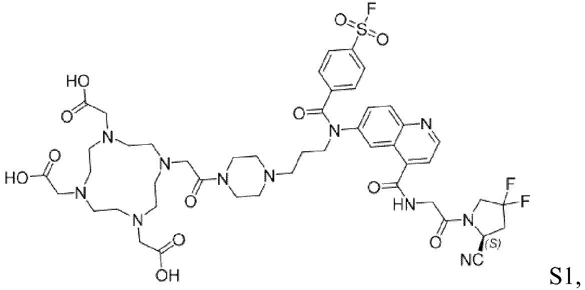

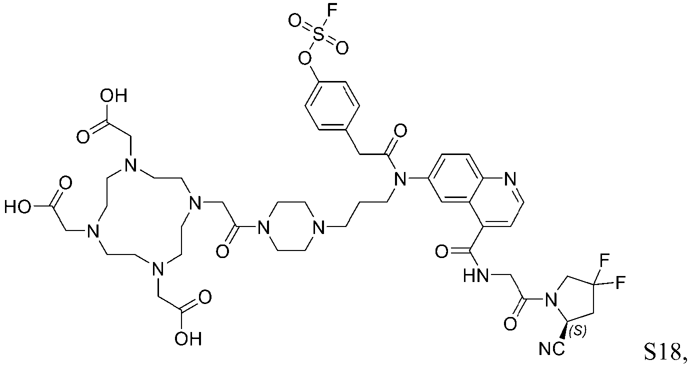

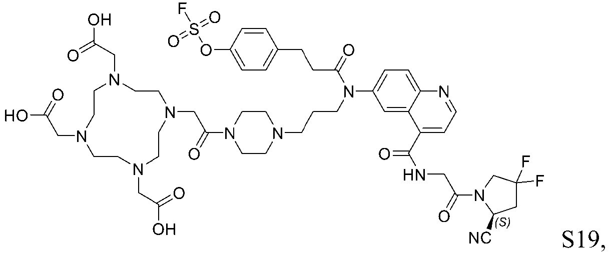

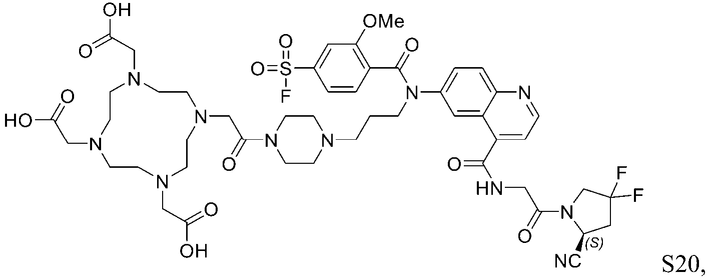

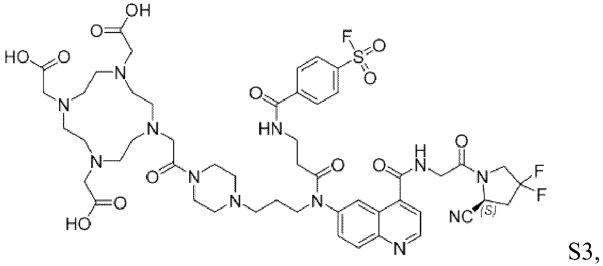

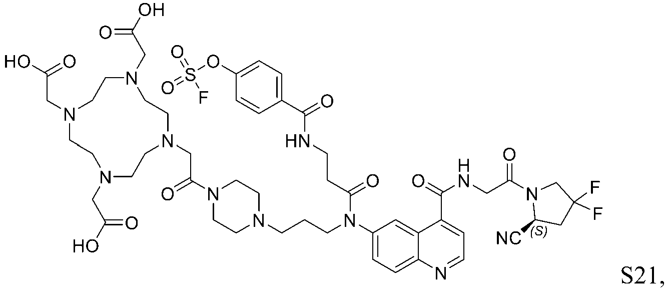

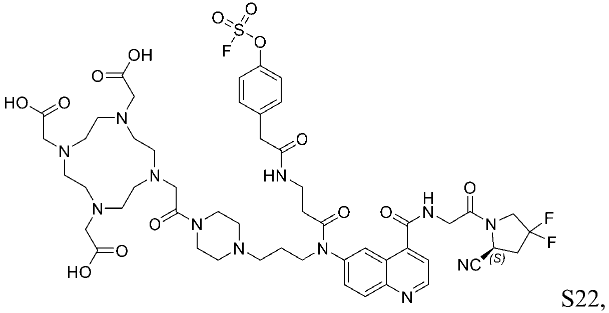

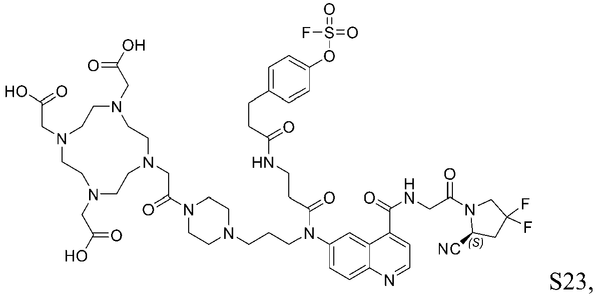

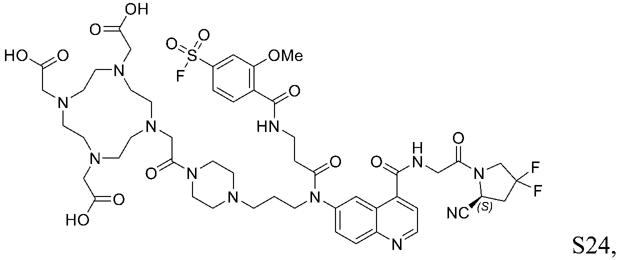

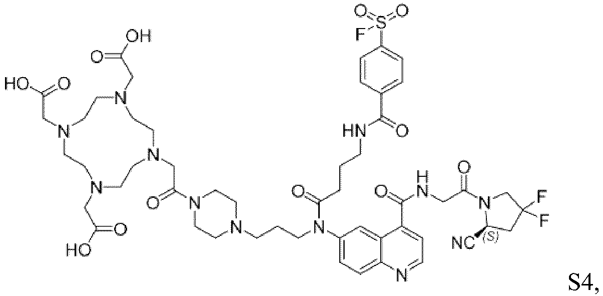

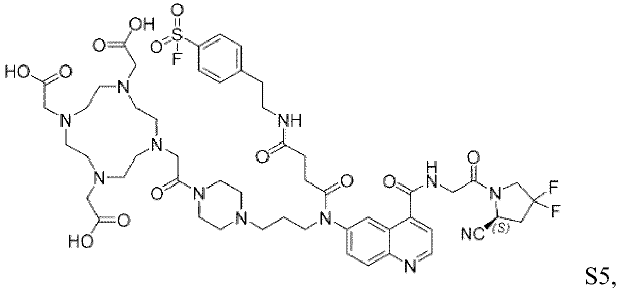

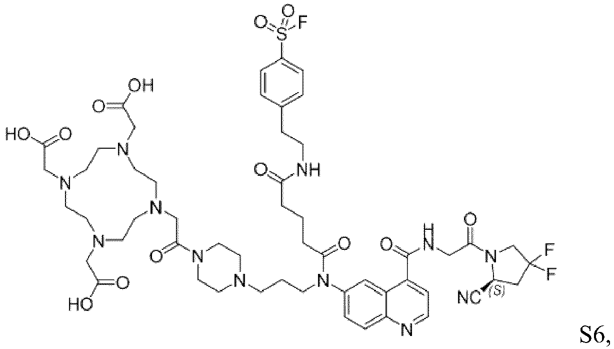

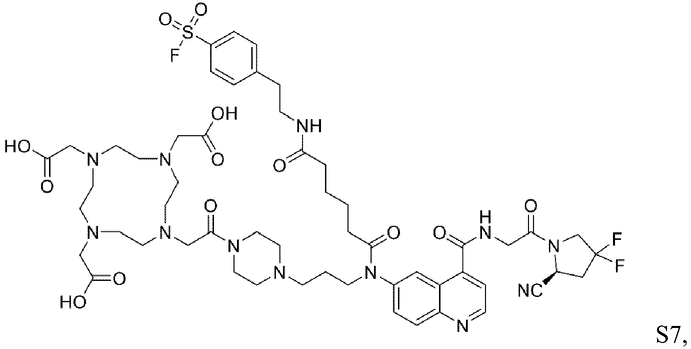

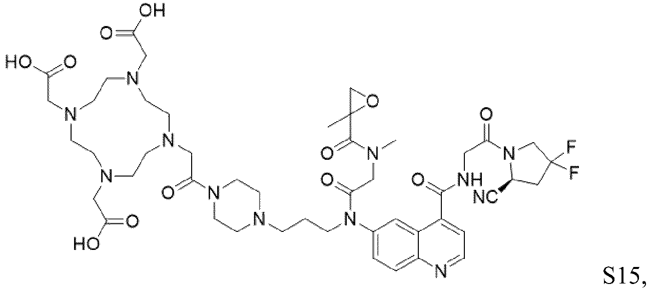

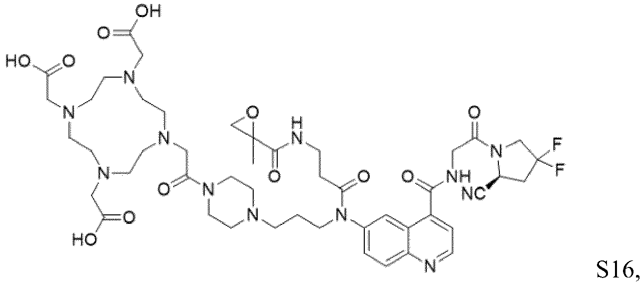

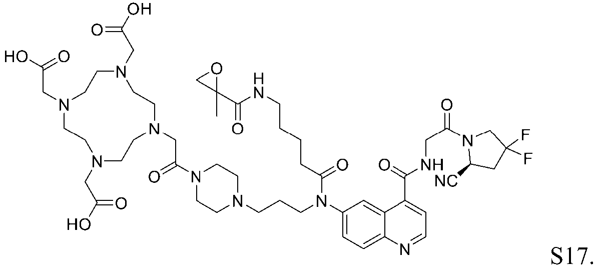

19. A compound represented by Formula (D), or a stereoisomer, tautomer, isotopically labeled form, pharmaceutically acceptable salt, or prodrug thereof, **characterized in that**: 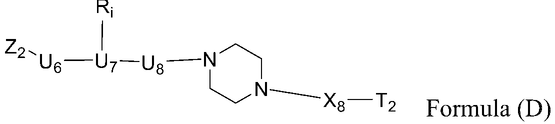
wherein Z₂ is a payload, T₂ is a targeting moiety,
U₆, U₇, U₈, and X₈ are the same or different, and are each independently selected from a linking group;
Rᵢ is selected from the structure represented by Formula (Q-2-4) or Formula (Q-5-2) below: 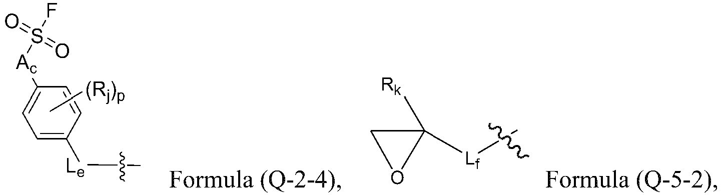
Lₑ, A_{c}, and L_{f} are the same or different, and are each independently selected from a linking group,
p is selected from an integer of 0 to 6 (e.g., 0, 1, 2, 3, 4, 5, 6),
Rⱼ and Rₖ are selected from hydrogen, halogen, -OH, cyano, nitro, amino, C₁₋₂₀ alkyl, C₁₋₂₀ alkoxy, and halogenated C₁₋₂₀ alkyl;
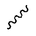 denotes the attachment site of the group.

20. The compound, or the stereoisomer, tautomer, isotopically labeled form, pharmaceutically acceptable salt, or prodrug thereof according to claim 19, **characterized in that**:
Rᵢ is selected from the structure represented by the following formula (Q-2-5):

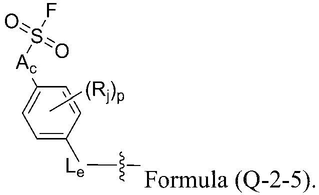

21. The compound, or the stereoisomer, tautomer, isotopically labeled form, pharmaceutically acceptable salt, or prodrug thereof according to claim 19 or 20, **characterized in that**:
U₇ is selected from a trivalent linking group; preferably, U₇ is each independently selected from N, C₁₋₂₀ alkanetriyl (C₁₋₁₀ alkanetriyl, C₁₋₆ alkanetriyl, C₁₋₄ alkanetriyl) optionally substituted with one or more R₀, and any combination thereof;
preferably, U₆, U₈, X₈, L_{f}, Lₑ, and A_{c} are the same or different, and are each independently selected from a divalent linking group; preferably, U₆, U₈, X₈, L_{f}, Lₑ, and A_{c} are each independently selected from a bond, -O-, -S-, -P-, -O-(HO-P=O)-O-, -N(R₀)-, -CO-, C₁₋₂₀ alkylene (C₁₋₁₀ alkylene, C₁₋₆ alkylene, C₁₋₄ alkylene) optionally substituted with one or more R₀, and any combination thereof;
preferably, A_{c} is selected from a bond;
preferably, Rⱼ and Rₖ are the same or different, and are each independently selected from hydrogen, halogen, hydroxy, cyano, nitro, amino, C₁₋₂₀ alkyl, C₁₋₂₀ alkoxy, and halogenated C₁₋₂₀ alkyl;
preferably, each R₀ is the same or different, and is independently selected from hydrogen, halogen, hydroxy, cyano, nitro, amino, C₁₋₁₀ alkyl, C₁₋₁₀ alkoxy, halogenated C₁₋₁₀ alkyl, and halogenated C₁₋₁₀ alkoxy; preferably, each R₀ is the same or different, and is independently selected from hydrogen, halogen, hydroxy, cyano, nitro, amino, C₁₋₆ alkyl, C₁₋₆ alkoxy, halogenated C₁₋₆ alkyl, and halogenated C₁₋₆ alkoxy; further preferably, each R₀ is the same or different, and is independently selected from hydrogen, halogen, hydroxy, cyano, nitro, amino, C₁₋₄ alkyl, C₁₋₄ alkoxy, halogenated C₁₋₄ alkyl, and halogenated C₁₋₄ alkoxy.

22. The compound, or stereoisomer, tautomer, isotopically labeled form, pharmaceutically acceptable salt, or prodrug thereof according to claim 20, **characterized in that**:
U₆ is selected from -NH-, -CONH-, and -C₀₋₄ alkyl CONH-;
U₇ is selected from C₁₋₄ straight or branched alkanetriyl, preferably methanetriyl;
U₈ is selected from -CO-;
X₈ is selected from #-O-C₁₋₁₀ alkylene-*, preferably #-O-C₁₋₆ alkylene-*, #-O-C₁₋₄ alkylene-*, and #-O-C₁₋₃ alkylene-*, wherein # denotes the attachment site to T₂, and * denotes the attachment site to the piperazine ring;
Lₑ is selected from -C₁₋₃ alkylene -, -CO-, -O-, -NH-, and a combination thereof, such as: -C₁₋₃ alkylene-, -C₁₋₃ alkylene-NH-CO-C₁₋₃ alkylene-, -CO-, -CO-C₁₋₃ alkylene-, -CO-C₁₋₃ alkylene-NH-CO-, -CO-C₁₋₃ alkylene-NH-CO-C₁₋₃ alkylene-;
Rₖ is selected from hydrogen, halogen, C₁₋₆ alkyl, and C₁₋₆ alkoxy;
Rj is selected from hydrogen, methyl, and methoxy;
L_{f} is selected from -C₁₋₄ alkylene-, -CO-, -O-, -NH-, and a combination thereof, such as: -C₁₋₄ alkylene-, -C₁₋₄ alkylene-CO-NH-C₁₋₄ alkylene-, -C₁₋₄ alkylene-, -C₁₋₄ alkylene-CO-NH-C₁₋₄ alkylene- CO-O-C₁₋₄ alkylene-;
R₀ is selected from hydrogen and C₁₋₄ alkyl, such as methyl;
A_{c} is selected from a bond or oxygen.

23. The compound, or the stereoisomer, tautomer, isotopically labeled form, pharmaceutically acceptable salt, or prodrug thereof according to claim 20, **characterized in that**:
the compound represented by Formula (D) is selected from the following compounds:

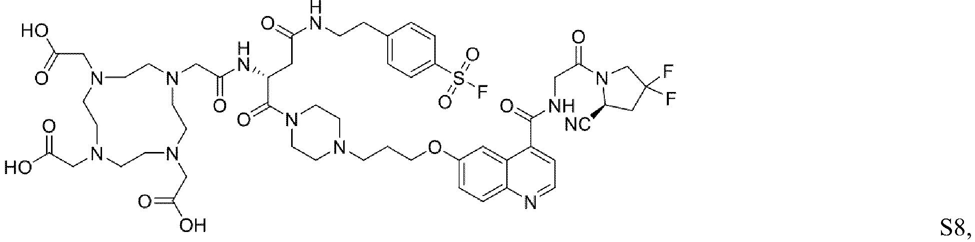

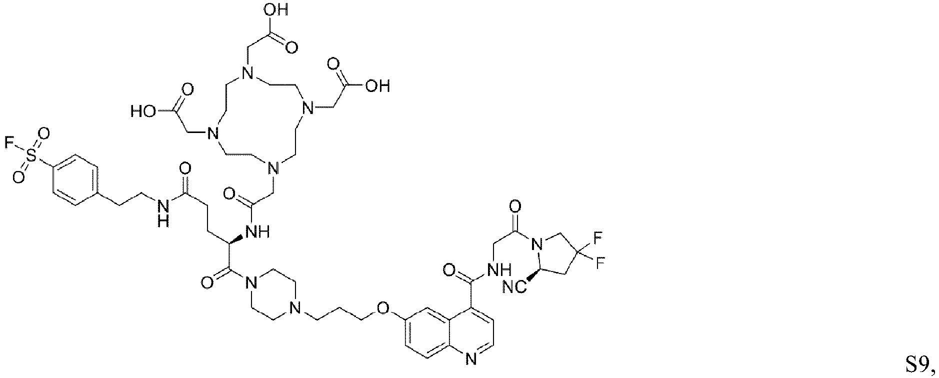

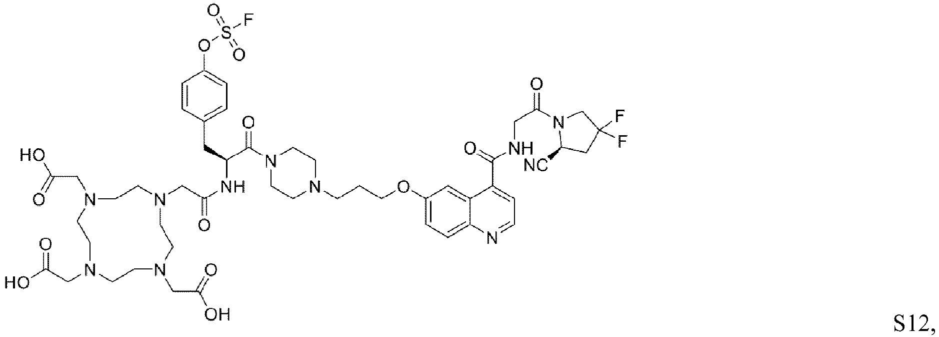

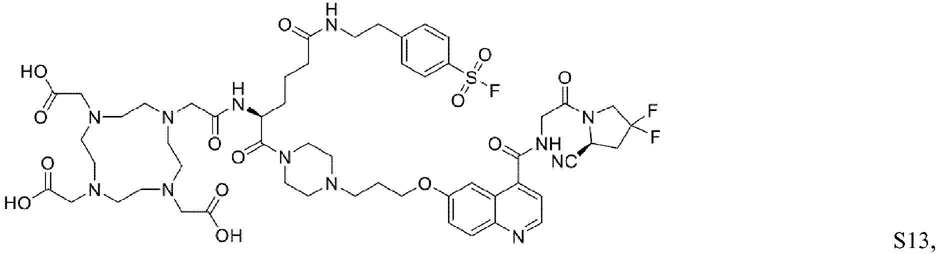

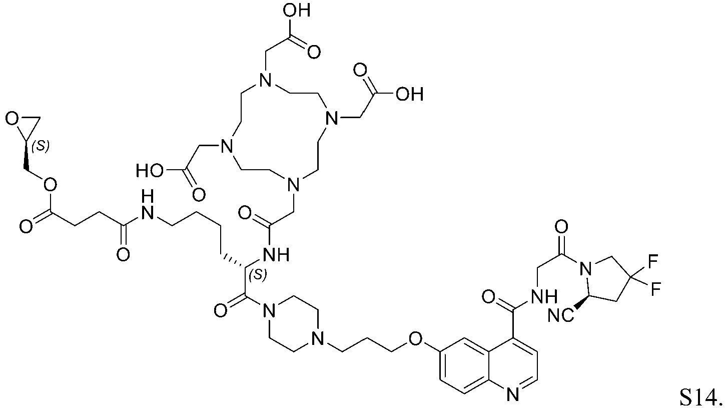

24. Use of the compound, or the stereoisomer, tautomer, isotopically labeled form, pharmaceutically acceptable salt, or prodrug thereof according to any one of claims 1-23 in the preparation of a FAP inhibitor.

25. A FAP-targeted nuclide probe, wherein the nuclide probe is the compound, or stereoisomer, tautomer, isotopically labeled form, pharmaceutically acceptable salt, or prodrug thereof according to any one of claims 1-23 labeled with a radionuclide.

26. A FAP-targeted nuclide probe, wherein the nuclide probe is the compound, or the stereoisomer, tautomer, isotopically labeled form, pharmaceutically acceptable salt, or prodrug thereof according to any one of claims 1-23 labeled with a radionuclide, and the radionuclide is a diagnostic radionuclide or a therapeutic radionuclide,
preferably, the diagnostic radionuclide is at least one of ⁶⁸Ga, ⁶⁷Ga, ⁶⁴Cu, ¹⁸F, ^{g6}Y, ⁸⁹Zr, ¹¹¹In, 99^{m}Tc, ¹¹C, ²⁰³Pb, ¹²³I, ¹²⁵I, ¹²⁴I, and Al¹⁸F;
preferably, the therapeutic radionuclide is at least one of ¹⁷⁷Lu, ⁹⁰Y, ¹²⁵I, ¹³¹I, ²¹¹At, ¹⁵³Sm, ¹⁸⁶Re, ¹⁸⁸Re, ⁶⁷Cu, ²²⁵Ac, ²²⁷Th, ²²³Ra, ²¹³Bi, ²¹²Bi, and ²¹²Pb.

27. A pharmaceutical composition, comprising the compound, or the stereoisomer, tautomer, isotopically labeled form, pharmaceutically acceptable salt, or prodrug thereof according to any one of claims 1-23, or the FAP-targeted nuclide probe according to claim 25 or 26; and at least one pharmaceutically acceptable excipient or carrier.

28. Use of the compound, or the stereoisomer, tautomer, isotopically labeled form, pharmaceutically acceptable salt, or prodrug thereof according to any one of claims 1-23, the inhibitor according to claim 24, the FAP-targeted nuclide probe according to claim 25 or 26, or the pharmaceutical composition according to claim 27 in the preparation of a FAP-targeted imaging agent and/or therapeutic agent.

29. Use of the compound, or the stereoisomer, tautomer, isotopically labeled form, pharmaceutically acceptable salt, or prodrug thereof according to any one of claims 1-23, the inhibitor according to claim 24, the FAP-targeted nuclide probe according to claim 25 or 26, or the pharmaceutical composition according to claim 27 in the preparation of drugs for diagnosing and/or treating a disease, wherein the disease is a disease **characterized by** overexpression of fibroblast activation protein (FAP).

30. A method for diagnosing and/or treating a disease, the method comprising administering a diagnostically and/or therapeutically effective amount of the compound, or the stereoisomer, tautomer, isotopically labeled form, pharmaceutically acceptable salt, or prodrug thereof according to any one of claims 1-23, the inhibitor according to claim 24, the FAP-targeted nuclide probe according to claim 25 or 26, or the pharmaceutical composition according to claim 27 to a subject to be diagnosed and/or treated, wherein the disease is a disease **characterized by** overexpression of fibroblast activation protein (FAP).

31. A kit, comprising the compound, or the stereoisomer, tautomer, isotopically labeled form, pharmaceutically acceptable salt, or prodrug thereof according to any one of claims 1-23, the inhibitor according to claim 24, the FAP-targeted nuclide probe according to claim 25 or 26, or the pharmaceutical composition according to claim 27; and instructions for diagnosing and/or treating a disease **characterized by** overexpression of fibroblast activation protein (FAP);
preferably, the disease **characterized by** overexpression of fibroblast activation protein (FAP) is selected from cancer, chronic inflammation, atherosclerosis, fibrosis (e.g., pulmonary fibrosis), tissue remodeling, scarring disease, rheumatoid arthritis, osteoarthritis, liver cirrhosis, and liver disease;
preferably, the cancer is selected from breast cancer, pancreatic cancer, small intestine cancer, colon cancer, rectal cancer, lung cancer, head and neck cancer, ovarian cancer, hepatocellular carcinoma, esophageal cancer, hypopharyngeal cancer, nasopharyngeal cancer, laryngeal cancer, myeloma cell, bladder cancer, cholangiocarcinoma, clear cell renal carcinoma, neuroendocrine tumor, carcinogenic osteomalacia, sarcoma, CUP(cancer of unknown primary), thymic cancer, glioma, neuroglioma, astrocytoma, cervical cancer, and prostate cancer.

32. A method for preparing the FAP-targeted nuclide probe according to claim 25 or 26, comprising:
1) providing the compound according to any one of claims 1-23; and
2) reacting the compound with the radionuclide according to claim 26 or a reagent providing the radionuclide in solution or on a column to complete labeling.

33. A kit or reagent combination for carrying out the preparation method of claim 32, comprising:
1) the compound according to any one of claims 1-23; and
2) the radionuclide according to claim 26 or a reagent providing the radionuclide.

34. A theranostic kit or reagent combination comprising:
1) the compound according to any one of claims 1-15; and
2) the compound according to any one of claims 16-23.

35. A compound represented by Formula (A-4), or a stereoisomer, tautomer, isotopically labeled form, pharmaceutically acceptable salt, or prodrug thereof, 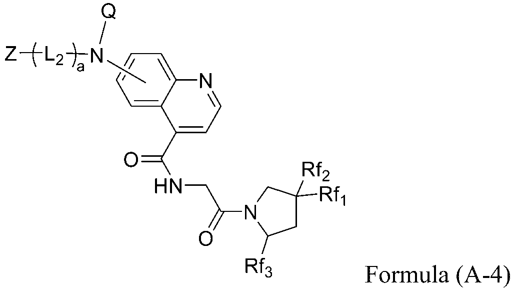
wherein Z is a payload,
Q is selected from the structures represented by the following Formulas (Q-1), (Q-2), (Q-3), (Q-4), and (Q-5): 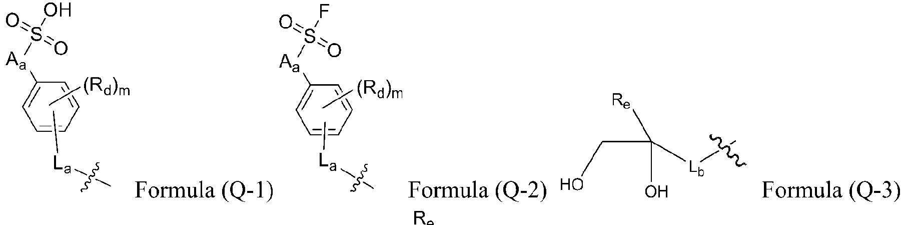 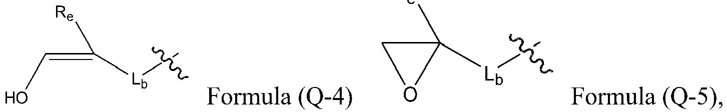
L₂, Aₐ, Lₐ, and L_{b} are the same or different, and are each independently selected from a linking group;
a and m are the same or different, and are each independently selected from an integer of 0 to 6;
Rₐ and Rₑ are the same or different, and are each independently selected from hydrogen, halogen, hydroxy, cyano, nitro, amino, C₁₋₂₀ alkyl, C₁₋₂₀ alkoxy, and halogenated C₁₋₂₀ alkyl;
Rf₁ and Rf₂ are independently selected from hydrogen and halogen, such as fluorine, chlorine, iodine, bromine, preferably fluorine;
Rf₃ is selected from hydrogen and cyano;
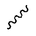 denotes the attachment site of the group.

36. The compound, or the stereoisomer, tautomer, isotopically labeled form, pharmaceutically acceptable salt, or prodrug thereof according to claim 35, **characterized in that**:
L₂, Aₐ, Lₐ, and L_{b} are the same or different, and are each independently selected from a divalent linking group; preferably, L₂, Aₐ, Lₐ, and L_{b} are each independently selected from a bond, -O-, -S-, -P-, -O-(HO-P=O)-O-, -NR₀-, -CO-, C₁₋₂₀ alkylene optionally substituted with one or more R₀, 3- to 20-membered heterocyclylene optionally substituted with one or more R₀, and any combination thereof; further preferably, L₂, Aₐ, Lₐ, and L_{b} are each independently selected from a bond, -O-, -S-, -P-, -O-(HO-P=O)-O-, -N(R₀)-, -CO-, C₁₋₁₀ alkylene (C₁₋₆ alkylene, C₁₋₄ alkylene) optionally substituted with one or more R₀, 3- to 20-membered heterocyclylene optionally substituted with one or more R₀ (3- to 10-membered heterocyclylene, 3- to 6-membered heterocyclylene), and any combination thereof; wherein each R₀ is the same or different, and is independently selected from hydrogen, halogen, hydroxy, cyano, nitro, amino, C₁₋₁₀ alkyl, C₁₋₁₀ alkoxy, halogenated C₁₋₁₀ alkyl, and halogenated C₁₋₁₀ alkoxy; preferably, each R₀ is the same or different, and is independently selected from hydrogen, halogen, hydroxy, cyano, nitro, amino, C₁₋₆ alkyl, C₁₋₆ alkoxy, halogenated C₁₋₆ alkyl, and halogenated C₁₋₆ alkoxy; further preferably, each R₀ is the same or different, and is independently selected from hydrogen, halogen, hydroxy, cyano, nitro, amino, C₁₋₄ alkyl, C₁₋₄ alkoxy, halogenated C₁₋₄ alkyl, and halogenated C₁₋₄ alkoxy;
preferably, the heterocyclylene comprises at least one N; preferably, the heterocyclylene is piperazinylene;
preferably, Aₐ is selected from a bond;
preferably, L₂ comprises 3- to 20-membered heterocyclylene; preferably, L₂ comprises heterocyclylene having one nitrogen atom or two nitrogen atoms preferably, L₂ comprises piperazinylene;
wherein each R₀ is the same or different, and is independently selected from hydrogen, halogen, hydroxy, cyano, nitro, amino, C₁₋₁₀ alkyl, C₁₋₁₀ alkoxy, halogenated C₁₋₁₀ alkyl, and halogenated C₁₋₁₀ alkoxy; preferably, each R₀ is the same or different, and is independently selected from hydrogen, halogen, hydroxy, cyano, nitro, amino, C₁₋₆ alkyl, C₁₋₆ alkoxy, halogenated C₁₋₆ alkyl, and halogenated C₁₋₆ alkoxy; further preferably, each R₀ is the same or different, and is independently selected from hydrogen, halogen, hydroxy, cyano, nitro, amino, C₁₋₄ alkyl, C₁₋₄ alkoxy, halogenated C₁₋₄ alkyl, and halogenated C₁₋₄ alkoxy.

37. The compound, or the stereoisomer, tautomer, isotopically labeled form, pharmaceutically acceptable salt, or prodrug thereof according to claim 36, **characterized in that**:
L₂ comprises the structure represented by the following Formula (L-1): 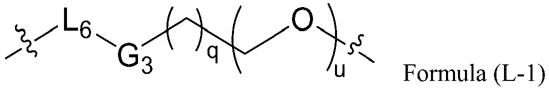
wherein q is an integer of 1 to 20, and u is an integer of 0 to 10;
G₃ is C₃₋₁₀ cycloalkyl, 3- to 10-membered heterocycloalkyl, 5- to 10-membered heteroaryl, or C₆₋₁₀ aryl;
L₆ is a bond or -CO-C₁₋₃ alkylene-NH-; and/or,
Lₐ and L_{b} are independently selected from the structure represented by the following Formula (L-2): 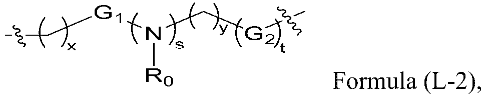
x is selected from 0, 1, and 2; s is 0 or 1; y is selected from 0, 1, 2, 3, and 4; t is 0 or 1;
G₁ or G₂ is independently -CH₂- or carbonyl, and they are not -CH₂- at the same time;
R₀ is each independently selected from hydrogen, halogen, hydroxy, cyano, nitro, amino, C₁₋₁₀ alkyl, C₁₋₁₀ alkoxy, halogenated C₁₋₁₀ alkyl, and halogenated C₁₋₁₀ alkoxy,
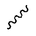 denotes the attachment site of the group.

38. The compound, or the stereoisomer, tautomer, isotopically labeled form, pharmaceutically acceptable salt, or prodrug thereof according to claim 37, **characterized in that**:
in formula (L-1), G₃ is selected from at least one of the following groups: 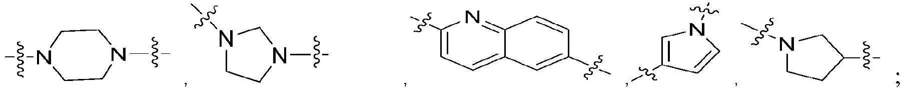
q is an integer of 1 to 5, and u is 0.

39. The compound, or the stereoisomer, tautomer, isotopically labeled form, pharmaceutically acceptable salt, or prodrug thereof according to claim 37, **characterized in that**:
Lₐ and L_{b} are independently selected from the groups represented by Formula (L-3) and Formula (L-4) below: 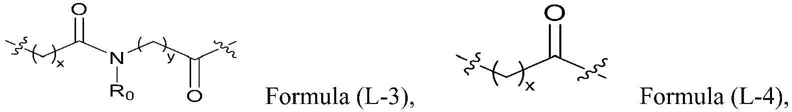
x is selected from 0, 1, and 2; y is selected from 0, 1, 2, 3, and 4; and R₀ is H or C₁₋₃ alkyl.

40. The compound, or the stereoisomer, tautomer, isotopically labeled form, pharmaceutically acceptable salt, or prodrug thereof according to any one of claims 35 to 39, **characterized in that**:
in formula (A-4), Q is selected from the groups represented by Formula (L-5) and Formula (L-6) below: 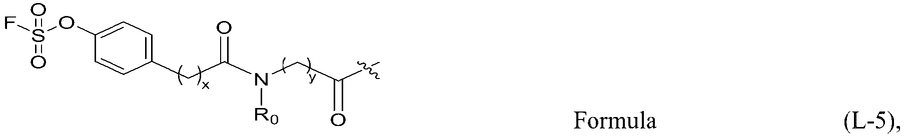 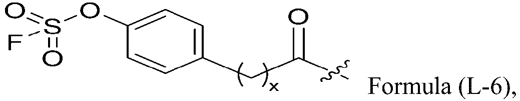
x is selected from 0, 1, and 2; y is selected from 0, 1, 2, 3, and 4; and R₀ is H or C₁₋₃ alkyl.

41. A compound represented by Formula (A-5), or a stereoisomer, tautomer, isotopically labeled form, pharmaceutically acceptable salt, or prodrug thereof, 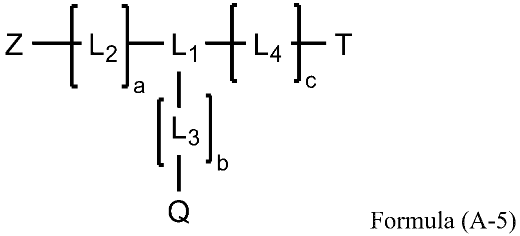
wherein Z is a payload, and T is a targeting moiety,
Q is selected from the structures represented by Formulas (Q-1), (Q-2), (Q-3), (Q-4), and (Q-5) below: 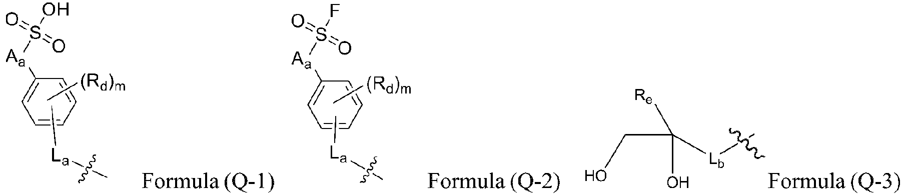 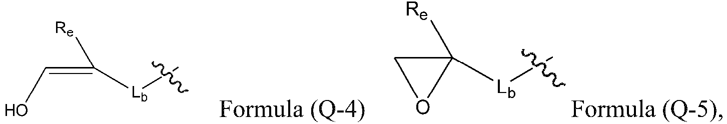
L₁, L₂, L₃, L₄, Aₐ, Lₐ, and L_{b} are the same or different, and are each independently selected from a bond and a linking group,
a, b, c, and m are the same or different and are each independently selected from an integer of 0 to 6 (e.g., 0, 1, 2, 3, 4, 5, 6),
R_{d} and Rₑ are the same or different, and are each independently selected from hydrogen, halogen, hydroxy, cyano, nitro, amino, C₁₋₂₀ alkyl, C₁₋₂₀ alkoxy, and halogenated C₁₋₂₀ alkyl;
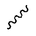 denotes the attachment site of the group;
wherein Lₐ and L_{b} are independently selected from the structure represented by Formula (L-2): 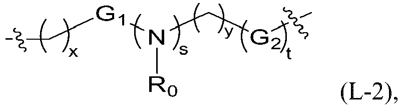
x is selected from 0, 1, and 2; s is 0 or 1; y is selected from 0, 1, 2, 3, and 4; t is 0 or 1;
G₁ or G₂ is independently -CH₂- or carbonyl, and they are not -CH₂- at the same time;
R₀ is independently selected from hydrogen, halogen, hydroxy, cyano, nitro, amino, C₁₋₁₀ alkyl, C₁₋₁₀ alkoxy, halogenated C₁₋₁₀ alkyl, and halogenated C₁₋₁₀ alkoxy,
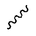 denotes the attachment site of the group.

42. The compound, or the stereoisomer, tautomer, isotopically labeled form, pharmaceutically acceptable salt, or prodrug thereof according to claim 41, **characterized in that**:
L₁ is selected from a trivalent linking group; preferably, L₁ is selected from N, C₁₋₂₀ alkanetriyl optionally substituted with one or more R₀, and any combination thereof; further preferably, L₁ is selected from N, C₁₋₁₀ alkanetriyl (C₁₋₆ alkanetriyl, C₁₋₄ alkanetriyl), and any combination thereof; and/or
Aₐ is selected from a divalent linking group; preferably, Aₐ is selected from a bond, -O-, -S-, -P-, -O-(HO-P=O)-O-, -N(R₀)-, -CO-, C₁₋₂₀ alkylene (C₁₋₁₀ alkylene, C₁₋₆ alkylene, C₁₋₄ alkylene) optionally substituted with one or more R₀, and any combination thereof; preferably, Aₐ is selected from a bond; and/or
L₂, L₃, L₄, and Aₐ are the same or different, and are each independently selected from a divalent linking group; preferably, L₂, L₃, L₄, and Aₐ are each independently selected from a bond, -O-, -S-, -P-, -O-(HO-P=O)-O-, -N(R₀)-, -CO-, C₁₋₂₀ alkylene optionally substituted with one or more R₀, 3- to 20-membered heterocyclylene optionally substituted with one or more R₀, and any combination thereof; further preferably, L₂, L₃, L₄, and Aₐ are each independently selected from a bond, -O-, -S-, -P-, -O-(HO-P=O)-O-, -N(R₀)-, -CO-, C₁₋₁₀ alkylene (C₁₋₆ alkylene, C₁₋₄ alkylene) optionally substituted with one or more R₀, 3- to 20-membered heterocyclylene optionally substituted with one or more R₀ (3- to 10-membered heterocyclylene, 3- to 6-membered heterocyclylene), and any combination thereof;
preferably, the heterocyclylene contains at least one N; preferably, the heterocyclylene is piperazinylene;
preferably, at least one of L₂ and L₄ comprises 3- to 20-membered heterocyclylene; preferably, at least one of L₂ and L₄ comprises heterocyclylene having one nitrogen atom or two nitrogen atoms; preferably, at least one of L₂ and L₄ comprises piperazinylene.

43. The compound, or the stereoisomer, tautomer, isotopically labeled form, pharmaceutically acceptable salt, or prodrug thereof according to claim 41 or 42, **characterized in that**:
the compound has the structure represented by Formula (A-4): 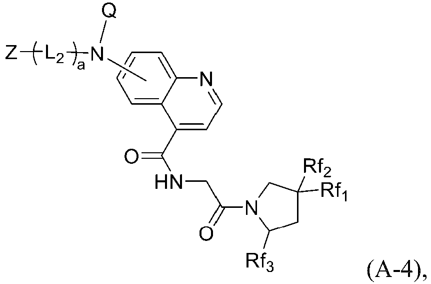
R_{f1} and R_{f2} are independently selected from hydrogen, and halogen, such as fluorine, chlorine, iodine, bromine, preferably fluorine;
R_{f3} is selected from hydrogen and cyano.

44. The compound, or the stereoisomer, tautomer, isotopically labeled form, pharmaceutically acceptable salt, or prodrug thereof according to claim 43, **characterized in that**:
Lₐ and L_{b} are independently selected from the groups represented by Formula (L-3) or Formula (L-4) below: 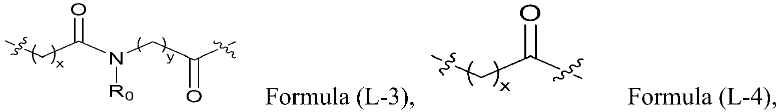
x is selected from 0, 1, and 2; y is selected from 0, 1, 2, 3, and 4; and R₀ is H or C₁₋₃ alkyl.

45. The compound, or the stereoisomer, tautomer, isotopically labeled form, pharmaceutically acceptable salt, or prodrug thereof according to any one of claim 41 to 42, **characterized in that**:
the compound has the structure represented by Formula (A-6): 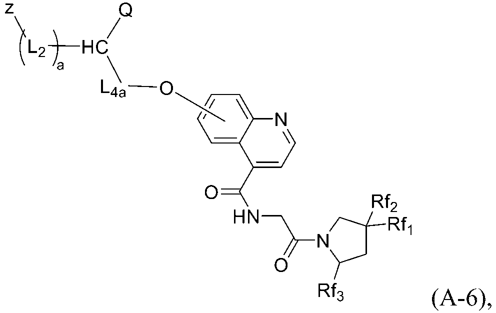
Rf₁ and Rf₂ are independently selected from hydrogen and halogen, such as fluorine, chlorine, iodine, bromine, preferably fluorine;
Rf₃ is selected from hydrogen and cyano;
L₄ₐ is selected from a bond, -O-, -S-, -P-, -O-(HO-P=O)-O-, -N(R₀)-, -CO-, C₁₋₂₀ alkylene optionally substituted with one or more R₀, 3- to 20-membered heterocyclylene optionally substituted with one or more R₀, and any combination thereof.

46. The compound, or the stereoisomer, tautomer, isotopically labeled form, pharmaceutically acceptable salt, or prodrug thereof according to claim 45, **characterized in that**:
Lₐ and L_{b} are independently selected from: 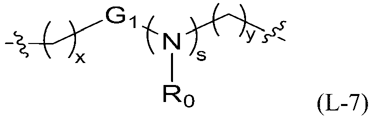
wherein x is selected from 0, 1, and 2; s is 0 or 1; and y is selected from 0, 1, 2, 3, and 4;
G₁ is -CH₂- or carbonyl.

47. The compound, or the stereoisomer, tautomer, isotopically labeled form, pharmaceutically acceptable salt, or prodrug thereof according to claim 46, **characterized in that**:
Lₐ and L_{b} are independently selected from -CH₂-, and the group shown below: 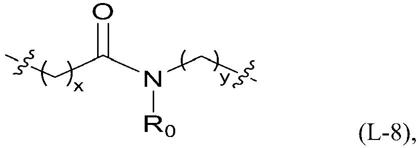
wherein x is selected from 0, 1, and 2, and y is selected from 0, 1, 2, 3, and 4.

48. The compound, or the stereoisomer, tautomer, isotopically labeled form, pharmaceutically acceptable salt, or prodrug thereof according to any one of claims 45 to 47, **characterized in that**,
L₄ₐ comprises the structure represented by Formula (L-9) below: 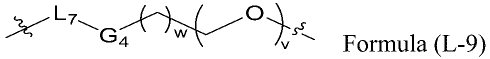
wherein w is an integer of 1 to 20, and v is an integer of 0 to 10;
G₄ is C₃-C₁₀ cyclic group, and L₇ is carbonyl or C₁₋₁₀ alkylene.

49. The compound, or the stereoisomer, tautomer, isotopically labeled form, pharmaceutically acceptable salt, or prodrug thereof according to any one of claims 45 to 48, **characterized in that**:
L₂ comprises -N(R₀)-, or the structure represented by Formula (L-1): 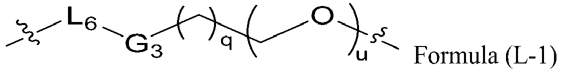
wherein q is an integer of 1 to 20, and u is an integer of 0 to 10;
G₃ is C₃₋₁₀ cycloalkyl, 3- to 10-membered heterocycloalkyl, 5- to 10-membered heteroaryl, or C₆₋₁₀ aryl;
L₆ is a bond or -CO-C₁₋₃ alkylene-NH-.

50. A compound represented by Formula (M-1): 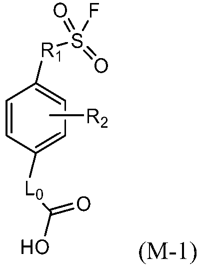
wherein R₁ is selected from a bond and O;
R₂ is selected from hydrogen, C₁₋₁₀ alkyl, and C₁₋₁₀ alkoxy;
L₀ comprises the structure represented by Formula (L-X): 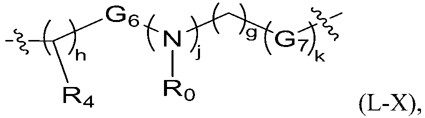
wherein h is selected from 0, 1, and 2; j is 0 or 1; g is selected from 0, 1, 2, 3, and 4; and k is 0, 1, or 2;
G₆ or G₇ is independently -CH₂- or carbonyl, and they are not -CH₂- at the same time;
R₄ is H, -NH₂ or amino group protected by a protecting group.

51. The compound according to claim 50, **characterized in that**
the compound of Formula (M-1) is selected from the compounds represented by Formula (M-2) and Formula (M-3): 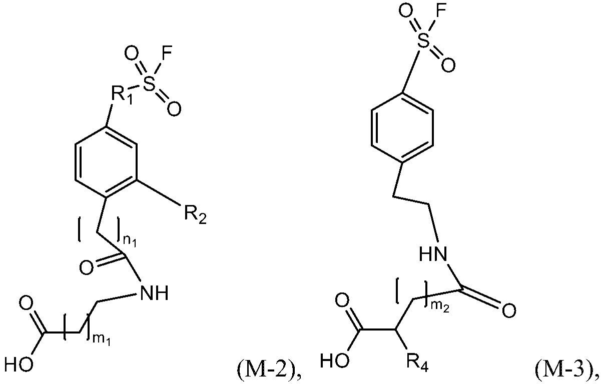
wherein R₂ is hydrogen or methoxy,
R₁ is a bond or oxygen,
n₁ is an integer of 0 to 2,
m₁ is an integer of 1 to 3,
m₂ is an integer of 1 to 3.

52. The compound according to claim 50, **characterized in that** it is selected from the compounds shown below:

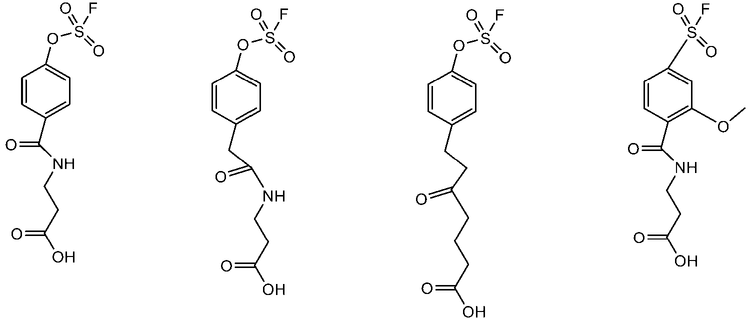

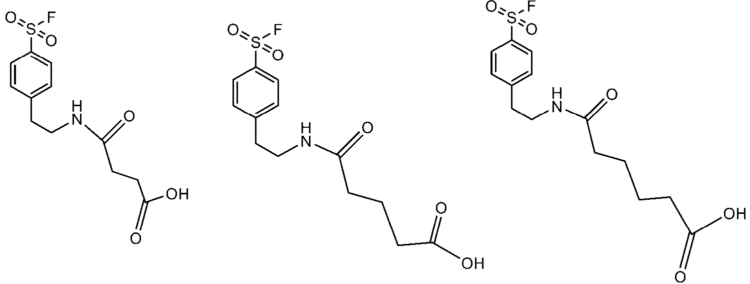

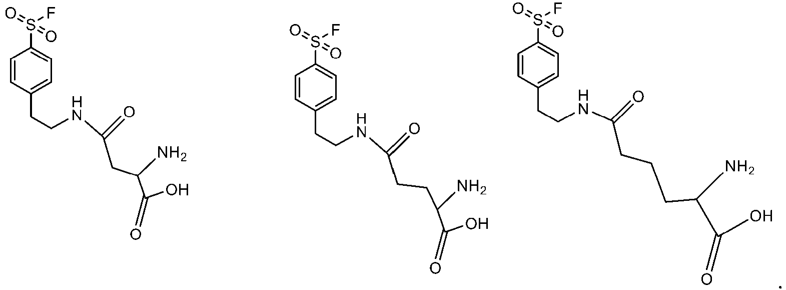

53. Use of the compound according to any one of claims 50 to 52 in the preparation of a radiopharmaceutical.

54. The use according to claim 53, wherein the radiopharmaceutical comprises the compound, or the stereoisomer, tautomer, isotopically labeled form, pharmaceutically acceptable salt, or prodrug thereof according to any one of claims 1-23 and 35-49.
